(19) Europäisches Patentamt
European Patent Office
Office européen des brevets

(11) Publication number: **0 593 998 A1**

# (12) EUROPEAN PATENT APPLICATION

(21) Application number: **93116309.1**

(22) Date of filing: **08.10.93**

(51) Int. Cl.5: **C07D 239/38**, A01N 43/54, C07D 239/60, C07D 401/12, C07D 409/12

(30) Priority: **23.10.92 JP 307813/92**
**08.06.93 JP 163335/93**

(43) Date of publication of application:
**27.04.94 Bulletin 94/17**

(84) Designated Contracting States:
**BE CH DE ES FR GB IT LI NL**

(71) Applicant: **NIHON BAYER AGROCHEM K.K.**
**10-8, Takanawa 4-chome**
**Minato-ku**
**Tokyo 108(JP)**

(72) Inventor: **Goto, Toshio**
**214-18, Koganei,**
**Kokubunji-machi**
**Shimotsuga-gun, Tochigi(JP)**
Inventor: **Kitagawa, Yoshinori**
**1085, Ara-machi**
**Moka-shi, Tochigi(JP)**
Inventor: **Hayakawa, Hidenori**
**3-7-1, Hachimangi**
**Hatogaya-shi, Saitama(JP)**
Inventor: **Shibuya, Katsuhiko**
**1425-2, Hitotonoya,**
**Oh-aza**
**Oyama-shi, Tochigi(JP)**
Inventor: **Ito, Seishi**
**1-39, Ekihigashidori**
**Oyama-shi, Tochigi(JP)**
Inventor: **Minegishi, Natsuko**
**1-9-31, Wakagi-sho**
**Oyama-shi, Tochigi(JP)**
Inventor: **Ukawa, Kazuhiro**
**1-23-13, Ekihigashidori**
**Oyama-shi, Tochigi(JP)**
Inventor: **Yamaoka, Tatsuya**
**934-7, Hitotonoya,**
**Oh-aza**
**Oyama-shi, Tochigi(JP)**
Inventor: **Ueno, Chieko**
**934-7, Hitotonoya,**
**Oh-aza**
**Oyama-shi, Tochigi(JP)**
Inventor: **Ito, Asami**
**934-7, Hitotonoya,**
**Oh-aza**
**Oyama-shi, Tochigi(JP)**
Inventor: **Kyo, Yoshiko**
**934-7, Hitotonoya,**
**Oh-aza**
**Oyama-shi, Tochigi(JP)**

(74) Representative: **Schumacher, Günter, Dr. et al**
**Bayer AG**
**Konzernverwaltung RP**
**Patentabteilung**
**D-51368 Leverkusen Bayerwerk (DE)**

(54) **Pyrimidinylthioalkane herbicides.**

(57) Novel pyrimidinylthioalkane derivatives of the formula (I)

EP 0 593 998 A1

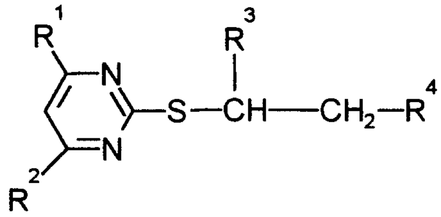

(I)

wherein $R^1$, $R^2$, $R^3$ and $R^4$ have the meanings as defined in the specification and the use of the new compounds as herbicides.

The present invention relates to novel pyrimidinylthioalkane derivatives, to processes for their preparation, and to their use as herbicides.

It has already been disclosed that a certain group of α-pyrimidinylthiocarboxylic acid derivatives is useful as herbicides (see Japanese Laid-Open Patent Application Nos. 85262/1990, 135963/1991 and 240777/1991).

There have now been found novel pyrimidinylthioalkane derivatives of the formula (I)

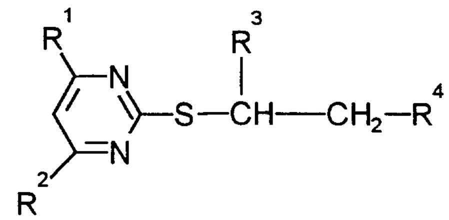

(I)

wherein

$R^1$ represents, $C_{1-4}$alkyl, $C_{1-4}$alkoxy, halogen, halogeno-$C_{1-4}$a1kyl, or halogeno-$C_{1-4}$alkoxy,

$R^2$ represents $C_{1-4}$alkoxy, halogen, halogeno-$C_{1-4}$alkyl or halogeno-$C_{1-4}$alkoxy,

$R^3$ represents optionally $C_{1-4}$alkyl substituted or unsubstituted $C_{3-7}$cycloalkyl or optionally substituted $C_{1-15}$alkyl,

$R^4$ represents halogen or

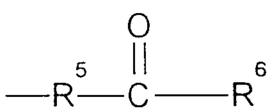

,

$R^5$ represents oxygen or sulfur,

$R^6$ represents hydrogen, in each case optionally substituted $C_{1-20}$ saturated aliphatic hydrocarbon, $C_{2-20}$ unsaturated aliphatic hydrocarbon, phenyl, $C_{3-8}$-cycloalkyl, $C_{3-8}$cycloalkenyl, $C_{1-4}$alkoxycarbonyl, carboxyl and its salts, an optionally substituted heterocyclic ring, an optionally substituted condensed heterocyclic ring, or the following formula:

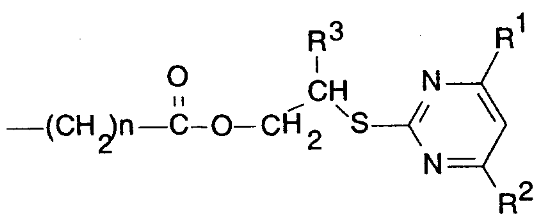

wherein $R^1$, $R^2$ and $R^3$ have the same meanings as mentioned above, and n represents an integer of 0 to 6 or

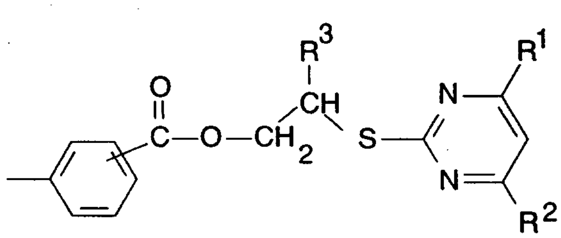

wherein, $R^1$, $R^2$ and $R^3$ have the same meanings as mentioned above.

Pyrimidinylthioalkane derivatives of the formula (I) are obtained when
(a) in the case where $R^4$ represents halogen:
compounds of the formula (II)

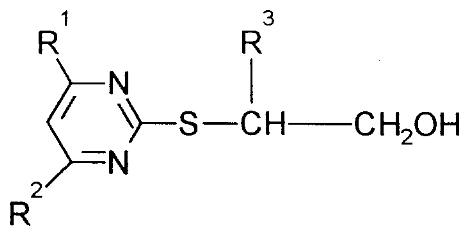

(II)

wherein
$R^1$, $R^2$ and $R^3$ have the same meanings as defined above,
are reacted with a halogenation agent, if appropriate, in the presence of inert solvents,
or
(b) in the case where the $R^4$ represents

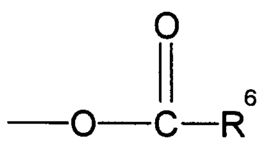
:

above mentioned compounds of the formula (II),
are reacted with acid halides of the formula (III)

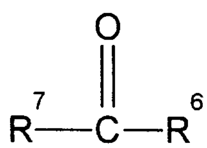

(III)

wherein $R^6$ has the meanings as defined above, and $R^7$ represents chlorine, bromine or iodine,
if appropriate, in the presence of inert solvents and, if appropriate in the presence of an acid binder,
or
(c) in the case where $R^4$ represents

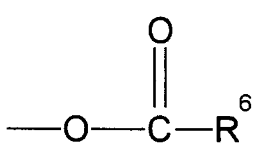
:

above mentioned compounds of the formula (II)
are reacted with acid anhydrides of the formula (IV)

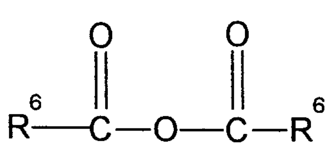

(IV)

wherein
$R^6$ has the same meanings as defined above and both $R^6$ may be the same or different to each other, if appropriate, in the presence of inert solvents and, if appropriate in the presence of an acid binder,
or
(d) in the case where $R^4$ represents

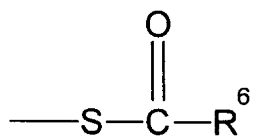

:

compounds of the formula (V)

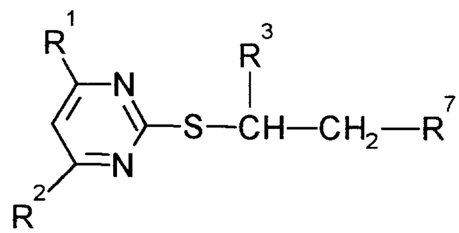

(V)

wherein
$R^1$, $R^2$, $R^3$ and $R^7$ have the same meanings as defined above,
are reacted with thiocarboxylic acids of the formula (VI)

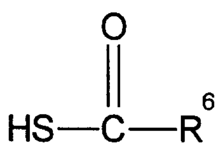

(VI)

wherein
$R^6$ has the same meaning as defined above,
in the presence of inert solvents and if appropriate in the presence of an acid binder.

The novel pyrimidinylthioalkane derivatives according to the invention exhibit powerful herbicidal properties.

Surprisingly, the pyrimidinylthioalkane derivatives according to the invention exhibit a substantially higher herbicidal action than those known from the prior art, for instance, the aforementioned Japanese Laid-open Patent Application Nos. 85262/1990, 135963/1991 and 240777/1991.

In the compounds of the formula (I) and their intermediates, the halogen represents fluoro, chloro, bromo and iodo, preferably chloro or fluoro.

$C_{1-4}$alkyl and the alkyl moiety of $C_{1-4}$alkoxy, $C_{1-4}$alkylthio, halogeno-$C_{1-4}$alkyl, halogeno-$C_{1-4}$alkoxy and halogeno-$C_{1-4}$alkylthio represents straight-chain or branched alkyl such as methyl, ethyl, n-propyl, isopropyl, or n-(iso-, sec- or tert-)butyl.

Saturated aliphatic hydrocarbon having 1 to 20 carbon atom(s) represents, straight-chain or branched alkyl, such as above mentioned $C_{1-4}$alkyl and n-(sec-, iso-)pentyl, hexyl, heptyl, octyl, nonyl, decyl, undecyl, dodecyl, tridecyl, tetradecyl, pentadecyl, hexadecyl, heptadecyl, octadecyl, nonadecyl, eicocyl, 1-ethyl-1-methylpropyl, 1,1-dimethylpropyl.

Unsaturated aliphatic hydrocarbon having 2 to 20 carbon atoms represents, straight-chain or branched alkenyl, straight-chain or branched alkynyl and straight-chain or branched alkadienyl, such as propargyl, 1-propynyl, 2-propenyl, vinyl, 1-propenyl, 9-decenyl, 8-tridecenyl, 10-nonadecenyl, 8-heptadecenyl, 8-pentadecenyl, 1,1-dimethyl-3-butenyl.

The heterocyclic ring represents 5- or 6-memberd cyclic groups having one to four hetero atom(s) each being independently nitrogen, sulfur and oxygen. Examples of heterocyclic rings are (without limiting) thiadiazolyl, thiazolyl, imidazolyl, pyrazolyl, furyl, thienyl, pyridyl, isoxazolyl, pyrimidinyl, pyridazinyl, pyrazinyl, and the like.

Condensed heterocyclic rings representing 9- or 10-membered bicyclo groups that have been formed by the above mentioned 5- or 6-membered heterocyclic group with phenyl, such as quinolyl, benzoxazolyl, benzothiazolyl, benzimidazolyl, phthalazinyl and so on.

Among the pyrimidinylthioalkane derivatives according to the invention of the formula (I), the preferred compounds are those wherein

$R^1$ represents, methoxy, difluoromethoxy or trifluoromethoxy,

$R^2$ represents, methoxy, difluoromethoxy or trifluoromethoxy,

$R^3$ represents, optionally $C_{1-2}$alkyl substituted $C_{3-6}$cycloalkyl or $C_{1-7}$alkyl which may be unsubstituted or substituted by at least one substituent selected from the group consisting of halogen, $C_{3-6}$cycloalkyl and phenyl which may be substituted by cyano, nitro, halogen, $C_{1-4}$alkyl, $C_{1-4}$ alkoxy, halogeno-$C_{1-4}$alkyl or halogeno-$C_{1-4}$alkoxy,

$R^4$ represents, chloro, bromo and

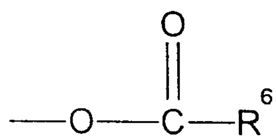

,

$R^6$ represents, hydrogen,

$R^6$ further represents, $C_{1-12}$alkyl which may be unsubstituted or substituted by at least one substituent selected from the group consisting of halogen, cyano, nitro, $C_{3-8}$cycloalkyl which may be substituted by $C_{1-4}$alkyl, $C_{1-4}$alkoxy, $C_{1-4}$alkylthio, halogeno-$C_{1-4}$alkoxy, halogeno-$C_{1-4}$alkylthio, carboxyl or its salt, $C_{1-4}$alkylcarbonyl, $C_{1-4}$alkoxycarbonyl, $C_{1-4}$alkylthiocarbonyl, amino, $C_{1-4}$alkylamino, and di-$C_{1-4}$alkylamino,

$R^6$ further represents phenyl-$C_{1-12}$alkyl, said phenyl is unsubstituted or substituted at least by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, and halogeno-$C_{1-4}$alkylthio,

$R^6$ further represents, phenoxy-$C_{1-12}$alkyl, said phenoxy may be unsubstituted or substituted at least by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, halogeno-$C_{1-4}$alkylthio, phenoxy which may be unsubstituted or substituted by one of the group consisting of halogen, $C_{1-4}$alkyl and halogeno-$C_{1-4}$alkyl,

pyridyloxy which may be unsubstituted or substituted by one of the group consisting of halogen, $C_{1-4}$alkyl, and halogeno-$C_{1-4}$alkyl,

quinoxalyloxy which may be unsubstituted or substituted by one of the group consisting of halogen, $C_{1-4}$alkyl, and halogeno-$C_{1-4}$alkyl,

benzothiazolyloxy which may be unsubstituted or substituted by one of the group consisting of halogen, $C_{1-4}$alkyl, and halogeno-$C_{1-4}$alkyl, and

benzoxazolyloxy which may be unsubstituted or substituted by one of the group consisting of halogen, $C_{1-4}$alkyl, and halogeno-$C_{1-4}$alkyl,

$R^6$ further represents, phenylthio-$C_{1-12}$alkyl, said phenylthio may be unsubstituted or substituted by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, halogeno-$C_{1-4}$alkylthio, phenyl-$C_{1-4}$alkoxy, phenoxy which may be unsubstituted or substituted by one of the group consisting of halogen, $C_{1-4}$alkyl and halogeno-$C_{1-4}$alkyl,

pyridyloxy which may be unsubstituted or substituted by one of the group consisting of halogen, $C_{1-4}$alkyl, and halogeno-$C_{1-4}$alkyl,

quinoxalyloxy which may be unsubstituted or substituted by one of the group consisting of halogen, $C_{1-4}$alkyl, and halogeno-$C_{1-4}$alkyl,

benzothiazolyloxy which may be unsubstituted or substituted by one of the group consisting of halogen, $C_{1-4}$alkyl, and halogeno-$C_{1-4}$alkyl, and

benzothiazolyloxy which may be unsubstituted or substituted by one of the group consisting of

halogen, $C_{1-4}$alkyl, and halogeno-$C_{1-4}$alkyl,

$R^6$ further represents, naphthyl-$C_{1-12}$alkyl or naphthoxy-$C_{1-12}$alkyl, said naphthyl or naphthoxy may be unsubstituted or substituted by one of the group consisting of cyano, nitro, halogen, $C_{1-4}$alkyl, $C_{1-4}$alkoxy,

$C_{1-4}$alkylthio, halogeno-$C_{1-4}$alkyl, halogeno-$C_{1-4}$alkoxy, halogeno-$C_{1-4}$alkylthio, phenyl-$C_{1-4}$alkoxy, phenoxy which may be unsubstituted or substituted by one of the group consisting of halogen, $C_{1-4}$alkyl and halogeno-$C_{1-4}$alkyl,

pyridyloxy which may be unsubstituted or substituted by one of the group consisting of halogen, $C_{1-4}$alkyl, and halogeno-$C_{1-4}$alkyl,

quinoxalyloxy which may be unsubstituted or substituted by one of the group consisting of halogen, $C_{1-4}$alkyl, and halogeno-$C_{1-4}$alkyl,

benzothiazolyloxy which may be unsubstituted or substituted by one of the group consisting of halogen, $C_{1-4}$alkyl, and halogeno-$C_{1-4}$alkyl, and

benzoxazolyloxy which may be unsubstituted or substituted by one of the group consisting of halogen, $C_{1-4}$alkyl, and halogeno-$C_{1-4}$alkyl,

$R^6$ further represents, $C_{1-4}$alkylsulfonyloxy-$C_{1-12}$alkyl, or benzenesulfonyl-$C_{1-12}$alkyl, said benzene may be unsubstituted or substituted by one of the group consisting of halogen and $C_{1-4}$alkyl,

$R^6$ further represents $C_{2-12}$alkenyl which may be unsubstituted or substituted by one of the group consisting of halogen, $C_{1-4}$alkyl, carboxyl or its salts and phenyl which may be substituted by halogen or $C_{1-4}$alkyl,

$R^6$ further represents $C_{3-12}$alkynyl, $C_{3-12}$alkadienyl,

$R^6$ further represents, phenyl which may be unsubstituted or substituted by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, and carboxyl or its salts,

$R^6$ further represents a 5- or 6-memberd heterocyclic group that has one to four heteroatom(s) each being independently oxygen, sulfur or nitrogen, said heterocyclic group is unsubstituted or substituted by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, phenyl which may be unsubstituted or substituted by halogen, nitro, $C_{1-4}$ alkyl, $C_{1-4}$ alkoxy, halogeno-$C_{1-4}$alkyl, halogeno-$C_{1-4}$alkoxy, phenoxy, and carboxyl or its salts,

$R^6$ further represents a 9- to 10-membered condensed heterocyclic group having one to four hetero atom(s) each being independently oxygen, sulfur or nitrogen, said condensed heterocyclic group may be unsubstituted or substituted by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, phenyl which may be unsubstituted or substituted by halogen, phenoxy or carboxyl and its salts,

$R^6$ further represents, $C_{3-8}$cycloalkyl which may be substituted by one of the group consisting of $C_{1-4}$alkyl or carboxyl and its salts, $C_{3-8}$cycloalkenyl which may be substituted by one of the group consisting of $C_{1-4}$alkyl, $C_{1-4}$alkoxycarbonyl or carboxyl and its salts,

$R^6$ further represents the following formulae

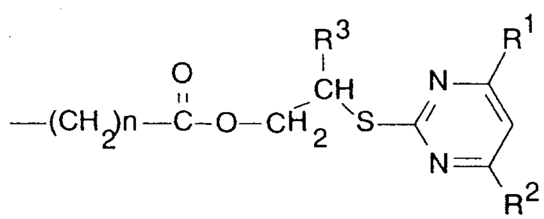

wherein, $R^1$, $R^2$ and $R^3$ have the same meanings as defined above, and n represents an integer of 0 to 6

or

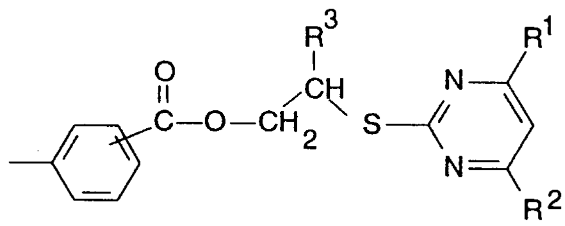

wherein $R^1$, $R^2$ and $R^3$ have the same meanings as defined above.

Very particularly preferred pyrimidinylthioalkane derivatives according to the invention of the formula (I) are those wherein

$R^1$ represents methoxy,

$R^2$ represents methoxy,

$R^3$ represents cyclopentyl which may be substituted by methyl, optionally unsubstituted or methyl-substituted cyclohexyl, $C_{1-4}$alkyl which may be unsubstituted or substituted by one of the group consisting of fluoro, chloro, bromo, cyclopropane, cyclopentane cyclohexane and phenyl which may be unsubstituted or substituted by one of the group consisting of cyano, nitro, fluoro, chloro, bromo, methyl, methoxy, trifluoromethyl and trifluoromethoxy,

$R^4$ represents chloro, bromo, or

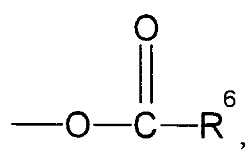

$R^6$ represents hydrogen,

$R^6$ further represents $C_{1-6}$alkyl which may be unsubstituted or substituted by one of the group consisting of fluoro, chloro, bromo, cyano, nitro, cyclopentyl which may be substituted by methyl, cyclohexyl which may be substituted by methyl, $C_{1-4}$alkoxy, carboxyl and its sodium salt, methylcarbonyl, methoxycarbonyl, amino and dimethylamino,

$R^6$ further represents phenyl-$C_{1-6}$alkyl, said phenyl may be unsubstituted or substituted by one of the group consisting of cyano, nitro, fluoro, chloro, methyl, methoxy or carboxyl,

$R^6$ further represents phenoxy-$C_{1-6}$alkyl, said phenoxy may be unsubstituted or substituted at least by one of the group consisting of cyano, nitro, fluoro, chloro, methyl, unsubstituted or substituted phenoxy (said substituent(s) is/are selected from the group consisting of fluoro, chloro and trifluoromethyl), unsubstituted or substituted pyridin-2-yloxy (said substituent(s) is/are selected from the group consisting of fluoro, chloro and trifluoromethyl), unsubstituted or substituted quinoxaline-2-yloxy (said substituent(s) is/are selected from the group consisting of fluoro, chloro and trifluoromethyl), unsubstituted or substituted benzothiazole-2-yloxy (said substituent(s) is/are selected from the group consisting of fluoro, chloro and trifluoromethyl) and unsubstituted or substituted benzoxazole-2-yloxy (said substituent(s) is/are selected from the group consisting of fluoro, chloro and trifluoromethyl),

$R^6$ further represents phenylthio-$C_{1-6}$alkyl, said phenylthio may be unsubstituted or substituted, at least by one of the group consisting of fluoro, chloro, methyl, phenylmethoxy, unsubstituted or substituted phenoxy (said substituent(s) is/are selected from the group consisting of fluoro, chloro, $C_{1-4}$alkyl or halogeno-$C_{1-4}$ alkyl), unsubstituted or substituted pyridin-2-yloxy (said substituent(s) is/are selected from the group consisting of fluoro, chloro or trifluoromethyl), unsubstituted or substituted quinoxaline-2-yloxy (said substituent(s) is/are selected from the group consisting of fluoro, chloro and trifluoromethyl), unsubstituted or substituted benzothiazole-2-yloxy (said substituent(s) is/are selected from the group consisting of fluoro, chloro and trifluoromethyl) and unsubstituted or substituted benzoxazole-2-yloxy (said substituent(s) is/are selected from the group consisting of fluoro, chloro or trifluoromethyl),

$R^6$ further represents, naphthyl-$C_{1-6}$alkyl, or naphthoxy-$C_{1-6}$alkyl, said naphthyl or naphthoxy may be unsubstituted or substituted, and the substituent(s) is/are phenoxy (which may be substituted by fluoro, chloro, or trifluoromethyl), and unsubstituted or substituted benzenesulfonyloxy (said

substituent(s) is/are selected from the group consisting of fluoro, chloro and methyl),

$R^6$ further represents unsubstituted or substituted $C_{3-7}$alkenyl, said substituent is selected from one of the group consisting of fluoro, chloro, bromo, carboxyl and unsubstituted or substituted phenyl (said substituents are selected from the group consisting of fluoro and chloro),

$R^6$ further represents $C_{3-6}$alkynyl, or unsubstituted or substituted phenyl, said substituent is selected from one of the group consisting of cyano, nitro, fluoro, chloro, bromo, ethyl, propyl, isopropyl, (n- or tert-)butyl, methoxy, ethoxy, trifluoromethyl and trifluoromethoxy,

$R^6$ further represents an unsubstituted or substituted 5- or 6-memberd heterocyclic ring, said heterocyclic group is selected from the group consisting of thienyl, thiazolyl, isoxazolyl, imidazolyl, pyrazolyl, furyl, pyridyl, pyrimidinyl, pyridazinyl, pyrazinyl and oxazolyl, and the substituent(s) is/are selected from the group consisting of fluoro, chloro, bromo, methyl, methoxy, methylthio, trifluoromethyl, trifluoromethoxy, carboxyl and phenyl which may be substituted by fluoro, chloro, nitro, methyl, ethyl methoxy and trifluoromethyl,

$R^6$ further represents an unsubstituted or substituted 9- or 10-membered condensed heterocyclic ring, said condensed heterocyclic group is selected from the group consisting of quinolyl and indolyl, and the substituent(s) is/are selected from the group consisting of fluoro, chloro, bromo, methyl, methoxy, methylthio, trifluoromethyl and trifluoromethoxy,

$R^6$ further represents unsubstituted or substituted cyclopropyl, cyclopentyl or cyclohexyl (said substituent(s) is/are selected from the group consisting of methyl, ethyl and carboxyl),

$R^6$ further represents unsubstituted or substituted cyclopentenyl or cyclohexenyl (said substituent(s) is/are selected from the group consisting of methyl, ethyl and carboxyl), methoxycarbonyl, carboxyl and its salts, and the following formulae

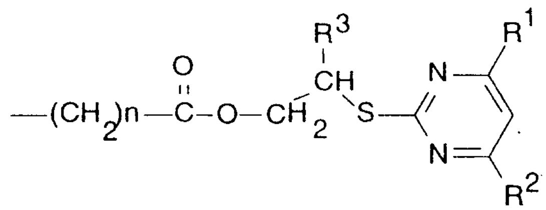

wherein $R^1$, $R^2$, and $R^3$ have the same meanings as defined above and n represents an integer of 0 to 6, or

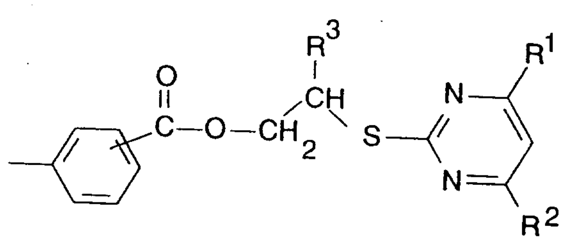

wherein $R^1$, $R^2$ and $R^3$ have the same meanings as defined above.

As the compounds represented by the formula (I) according to the invention may be exemplified the following compounds shown in Table 1, 2 and 3, in addition to those which will be given in the Examples hereinafter shown.

Table 1

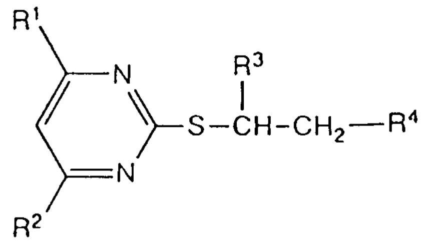

| $R^1$ | $R^2$ | $R^3$ | $R^4$ |
|---|---|---|---|
| $OCH_3$ | $OCH_3$ | $C_2H_5$ | Cl |
| $OCH_3$ | $OCH_3$ | $CH_2CH_2CH_3$ | Cl |
| $CH_3$ | $CH_3$ | $CH(CH_3)_2$ | Cl |
| $OCHF_2$ | $OCHF_2$ | $CH(CH_3)_2$ | Cl |
| $OCF_3$ | $OCF_3$ | $CH(CH_3)_2$ | Cl |
| $OCH_3$ | $OCH_3$ | $CH(CH_3)_2$ | Cl |
| $OCH_3$ | $OCH_3$ | $CH_2CH_2CH_2CH_3$ | Cl |
| $OCH_3$ | $OCH_3$ | $CH_2CH(CH_3)_2$ | Cl |
| $OCH_3$ | $OCH_3$ | $CH(CH_3)CH_2CH_3$ | Cl |
| $CH_3$ | $CH_3$ | $C(CH_3)_3$ | Cl |
| $OCH_3$ | $OCH_3$ | $C(CH_3)_3$ | Cl |
| $OCHF_2$ | $OCHF_2$ | $C(CH_3)_3$ | Cl |
| $OCF_3$ | $OCF_3$ | $C(CH_3)_3$ | Cl |
| $OCH_3$ | $OCH_3$ | $(CH_2)_4CH_3$ | Cl |
| $OCH_3$ | $OCH_3$ | $CH(CH_3)(CH_2)_2CH_3$ | Cl |
| $OCH_3$ | $OCH_3$ | $CH(C_2H_5)_2$ | Cl |
| $OCH_3$ | $OCH_3$ | $C(CH_3)_2CH_2CH_3$ | Cl |
| $OCH_3$ | $OCH_3$ | $CH_2C(CH_3)_3$ | Cl |
| $OCH_3$ | $OCH_3$ | $(CH_2)_2CH(CH_3)_2$ | Cl |
| $OCH_3$ | $OCH_3$ | $(CH_2)_5CH_3$ | Cl |

Table 1 (continued)

| $R^1$ | $R^2$ | $R^3$ | $R^4$ |
|---|---|---|---|
| $OCH_3$ | $OCH_3$ | $CH(CH_3)(CH_2)_3CH_3$ | Cl |
| $OCH_3$ | $OCH_3$ | $C(CH_3)_2CH_2CH_2CH_3$ | Cl |
| $OCH_3$ | $OCH_3$ | $C(C_2H_5)_2CH_3$ | Cl |
| $OCH_3$ | $OCH_3$ | $CH(CH_3)(CH_2)_4CH_3$ | Cl |
| $OCH_3$ | $OCH_3$ | $C(CH_3)_2(CH_2)_3CH_3$ | Cl |
| $OCH_3$ | $OCH_3$ | $CH(CH_3)(CH_2)_5CH_3$ | Cl |
| $OCH_3$ | $OCH_3$ | $C(CH_3)_2(CH_2)_4CH_3$ | Cl |
| $OCH_3$ | $OCH_3$ | $C(C_2H_5)_3$ | Cl |
| $OCH_3$ | $OCH_3$ | 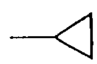 | Cl |
| $OCH_3$ | $OCH_3$ | 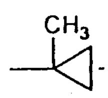 | Cl |
| $OCH_3$ | $OCH_3$ | 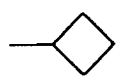 | Cl |
| $OCH_3$ | $OCH_3$ | 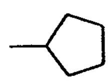 | Cl |
| $OCH_3$ | $OCH_3$ | 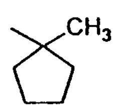 | Cl |
| $OCH_3$ | $OCH_3$ | 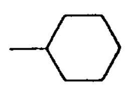 | Cl |
| $OCH_3$ | $OCH_3$ | 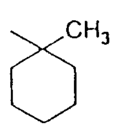 | Cl |
| $OCH_3$ | $OCH_3$ | 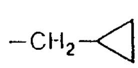 | Cl |

Table 1 (continued)

| $R^1$ | $R^2$ | $R^3$ | $R^4$ |
|---|---|---|---|
| $OCH_3$ | $OCH_3$ | 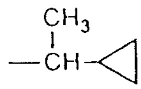 | Cl |
| $OCH_3$ | $OCH_3$ | 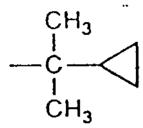 | Cl |
| $OCH_3$ | $OCH_3$ | 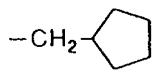 | Cl |
| $OCH_3$ | $OCH_3$ | 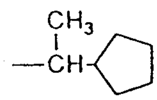 | Cl |
| $OCH_3$ | $OCH_3$ | 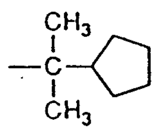 | Cl |
| $OCH_3$ | $OCH_3$ | 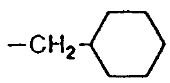 | Cl |
| $OCH_3$ | $OCH_3$ | 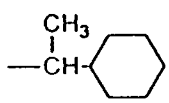 | Cl |
| $OCH_3$ | $OCH_3$ | 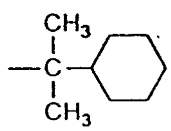 | Cl |
| $OCH_3$ | $OCH_3$ | 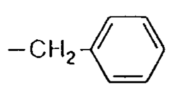 | Cl |

Table 1 (continued)

| $R^1$ | $R^2$ | $R^3$ | $R^4$ |
|---|---|---|---|
| $OCH_3$ | $OCH_3$ | 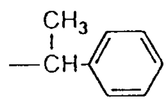 | Cl |
| $OCH_3$ | $OCH_3$ | 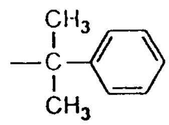 | Cl |
| $OCH_3$ | $OCH_3$ | 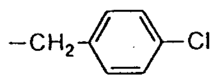 | Cl |
| $OCH_3$ | $OCH_3$ | 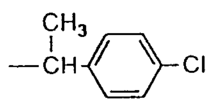 | Cl |
| $OCH_3$ | $OCH_3$ | 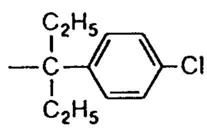 | Cl |
| $OCH_3$ | $OCH_3$ | 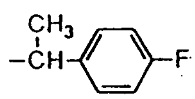 | Cl |
| $OCH_3$ | $OCH_3$ | 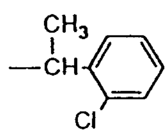 | Cl |
| $OCH_3$ | $OCH_3$ | $CH_2CF_3$ | Cl |
| $OCH_3$ | $OCH_3$ | $CH_2CH_2Cl$ | Cl |
| $OCH_3$ | $OCH_3$ | $C_2H_5$ | Br |
| $OCH_3$ | $OCH_3$ | $CH_2CH_2CH_3$ | Br |
| $OCH_3$ | $OCH_3$ | $CH(CH_3)_2$ | Br |
| $OCHF_2$ | $OCHF_2$ | $CH(CH_3)_2$ | Br |
| $OCF_3$ | $OCF_3$ | $CH(CH_3)_2$ | Br |

Table 1 (continued)

| $R^1$ | $R^2$ | $R^3$ | $R^4$ |
|---|---|---|---|
| $OCH_3$ | $OCH_3$ | $CH_2CH_2CH_2CH_3$ | Br |
| $OCH_3$ | $OCH_3$ | $CH_2CH(CH_3)_2$ | Br |
| $OCH_3$ | $OCH_3$ | $CH(CH_3)CH_2CH_3$ | Br |
| $OCH_3$ | $OCH_3$ | $C(CH_3)_3$ | Br |
| $OCH_3$ | $OCH_3$ | $(CH_2)_4CH_3$ | Br |
| $OCH_3$ | $OCH_3$ | $CH(CH_3)(CH_2)_2CH_3$ | Br |
| $OCH_3$ | $OCH_3$ | $CH(C_2H_5)_2$ | Br |
| $OCH_3$ | $OCH_3$ | $C(CH_3)_2CH_2CH_3$ | Br |
| $OCH_3$ | $OCH_3$ | $CH_2C(CH_3)_3$ | Br |
| $OCH_3$ | $OCH_3$ | $(CH_2)_2CH(CH_3)_2$ | Br |
| $OCH_3$ | $OCH_3$ | $(CH_2)_5CH_3$ | Br |
| $OCH_3$ | $OCH_3$ | $CH(CH_3)(CH_2)_3CH_3$ | Br |
| $OCH_3$ | $OCH_3$ | $C(CH_3)_2CH_2CH_2CH_3$ | Br |
| $OCH_3$ | $OCH_3$ | $C(C_2H_5)_2CH_3$ | Br |
| $OCH_3$ | $OCH_3$ | $CH(CH_3)(CH_2)_4CH_3$ | Br |
| $OCH_3$ | $OCH_3$ | $C(CH_3)_2(CH_2)_3CH_3$ | Br |
| $OCH_3$ | $OCH_3$ | $CH(CH_3)(CH_2)_5CH_3$ | Br |
| $OCH_3$ | $OCH_3$ | $C(CH_3)_2(CH_2)_4CH_3$ | Br |
| $OCH_3$ | $OCH_3$ | $C(C_2H_5)_3$ | Br |
| $OCH_3$ | $OCH_3$ | 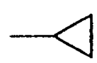 | Br |
| $OCH_3$ | $OCH_3$ | 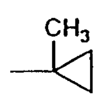 | Br |
| $OCH_3$ | $OCH_3$ | 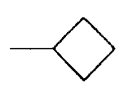 | Br |
| $OCH_3$ | $OCH_3$ | 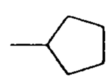 | Br |

Table 1 (continued)

| $R^1$ | $R^2$ | $R^3$ | $R^4$ |
|---|---|---|---|
| $OCH_3$ | $OCH_3$ | 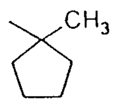 | Br |
| $OCH_3$ | $OCH_3$ | 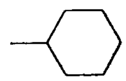 | Br |
| $OCH_3$ | $OCH_3$ | 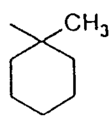 | Br |
| $OCH_3$ | $OCH_3$ | 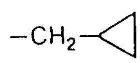 | Br |
| $OCH_3$ | $OCH_3$ | 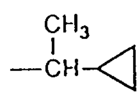 | Br |
| $OCH_3$ | $OCH_3$ | 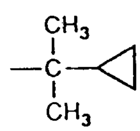 | Br |
| $OCH_3$ | $OCH_3$ | 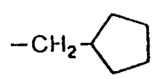 | Br |
| $OCH_3$ | $OCH_3$ | 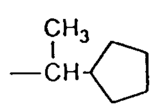 | Br |
| $OCH_3$ | $OCH_3$ | 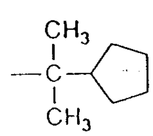 | Br |
| $OCH_3$ | $OCH_3$ | 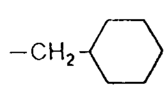 | Br |

Table 1 (continued)

| $R^1$ | $R^2$ | $R^3$ | $R^4$ |
|---|---|---|---|
| $OCH_3$ | $OCH_3$ | 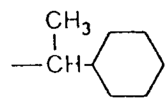 | Br |
| $OCH_3$ | $OCH_3$ | 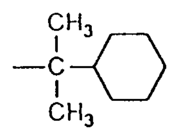 | Br |
| $OCH_3$ | $OCH_3$ | 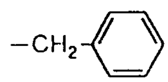 | Br |
| $OCH_3$ | $OCH_3$ | 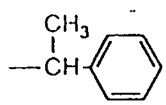 | Br |
| $OCH_3$ | $OCH_3$ | 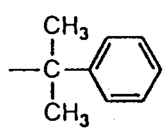 | Br |
| $OCH_3$ | $OCH_3$ | 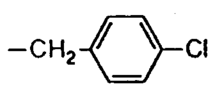 | Br |
| $OCH_3$ | $OCH_3$ | 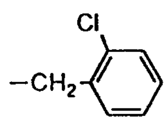 | Br |
| $OCH_3$ | $OCH_3$ | 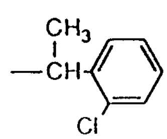 | Br |
| $OCH_3$ | $OCH_3$ | 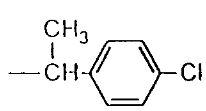 | Br |

Table 1 (continued)

| $R^1$ | $R^2$ | $R^3$ | $R^4$ |
|---|---|---|---|
| $OCH_3$ | $OCH_3$ | 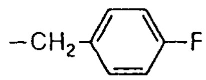 | Br |
| $OCH_3$ | $OCH_3$ | 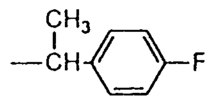 | Br |
| $OCH_3$ | $OCH_3$ | 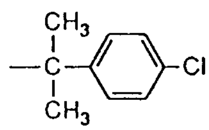 | Br |
| $OCH_3$ | $OCH_3$ | $CH_2CF_3$ | Br |
| $OCH_3$ | $OCH_3$ | $CH_2CH_2Cl$ | Br |
| $OCH_3$ | $OCH_3$ | $C(CH_3)_2CH_2Br$ | 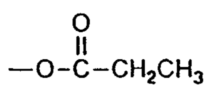 |
| $OCH_3$ | $OCH_3$ | $C(CH_3)_2CH_2Br$ | 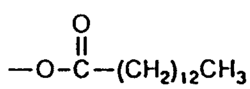 |
| $OCH_3$ | $OCH_3$ | $C(CH_3)_2CH_2Br$ | 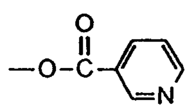 |
| $OCH_3$ | $OCH_3$ | $C(CH_3)_2CH_2Br$ | 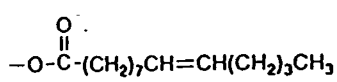 |
| $OCH_3$ | $OCH_3$ | $C(CH_3)_2CH_2Br$ | Cl |
| $OCH_3$ | $OCH_3$ | $C(CH_3)_2CH_2Br$ | Br |
| $OCH_3$ | $OCH_3$ | $C(CH_3)_2CH_2Cl$ | 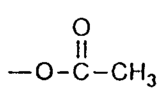 |

Table 1 (continued)

| $R^1$ | $R^2$ | $R^3$ | $R^4$ |
|---|---|---|---|
| $OCH_3$ | $OCH_3$ | $C(CH_3)_2CH_2Cl$ | 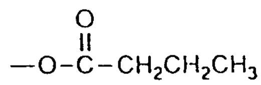 |
| $OCH_3$ | $OCH_3$ | $C(CH_3)_2CH_2Cl$ | 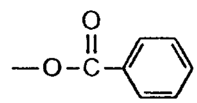 |
| $OCH_3$ | $OCH_3$ | $C(CH_3)_2CH_2Cl$ | 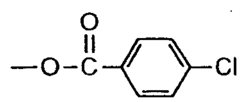 |
| $OCH_3$ | $OCH_3$ | $C(CH_3)_2CH_2Cl$ | Cl |
| $OCH_3$ | $OCH_3$ | $C(CH_3)_2CH_2Cl$ | Br |
| $OCH_3$ | $OCH_3$ | $C_2H_5$ | 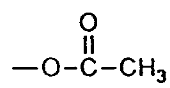 |
| $OCH_3$ | $OCH_3$ | $CH_2CH_2CH_3$ | 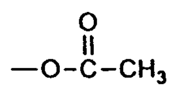 |
| $OCH_3$ | $OCH_3$ | $CH(CH_3)_2$ | 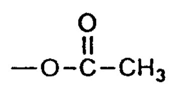 |
| $OCHF_2$ | $OCHF_2$ | $CH(CH_3)_2$ | 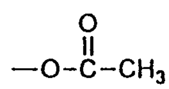 |
| $OCF_3$ | $OCF_3$ | $CH(CH_3)_2$ | 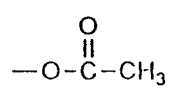 |
| $OCH_3$ | $OCH_3$ | $CH_2CH(CH_3)_2$ | 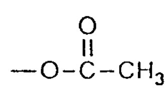 |

Table 1 (continued)

| $R^1$ | $R^2$ | $R^3$ | $R^4$ |
|---|---|---|---|
| $OCH_3$ | $OCH_3$ | $CH(CH_3)CH_2CH_3$ | $-O-\overset{\overset{O}{\|}}{C}-CH_3$ |
| $OCH_3$ | $OCH_3$ | $C(CH_3)_3$ | $-O-\overset{\overset{O}{\|}}{C}-CH_3$ |
| $OCHF_2$ | $OCHF_2$ | $C(CH_3)_3$ | $-O-\overset{\overset{O}{\|}}{C}-CH_3$ |
| $OCF_3$ | $OCF_3$ | $C(CH_3)_3$ | $-O-\overset{\overset{O}{\|}}{C}-CH_3$ |
| $OCH_3$ | $OCH_3$ | $(CH_2)_4CH_3$ | $-O-\overset{\overset{O}{\|}}{C}-CH_3$ |
| $OCH_3$ | $OCH_3$ | $CH(CH_3)(CH_2)_2CH_3$ | $-O-\overset{\overset{O}{\|}}{C}-CH_3$ |
| $OCH_3$ | $OCH_3$ | $CH(C_2H_5)_2$ | $-O-\overset{\overset{O}{\|}}{C}-CH_3$ |
| $OCH_3$ | $OCH_3$ | $C(CH_3)_2CH_2CH_3$ | $-O-\overset{\overset{O}{\|}}{C}-CH_3$ |
| $OCH_3$ | $OCH_3$ | $CH_2C(CH_3)_3$ | $-O-\overset{\overset{O}{\|}}{C}-CH_3$ |
| $OCH_3$ | $OCH_3$ | $(CH_2)_2CH(CH_3)_2$ | $-O-\overset{\overset{O}{\|}}{C}-CH_3$ |
| $OCH_3$ | $OCH_3$ | $(CH_2)_5CH_3$ | $-O-\overset{\overset{O}{\|}}{C}-CH_3$ |

Table 1 (continued)

| $R^1$ | $R^2$ | $R^3$ | $R^4$ |
|---|---|---|---|
| $OCH_3$ | $OCH_3$ | $CH(CH_3)(CH_2)_3CH_3$ | 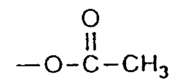 |
| $OCH_3$ | $OCH_3$ | $C(CH_3)_2CH_2CH_2CH_3$ | 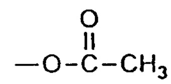 |
| $OCH_3$ | $OCH_3$ | $C(C_2H_5)_2CH_3$ | 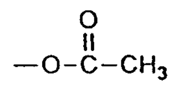 |
| $OCH_3$ | $OCH_3$ | $CH(CH_3)(CH_2)_4CH_3$ | 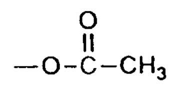 |
| $OCH_3$ | $OCH_3$ | $C(CH_3)_2(CH_2)_3CH_3$ | 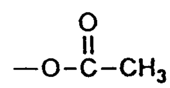 |
| $OCH_3$ | $OCH_3$ | $CH(CH_3)(CH_2)_5CH_3$ | 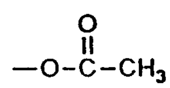 |
| $OCH_3$ | $OCH_3$ | $C(CH_3)_2(CH_2)_4CH_3$ | 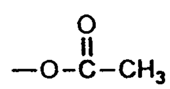 |
| $OCH_3$ | $OCH_3$ | $C(C_2H_5)_3$ | 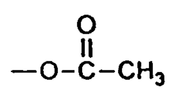 |
| $OCH_3$ | $OCH_3$ | 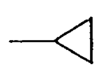 | 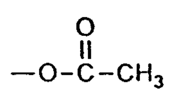 |
| $OCH_3$ | $OCH_3$ | 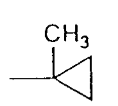 | 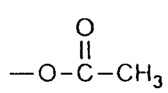 |

Table 1 (continued)

| $R^1$ | $R^2$ | $R^3$ | $R^4$ |
|---|---|---|---|
| $OCH_3$ | $OCH_3$ | 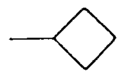 | 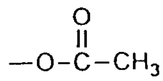 |
| $OCH_3$ | $OCH_3$ | 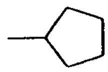 | 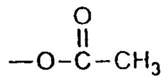 |
| $OCH_3$ | $OCH_3$ | 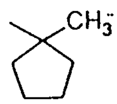 | 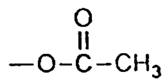 |
| $OCH_3$ | $OCH_3$ | 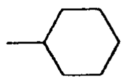 | 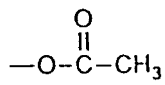 |
| $OCH_3$ | $OCH_3$ | 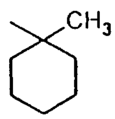 | 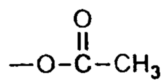 |
| $OCH_3$ | $OCH_3$ | 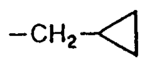 | 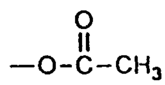 |
| $OCH_3$ | $OCH_3$ | 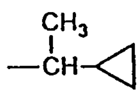 | 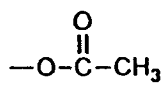 |
| $OCH_3$ | $OCH_3$ | 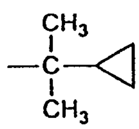 | 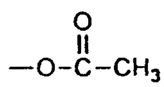 |
| $OCH_3$ | $OCH_3$ | 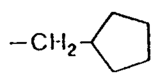 | 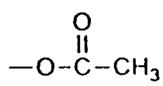 |
| $OCH_3$ | $OCH_3$ | 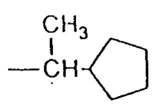 | 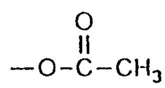 |

Table 1 (continued)

| $R^1$ | $R^2$ | $R^3$ | $R^4$ |
|---|---|---|---|
| $OCH_3$ | $OCH_3$ | 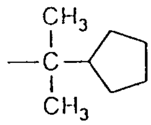 | 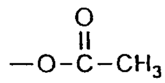 |
| $OCH_3$ | $OCH_3$ | 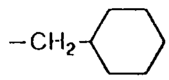 | 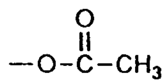 |
| $OCH_3$ | $OCH_3$ | 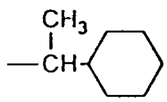 | 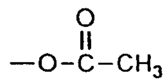 |
| $OCH_3$ | $OCH_3$ | 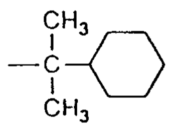 | 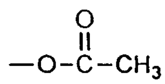 |
| $OCH_3$ | $OCH_3$ | 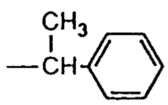 | 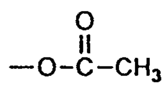 |
| $OCH_3$ | $OCH_3$ | 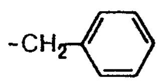 | 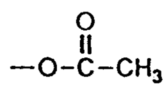 |
| $OCH_3$ | $OCH_3$ | 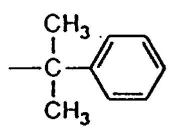 | 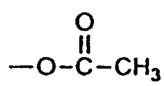 |
| $OCH_3$ | $OCH_3$ | 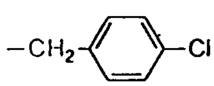 | 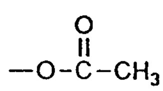 |
| $OCH_3$ | $OCH_3$ | 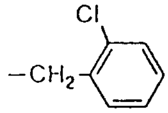 | 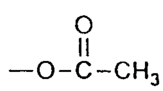 |

Table 1 (continued)

| $R^1$ | $R^2$ | $R^3$ | $R^4$ |
|---|---|---|---|
| $OCH_3$ | $OCH_3$ | 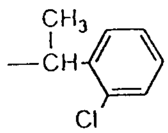 | 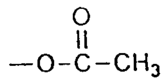 |
| $OCH_3$ | $OCH_3$ | 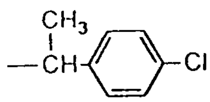 | 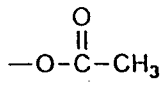 |
| $OCH_3$ | $OCH_3$ | 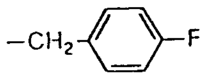 | 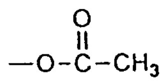 |
| $OCH_3$ | $OCH_3$ | 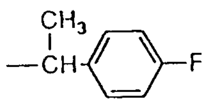 | 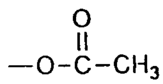 |
| $OCH_3$ | $OCH_3$ | 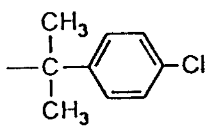 | 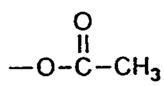 |
| $OCH_3$ | $OCH_3$ | $C_2H_5$ | 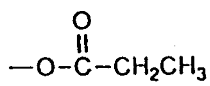 |
| $OCH_3$ | $OCH_3$ | $CH_2CH_2CH_3$ | 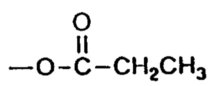 |
| $OCH_3$ | $OCH_3$ | $CH(CH_3)_2$ | 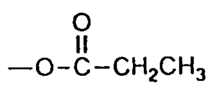 |
| $OCH_3$ | $OCH_3$ | $CH_2CH_2CH_2CH_3$ | 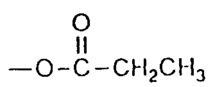 |
| $OCH_3$ | $OCH_3$ | $CH_2CH(CH_3)_2$ | 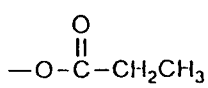 |

Table 1 (continued)

| $R^1$ | $R^2$ | $R^3$ | $R^4$ |
|---|---|---|---|
| $OCH_3$ | $OCH_3$ | $CH(CH_3)CH_2CH_3$ | 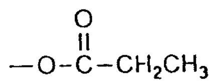 |
| $OCH_3$ | $OCH_3$ | $C(CH_3)_3$ | 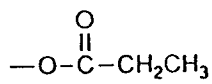 |
| $OCH_3$ | $OCH_3$ | $(CH_2)_4CH_3$ | 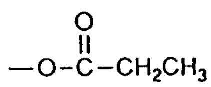 |
| $OCH_3$ | $OCH_3$ | $CH(CH_3)(CH_2)_2CH_3$ | 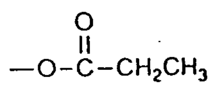 |
| $OCH_3$ | $OCH_3$ | $CH(C_2H_5)_2$ | 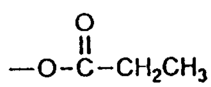 |
| $OCH_3$ | $OCH_3$ | $C(CH_3)_2CH_2CH_3$ | 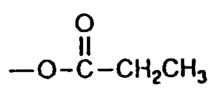 |
| $OCH_3$ | $OCH_3$ | $CH_2C(CH_3)_3$ | 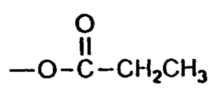 |
| $OCH_3$ | $OCH_3$ | $(CH_2)_2CH(CH_3)_2$ | 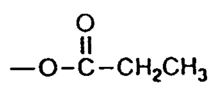 |
| $OCH_3$ | $OCH_3$ | $(CH_2)_5CH_3$ | 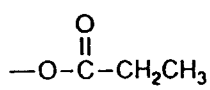 |
| $OCH_3$ | $OCH_3$ | $CH(CH_3)(CH_2)_3CH_3$ | 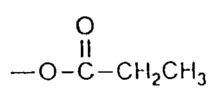 |
| $OCH_3$ | $OCH_3$ | $C(CH_3)_2CH_2CH_2CH_3$ | 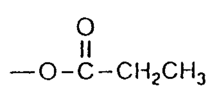 |

Table 1 (continued)

| $R^1$ | $R^2$ | $R^3$ | $R^4$ |
|---|---|---|---|
| $OCH_3$ | $OCH_3$ | $C(C_2H_5)_2CH_3$ | 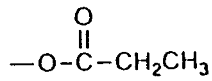 |
| $OCH_3$ | $OCH_3$ | $CH(CH_3)(CH_2)_4CH_3$ | 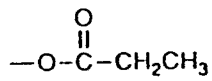 |
| $OCH_3$ | $OCH_3$ | $C(CH_3)_2(CH_2)_3CH_3$ | 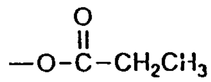 |
| $OCH_3$ | $OCH_3$ | $CH(CH_3)(CH_2)_5CH_3$ | 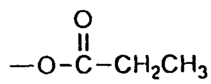 |
| $OCH_3$ | $OCH_3$ | $C(CH_3)_2(CH_2)_4CH_3$ | 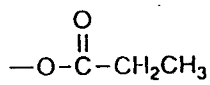 |
| $OCH_3$ | $OCH_3$ | $C(C_2H_5)_3$ | 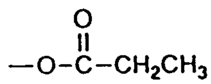 |
| $OCH_3$ | $OCH_3$ | 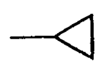 | 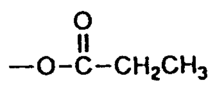 |
| $OCH_3$ | $OCH_3$ | 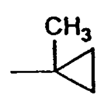 | 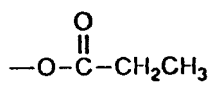 |
| $OCH_3$ | $OCH_3$ | 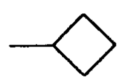 | 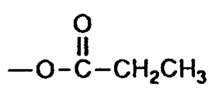 |
| $OCH_3$ | $OCH_3$ | 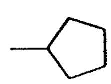 | 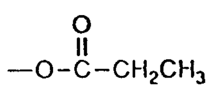 |
| $OCH_3$ | $OCH_3$ | 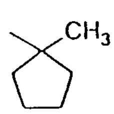 | 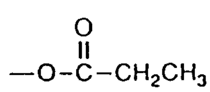 |

Table 1 (continued)

| $R^1$ | $R^2$ | $R^3$ | $R^4$ |
|---|---|---|---|
| $OCH_3$ | $OCH_3$ | 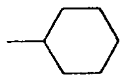 | 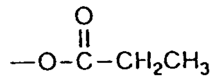 |
| $OCH_3$ | $OCH_3$ | 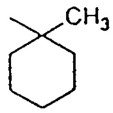 | 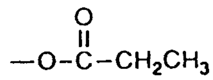 |
| $OCH_3$ | $OCH_3$ | 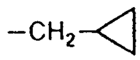 | 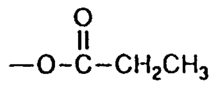 |
| $OCH_3$ | $OCH_3$ | 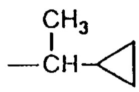 | 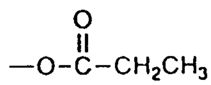 |
| $OCH_3$ | $OCH_3$ | 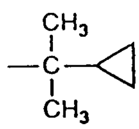 | 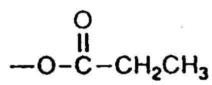 |
| $OCH_3$ | $OCH_3$ | 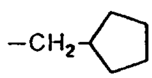 | 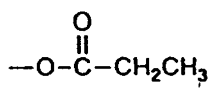 |
| $OCH_3$ | $OCH_3$ | 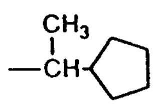 | 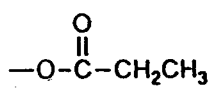 |
| $OCH_3$ | $OCH_3$ | 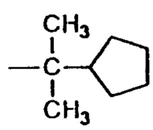 | 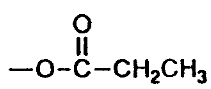 |
| $OCH_3$ | $OCH_3$ | 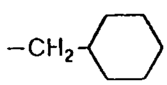 | 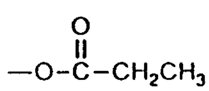 |
| $OCH_3$ | $OCH_3$ | 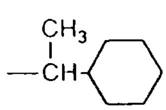 | 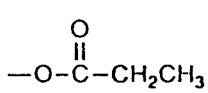 |

Table 1 (continued)

| $R^1$ | $R^2$ | $R^3$ | $R^4$ |
|---|---|---|---|
| $OCH_3$ | $OCH_3$ | 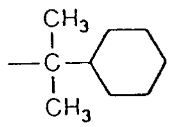 | 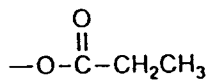 |
| $OCH_3$ | $OCH_3$ | 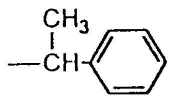 | 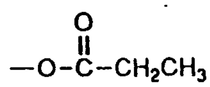 |
| $OCH_3$ | $OCH_3$ | 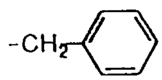 | 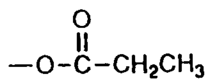 |
| $OCH_3$ | $OCH_3$ | 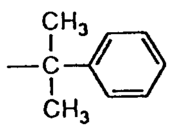 | 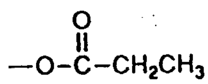 |
| $OCH_3$ | $OCH_3$ | 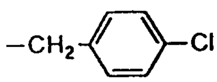 | 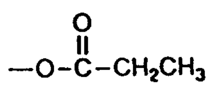 |
| $OCH_3$ | $OCH_3$ | 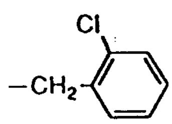 | 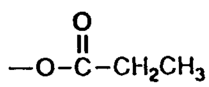 |
| $OCH_3$ | $OCH_3$ | 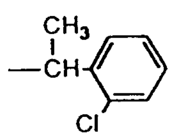 | 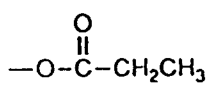 |
| $OCH_3$ | $OCH_3$ | 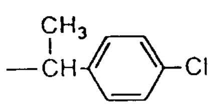 | 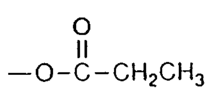 |
| $OCH_3$ | $OCH_3$ | 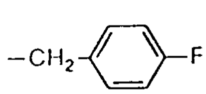 | 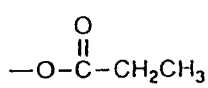 |

Table 1 (continued)

| $R^1$ | $R^2$ | $R^3$ | $R^4$ |
|---|---|---|---|
| $OCH_3$ | $OCH_3$ | 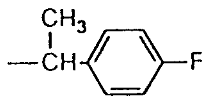 | 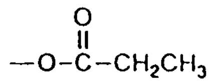 |
| $OCH_3$ | $OCH_3$ | 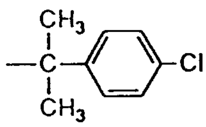 | 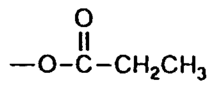 |
| $OCH_3$ | $OCH_3$ | $C_2H_5$ | 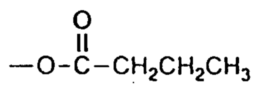 |
| $OCH_3$ | $OCH_3$ | $CH_2CH_2CH_3$ | 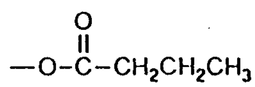 |
| $OCH_3$ | $OCH_3$ | $CH(CH_3)_2$ | 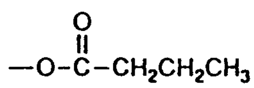 |
| $OCH_3$ | $OCH_3$ | $CH_2CH_2CH_2CH_3$ | 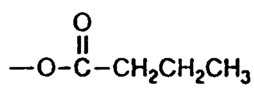 |
| $OCH_3$ | $OCH_3$ | $CH_2CH(CH_3)_2$ | 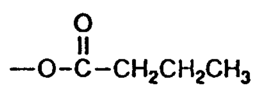 |
| $OCH_3$ | $OCH_3$ | $CH(CH_3)CH_2CH_3$ | 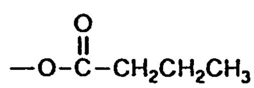 |
| $OCH_3$ | $OCH_3$ | $C(CH_3)_3$ | 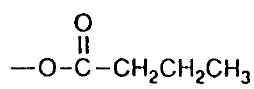 |
| $OCH_3$ | $OCH_3$ | $(CH_2)_4CH_3$ | 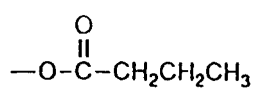 |

Table 1 (continued)

| $R^1$ | $R^2$ | $R^3$ | $R^4$ |
|---|---|---|---|
| $OCH_3$ | $OCH_3$ | $CH(CH_3)(CH_2)_2CH_3$ | 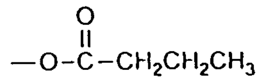 |
| $OCH_3$ | $OCH_3$ | $CH(C_2H_5)_2$ | 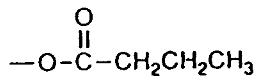 |
| $OCH_3$ | $OCH_3$ | $C(CH_3)_2CH_2CH_3$ | 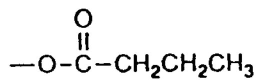 |
| $OCH_3$ | $OCH_3$ | $CH_2C(CH_3)_3$ | 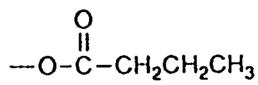 |
| $OCH_3$ | $OCH_3$ | $(CH_2)_2CH(CH_3)_2$ | 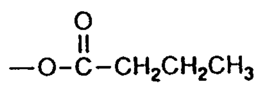 |
| $OCH_3$ | $OCH_3$ | $(CH_2)_5CH_3$ | 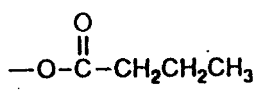 |
| $OCH_3$ | $OCH_3$ | $CH(CH_3)(CH_2)_3CH_3$ | 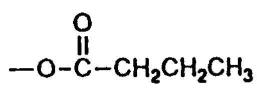 |
| $OCH_3$ | $OCH_3$ | $C(CH_3)_2CH_2CH_2CH_3$ | 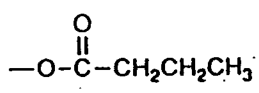 |
| $OCH_3$ | $OCH_3$ | $C(C_2H_5)_2CH_3$ | 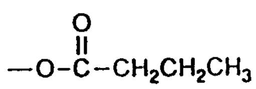 |
| $OCH_3$ | $OCH_3$ | $CH(CH_3)(CH_2)_4CH_3$ | 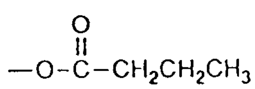 |
| $OCH_3$ | $OCH_3$ | $C(CH_3)_2(CH_2)_3CH_3$ | 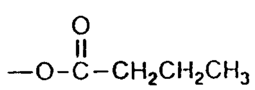 |

Table 1 (continued)

| $R^1$ | $R^2$ | $R^3$ | $R^4$ |
|---|---|---|---|
| $OCH_3$ | $OCH_3$ | $CH(CH_3)(CH_2)_5CH_3$ | $-O-\overset{O}{\overset{\|}{C}}-CH_2CH_2CH_3$ |
| $OCH_3$ | $OCH_3$ | $C(CH_3)_2(CH_2)_4CH_3$ | $-O-\overset{O}{\overset{\|}{C}}-CH_2CH_2CH_3$ |
| $OCH_3$ | $OCH_3$ | $C(C_2H_5)_3$ | $-O-\overset{O}{\overset{\|}{C}}-CH_2CH_2CH_3$ |
| $OCH_3$ | $OCH_3$ | 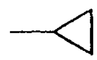 | $-O-\overset{O}{\overset{\|}{C}}-CH_2CH_2CH_3$ |
| $OCH_3$ | $OCH_3$ | 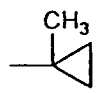 | $-O-\overset{O}{\overset{\|}{C}}-CH_2CH_2CH_3$ |
| $OCH_3$ | $OCH_3$ | 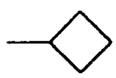 | $-O-\overset{O}{\overset{\|}{C}}-CH_2CH_2CH_3$ |
| $OCH_3$ | $OCH_3$ | 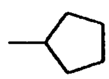 | $-O-\overset{O}{\overset{\|}{C}}-CH_2CH_2CH_3$ |
| $OCH_3$ | $OCH_3$ | 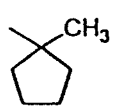 | $-O-\overset{O}{\overset{\|}{C}}-CH_2CH_2CH_3$ |
| $OCH_3$ | $OCH_3$ | 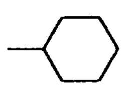 | $-O-\overset{O}{\overset{\|}{C}}-CH_2CH_2CH_3$ |
| $OCH_3$ | $OCH_3$ | 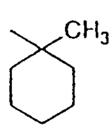 | $-O-\overset{O}{\overset{\|}{C}}-CH_2CH_2CH_3$ |

Table 1 (continued)

| $R^1$ | $R^2$ | $R^3$ | $R^4$ |
|---|---|---|---|
| $OCH_3$ | $OCH_3$ | 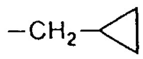 | 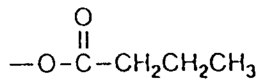 |
| $OCH_3$ | $OCH_3$ | 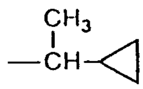 | 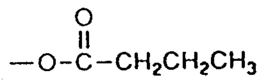 |
| $OCH_3$ | $OCH_3$ | 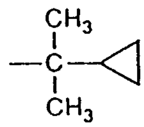 | 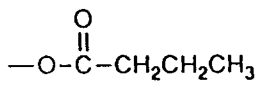 |
| $OCH_3$ | $OCH_3$ | 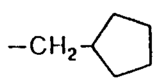 | 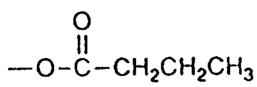 |
| $OCH_3$ | $OCH_3$ | 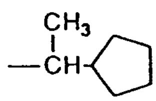 | 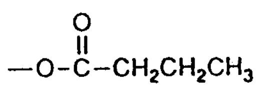 |
| $OCH_3$ | $OCH_3$ | 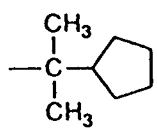 | 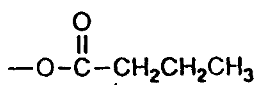 |
| $OCH_3$ | $OCH_3$ | 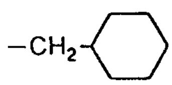 | 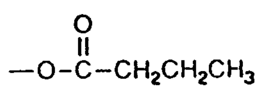 |
| $OCH_3$ | $OCH_3$ | 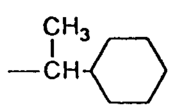 | 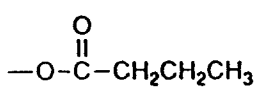 |
| $OCH_3$ | $OCH_3$ | 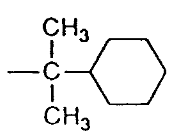 | 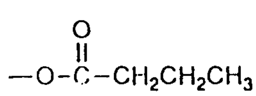 |
| $OCH_3$ | $OCH_3$ | 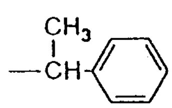 | 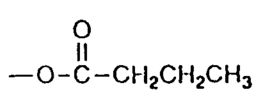 |

Table 1 (continued)

| $R^1$ | $R^2$ | $R^3$ | $R^4$ |
|---|---|---|---|
| $OCH_3$ | $OCH_3$ | 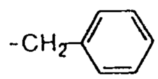 | 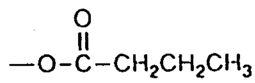 |
| $OCH_3$ | $OCH_3$ | 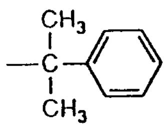 | 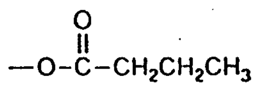 |
| $OCH_3$ | $OCH_3$ | 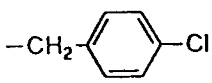 | 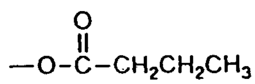 |
| $OCH_3$ | $OCH_3$ | 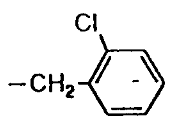 | 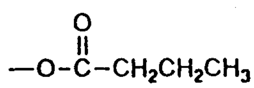 |
| $OCH_3$ | $OCH_3$ | 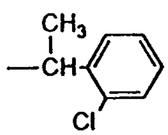 | 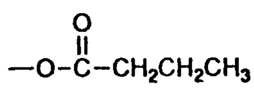 |
| $OCH_3$ | $OCH_3$ | 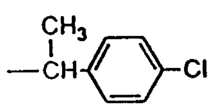 | 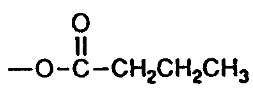 |
| $OCH_3$ | $OCH_3$ | 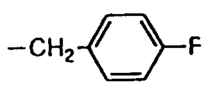 | 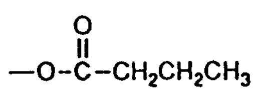 |
| $OCH_3$ | $OCH_3$ | 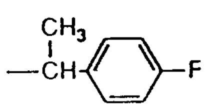 | 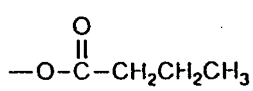 |
| $OCH_3$ | $OCH_3$ | 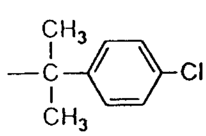 | 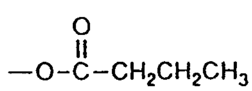 |

Table 1 (continued)

| $R^1$ | $R^2$ | $R^3$ | $R^4$ |
|---|---|---|---|
| $OCH_3$ | $OCH_3$ | $C_2H_5$ | 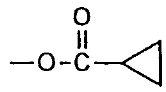 |
| $OCH_3$ | $OCH_3$ | $CH_2CH_2CH_3$ | 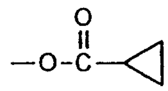 |
| $OCH_3$ | $OCH_3$ | $CH(CH_3)_2$ | 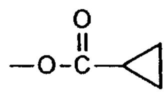 |
| $OCH_3$ | $OCH_3$ | $CH_2CH_2CH_2CH_3$ | 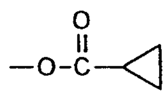 |
| $OCH_3$ | $OCH_3$ | $CH_2CH(CH_3)_2$ | 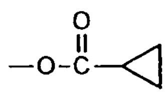 |
| $OCH_3$ | $OCH_3$ | $CH(CH_3)CH_2CH_3$ | 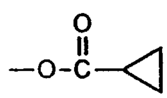 |
| $OCH_3$ | $OCH_3$ | $C(CH_3)_3$ | 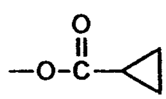 |
| $OCH_3$ | $OCH_3$ | $(CH_2)_4CH_3$ | 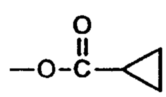 |
| $OCH_3$ | $OCH_3$ | $CH(CH_3)(CH_2)_2CH_3$ | 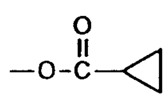 |
| $OCH_3$ | $OCH_3$ | $CH(C_2H_5)_2$ | 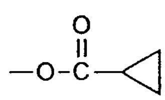 |

Table 1 (continued)

| $R^1$ | $R^2$ | $R^3$ | $R^4$ |
|---|---|---|---|
| $OCH_3$ | $OCH_3$ | $C(CH_3)_2CH_2CH_3$ | 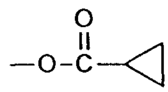 |
| $OCH_3$ | $OCH_3$ | $CH_2C(CH_3)_3$ | 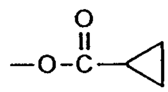 |
| $OCH_3$ | $OCH_3$ | $(CH_2)_2CH(CH_3)_2$ | 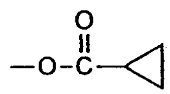 |
| $OCH_3$ | $OCH_3$ | $(CH_2)_5CH_3$ | 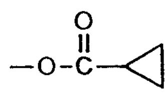 |
| $OCH_3$ | $OCH_3$ | $CH(CH_3)(CH_2)_3CH_3$ | 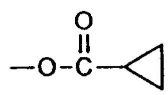 |
| $OCH_3$ | $OCH_3$ | $C(CH_3)_2CH_2CH_2CH_3$ | 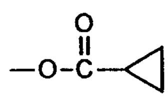 |
| $OCH_3$ | $OCH_3$ | $C(C_2H_5)_2CH_3$ | 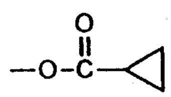 |
| $OCH_3$ | $OCH_3$ | $CH(CH_3)(CH_2)_4CH_3$ | 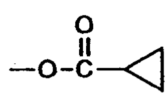 |
| $OCH_3$ | $OCH_3$ | $C(CH_3)_2(CH_2)_3CH_3$ | 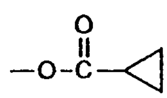 |
| $OCH_3$ | $OCH_3$ | $CH(CH_3)(CH_2)_5CH_3$ | 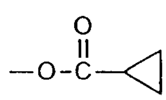 |

Table 1 (continued)

| $R^1$ | $R^2$ | $R^3$ | $R^4$ |
|---|---|---|---|
| $OCH_3$ | $OCH_3$ | $C(CH_3)_2(CH_2)_4CH_3$ | 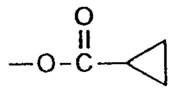 |
| $OCH_3$ | $OCH_3$ | $C(C_2H_5)_3$ | 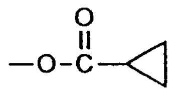 |
| $OCH_3$ | $OCH_3$ | 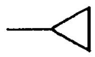 | 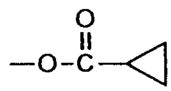 |
| $OCH_3$ | $OCH_3$ | 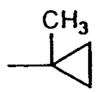 | 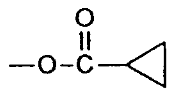 |
| $OCH_3$ | $OCH_3$ | 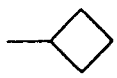 | 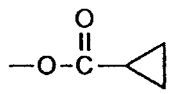 |
| $OCH_3$ | $OCH_3$ | 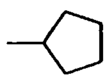 | 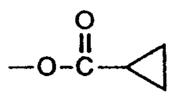 |
| $OCH_3$ | $OCH_3$ | 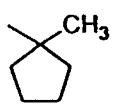 | 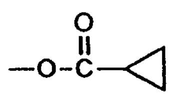 |
| $OCH_3$ | $OCH_3$ | 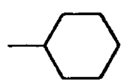 | 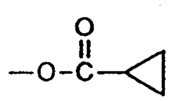 |
| $OCH_3$ | $OCH_3$ | 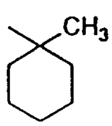 | 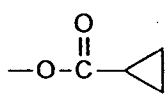 |
| $OCH_3$ | $OCH_3$ | 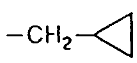 | 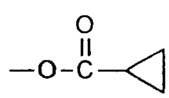 |

Table 1 (continued)

| $R^1$ | $R^2$ | $R^3$ | $R^4$ |
|---|---|---|---|
| $OCH_3$ | $OCH_3$ | 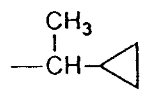 | 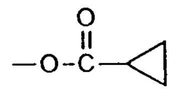 |
| $OCH_3$ | $OCH_3$ | 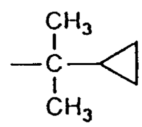 | 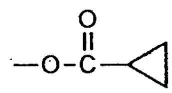 |
| $OCH_3$ | $OCH_3$ | 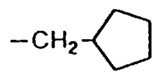 | 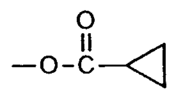 |
| $OCH_3$ | $OCH_3$ | 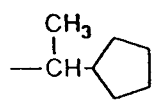 | 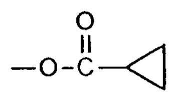 |
| $OCH_3$ | $OCH_3$ | 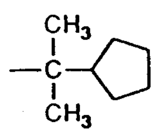 | 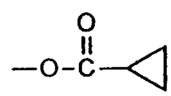 |
| $OCH_3$ | $OCH_3$ | 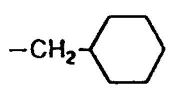 | 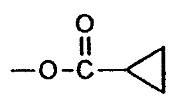 |
| $OCH_3$ | $OCH_3$ | 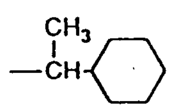 | 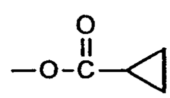 |
| $OCH_3$ | $OCH_3$ | 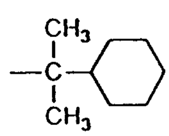 | 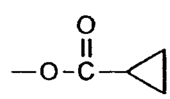 |
| $OCH_3$ | $OCH_3$ | 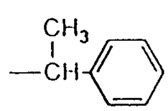 | 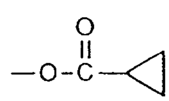 |

Table 1 (continued)

| $R^1$ | $R^2$ | $R^3$ | $R^4$ |
|---|---|---|---|
| $OCH_3$ | $OCH_3$ | 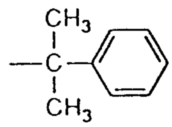 | 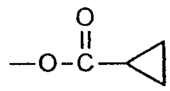 |
| $OCH_3$ | $OCH_3$ | 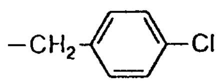 | 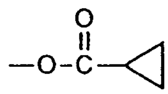 |
| $OCH_3$ | $OCH_3$ | 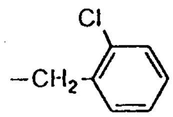 | 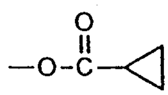 |
| $OCH_3$ | $OCH_3$ | 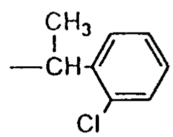 | 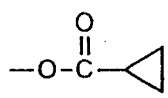 |
| $OCH_3$ | $OCH_3$ | 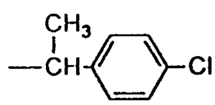 | 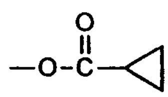 |
| $OCH_3$ | $OCH_3$ | 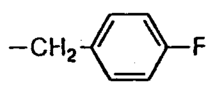 | 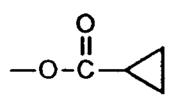 |
| $OCH_3$ | $OCH_3$ | 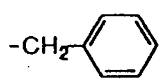 | 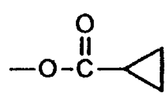 |
| $OCH_3$ | $OCH_3$ | 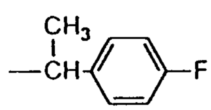 | 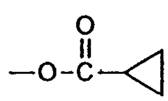 |
| $OCH_3$ | $OCH_3$ | 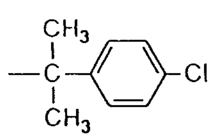 | 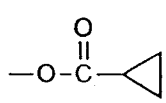 |

Table 1 (continued)

| $R^1$ | $R^2$ | $R^3$ | $R^4$ |
|---|---|---|---|
| $OCH_3$ | $OCH_3$ | $C_2H_5$ | $-O-\overset{\overset{O}{\|}}{C}-CH_2CH_2CH_2CH_3$ |
| $OCH_3$ | $OCH_3$ | $CH_2CH_2CH_3$ | $-O-\overset{\overset{O}{\|}}{C}-CH_2CH_2CH_2CH_3$ |
| $OCH_3$ | $OCH_3$ | $CH(CH_3)_2$ | $-O-\overset{\overset{O}{\|}}{C}-CH_2CH_2CH_2CH_3$ |
| $OCH_3$ | $OCH_3$ | $CH_2CH_2CH_2CH_3$ | $-O-\overset{\overset{O}{\|}}{C}-CH_2CH_2CH_2CH_3$ |
| $OCH_3$ | $OCH_3$ | $CH_2CH(CH_3)_2$ | $-O-\overset{\overset{O}{\|}}{C}-CH_2CH_2CH_2CH_3$ |
| $OCH_3$ | $OCH_3$ | $CH(CH_3)CH_2CH_3$ | $-O-\overset{\overset{O}{\|}}{C}-CH_2CH_2CH_2CH_3$ |
| $OCH_3$ | $OCH_3$ | $C(CH_3)_3$ | $-O-\overset{\overset{O}{\|}}{C}-CH_2CH_2CH_2CH_3$ |
| $OCH_3$ | $OCH_3$ | $(CH_2)_4CH_3$ | $-O-\overset{\overset{O}{\|}}{C}-CH_2CH_2CH_2CH_3$ |
| $OCH_3$ | $OCH_3$ | $CH(CH_3)(CH_2)_2CH_3$ | $-O-\overset{\overset{O}{\|}}{C}-CH_2CH_2CH_2CH_3$ |
| $OCH_3$ | $OCH_3$ | $CH(C_2H_5)_2$ | $-O-\overset{\overset{O}{\|}}{C}-CH_2CH_2CH_2CH_3$ |
| $OCH_3$ | $OCH_3$ | $C(CH_3)_2CH_2CH_3$ | $-O-\overset{\overset{O}{\|}}{C}-CH_2CH_2CH_2CH_3$ |

Table 1 (continued)

| $R^1$ | $R^2$ | $R^3$ | $R^4$ |
|---|---|---|---|
| $OCH_3$ | $OCH_3$ | $CH_2C(CH_3)_3$ | $-O-\overset{\overset{O}{\Vert}}{C}-CH_2CH_2CH_2CH_3$ |
| $OCH_3$ | $OCH_3$ | $(CH_2)_2CH(CH_3)_2$ | $-O-\overset{\overset{O}{\Vert}}{C}-CH_2CH_2CH_2CH_3$ |
| $OCH_3$ | $OCH_3$ | $(CH_2)_5CH_3$ | $-O-\overset{\overset{O}{\Vert}}{C}-CH_2CH_2CH_2CH_3$ |
| $OCH_3$ | $OCH_3$ | $CH(CH_3)(CH_2)_3CH_3$ | $-O-\overset{\overset{O}{\Vert}}{C}-CH_2CH_2CH_2CH_3$ |
| $OCH_3$ | $OCH_3$ | $C(CH_3)_2CH_2CH_2CH_3$ | $-O-\overset{\overset{O}{\Vert}}{C}-CH_2CH_2CH_2CH_3$ |
| $OCH_3$ | $OCH_3$ | $C(C_2H_5)_2CH_3$ | $-O-\overset{\overset{O}{\Vert}}{C}-CH_2CH_2CH_2CH_3$ |
| $OCH_3$ | $OCH_3$ | $CH(CH_3)(CH_2)_4CH_3$ | $-O-\overset{\overset{O}{\Vert}}{C}-CH_2CH_2CH_2CH_3$ |
| $OCH_3$ | $OCH_3$ | $C(CH_3)_2(CH_2)_3CH_3$ | $-O-\overset{\overset{O}{\Vert}}{C}-CH_2CH_2CH_2CH_3$ |
| $OCH_3$ | $OCH_3$ | $CH(CH_3)(CH_2)_5CH_3$ | $-O-\overset{\overset{O}{\Vert}}{C}-CH_2CH_2CH_2CH_3$ |
| $OCH_3$ | $OCH_3$ | $C(CH_3)_2(CH_2)_4CH_3$ | $-O-\overset{\overset{O}{\Vert}}{C}-CH_2CH_2CH_2CH_3$ |
| $OCH_3$ | $OCH_3$ | $C(C_2H_5)_3$ | $-O-\overset{\overset{O}{\Vert}}{C}-CH_2CH_2CH_2CH_3$ |

Table 1 (continued)

| $R^1$ | $R^2$ | $R^3$ | $R^4$ |
|---|---|---|---|
| $OCH_3$ | $OCH_3$ | 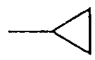 | 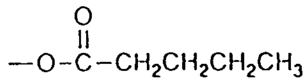 |
| $OCH_3$ | $OCH_3$ | 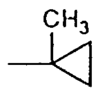 | 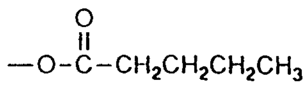 |
| $OCH_3$ | $OCH_3$ | 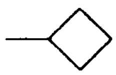 | 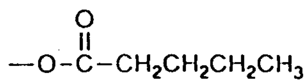 |
| $OCH_3$ | $OCH_3$ | 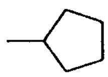 | 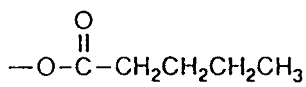 |
| $OCH_3$ | $OCH_3$ | 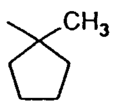 | 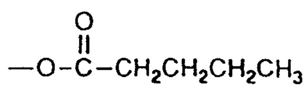 |
| $OCH_3$ | $OCH_3$ | 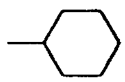 | 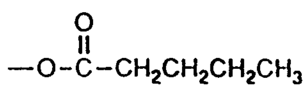 |
| $OCH_3$ | $OCH_3$ | 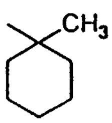 | 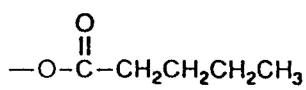 |
| $OCH_3$ | $OCH_3$ | 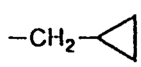 | 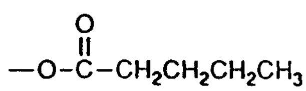 |
| $OCH_3$ | $OCH_3$ | 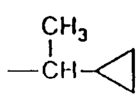 | 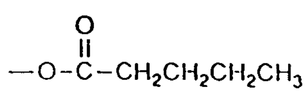 |
| $OCH_3$ | $OCH_3$ | 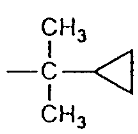 | 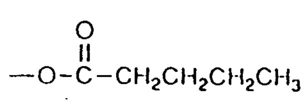 |

Table 1 (continued)

| $R^1$ | $R^2$ | $R^3$ | $R^4$ |
|---|---|---|---|
| $OCH_3$ | $OCH_3$ | 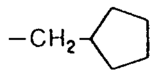 | 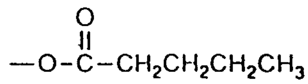 |
| $OCH_3$ | $OCH_3$ | 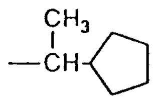 | 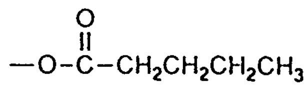 |
| $OCH_3$ | $OCH_3$ | 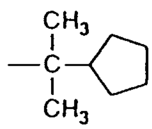 | 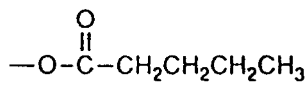 |
| $OCH_3$ | $OCH_3$ | 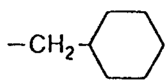 | 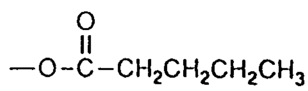 |
| $OCH_3$ | $OCH_3$ | 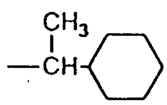 | 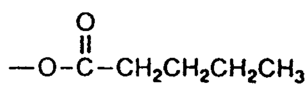 |
| $OCH_3$ | $OCH_3$ | 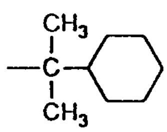 | 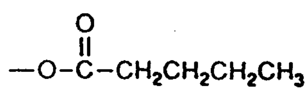 |
| $OCH_3$ | $OCH_3$ | 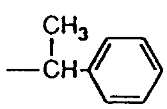 | 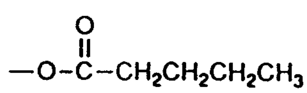 |
| $OCH_3$ | $OCH_3$ | 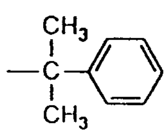 | 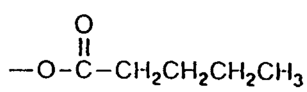 |
| $OCH_3$ | $OCH_3$ | 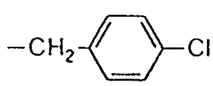 | 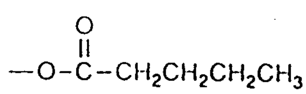 |

Table 1 (continued)

| $R^1$ | $R^2$ | $R^3$ | $R^4$ |
|---|---|---|---|
| $OCH_3$ | $OCH_3$ | 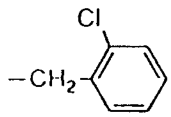 | 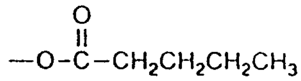 |
| $OCH_3$ | $OCH_3$ | 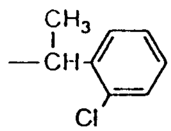 | 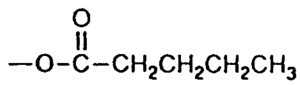 |
| $OCH_3$ | $OCH_3$ | 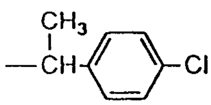 | 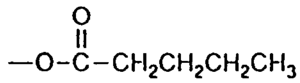 |
| $OCH_3$ | $OCH_3$ | 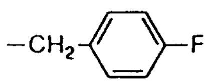 | 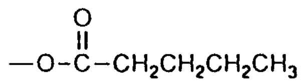 |
| $OCH_3$ | $OCH_3$ | 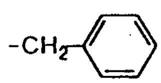 | 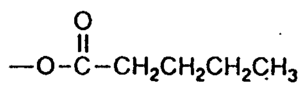 |
| $OCH_3$ | $OCH_3$ | 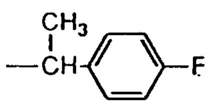 | 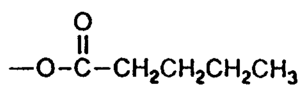 |
| $OCH_3$ | $OCH_3$ | 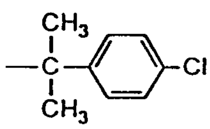 | 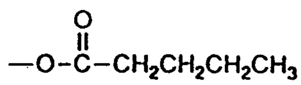 |
| $OCH_3$ | $OCH_3$ | $C_2H_5$ | 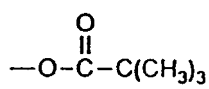 |
| $OCH_3$ | $OCH_3$ | $CH_2CH_2CH_3$ | 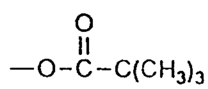 |
| $OCH_3$ | $OCH_3$ | $CH(CH_3)_2$ | 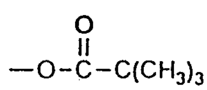 |

Table 1 (continued)

| $R^1$ | $R^2$ | $R^3$ | $R^4$ |
|---|---|---|---|
| $OCH_3$ | $OCH_3$ | $CH_2CH_2CH_2CH_3$ | 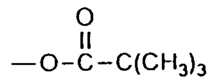 |
| $OCH_3$ | $OCH_3$ | $CH_2CH(CH_3)_2$ | 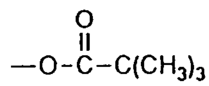 |
| $OCH_3$ | $OCH_3$ | $CH(CH_3)CH_2CH_3$ | 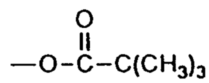 |
| $OCH_3$ | $OCH_3$ | $C(CH_3)_3$ | 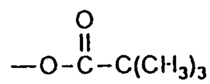 |
| $OCH_3$ | $OCH_3$ | $(CH_2)_4CH_3$ | 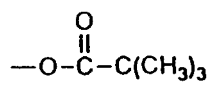 |
| $OCH_3$ | $OCH_3$ | $CH(CH_3)(CH_2)_2CH_3$ | 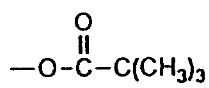 |
| $OCH_3$ | $OCH_3$ | $CH(C_2H_5)_2$ | 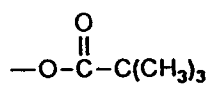 |
| $OCH_3$ | $OCH_3$ | $C(CH_3)_2CH_2CH_3$ | 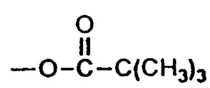 |
| $OCH_3$ | $OCH_3$ | $CH_2C(CH_3)_3$ | 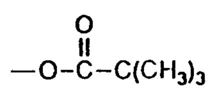 |
| $OCH_3$ | $OCH_3$ | $(CH_2)_2CH(CH_3)_2$ | 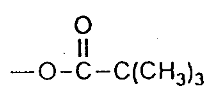 |
| $OCH_3$ | $OCH_3$ | $(CH_2)_5CH_3$ | 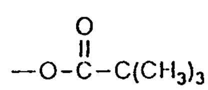 |

Table 1 (continued)

| $R^1$ | $R^2$ | $R^3$ | $R^4$ |
|---|---|---|---|
| $OCH_3$ | $OCH_3$ | $CH(CH_3)(CH_2)_3CH_3$ | 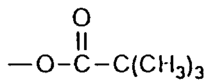 |
| $OCH_3$ | $OCH_3$ | $C(CH_3)_2CH_2CH_2CH_3$ | 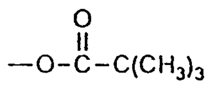 |
| $OCH_3$ | $OCH_3$ | $C(C_2H_5)_2CH_3$ | 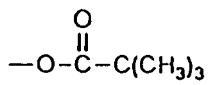 |
| $OCH_3$ | $OCH_3$ | $CH(CH_3)(CH_2)_4CH_3$ | 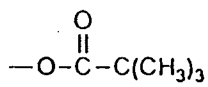 |
| $OCH_3$ | $OCH_3$ | $C(CH_3)_2(CH_2)_3CH_3$ | 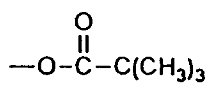 |
| $OCH_3$ | $OCH_3$ | $CH(CH_3)(CH_2)_5CH_3$ | 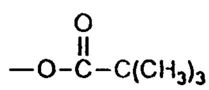 |
| $OCH_3$ | $OCH_3$ | $C(CH_3)_2(CH_2)_4CH_3$ | 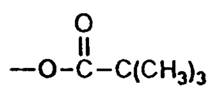 |
| $OCH_3$ | $OCH_3$ | $C(C_2H_5)_3$ | 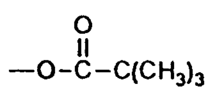 |
| $OCH_3$ | $OCH_3$ | 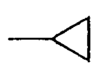 | 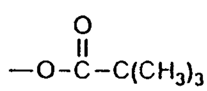 |
| $OCH_3$ | $OCH_3$ | 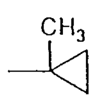 | 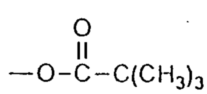 |

Table 1 (continued)

| $R^1$ | $R^2$ | $R^3$ | $R^4$ |
|---|---|---|---|
| $OCH_3$ | $OCH_3$ | 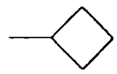 | 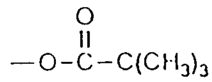 |
| $OCH_3$ | $OCH_3$ | 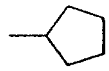 | 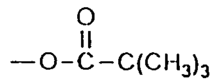 |
| $OCH_3$ | $OCH_3$ | 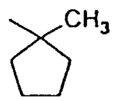 | 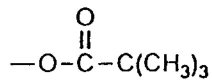 |
| $OCH_3$ | $OCH_3$ | 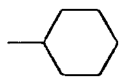 | 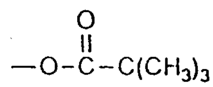 |
| $OCH_3$ | $OCH_3$ | 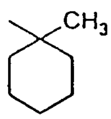 | 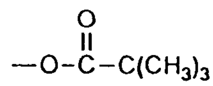 |
| $OCH_3$ | $OCH_3$ | 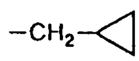 | 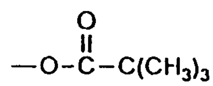 |
| $OCH_3$ | $OCH_3$ | 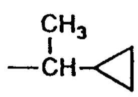 | 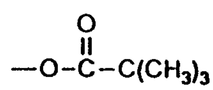 |
| $OCH_3$ | $OCH_3$ | 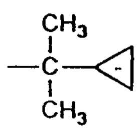 | 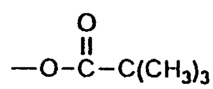 |
| $OCH_3$ | $OCH_3$ | 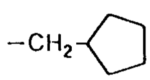 | 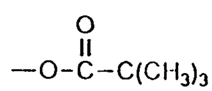 |
| $OCH_3$ | $OCH_3$ | 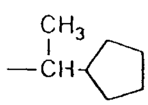 | 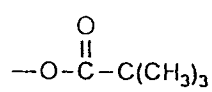 |

Table 1 (continued)

| $R^1$ | $R^2$ | $R^3$ | $R^4$ |
|---|---|---|---|
| $OCH_3$ | $OCH_3$ | 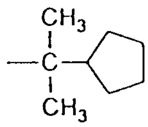 | 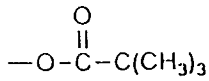 |
| $OCH_3$ | $OCH_3$ | 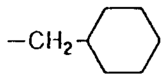 | 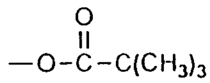 |
| $OCH_3$ | $OCH_3$ | 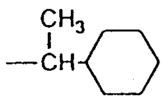 | 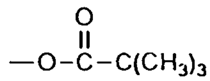 |
| $OCH_3$ | $OCH_3$ | 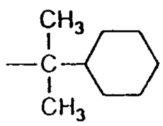 | 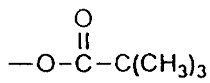 |
| $OCH_3$ | $OCH_3$ | 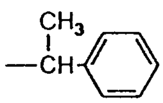 | 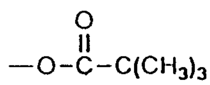 |
| $OCH_3$ | $OCH_3$ | 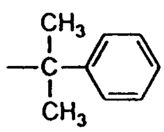 | 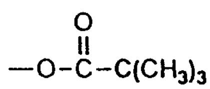 |
| $OCH_3$ | $OCH_3$ | 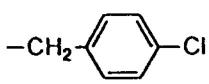 | 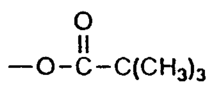 |
| $OCH_3$ | $OCH_3$ | 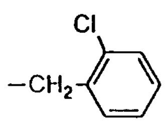 | 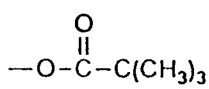 |
| $OCH_3$ | $OCH_3$ | 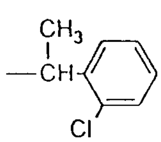 | 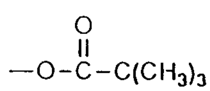 |

Table 1 (continued)

| $R^1$ | $R^2$ | $R^3$ | $R^4$ |
|---|---|---|---|
| $OCH_3$ | $OCH_3$ | 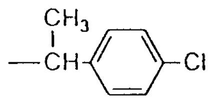 | 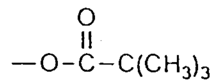 |
| $OCH_3$ | $OCH_3$ | 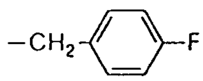 | 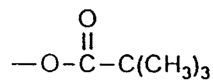 |
| $OCH_3$ | $OCH_3$ | 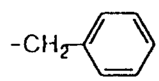 | 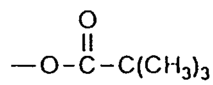 |
| $OCH_3$ | $OCH_3$ | 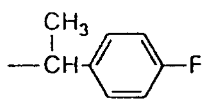 | 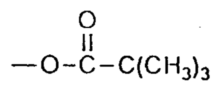 |
| $OCH_3$ | $OCH_3$ | 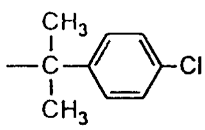 | 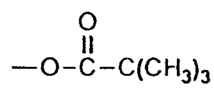 |
| $OCH_3$ | $OCH_3$ | $C_2H_5$ | 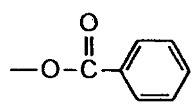 |
| $OCH_3$ | $OCH_3$ | $CH_2CH_2CH_3$ | 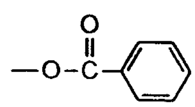 |
| $OCH_3$ | $OCH_3$ | $CH(CH_3)_2$ | 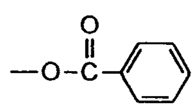 |
| $OCHF_2$ | $OCHF_2$ | $CH(CH_3)_2$ | 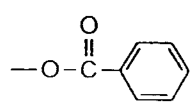 |
| $OCF_3$ | $OCF_3$ | $CH(CH_3)_2$ | 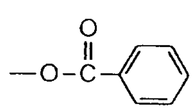 |

Table 1 (continued)

| $R^1$ | $R^2$ | $R^3$ | $R^4$ |
|---|---|---|---|
| $OCH_3$ | $OCH_3$ | $CH_2CH_2CH_2CH_3$ | 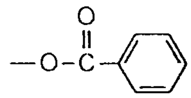 |
| $OCH_3$ | $OCH_3$ | $CH_2CH(CH_3)_2$ | 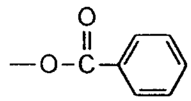 |
| $OCH_3$ | $OCH_3$ | $CH(CH_3)CH_2CH_3$ | 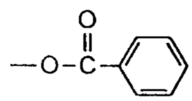 |
| $OCH_3$ | $OCH_3$ | $C(CH_3)_3$- | 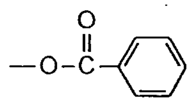 |
| $OCH_3$ | $OCH_3$ | $(CH_2)_4CH_3$ | 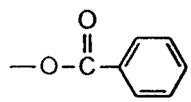 |
| $OCH_3$ | $OCH_3$ | $CH(CH_3)(CH_2)_2CH_3$ | 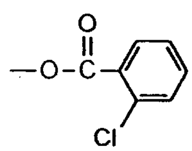 |
| $OCH_3$ | $OCH_3$ | $CH(C_2H_5)_2$ | 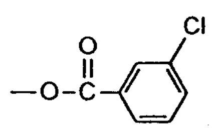 |
| $OCH_3$ | $OCH_3$ | $CH(C_2H_5)_2$ | 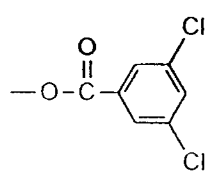 |

Table 1 (continued)

| $R^1$ | $R^2$ | $R^3$ | $R^4$ |
|---|---|---|---|
| $OCH_3$ | $OCH_3$ | $CH(C_2H_5)_2$ | 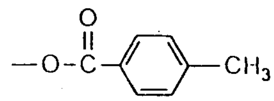 |
| $OCH_3$ | $OCH_3$ | $C(CH_3)_2CH_2CH_3$ | 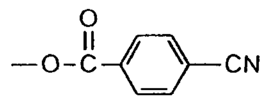 |
| $OCH_3$ | $OCH_3$ | $CH_2C(CH_3)_3$ | 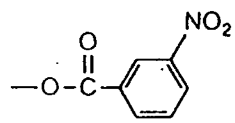 |
| $OCH_3$ | $OCH_3$ | $(CH_2)_2CH(CH_3)_2$ | 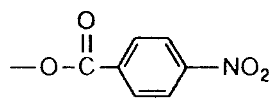 |
| $OCH_3$ | $OCH_3$ | $(CH_2)_5CH_3$ | 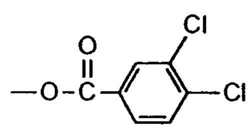 |
| $OCH_3$ | $OCH_3$ | $CH(CH_3)(CH_2)_3CH_3$ | 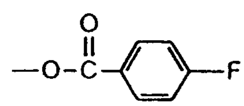 |
| $OCH_3$ | $OCH_3$ | $C(CH_3)_2CH_2CH_2CH_3$ | 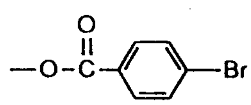 |
| $OCH_3$ | $OCH_3$ | $C(C_2H_5)_2CH_3$ | 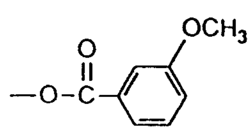 |
| $OCH_3$ | $OCH_3$ | $CH(CH_3)(CH_2)_4CH_3$ | 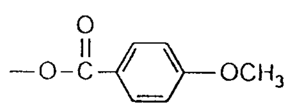 |

Table 1 (continued)

| $R^1$ | $R^2$ | $R^3$ | $R^4$ |
|---|---|---|---|
| $OCH_3$ | $OCH_3$ | $C(CH_3)_2(CH_2)_3CH_3$ | 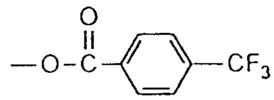 |
| $OCH_3$ | $OCH_3$ | $CH(CH_3)(CH_2)_5CH_3$ | 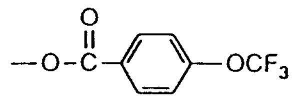 |
| $OCH_3$ | $OCH_3$ | $C(CH_3)_2(CH_2)_4CH_3$ | 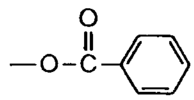 |
| $OCH_3$ | $OCH_3$ | $C(C_2H_5)_3$ | 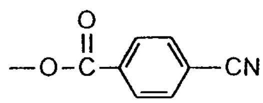 |
| $OCH_3$ | $OCH_3$ | 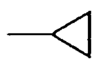 | 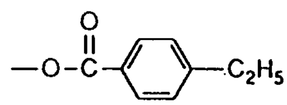 |
| $OCH_3$ | $OCH_3$ | 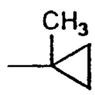 | 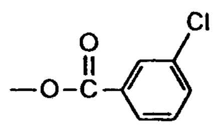 |
| $OCH_3$ | $OCH_3$ | 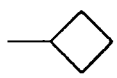 | 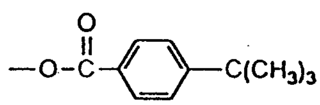 |
| $OCH_3$ | $OCH_3$ | 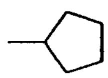 | 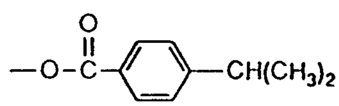 |
| $OCH_3$ | $OCH_3$ | 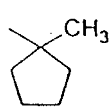 | 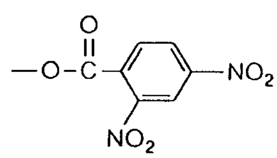 |

Table 1 (continued)

| $R^1$ | $R^2$ | $R^3$ | $R^4$ |
|---|---|---|---|
| $OCH_3$ | $OCH_3$ | 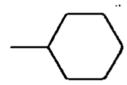 | 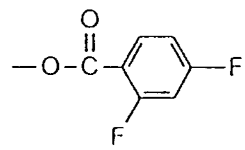 |
| $OCH_3$ | $OCH_3$ | 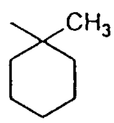 | 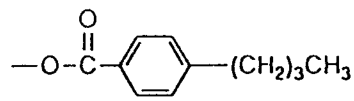 |
| $OCH_3$ | $OCH_3$ | 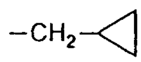 | 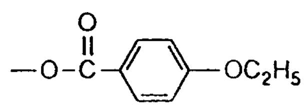 |
| $OCH_3$ | $OCH_3$ | 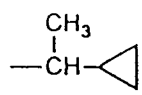 | 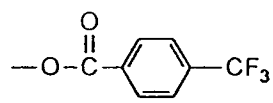 |
| $OCH_3$ | $OCH_3$ | 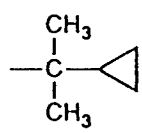 | 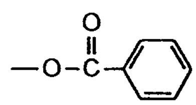 |
| $OCH_3$ | $OCH_3$ | 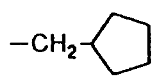 | 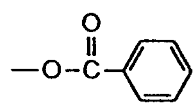 |
| $OCH_3$ | $OCH_3$ | 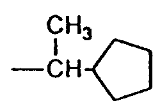 | 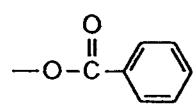 |
| $OCH_3$ | $OCH_3$ | 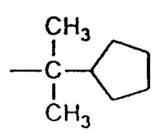 | 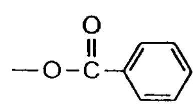 |
| $OCH_3$ | $OCH_3$ | 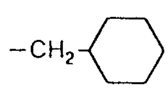 | 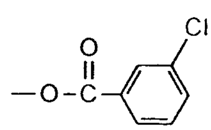 |

Table 1 (continued)

| $R^1$ | $R^2$ | $R^3$ | $R^4$ |
|---|---|---|---|
| $OCH_3$ | $OCH_3$ | 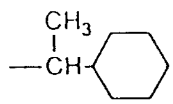 | 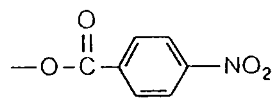 |
| $OCH_3$ | $OCH_3$ | 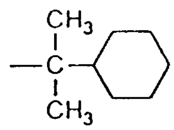 | 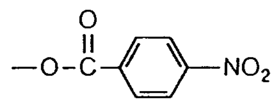 |
| $OCH_3$ | $OCH_3$ | 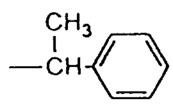 | 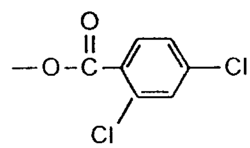 |
| $OCH_3$ | $OCH_3$ | 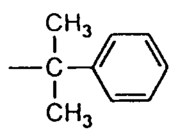 | 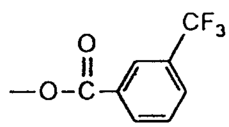 |
| $OCH_3$ | $OCH_3$ | 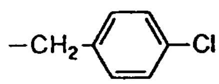 | 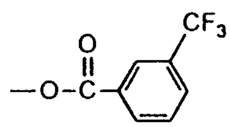 |
| $OCH_3$ | $OCH_3$ | 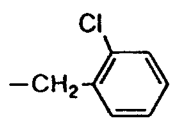 | 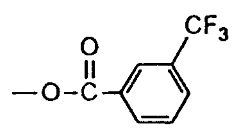 |
| $OCH_3$ | $OCH_3$ | 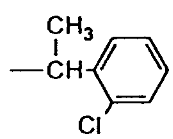 | 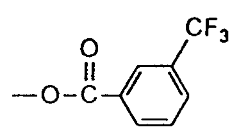 |
| $OCH_3$ | $OCH_3$ | 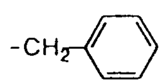 | 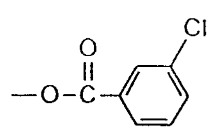 |

Table 1 (continued)

| $R^1$ | $R^2$ | $R^3$ | $R^4$ |
|---|---|---|---|
| $OCH_3$ | $OCH_3$ | 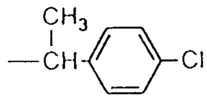 | 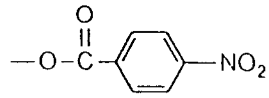 |
| $OCH_3$ | $OCH_3$ | 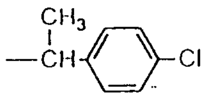 | 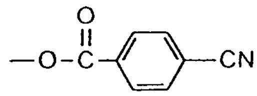 |
| $OCH_3$ | $OCH_3$ | 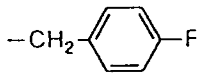 | 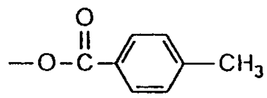 |
| $OCH_3$ | $OCH_3$ | 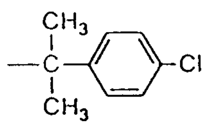 | 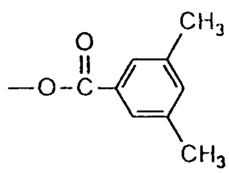 |
| $OCH_3$ | $OCH_3$ | $C_2H_5$ | 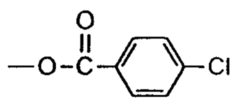 |
| $OCH_3$ | $OCH_3$ | $CH_2CH_2CH_3$ | 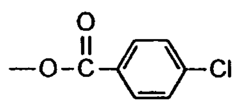 |
| $OCH_3$ | $OCH_3$ | $CH(CH_3)_2$ | 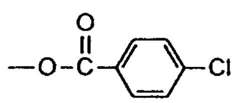 |
| $OCH_3$ | $OCH_3$ | $CH_2CH_2CH_2CH_3$ | 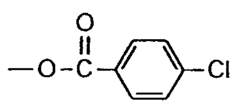 |
| $OCH_3$ | $OCH_3$ | $CH_2CH(CH_3)_2$ | 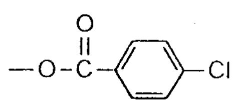 |

Table 1 (continued)

| $R^1$ | $R^2$ | $R^3$ | $R^4$ |
|---|---|---|---|
| $OCH_3$ | $OCH_3$ | $CH(CH_3)CH_2CH_3$ | 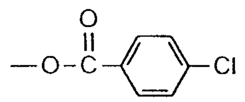 |
| $OCH_3$ | $OCH_3$ | $C(CH_3)_3$ | 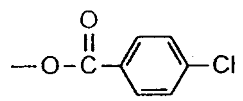 |
| $OCH_3$ | $OCH_3$ | $(CH_2)_4CH_3$ | 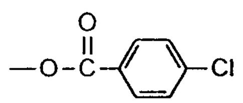 |
| $OCH_3$ | $OCH_3$ | $CH(CH_3)(CH_2)_2CH_3$ | 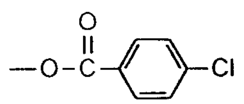 |
| $OCH_3$ | $OCH_3$ | $CH(C_2H_5)_2$ | 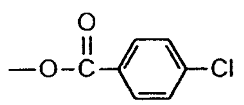 |
| $OCH_3$ | $OCH_3$ | $C(CH_3)_2CH_2CH_3$ | 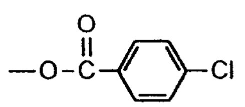 |
| $OCH_3$ | $OCH_3$ | $CH_2C(CH_3)_3$ | 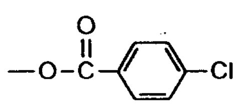 |
| $OCH_3$ | $OCH_3$ | $(CH_2)_2CH(CH_3)_2$ | 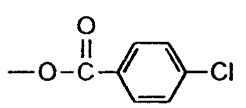 |
| $OCH_3$ | $OCH_3$ | $(CH_2)_5CH_3$ | 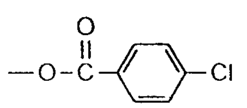 |
| $OCH_3$ | $OCH_3$ | $CH(CH_3)(CH_2)_3CH_3$ | 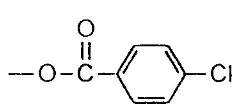 |

Table 1 (continued)

| $R^1$ | $R^2$ | $R^3$ | $R^4$ |
|---|---|---|---|
| $OCH_3$ | $OCH_3$ | $C(CH_3)_2CH_2CH_2CH_3$ | 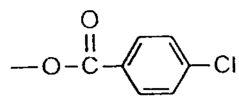 |
| $OCH_3$ | $OCH_3$ | $C(C_2H_5)_2CH_3$ | 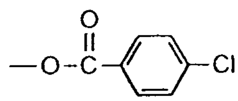 |
| $OCH_3$ | $OCH_3$ | $CH(CH_3)(CH_2)_4CH_3$ | 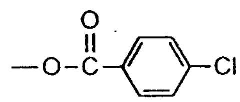 |
| $OCH_3$ | $OCH_3$ | $C(CH_3)_2(CH_2)_3CH_3$ | 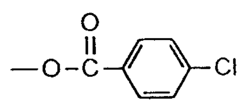 |
| $OCH_3$ | $OCH_3$ | $CH(CH_3)(CH_2)_5CH_3$ | 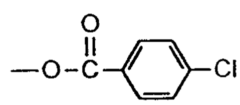 |
| $OCH_3$ | $OCH_3$ | $C(CH_3)_2(CH_2)_4CH_3$ | 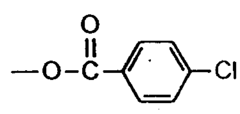 |
| $OCH_3$ | $OCH_3$ | $C(C_2H_5)_3$ | 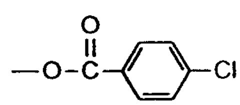 |
| $OCH_3$ | $OCH_3$ | 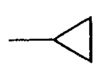 | 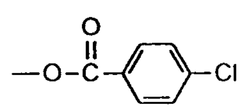 |
| $OCH_3$ | $OCH_3$ | 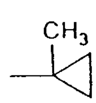 | 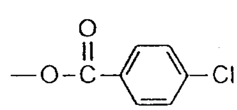 |

Table 1 (continued)

| $R^1$ | $R^2$ | $R^3$ | $R^4$ |
|---|---|---|---|
| $OCH_3$ | $OCH_3$ | 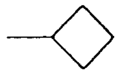 | 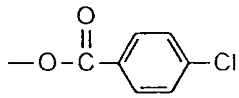 |
| $OCH_3$ | $OCH_3$ | 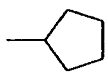 | 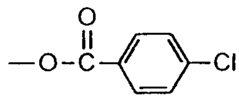 |
| $OCH_3$ | $OCH_3$ | 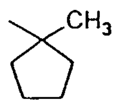 | 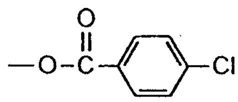 |
| $OCH_3$ | $OCH_3$ | 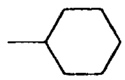 | 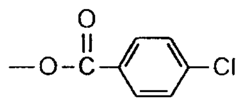 |
| $OCH_3$ | $OCH_3$ | 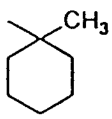 | 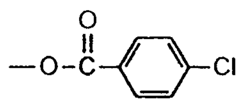 |
| $OCH_3$ | $OCH_3$ | 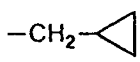 | 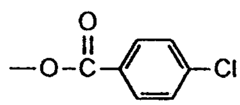 |
| $OCH_3$ | $OCH_3$ | 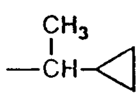 | 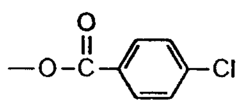 |
| $OCH_3$ | $OCH_3$ | 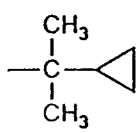 | 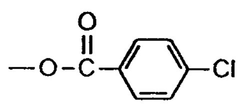 |
| $OCH_3$ | $OCH_3$ | 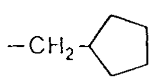 | 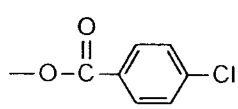 |

Table 1 (continued)

| $R^1$ | $R^2$ | $R^3$ | $R^4$ |
|---|---|---|---|
| $OCH_3$ | $OCH_3$ | 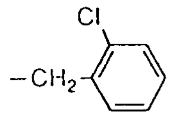 | 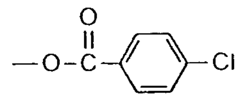 |
| $OCH_3$ | $OCH_3$ | 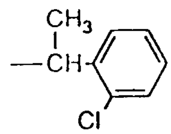 | 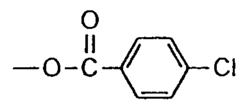 |
| $OCH_3$ | $OCH_3$ | 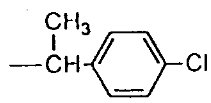 | 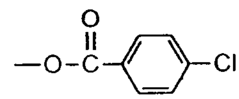 |
| $OCH_3$ | $OCH_3$ | 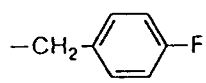 | 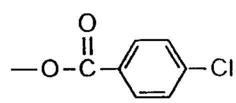 |
| $OCH_3$ | $OCH_3$ | 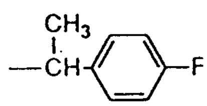 | 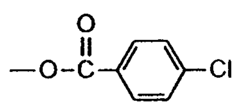 |
| $OCH_3$ | $OCH_3$ | 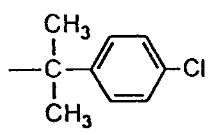 | 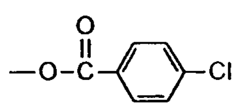 |
| $OCH_3$ | $OCH_3$ | $C_2H_5$ | 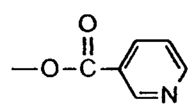 |
| $OCH_3$ | $OCH_3$ | $CH_2CH_2CH_3$ | 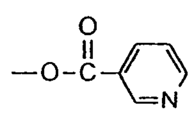 |
| $OCH_3$ | $OCH_3$ | $CH(CH_3)_2$ | 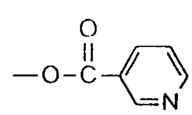 |

Table 1 (continued)

| $R^1$ | $R^2$ | $R^3$ | $R^4$ |
|---|---|---|---|
| $OCH_3$ | $OCH_3$ | $CH_2CH_2CH_2CH_3$ | 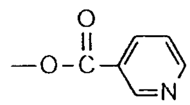 |
| $OCH_3$ | $OCH_3$ | $CH_2CH(CH_3)_2$ | 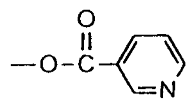 |
| $OCH_3$ | $OCH_3$ | $CH(CH_3)CH_2CH_3$ | 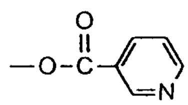 |
| $OCH_3$ | $OCH_3$ | $C(CH_3)_3$ | 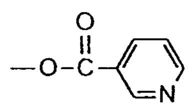 |
| $OCH_3$ | $OCH_3$ | $(CH_2)_4CH_3$ | 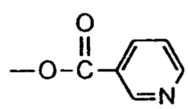 |
| $OCH_3$ | $OCH_3$ | $CH(CH_3)(CH_2)_2CH_3$ | 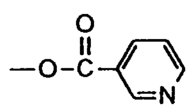 |
| $OCH_3$ | $OCH_3$ | $CH(C_2H_5)_2$ | 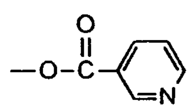 |
| $OCH_3$ | $OCH_3$ | $C(CH_3)_2CH_2CH_3$ | 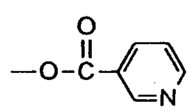 |
| $OCH_3$ | $OCH_3$ | $C(CH_3)_3$ | 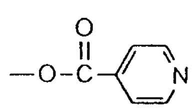 |

Table 1 (continued)

| $R^1$ | $R^2$ | $R^3$ | $R^4$ |
|---|---|---|---|
| $OCH_3$ | $OCH_3$ | $CH(CH_3)_2$ | 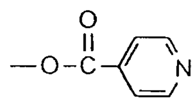 |
| $OCH_3$ | $OCH_3$ | $C(CH_3)_3$ | 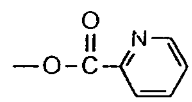 |
| $OCH_3$ | $OCH_3$ | $CH(CH_3)_2$ | 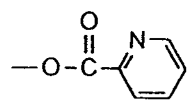 |
| $OCH_3$ | $OCH_3$ | $C(CH_3)_3$ | 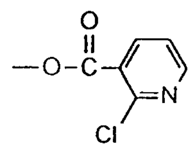 |
| $OCH_3$ | $OCH_3$ | $CH(CH_3)_2$ | 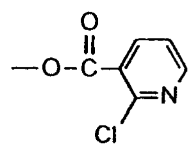 |
| $OCH_3$ | $OCH_3$ | $C(CH_3)_3$ | 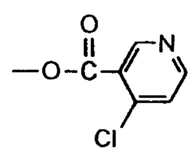 |
| $OCH_3$ | $OCH_3$ | $CH(CH_3)_2$ | 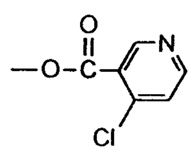 |
| $OCH_3$ | $OCH_3$ | $C(CH_3)_3$ | 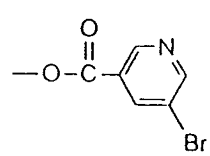 |

Table 1 (continued)

| $R^1$ | $R^2$ | $R^3$ | $R^4$ |
|---|---|---|---|
| $OCH_3$ | $OCH_3$ | $CH(CH_3)_2$ | 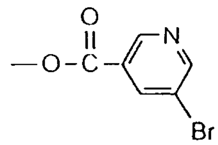 |
| $OCH_3$ | $OCH_3$ | 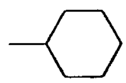 | 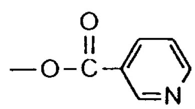 |
| $OCH_3$ | $OCH_3$ | 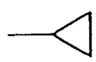 | 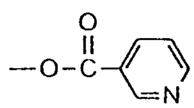 |
| $OCH_3$ | $OCH_3$ | $(CH_2)_5CH_3$ | 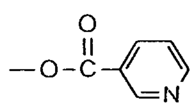 |
| $OCH_3$ | $OCH_3$ | $CH(CH_3)(CH_2)_3CH_3$ | 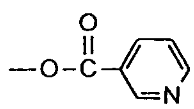 |
| $OCH_3$ | $OCH_3$ | 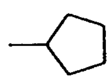 | 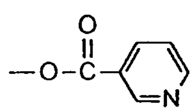 |
| $OCH_3$ | $OCH_3$ | $CH(CH_3)_2$ | 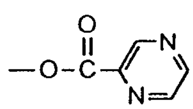 |
| $OCH_3$ | $OCH_3$ | C(CH3)3 | 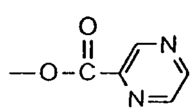 |
| $OCH_3$ | $OCH_3$ | C(CH3)3 | 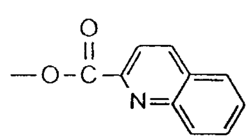 |

Table 1 (continued)

| $R^1$ | $R^2$ | $R^3$ | $R^4$ |
|---|---|---|---|
| $OCH_3$ | $OCH_3$ | $CH(CH_3)_2$ | 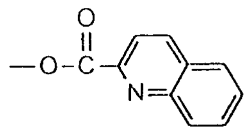 |
| $OCH_3$ | $OCH_3$ | 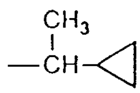 | 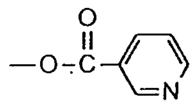 |
| $OCH_3$ | $OCH_3$ | C(CH3)3 | 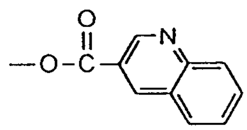 |
| $OCH_3$ | $OCH_3$ | $CH(CH_3)_2$ | 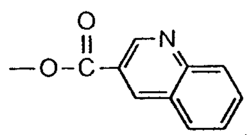 |
| $OCH_3$ | $OCH_3$ | C(CH3)3 | 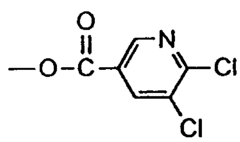 |
| $OCH_3$ | $OCH_3$ | 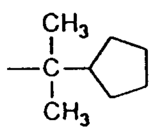 | 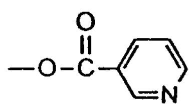 |
| $OCH_3$ | $OCH_3$ | $CH(CH_3)_2$ | 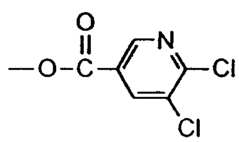 |
| $OCH_3$ | $OCH_3$ | C(CH3)3 | 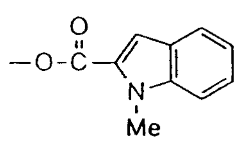 |

Table 1 (continued)

| $R^1$ | $R^2$ | $R^3$ | $R^4$ |
| --- | --- | --- | --- |
| $OCH_3$ | $OCH_3$ | 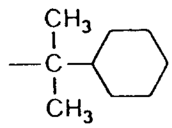 | 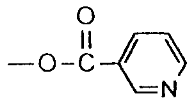 |
| $OCH_3$ | $OCH_3$ | $CH(CH_3)_2$ | 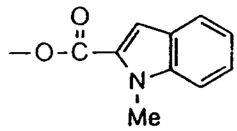 |
| $OCH_3$ | $OCH_3$ | 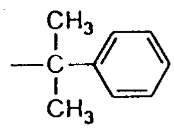 | 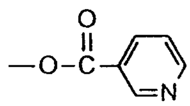 |
| $OCH_3$ | $OCH_3$ | C(CH3)3 | 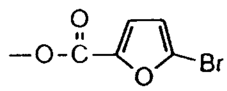 |
| $OCH_3$ | $OCH_3$ | 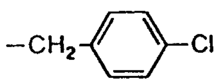 | 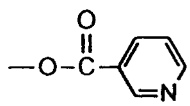 |
| $OCH_3$ | $OCH_3$ | 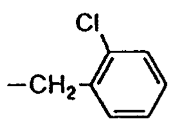 | 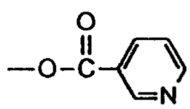 |
| $OCH_3$ | $OCH_3$ | $CH(CH_3)_2$ | 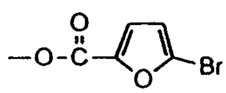 |
| $OCH_3$ | $OCH_3$ | C(CH3)3 | 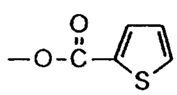 |
| $OCH_3$ | $OCH_3$ | 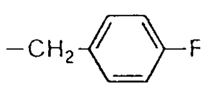 | 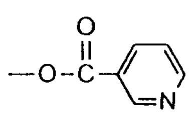 |

Table 1 (continued)

| $R^1$ | $R^2$ | $R^3$ | $R^4$ |
|---|---|---|---|
| $OCH_3$ | $OCH_3$ | $CH(CH_3)_2$ | 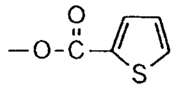 |
| $OCH_3$ | $OCH_3$ | $CH(CH_3)_2$ | 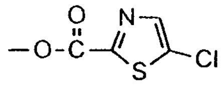 |
| $OCH_3$ | $OCH_3$ | $C_2H_5$ | 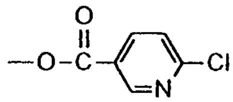 |
| $OCH_3$ | $OCH_3$ | $CH_2CH_2CH_3$ | 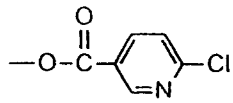 |
| $OCH_3$ | $OCH_3$ | $CH(CH_3)_2$ | 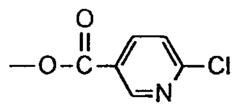 |
| $OCH_3$ | $OCH_3$ | $CH_2CH_2CH_2CH_3$ | 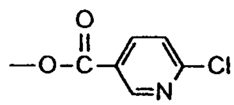 |
| $OCH_3$ | $OCH_3$ | $CH_2CH(CH_3)_2$ | 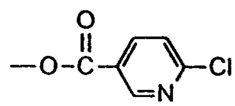 |
| $OCH_3$ | $OCH_3$ | $CH(CH_3)CH_2CH_3$ | 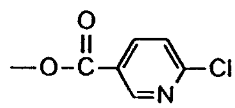 |
| $OCH_3$ | $OCH_3$ | $C(CH_3)_3$ | 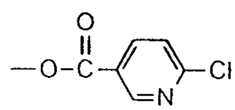 |
| $OCH_3$ | $OCH_3$ | $(CH_2)_4CH_3$ | 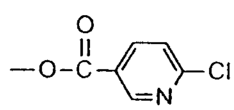 |

Table 1 (continued)

| $R^1$ | $R^2$ | $R^3$ | $R^4$ |
|---|---|---|---|
| $OCH_3$ | $OCH_3$ | C(CH3)3 | 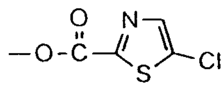 |
| $OCH_3$ | $OCH_3$ | $CH(C_2H_5)_2$ | 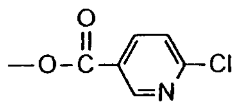 |
| $OCH_3$ | $OCH_3$ | $C(CH_3)_2CH_2CH_3$ | 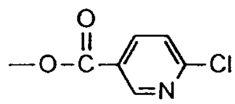 |
| $OCH_3$ | $OCH_3$ | C(CH3)3 | 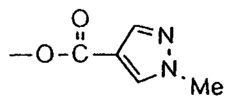 |
| $OCH_3$ | $OCH_3$ | CH(CH3)2 | 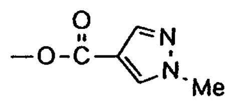 |
| $OCH_3$ | $OCH_3$ | $C(CH_3)_2CH_2CH_2CH_3$ | 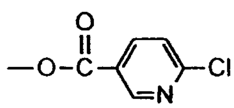 |
| $OCH_3$ | $OCH_3$ | $C(C_2H_5)_2CH_3$ | 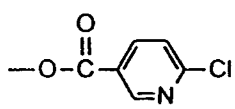 |
| $OCH_3$ | $OCH_3$ | $C(CH_3)_2(CH_2)_3CH_3$ | 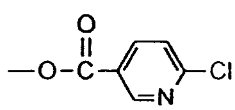 |
| $OCH_3$ | $OCH_3$ | 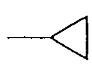 | 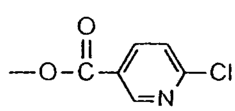 |

Table 1 (continued)

| $R^1$ | $R^2$ | $R^3$ | $R^4$ |
|---|---|---|---|
| $OCH_3$ | $OCH_3$ | 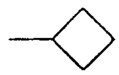 | 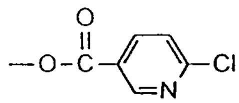 |
| $OCH_3$ | $OCH_3$ | 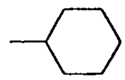 | 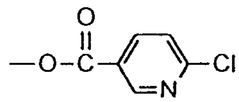 |
| $OCH_3$ | $OCH_3$ | 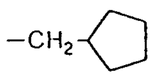 | 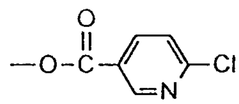 |
| $OCH_3$ | $OCH_3$ | 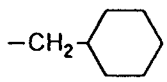 | 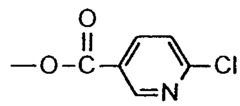 |
| $OCH_3$ | $OCH_3$ | 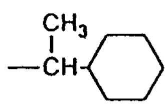 | 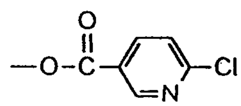 |
| $OCH_3$ | $OCH_3$ | 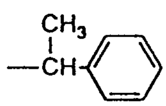 | 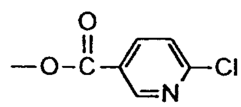 |
| $OCH_3$ | $OCH_3$ | 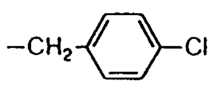 | 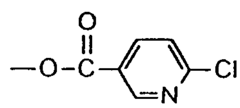 |
| $OCH_3$ | $OCH_3$ | 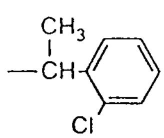 | 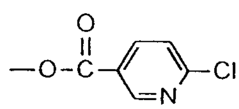 |

Table 1 (continued)

| $R^1$ | $R^2$ | $R^3$ | $R^4$ |
|---|---|---|---|
| $CCH_3$ | $OCH_3$ | 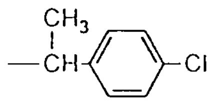 | 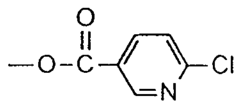 |
| $CCH_3$ | $OCH_3$ | 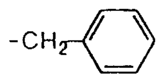 | 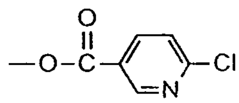 |
| $CCH_3$ | $OCH_3$ | 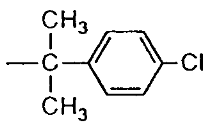 | 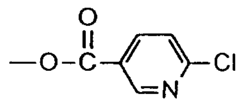 |
| $OCH_3$ | $OCH_3$ | $C_2H_5$ | 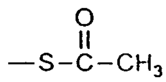 |
| $OCH_3$ | $OCH_3$ | $CH_2CH_2CH_3$ | 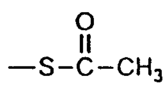 |
| $OCH_3$ | $OCH_3$ | $CH(CH_3)_2$ | 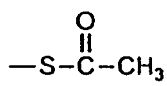 |
| $OCH_3$ | $OCH_3$ | $CH_2CH_2CH_2CH_3$ | 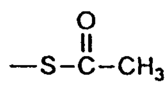 |
| $OCH_3$ | $OCH_3$ | $CH_2CH(CH_3)_2$ | 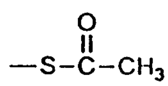 |
| $OCH_3$ | $OCH_3$ | $CH(CH_3)CH_2CH_3$ | 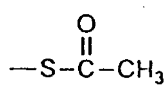 |
| $OCH_3$ | $OCH_3$ | $C(CH_3)_3$ | 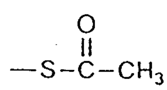 |

Table 1 (continued)

| $R^1$ | $R^2$ | $R^3$ | $R^4$ |
|---|---|---|---|
| $OCH_3$ | $OCH_3$ | $(CH_2)_4CH_3$ | 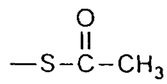 |
| $OCH_3$ | $OCH_3$ | $CH(CH_3)(CH_2)_2CH_3$ | 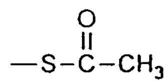 |
| $OCH_3$ | $OCH_3$ | $CH(C_2H_5)_2$ | 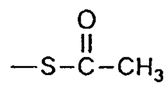 |
| $OCH_3$ | $OCH_3$ | $C(CH_3)_2CH_2CH_3$ | 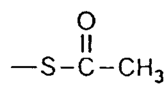 |
| $OCH_3$ | $OCH_3$ | $(CH_2)_2CH(CH_3)_2$ | 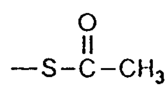 |
| $OCH_3$ | $OCH_3$ | $CH(CH_3)(CH_2)_3CH_3$ | 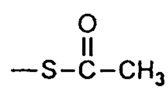 |
| $OCH_3$ | $OCH_3$ | $C(CH_3)_2CH_2CH_2CH_3$ | 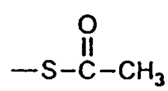 |
| $OCH_3$ | $OCH_3$ | $C(C_2H_5)_3$ | 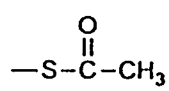 |
| $OCH_3$ | $OCH_3$ | 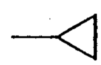 | 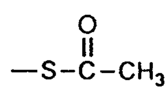 |
| $OCH_3$ | $OCH_3$ | 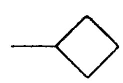 | 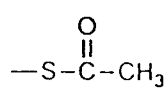 |
| $OCH_3$ | $OCH_3$ | 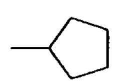 | 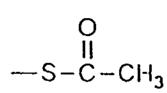 |

Table 1 (continued)

| $R^1$ | $R^2$ | $R^3$ | $R^4$ |
|---|---|---|---|
| $OCH_3$ | $OCH_3$ | 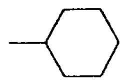 | 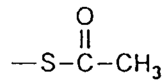 |
| $OCH_3$ | $OCH_3$ | 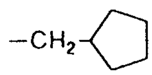 | 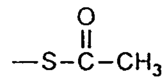 |
| $OCH_3$ | $OCH_3$ | 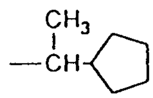 | 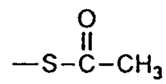 |
| $OCH_3$ | $OCH_3$ | 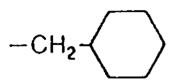 | 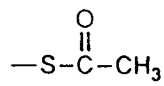 |
| $OCH_3$ | $OCH_3$ | 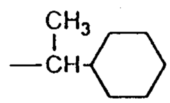 | 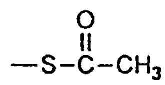 |
| $OCH_3$ | $OCH_3$ | 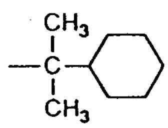 | 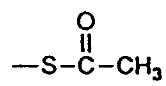 |
| $OCH_3$ | $OCH_3$ | 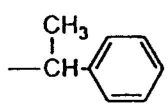 | 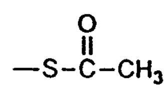 |
| $OCH_3$ | $OCH_3$ | 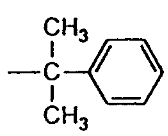 | 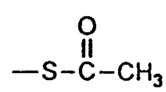 |
| $OCH_3$ | $OCH_3$ | 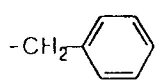 | 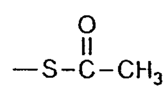 |
| $OCH_3$ | $OCH_3$ | $C_2H_5$ | 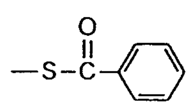 |

Table 1 (continued)

| $R^1$ | $R^2$ | $R^3$ | $R^4$ |
|---|---|---|---|
| $OCH_3$ | $OCH_3$ | $CH_2CH_2CH_3$ | 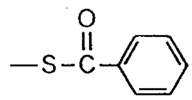 |
| $OCH_3$ | $OCH_3$ | $CH(CH_3)_2$ | 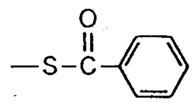 |
| $OCH_3$ | $OCH_3$ | $CH_2CH_2CH_2CH_3$ | 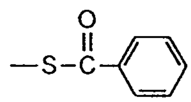 |
| $OCH_3$ | $OCH_3$ | $CH_2CH(CH_3)_2$ | 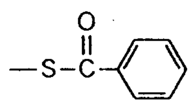 |
| $OCH_3$ | $OCH_3$ | $CH(CH_3)CH_2CH_3$ | 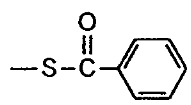 |
| $OCH_3$ | $OCH_3$ | $C(CH_3)_3$ | 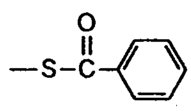 |
| $OCH_3$ | $OCH_3$ | $(CH_2)_4CH_3$ | 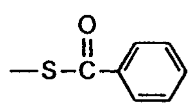 |
| $OCH_3$ | $OCH_3$ | $CH(CH_3)(CH_2)_2CH_3$ | 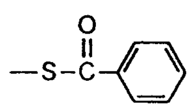 |
| $OCH_3$ | $OCH_3$ | $CH(C_2H_5)_2$ | 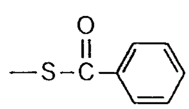 |

Table 1 (continued)

| $R^1$ | $R^2$ | $R^3$ | $R^4$ |
|---|---|---|---|
| $OCH_3$ | $OCH_3$ | $C(CH_3)_2CH_2CH_3$ | 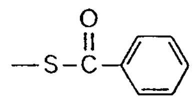 |
| $OCH_3$ | $OCH_3$ | $(CH_2)_2CH(CH_3)_2$ | 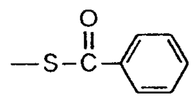 |
| $OCH_3$ | $OCH_3$ | $(CH_2)_5CH_3$ | 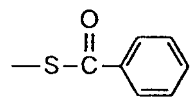 |
| $OCH_3$ | $OCH_3$ | 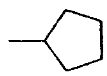 | 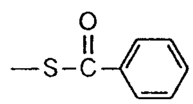 |
| $OCH_3$ | $OCH_3$ | 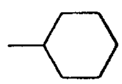 | 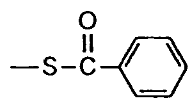 |
| $OCH_3$ | $OCH_3$ | 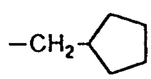 | 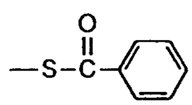 |
| $OCH_3$ | $OCH_3$ | 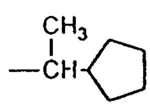 | 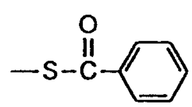 |
| $OCH_3$ | $OCH_3$ | 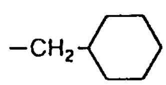 | 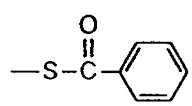 |
| $OCH_3$ | $OCH_3$ | $CH_2CH(CH_3)_2$ | 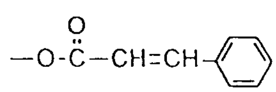 |
| $OCH_3$ | $OCH_3$ | $CH(CH_3)CH_2CH_2CH_3$ | 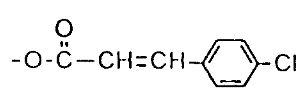 |

Table 1 (continued)

| $R^1$ | $R^2$ | $R^3$ | $R^4$ |
|---|---|---|---|
| $OCH_3$ | $OCH_3$ | $CH_2CH(CH_3)_2$ | $-O-\overset{O}{\overset{\|}{C}}-(CH_2)_4CH_3$ |
| $OCH_3$ | $OCH_3$ | $CH(CH_3)CH_2CH_2CH_3$ | $-O-\overset{O}{\overset{\|}{C}}-(CH_2)_5CH_3$ |
| $OCH_3$ | $OCH_3$ | $CH_2CH(CH_3)_2$ | $-O-\overset{O}{\overset{\|}{C}}-(CH_2)_6CH_3$ |
| $OCH_3$ | $OCH_3$ | $CH(CH_3)CH_2CH_2CH_3$ | $-O-\overset{O}{\overset{\|}{C}}-(CH_2)_7CH_3$ |
| $OCH_3$ | $OCH_3$ | $CH_2CH(CH_3)_2$ | $-O-\overset{O}{\overset{\|}{C}}-(CH_2)_8CH_3$ |
| $OCH_3$ | $OCH_3$ | $CH(CH_3)CH_2CH_2CH_3$ | $-O-\overset{O}{\overset{\|}{C}}-(CH_2)_9CH_3$ |
| $OCH_3$ | $OCH_3$ | $CH_2CH(CH_3)_2$ | $-O-\overset{O}{\overset{\|}{C}}-(CH_2)_{10}CH_3$ |
| $OCH_3$ | $OCH_3$ | $CH(CH_3)CH_2CH_2CH_3$ | $-O-\overset{O}{\overset{\|}{C}}-(CH_2)_{11}CH_3$ |
| $OCH_3$ | $OCH_3$ | $CH_2CH(CH_3)_2$ | $-O-\overset{O}{\overset{\|}{C}}-(CH_2)_{12}CH_3$ |
| $OCH_3$ | $OCH_3$ | $CH(CH_3)CH_2CH_2CH_3$ | $-O-\overset{O}{\overset{\|}{C}}-(CH_2)_{13}CH_3$ |
| $OCH_3$ | $OCH_3$ | $CH_2CH(CH_3)_2$ | $-O-\overset{O}{\overset{\|}{C}}-(CH_2)_{14}CH_3$ |
| $OCH_3$ | $OCH_3$ | $CH(CH_3)CH_2CH_2CH_3$ | $-O-\overset{O}{\overset{\|}{C}}-(CH_2)_{15}CH_3$ |

Table 1 (continued)

| $R^1$ | $R^2$ | $R^3$ | $R^4$ |
|---|---|---|---|
| $OCH_3$ | $OCH_3$ | $CH_2CH(CH_3)_2$ | $-O-\overset{O}{\overset{\|}{C}}-(CH_2)_{16}CH_3$ |
| $OCH_3$ | $OCH_3$ | $CH(CH_3)CH_2CH_2CH_3$ | $-O-\overset{O}{\overset{\|}{C}}-(CH_2)_{17}CH_3$ |
| $OCH_3$ | $OCH_3$ | $CH_2CH(CH_3)_2$ | $-O-\overset{O}{\overset{\|}{C}}-(CH_2)_{19}CH_3$ |
| $OCH_3$ | $OCH_3$ | $CH(CH_3)CH_2CH_2CH_3$ | $-O-\overset{O}{\overset{\|}{C}}-(CH_2)_9CH=CH(CH_2)_7CH_3$ |
| $OCH_3$ | $OCH_3$ | $CH_2CH(CH_3)_2$ | $-O-\overset{O}{\overset{\|}{C}}-(CH_2)_7CH=CH(CH_2)_7CH_3$ |
| $OCH_3$ | $OCH_3$ | $CH(CH_3)CH_2CH_2CH_3$ | $-O-\overset{O}{\overset{\|}{C}}-(CH_2)_7CH=CH(CH_2)_5CH_3$ |
| $OCH_3$ | $OCH_3$ | $CH_2CH(CH_3)_2$ | $-O-\overset{O}{\overset{\|}{C}}-(CH_2)_7CH=CH(CH_2)_3CH_3$ |
| $OCH_3$ | $OCH_3$ | $CH(CH_3)CH_2CH_2CH_3$ | $-O-\overset{O}{\overset{\|}{C}}-(CH_2)_8CH=CH_2$ |
| $OCH_3$ | $OCH_3$ | $CH_2CH(CH_3)_2$ | $-O-\overset{O}{\overset{\|}{C}}-CH_2\overset{CH_3}{\overset{\|}{C}}H(CH_2)_2CH=C(CH_3)_2$ |
| $OCH_3$ | $OCH_3$ | $CH(CH_3)CH_2CH_2CH_3$ | 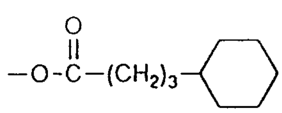 |

Table 1 (continued)

| $R^1$ | $R^2$ | $R^3$ | $R^4$ |
|---|---|---|---|
| $OCH_3$ | $OCH_3$ | $CH_2CH(CH_3)_2$ | 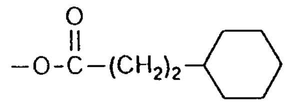 |
| $OCH_3$ | $OCH_3$ | $CH(CH_3)CH_2CH_2CH_3$ | 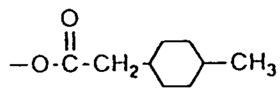 |
| $OCH_3$ | $OCH_3$ | $CH_2CH(CH_3)_2$ | 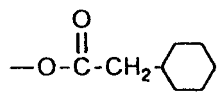 |
| $OCH_3$ | $OCH_3$ | $CH(CH_3)CH_2CH_2CH_3$ | 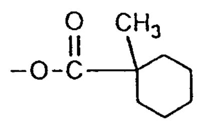 |
| $OCH_3$ | $OCH_3$ | $CH_2CH(CH_3)_2$ | 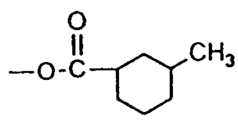 |
| $OCH_3$ | $OCH_3$ | $CH(CH_3)CH_2CH_2CH_3$ | 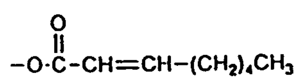 |
| $OCH_3$ | $OCH_3$ | $CH_2CH(CH_3)_2$ | 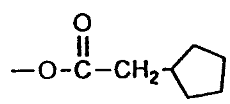 |
| $OCH_3$ | $OCH_3$ | $CH(CH_3)CH_2CH_2CH_3$ | 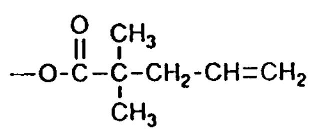 |
| $OCH_3$ | $OCH_3$ | $CH_2CH(CH_3)_2$ | 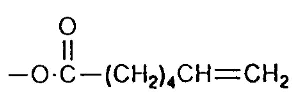 |

Table 1 (continued)

| $R^1$ | $R^2$ | $R^3$ | $R^4$ |
|---|---|---|---|
| $OCH_3$ | $OCH_3$ | $CH(CH_3)CH_2CH_2CH_3$ | 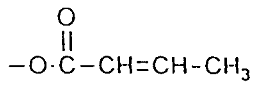 |
| $OCH_3$ | $OCH_3$ | $CH_2CH(CH_3)_2$ | 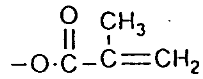 |
| $OCH_3$ | $OCH_3$ | $CH(CH_3)CH_2CH_2CH_3$ | 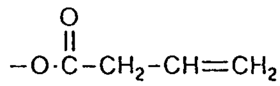 |
| $OCH_3$ | $OCH_3$ | $CH_2CH(CH_3)_2$ | 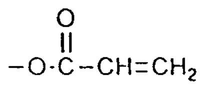 |
| $OCH_3$ | $OCH_3$ | $CH(CH_3)CH_2CH_2CH_3$ | 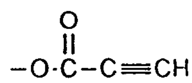 |
| $OCH_3$ | $OCH_3$ | $CH_2CH(CH_3)_2$ | 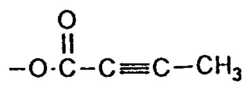 |
| $OCH_3$ | $OCH_3$ | $CH(CH_3)CH_2CH_2CH_3$ | 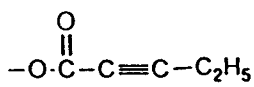 |
| $OCH_3$ | $OCH_3$ | $CH_2CH(CH_3)_2$ | 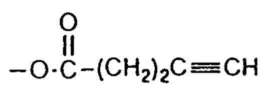 |
| $OCH_3$ | $OCH_3$ | $CH(CH_3)CH_2CH_2CH_3$ | 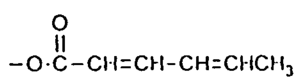 |
| $OCH_3$ | $OCH_3$ | $CH_2CH(CH_3)_2$ | 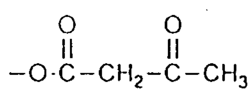 |

Table 1 (continued)

| $R^1$ | $R^2$ | $R^3$ | $R^4$ |
|---|---|---|---|
| $OCH_3$ | $OCH_3$ | $CH(CH_3)CH_2CH_2CH_3$ | 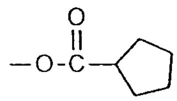 |
| $OCH_3$ | $OCH_3$ | $CH_2CH(CH_3)_2$ | 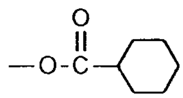 |
| $OCH_3$ | $OCH_3$ | $CH(CH_3)CH_2CH_2CH_3$ | 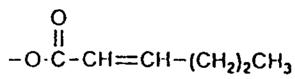 |
| $OCH_3$ | $OCH_3$ | $CH_2CH(CH_3)_2$ | 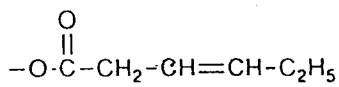 |
| $OCH_3$ | $OCH_3$ | $CH(CH_3)CH_2CH_2CH_3$ | 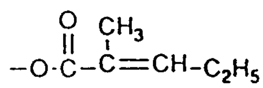 |
| $OCH_3$ | $OCH_3$ | $CH_2CH(CH_3)_2$ | 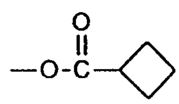 |
| $OCH_3$ | $OCH_3$ | $CH(CH_3)CH_2CH_2CH_3$ | 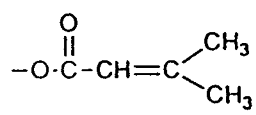 |
| $OCH_3$ | $OCH_3$ | $CH_2CH(CH_3)_2$ | 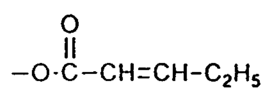 |
| $OCH_3$ | $OCH_3$ | $CH(CH_3)CH_2CH_2CH_3$ | 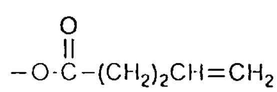 |
| $OCH_3$ | $OCH_3$ | $CH_2CH(CH_3)_2$ | 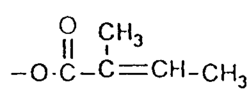 |

Table 1 (continued)

| $R^1$ | $R^2$ | $R^3$ | $R^4$ |
|---|---|---|---|
| $OCH_3$ | $OCH_3$ | $CH(CH_3)CH_2CH_2CH_3$ | 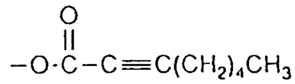 |
| $OCH_3$ | $OCH_3$ | $CH_2CH(CH_3)_2$ | 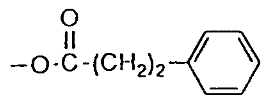 |
| $OCH_3$ | $OCH_3$ | $CH(CH_3)CH_2CH_2CH_3$ | 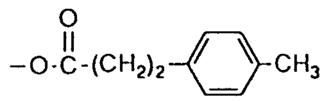 |
| $OCH_3$ | $OCH_3$ | $CH_2CH(CH_3)_2$ | 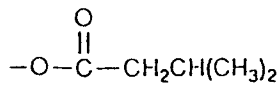 |
| $OCH_3$ | $OCH_3$ | $CH(CH_3)CH_2CH_2CH_3$ | 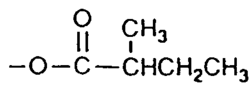 |
| $OCH_3$ | $OCH_3$ | $CH_2CH(CH_3)_2$ | 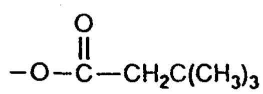 |
| $OCH_3$ | $OCH_3$ | $CH(CH_3)CH_2CH_2CH_3$ | 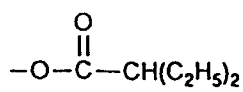 |
| $OCH_3$ | $OCH_3$ | $CH_2CH(CH_3)_2$ | 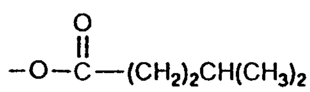 |
| $OCH_3$ | $OCH_3$ | $CH(CH_3)CH_2CH_2CH_3$ | 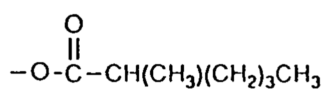 |
| $OCH_3$ | $OCH_3$ | $CH_2CH(CH_3)_2$ | 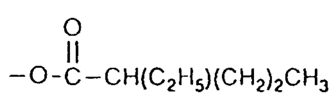 |

Table 1 (continued)

| $R^1$ | $R^2$ | $R^3$ | $R^4$ |
|---|---|---|---|
| $OCH_3$ | $OCH_3$ | $CH(CH_3)CH_2CH_2CH_3$ | 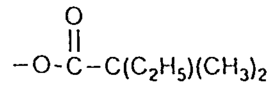 |
| $OCH_3$ | $OCH_3$ | $CH_2CH(CH_3)_2$ | 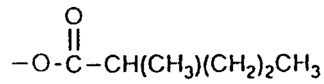 |
| $OCH_3$ | $OCH_3$ | $CH(CH_3)CH_2CH_2CH_3$ | 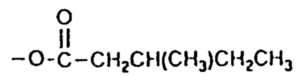 |
| $OCH_3$ | $OCH_3$ | $CH_2CH(CH_3)_2$ | 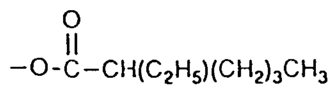 |
| $OCH_3$ | $OCH_3$ | $CH(CH_3)CH_2CH_2CH_3$ | 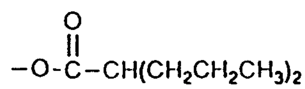 |
| $OCH_3$ | $OCH_3$ | $CH(CH_3)_2$ | 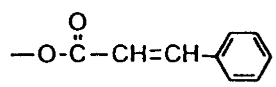 |
| $OCH_3$ | $OCH_3$ | $CH(CH_3)CH_2CH_3$ | 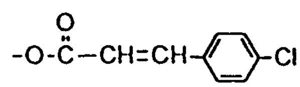 |
| $OCH_3$ | $OCH_3$ | $CH(CH_3)_2$ | 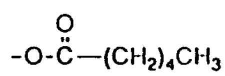 |
| $OCH_3$ | $OCH_3$ | $CH(CH_3)CH_2CH_3$ | 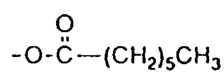 |
| $OCH_3$ | $OCH_3$ | $CH(CH_3)_2$ | 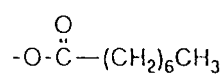 |
| $OCH_3$ | $OCH_3$ | $CH(CH_3)CH_2CH_3$ | 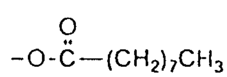 |

Table 1 (continued)

| $R^1$ | $R^2$ | $R^3$ | $R^4$ |
|---|---|---|---|
| $OCH_3$ | $OCH_3$ | $CH(CH_3)_2$ | $-O-\overset{O}{\overset{\|}{C}}-(CH_2)_8CH_3$ |
| $OCH_3$ | $OCH_3$ | $CH(CH_3)CH_2CH_3$ | $-O-\overset{O}{\overset{\|}{C}}-(CH_2)_9CH_3$ |
| $OCH_3$ | $OCH_3$ | $CH(CH_3)_2$ | $-O-\overset{O}{\overset{\|}{C}}-(CH_2)_{10}CH_3$ |
| $OCH_3$ | $OCH_3$ | $CH(CH_3)CH_2CH_3$ | $-O-\overset{O}{\overset{\|}{C}}-(CH_2)_{11}CH_3$ |
| $OCH_3$ | $OCH_3$ | $CH(CH_3)_2$ | $-O-\overset{O}{\overset{\|}{C}}-(CH_2)_{12}CH_3$ |
| $OCH_3$ | $OCH_3$ | $CH(CH_3)CH_2CH_3$ | $-O-\overset{O}{\overset{\|}{C}}-(CH_2)_{13}CH_3$ |
| $OCH_3$ | $OCH_3$ | $CH(CH_3)_2$ | $-O-\overset{O}{\overset{\|}{C}}-(CH_2)_{14}CH_3$ |
| $OCH_3$ | $OCH_3$ | $CH(CH_3)CH_2CH_3$ | $-O-\overset{O}{\overset{\|}{C}}-(CH_2)_{15}CH_3$ |
| $OCH_3$ | $OCH_3$ | $CH(CH_3)_2$ | $-O-\overset{O}{\overset{\|}{C}}-(CH_2)_{16}CH_3$ |
| $OCH_3$ | $OCH_3$ | $CH(CH_3)CH_2CH_3$ | $-O-\overset{O}{\overset{\|}{C}}-(CH_2)_{17}CH_3$ |
| $OCH_3$ | $OCH_3$ | $CH(CH_3)CH_2CH_3$ | $-O-\overset{O}{\overset{\|}{C}}-(CH_2)_{19}CH_3$ |
| $OCH_3$ | $OCH_3$ | $CH(CH_3)_2$ | $-O-\overset{O}{\overset{\|}{C}}-(CH_2)_9CH=CH(CH_2)_7CH_3$ |

Table 1 (continued)

| R1 | R2 | R3 | R4 |
|---|---|---|---|
| $OCH_3$ | $OCH_3$ | $C(CH_3)_3$ | 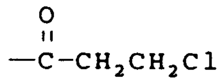 |
| $OCH_3$ | $OCH_3$ | $C(CH_3)_3$ | 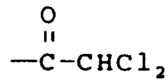 |
| $OCH_3$ | $OCH_3$ | $C(CH_3)_3$ | 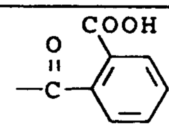 |
| $OCH_3$ | $OCH_3$ | $C(CH_3)_3$ | 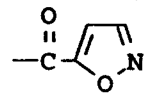 |
| $OCH_3$ | $OCH_3$ | $C(CH_3)_3$ | 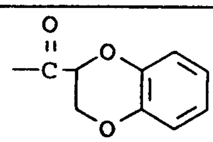 |
| $OCH_3$ | $OCH_3$ | $C(CH_3)_3$ | 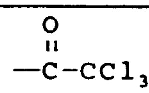 |
| $OCH_3$ | $OCH_3$ | $C(CH_3)_3$ | 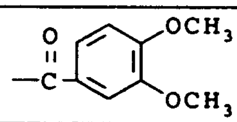 |
| $OCH_3$ | $OCH_3$ | $C(CH_3)_3$ | 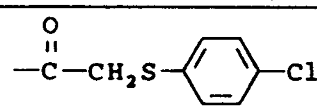 |
| $OCH_3$ | $OCH_3$ | $C(CH_3)_3$ | 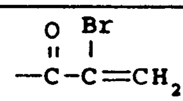 |
| $OCH_3$ | $OCH_3$ | $C(CH_3)_3$ | 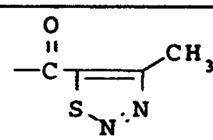 |
| $OCH_3$ | $OCH_3$ | $C(CH_3)_3$ | 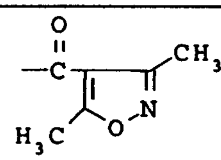 |
| $OCH_3$ | $OCH_3$ | $C(CH_3)_3$ | 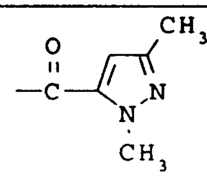 |

Table 1 (continued)

| R1 | R2 | R3 | R4 |
|---|---|---|---|
| $OCH_3$ | $OCH_3$ | $C(CH_3)_3$ | 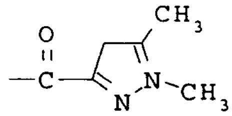 |
| $OCH_3$ | $OCH_3$ | $C(CH_3)_3$ | 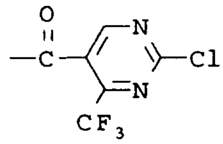 |
| $OCH_3$ | $OCH_3$ | $C(CH_3)_3$ | 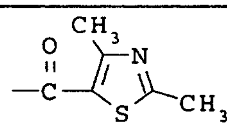 |
| $OCH_3$ | $OCH_3$ | $CH(CH_3)_2$ | 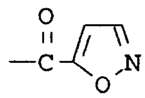 |
| $OCH_3$ | $OCH_3$ | $CH(CH_3)_2$ | 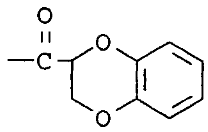 |
| $OCH_3$ | $OCH_3$ | $CH(CH_3)_2$ | 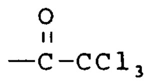 |
| $OCH_3$ | $OCH_3$ | $CH(CH_3)_2$ | 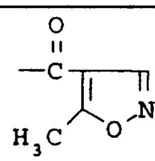 |
| $OCH_3$ | $OCH_3$ | $C(CH_3)_3$ | 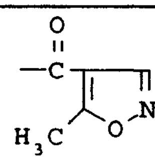 |
| $OCH_3$ | $OCH_3$ | $CH(CH_3)_2$ | 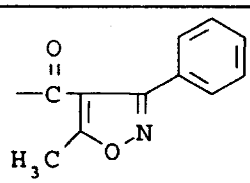 |
| $OCH_3$ | $OCH_3$ | $C(CH_3)_3$ | 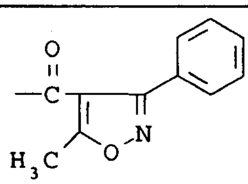 |

Table 1 (continued)

| R1 | R2 | R3 | R4 |
|---|---|---|---|
| $OCH_3$ | $OCH_3$ | $C(CH_3)_3$ | 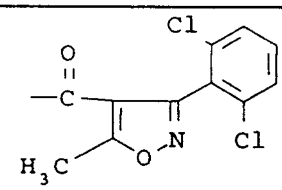 |
| $OCH_3$ | $OCH_3$ | $CH(CH_3)_2$ | 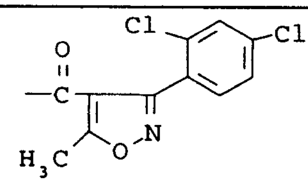 |
| $OCH_3$ | $OCH_3$ | $C(CH_3)_3$ | 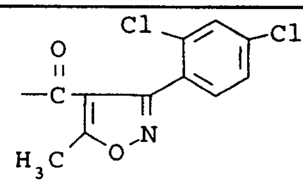 |
| $OCH_3$ | $OCH_3$ | $C(CH_3)_3$ | 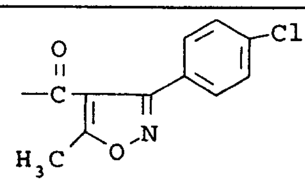 |
| $OCH_3$ | $OCH_3$ | $C(CH_3)_3$ | 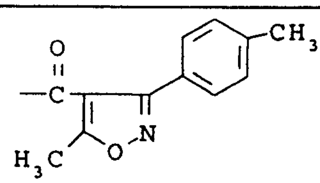 |
| $OCH_3$ | $OCH_3$ | $C(CH_3)_3$ | 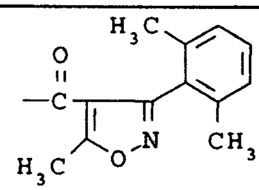 |
| $OCH_3$ | $OCH_3$ | $C(CH_3)_3$ | 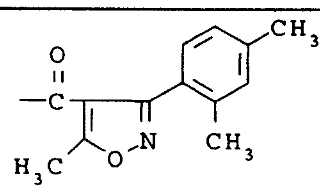 |
| $OCH_3$ | $OCH_3$ | $C(CH_3)_3$ | 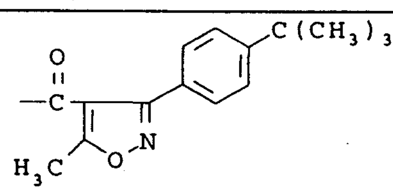 |

Table 1 (continued)

| R1 | R2 | R3 | R4 |
|---|---|---|---|
| $OCH_3$ | $OCH_3$ | $CH(CH_3)_2$ | 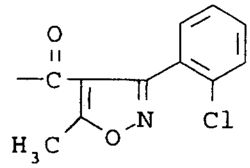 |
| $OCH_3$ | $OCH_3$ | $C(CH_3)_3$ | 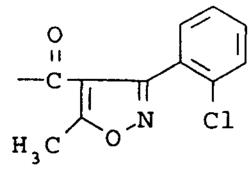 |
| $OCH_3$ | $OCH_3$ | $C(CH_3)_3$ | 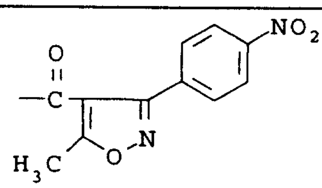 |
| $OCH_3$ | $OCH_3$ | $C(CH_3)_3$ | 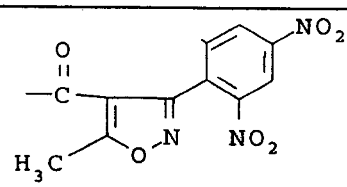 |
| $OCH_3$ | $OCH_3$ | $CH(CH_3)_2$ | 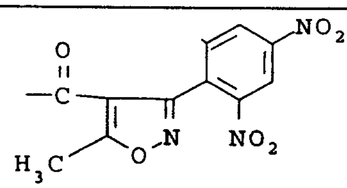 |
| $OCH_3$ | $OCH_3$ | $CH(CH_3)_2$ | 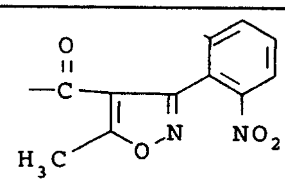 |
| $OCH_3$ | $OCH_3$ | $C(CH_3)_3$ | 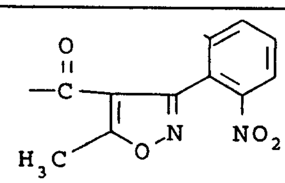 |
| $OCH_3$ | $OCH_3$ | $C(CH_3)_3$ | 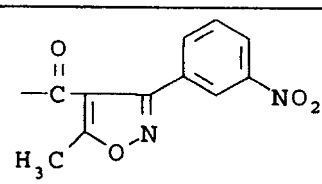 |

Table 1 (continued)

| R1 | R2 | R3 | R4 |
|---|---|---|---|
| $OCH_3$ | $OCH_3$ | $CH(CH_3)_2$ | 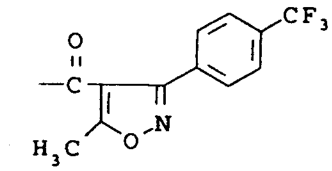 |
| $OCH_3$ | $OCH_3$ | $C(CH_3)_3$ | 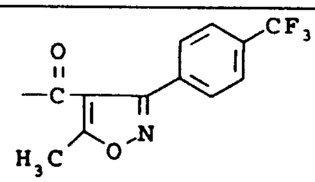 |
| $OCH_3$ | $OCH_3$ | $C(CH_3)_3$ | 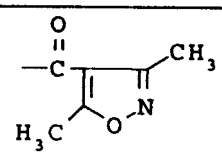 |
| $OCH_3$ | $OCH_3$ | $C(CH_3)_3$ | 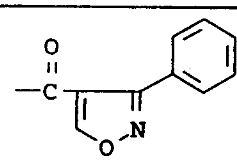 |
| $OCH_3$ | $OCH_3$ | $C(CH_3)_3$ | 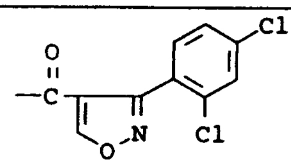 |
| $OCH_3$ | $OCH_3$ | $C(CH_3)_3$ | 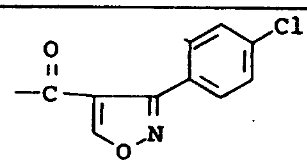 |
| $OCH_3$ | $OCH_3$ | $CH(CH_3)_2$ | 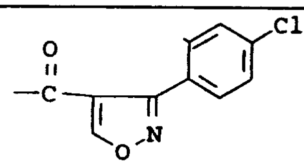 |
| $OCH_3$ | $OCH_3$ | $C(CH_3)_3$ | 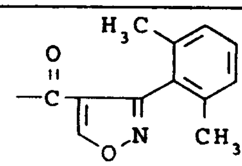 |
| $OCH_3$ | $OCH_3$ | $CH(CH_3)_2$ | 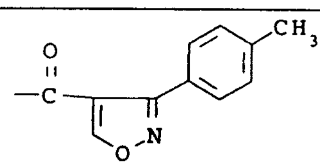 |

Table 1 (continued)

| R1 | R2 | R3 | R4 |
|---|---|---|---|
| $OCH_3$ | $OCH_3$ | $C(CH_3)_3$ | 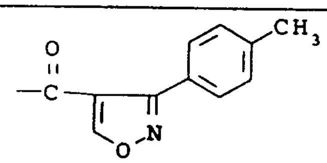 |
| $OCH_3$ | $OCH_3$ | $C(CH_3)_3$ | 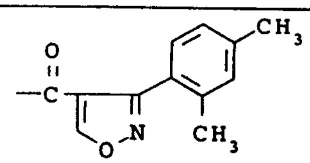 |
| $OCH_3$ | $OCH_3$ | $CH(CH_3)_2$ | 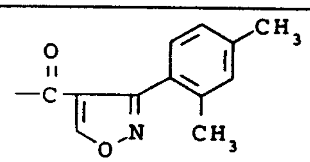 |
| $OCH_3$ | $OCH_3$ | $C(CH_3)_3$ | 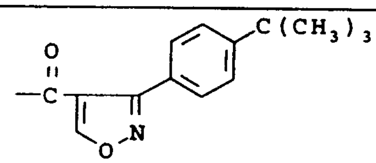 |
| $OCH_3$ | $OCH_3$ | $C(CH_3)_3$ | 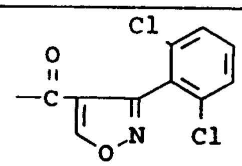 |
| $OCH_3$ | $OCH_3$ | $CH(CH_3)_2$ | 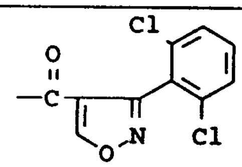 |
| $OCH_3$ | $OCH_3$ | $C(CH_3)_3$ | 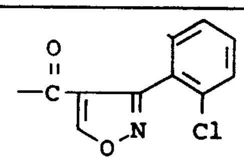 |
| $OCH_3$ | $OCH_3$ | $C(CH_3)_3$ | 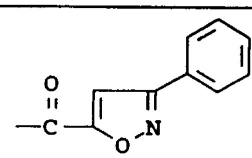 |
| $OCH_3$ | $OCH_3$ | $C(CH_3)_3$ | 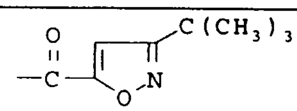 |
| $OCH_3$ | $OCH_3$ | $CH(CH_3)_2$ | 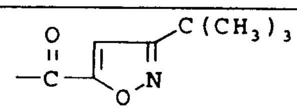 |

Table 1 (continued)

| R1 | R2 | R3 | R4 |
|---|---|---|---|
| $OCH_3$ | $OCH_3$ | $C(CH_3)_3$ | 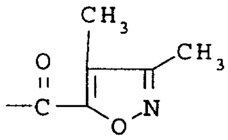 |
| $OCH_3$ | $OCH_3$ | $C(CH_3)_3$ | 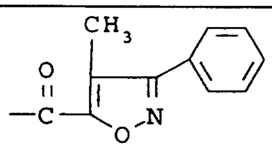 |
| $OCH_3$ | $OCH_3$ | $C(CH_3)_3$ | 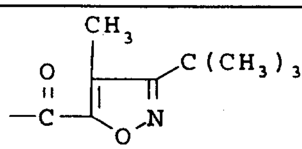 |
| $OCH_3$ | $OCH_3$ | $C(CH_3)_3$ | 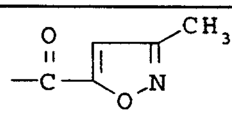 |
| $OCH_3$ | $OCH_3$ | $C(CH_3)_3$ | 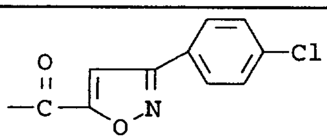 |
| $OCH_3$ | $OCH_3$ | $CH(CH_3)_2$ | 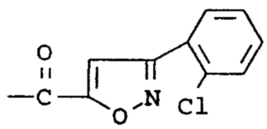 |
| $OCH_3$ | $OCH_3$ | $C(CH_3)_3$ | 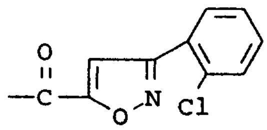 |
| $OCH_3$ | $OCH_3$ | $CH(CH_3)_2$ | 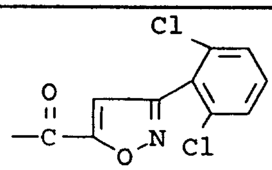 |
| $OCH_3$ | $OCH_3$ | $C(CH_3)_3$ | 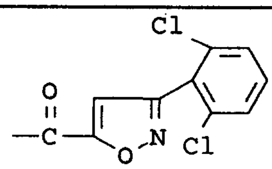 |
| $OCH_3$ | $OCH_3$ | $C(CH_3)_3$ | 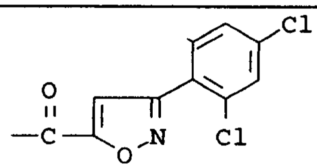 |

Table 1 (continued)

| R1 | R2 | R3 | R4 |
|---|---|---|---|
| $OCH_3$ | $OCH_3$ | $CH(CH_3)_2$ | 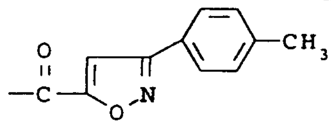 |
| $OCH_3$ | $OCH_3$ | $C(CH_3)_3$ | 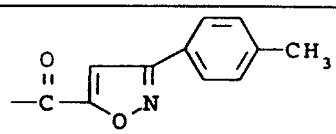 |
| $OCH_3$ | $OCH_3$ | $C(CH_3)_3$ | 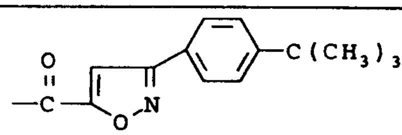 |
| $OCH_3$ | $OCH_3$ | $C(CH_3)_3$ | 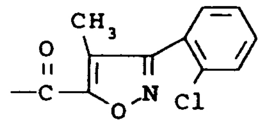 |
| $OCH_3$ | $OCH_3$ | $C(CH_3)_3$ | 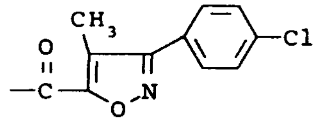 |
| $OCH_3$ | $OCH_3$ | $CH(CH_3)_2$ | 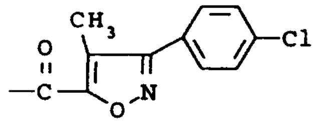 |
| $OCH_3$ | $OCH_3$ | $C(CH_3)_3$ | 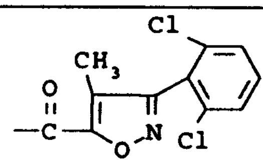 |
| $OCH_3$ | $OCH_3$ | $C(CH_3)_3$ | 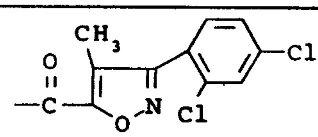 |
| $OCH_3$ | $OCH_3$ | $C(CH_3)_3$ | 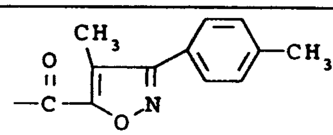 |
| $OCH_3$ | $OCH_3$ | $C(CH_3)_3$ | 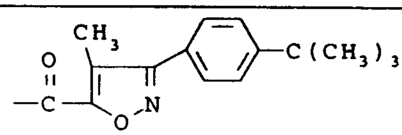 |
| $OCH_3$ | $OCH_3$ | $CH(CH_3)_2$ | 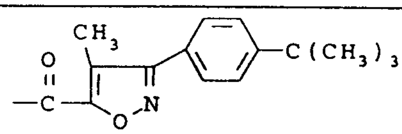 |

Table 1 (continued)

| R1 | R2 | R3 | R4 |
|---|---|---|---|
| $OCH_3$ | $OCH_3$ | $C(CH_3)_3$ | 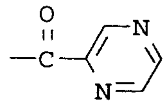 |
| $OCH_3$ | $OCH_3$ | $CH(CH_3)_2$ | 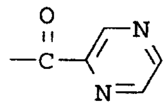 |
| $OCH_3$ | $OCH_3$ | $CH(CH_3)_2$ | 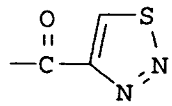 |
| $OCH_3$ | $OCH_3$ | $C(CH_3)_3$ | 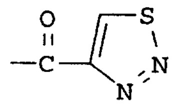 |
| $OCH_3$ | $OCH_3$ | $CH(CH_3)_2$ | 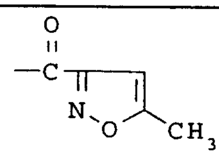 |
| $OCH_3$ | $OCH_3$ | $C(CH_3)_3$ | 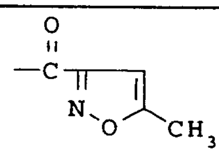 |
| $OCH_3$ | $OCH_3$ | $C(CH_3)_3$ | 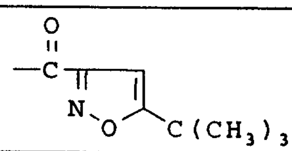 |
| $OCH_3$ | $OCH_3$ | $C(CH_3)_3$ | 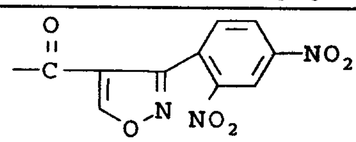 |
| $OCH_3$ | $OCH_3$ | $CH(CH_3)_2$ | 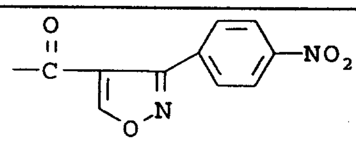 |
| $OCH_3$ | $OCH_3$ | $C(CH_3)_3$ | 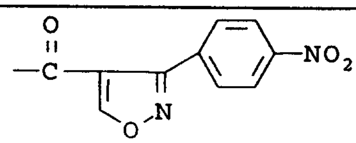 |
| $OCH_3$ | $OCH_3$ | $C(CH_3)_3$ | 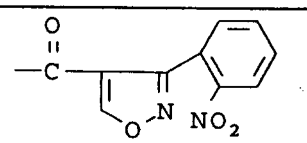 |

Table 1 (continued)

| R1 | R2 | R3 | R4 |
|---|---|---|---|
| $OCH_3$ | $OCH_3$ | $C(CH_3)_3$ | 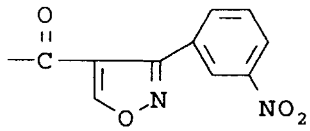 |
| $OCH_3$ | $OCH_3$ | $C(CH_3)_3$ | 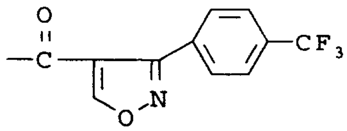 |
| $OCH_3$ | $OCH_3$ | $CH(CH_3)_2$ | 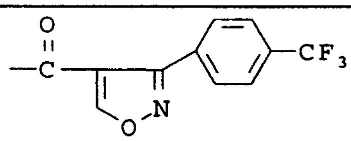 |

Table 1 (continued)

| $R^1$ | $R^2$ | $R^3$ | $R^4$ |
|---|---|---|---|
| $OCH_3$ | $OCH_3$ | $C(CH_3)_3$ | 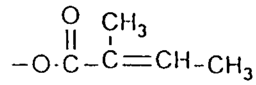 |
| $OCH_3$ | $OCH_3$ | $C(CH_3)_3$ | 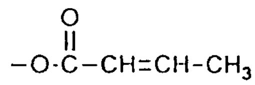 |
| $OCH_3$ | $OCH_3$ | $C(CH_3)_3$ | 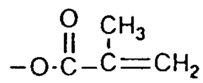 |
| $OCH_3$ | $OCH_3$ | $C(CH_3)_3$ | 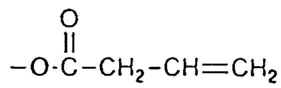 |
| $OCH_3$ | $OCH_3$ | $C(CH_3)_3$ | 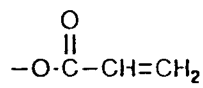 |
| $OCH_3$ | $OCH_3$ | $C(CH_3)_3$ | 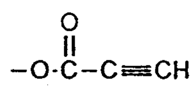 |
| $OCH_3$ | $OCH_3$ | $C(CH_3)_3$ | 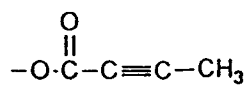 |
| $OCH_3$ | $OCH_3$ | $C(CH_3)_3$ | 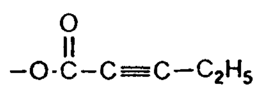 |
| $OCH_3$ | $OCH_3$ | $C(CH_3)_3$ | 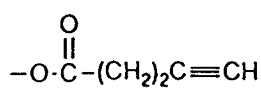 |
| $OCH_3$ | $OCH_3$ | $C(CH_3)_3$ | 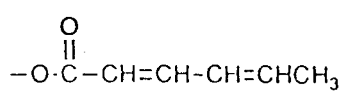 |
| $OCH_3$ | $OCH_3$ | $C(CH_3)_3$ | 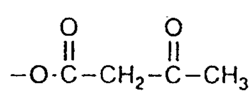 |

Table 1 (continued)

| $R^1$ | $R^2$ | $R^3$ | $R^4$ |
|---|---|---|---|
| $OCH_3$ | $OCH_3$ | $C(CH_3)_3$ | 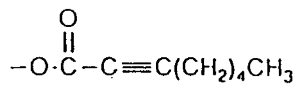 |
| $OCH_3$ | $OCH_3$ | $C(CH_3)_3$ | 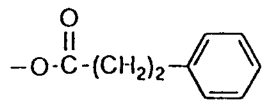 |
| $OCH_3$ | $OCH_3$ | $C(CH_3)_3$ | 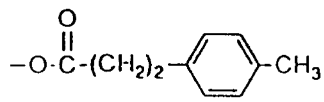 |
| $OCH_3$ | $OCH_3$ | $C(CH_3)_3$ | 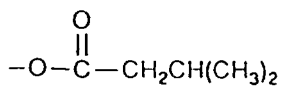 |
| $OCH_3$ | $OCH_3$ | $C(CH_3)_3$ | 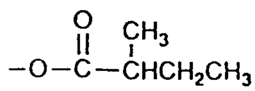 |
| $OCH_3$ | $OCH_3$ | $C(CH_3)_3$ | 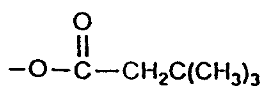 |
| $OCH_3$ | $OCH_3$ | $C(CH_3)_3$ | 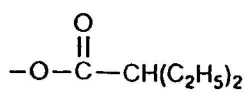 |
| $OCH_3$ | $OCH_3$ | $C(CH_3)_3$ | 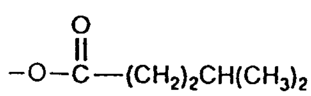 |
| $OCH_3$ | $OCH_3$ | $C(CH_3)_3$ | 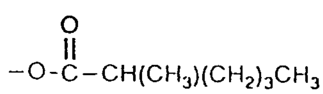 |
| $OCH_3$ | $OCH_3$ | $C(CH_3)_3$ | 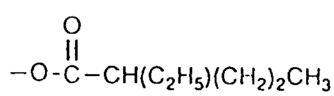 |

Table 1 (continued)

| $R^1$ | $R^2$ | $R^3$ | $R^4$ |
|---|---|---|---|
| $OCH_3$ | $OCH_3$ | $C(CH_3)_3$ | 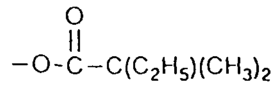 |
| $OCH_3$ | $OCH_3$ | $C(CH_3)_3$ | 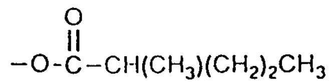 |
| $OCH_3$ | $OCH_3$ | $C(CH_3)_3$ | 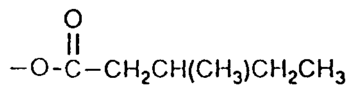 |
| $OCH_3$ | $OCH_3$ | $C(CH_3)_3$ | 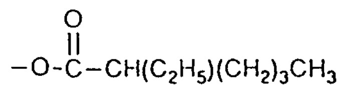 |
| $OCH_3$ | $OCH_3$ | $C(CH_3)_3$ | 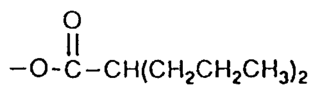 |
| $OCH_3$ | $OCH_3$ | 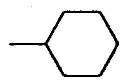 | 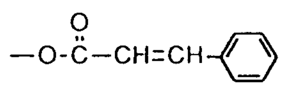 |
| $OCH_3$ | $OCH_3$ | 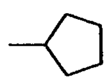 | 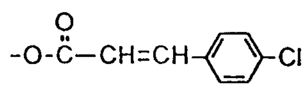 |
| $OCH_3$ | $OCH_3$ | $C(C_2H_5)(CH_3)_2$ | 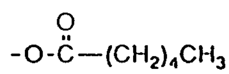 |
| $OCH_3$ | $OCH_3$ | 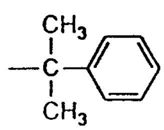 | 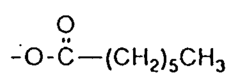 |
| $OCH_3$ | $OCH_3$ | 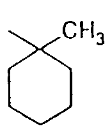 | 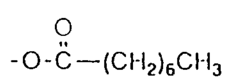 |

Table 1 (continued)

| $R^1$ | $R^2$ | $R^3$ | $R^4$ |
|---|---|---|---|
| $OCH_3$ | $OCH_3$ | 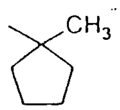 | $-O-\overset{O}{\overset{\|}{C}}-(CH_2)_7CH_3$ |
| $OCH_3$ | $OCH_3$ | $CH_2C(CH_3)_3$ | $-O-\overset{O}{\overset{\|}{C}}-(CH_2)_8CH_3$ |
| $OCH_3$ | $OCH_3$ | 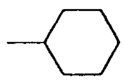 | $-O-\overset{O}{\overset{\|}{C}}-(CH_2)_9CH_3$ |
| $OCH_3$ | $OCH_3$ | $C(C_2H_5)(CH_3)_2$ | $-O-\overset{O}{\overset{\|}{C}}-(CH_2)_{10}CH_3$ |
| $OCH_3$ | $OCH_3$ | 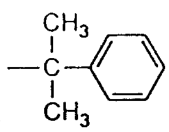 | $-O-\overset{O}{\overset{\|}{C}}-(CH_2)_{11}CH_3$ |
| $OCH_3$ | $OCH_3$ | 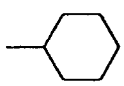 | $-O-\overset{O}{\overset{\|}{C}}-(CH_2)_{12}CH_3$ |
| $OCH_3$ | $OCH_3$ | 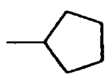 | $-O-\overset{O}{\overset{\|}{C}}-(CH_2)_{13}CH_3$ |
| $OCH_3$ | $OCH_3$ | 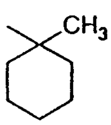 | $-O-\overset{O}{\overset{\|}{C}}-(CH_2)_{14}CH_3$ |
| $OCH_3$ | $OCH_3$ | 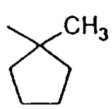 | $-O-\overset{O}{\overset{\|}{C}}-(CH_2)_{15}CH_3$ |
| $OCH_3$ | $OCH_3$ | 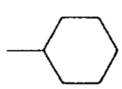 | $-O-\overset{O}{\overset{\|}{C}}-(CH_2)_{16}CH_3$ |
| $OCH_3$ | $OCH_3$ | $C(C_2H_5)(CH_3)_2$ | $-O-\overset{O}{\overset{\|}{C}}-(CH_2)_{17}CH_3$ |

Table 1 (continued)

| $R^1$ | $R^2$ | $R^3$ | $R^4$ |
|---|---|---|---|
| $OCH_3$ | $OCH_3$ | 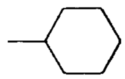 | $-O-\overset{O}{\overset{\|}{C}}-(CH_2)_{19}CH_3$ |
| $OCH_3$ | $OCH_3$ | 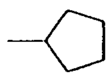 | $-O-\overset{O}{\overset{\|}{C}}-(CH_2)_9CH=CH(CH_2)_7CH_3$ |
| $OCH_3$ | $OCH_3$ | 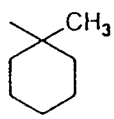 | $-O-\overset{O}{\overset{\|}{C}}-(CH_2)_7CH=CH(CH_2)_7CH_3$ |
| $OCH_3$ | $OCH_3$ | 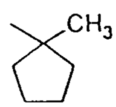 | $-O-\overset{O}{\overset{\|}{C}}-(CH_2)_7CH=CH(CH_2)_5CH_3$ |
| $OCH_3$ | $OCH_3$ | $C(C_2H_5)(CH_3)_2$ | $-O-\overset{O}{\overset{\|}{C}}-(CH_2)_7CH=CH(CH_2)_3CH_3$ |
| $OCH_3$ | $OCH_3$ | $C(C_2H_5)(CH_3)_2$ | $-O-\overset{O}{\overset{\|}{C}}-(CH_2)_8CH=CH_2$ |
| $OCH_3$ | $OCH_3$ | 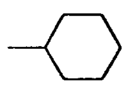 | $-O-\overset{O}{\overset{\|}{C}}-CH_2\overset{CH_3}{\overset{\|}{C}}H(CH_2)_2CH=C(CH_3)_2$ |
| $OCH_3$ | $OCH_3$ | 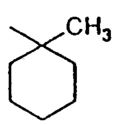 | 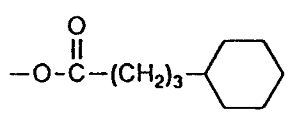 |
| $OCH_3$ | $OCH_3$ | 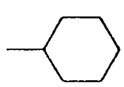 | 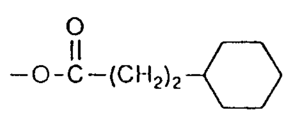 |
| $OCH_3$ | $OCH_3$ | 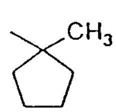 | 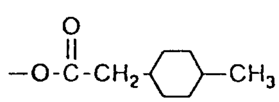 |

Table 1 (continued)

| $R^1$ | $R^2$ | $R^3$ | $R^4$ |
|---|---|---|---|
| $OCH_3$ | $OCH_3$ | 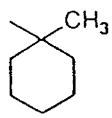 | 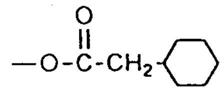 |
| $OCH_3$ | $OCH_3$ | 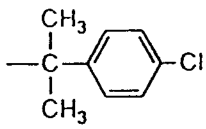 | 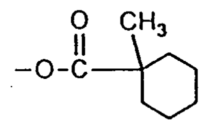 |
| $OCH_3$ | $OCH_3$ | $C(C_2H_5)(CH_3)_2$ | 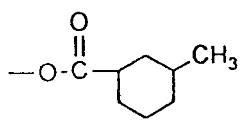 |
| $OCH_3$ | $OCH_3$ | $C(C_2H_5)(CH_3)_2$ | 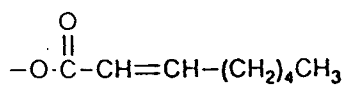 |
| $OCH_3$ | $OCH_3$ | 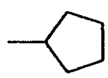 | 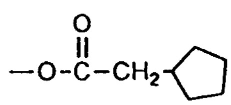 |
| $OCH_3$ | $OCH_3$ | 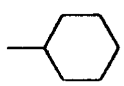 | 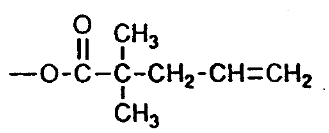 |
| $OCH_3$ | $OCH_3$ | 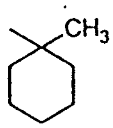 | 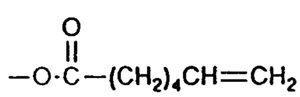 |
| $OCH_3$ | $OCH_3$ | 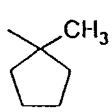 | 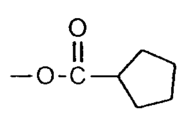 |
| $OCH_3$ | $OCH_3$ | 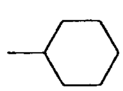 | 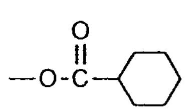 |

Table 1 (continued)

| $R^1$ | $R^2$ | $R^3$ | $R^4$ |
|---|---|---|---|
| $OCH_3$ | $OCH_3$ | 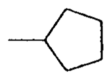 | 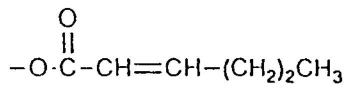 |
| $OCH_3$ | $OCH_3$ | $C(C_2H_5)(CH_3)_2$ | 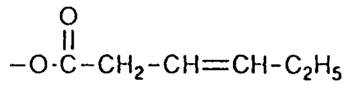 |
| $OCH_3$ | $OCH_3$ | 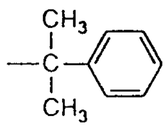 | 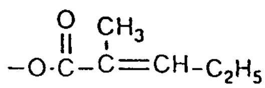 |
| $OCH_3$ | $OCH_3$ | $C(C_2H_5)(CH_3)_2$ | 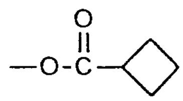 |
| $OCH_3$ | $OCH_3$ | $C(C_2H_5)(CH_3)_2$ | 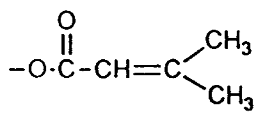 |
| $OCH_3$ | $OCH_3$ | 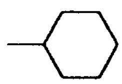 | 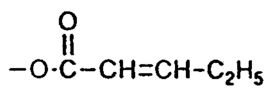 |
| $OCH_3$ | $OCH_3$ | 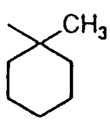 | 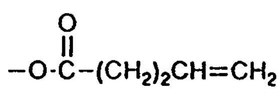 |
| $OCH_3$ | $OCH_3$ | 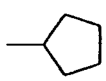 | 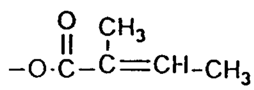 |
| $OCH_3$ | $OCH_3$ | 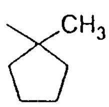 | 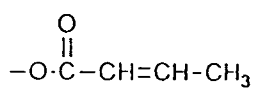 |

Table 1 (continued)

| $R^1$ | $R^2$ | $R^3$ | $R^4$ |
|---|---|---|---|
| $OCH_3$ | $OCH_3$ | $C(C_2H_5)(CH_3)_2$ | 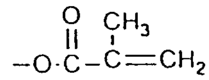 |
| $OCH_3$ | $OCH_3$ | 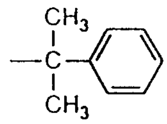 | 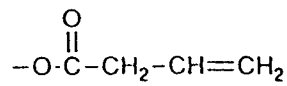 |
| $OCH_3$ | $OCH_3$ | 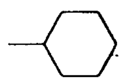 | 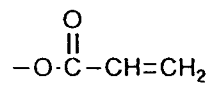 |
| $OCH_3$ | $OCH_3$ | 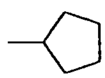 | 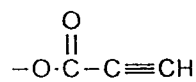 |
| $OCH_3$ | $OCH_3$ | 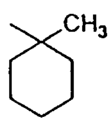 | 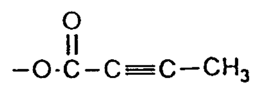 |
| $OCH_3$ | $OCH_3$ | 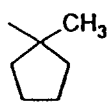 | 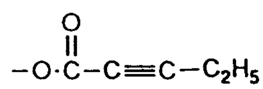 |
| $OCH_3$ | $OCH_3$ | 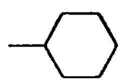 | 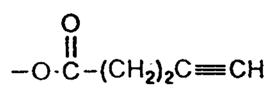 |
| $OCH_3$ | $OCH_3$ | 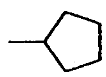 | 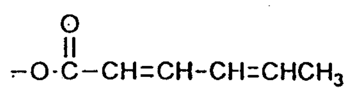 |
| $OCH_3$ | $OCH_3$ | $C(C_2H_5)(CH_3)_2$ | 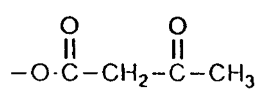 |
| $OCH_3$ | $OCH_3$ | 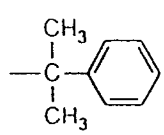 | 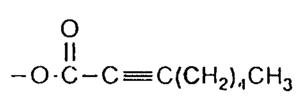 |

Table 1 (continued)

| $R^1$ | $R^2$ | $R^3$ | $R^4$ |
|---|---|---|---|
| $OCH_3$ | $OCH_3$ | 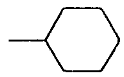 | 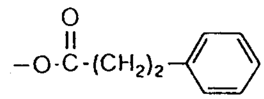 |
| $OCH_3$ | $OCH_3$ | 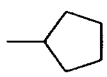 | 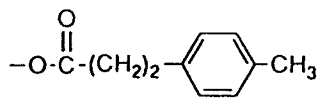 |
| $OCH_3$ | $OCH_3$ | 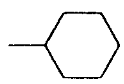 | 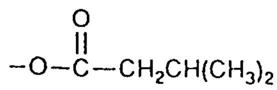 |
| $OCH_3$ | $OCH_3$ | 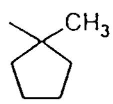 | 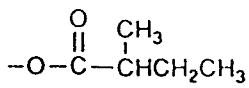 |
| $OCH_3$ | $OCH_3$ | 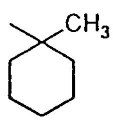 | 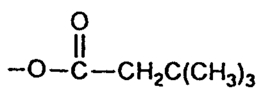 |
| $OCH_3$ | $OCH_3$ | $C(C_2H_5)(CH_3)_2$ | 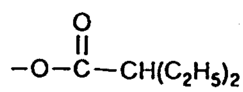 |
| $OCH_3$ | $OCH_3$ | 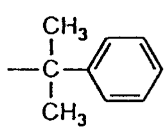 | 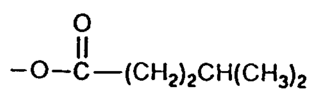 |
| $OCH_3$ | $OCH_3$ | 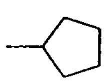 | 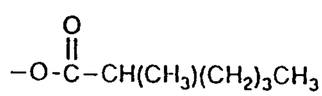 |
| $OCH_3$ | $OCH_3$ | 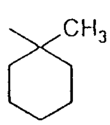 | 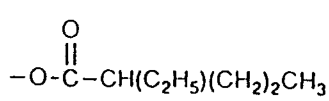 |

Table 1 (continued)

| $R^1$ | $R^2$ | $R^3$ | $R^4$ |
|---|---|---|---|
| $OCH_3$ | $OCH_3$ | $C(C_2H_5)(CH_3)_2$ | 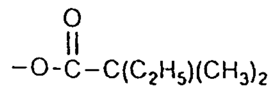 |
| $OCH_3$ | $OCH_3$ | $C(C_2H_5)(CH_3)_2$ | 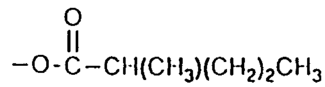 |
| $OCH_3$ | $OCH_3$ | $C(C_2H_5)(CH_3)_2$ | 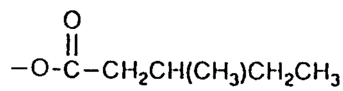 |
| $OCH_3$ | $OCH_3$ | 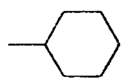 | 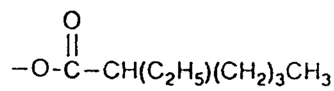 |
| $OCH_3$ | $OCH_3$ | 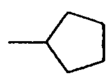 | 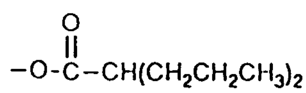 |
| $OCH_3$ | $OCH_3$ | $CH(CH_3)_2$ | 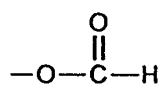 |
| $OCH_3$ | $OCH_3$ | $C(CH_3)_3$ | 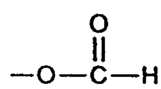 |
| $OCH_3$ | $OCH_3$ | $CH(CH_3)_2$ | 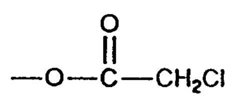 |
| $OCHF_2$ | $OCHF_2$ | $CH(CH_3)_2$ | 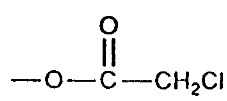 |
| $OCF_3$ | $OCF_3$ | $CH(CH_3)_2$ | 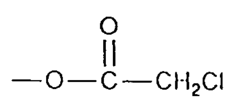 |

Table 1 (continued)

| $R^1$ | $R^2$ | $R^3$ | $R^4$ |
|---|---|---|---|
| $OCH_3$ | $OCH_3$ | $C(CH_3)_3$ | 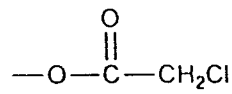 |
| $OCHF_2$ | $OCHF_2$ | $C(CH_3)_3$ | 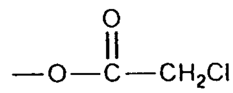 |
| $OCF_3$ | $OCF_3$ | $C(CH_3)_3$ | 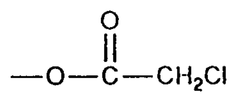 |
| $OCH_3$ | $OCH_3$ | $CH(CH_3)_2$ | 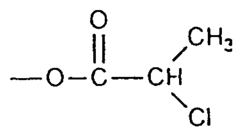 |
| $OCH_3$ | $OCH_3$ | $C(CH_3)_3$ | 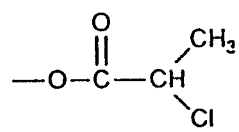 |
| $OCH_3$ | $OCH_3$ | $CH(CH_3)_2$ | 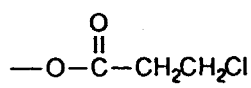 |
| $OCH_3$ | $OCH_3$ | $C(CH_3)_3$ | 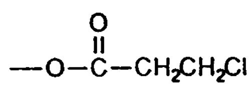 |
| $OCH_3$ | $OCH_3$ | $CH(CH_3)_2$ | 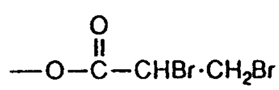 |
| $OCH_3$ | $OCH_3$ | $C(CH_3)_3$ | 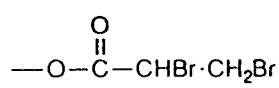 |
| $OCH_3$ | $OCH_3$ | $CH(CH_3)_2$ | 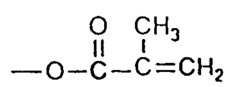 |

Table 1 (continued)

| $R^1$ | $R^2$ | $R^3$ | $R^4$ |
|---|---|---|---|
| $OCH_3$ | $OCH_3$ | $CH(CH_3)_2$ | 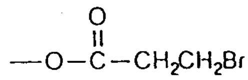 |
| $OCH_3$ | $OCH_3$ | $C(CH_3)_3$ | 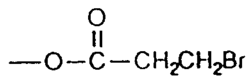 |
| $OCH_3$ | $OCH_3$ | $CH(CH_3)_2$ | 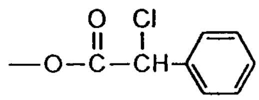 |
| $OCH_3$ | $OCH_3$ | $C(CH_3)_3$ | 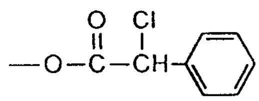 |
| $OCH_3$ | $OCH_3$ | $CH(CH_3)_2$ | 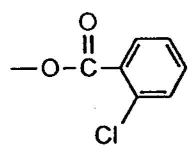 |
| $OCH_3$ | $OCH_3$ | $C(CH_3)_3$ | 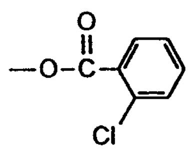 |
| $OCH_3$ | $OCH_3$ | $CH(CH_3)_2$ | 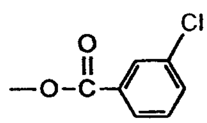 |
| $OCH_3$ | $OCH_3$ | $C(CH_3)_3$ | 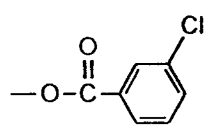 |
| $OCH_3$ | $OCH_3$ | $CH(CH_3)_2$ | 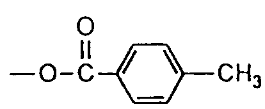 |

Table 1 (continued)

| $R^1$ | $R^2$ | $R^3$ | $R^4$ |
|---|---|---|---|
| $OCH_3$ | $OCH_3$ | $C(CH_3)_3$ | 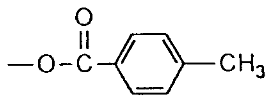 |
| $OCH_3$ | $OCH_3$ | $CH(CH_3)_2$ | 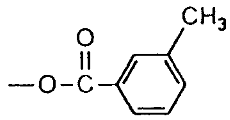 |
| $OCH_3$ | $OCH_3$ | $C(CH_3)_3$ | 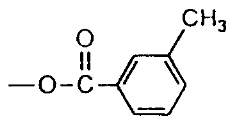 |
| $OCH_3$ | $OCH_3$ | $CH(CH_3)_2$ | 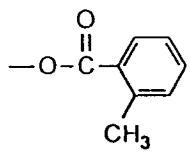 |
| $OCH_3$ | $OCH_3$ | $C(CH_3)_3$ | 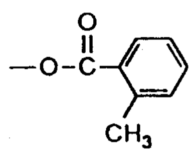 |
| $OCH_3$ | $OCH_3$ | $CH(CH_3)_2$ | 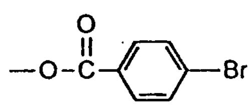 |
| $OCH_3$ | $OCH_3$ | $C(CH_3)_3$ | 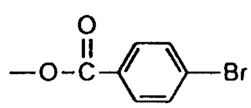 |
| $OCH_3$ | $OCH_3$ | $CH(CH_3)_2$ | 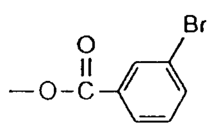 |

Table 1 (continued)

| $R^1$ | $R^2$ | $R^3$ | $R^4$ |
|---|---|---|---|
| $OCH_3$ | $OCH_3$ | $C(CH_3)_3$ | 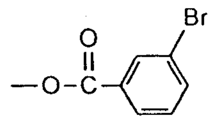 |
| $OCH_3$ | $OCH_3$ | $CH(CH_3)_2$ | 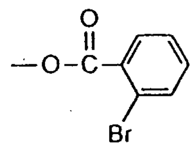 |
| $OCH_3$ | $OCH_3$ | $C(CH_3)_3$ | 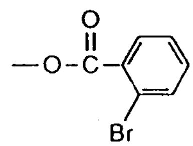 |
| $OCH_3$ | $OCH_3$ | $CH(CH_3)_2$ | 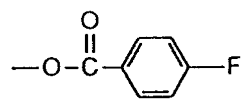 |
| $OCH_3$ | $OCH_3$ | $C(CH_3)_3$ | 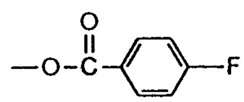 |
| $OCH_3$ | $OCH_3$ | $CH(CH_3)_2$ | 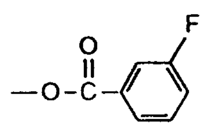 |
| $OCH_3$ | $OCH_3$ | $C(CH_3)_3$ | 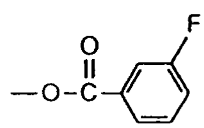 |
| $OCH_3$ | $OCH_3$ | $CH(CH_3)_2$ | 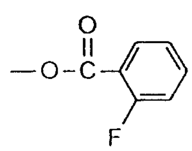 |

Table 1 (continued)

| $R^1$ | $R^2$ | $R^3$ | $R^4$ |
|---|---|---|---|
| $OCH_3$ | $OCH_3$ | $C(CH_3)_3$ | 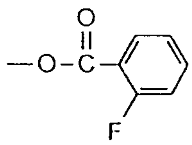 |
| $OCH_3$ | $OCH_3$ | $CH(CH_3)_2$ | 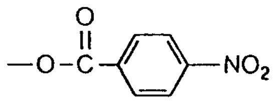 |
| $OCH_3$ | $OCH_3$ | $C(CH_3)_3$ | 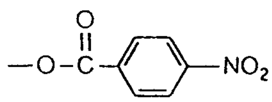 |
| $OCH_3$ | $OCH_3$ | $CH(CH_3)_2$ | 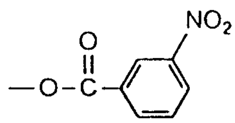 |
| $OCH_3$ | $OCH_3$ | $C(CH_3)_3$ | 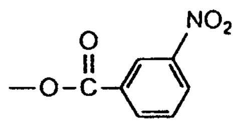 |
| $OCH_3$ | $OCH_3$ | $CH(CH_3)_2$ | 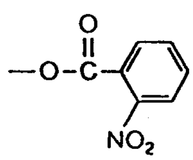 |
| $OCH_3$ | $OCH_3$ | $C(CH_3)_3$ | 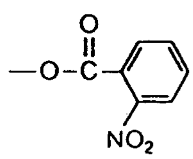 |
| $OCH_3$ | $OCH_3$ | $CH(CH_3)_2$ | 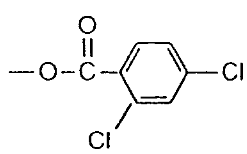 |

Table 1 (continued)

| $R^1$ | $R^2$ | $R^3$ | $R^4$ |
|---|---|---|---|
| $OCH_3$ | $OCH_3$ | $C(CH_3)_3$ | 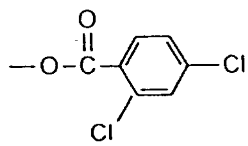 |
| $OCH_3$ | $OCH_3$ | $CH(CH_3)_2$ | 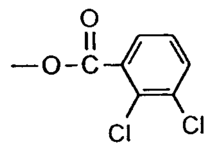 |
| $OCH_3$ | $OCH_3$ | $C(CH_3)_3$ | 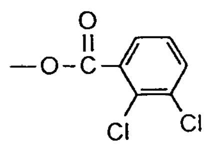 |
| $OCH_3$ | $OCH_3$ | $CH(CH_3)_2$ | 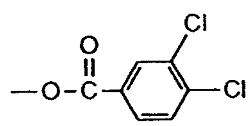 |
| $OCH_3$ | $OCH_3$ | $C(CH_3)_3$ | 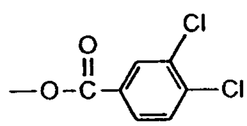 |
| $OCH_3$ | $OCH_3$ | $CH(CH_3)_2$ | 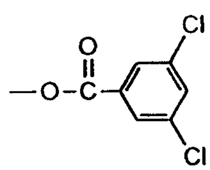 |
| $OCH_3$ | $OCH_3$ | $C(CH_3)_3$ | 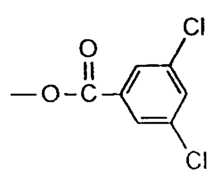 |

Table 1 (continued)

| $R^1$ | $R^2$ | $R^3$ | $R^4$ |
|---|---|---|---|
| $OCH_3$ | $OCH_3$ | $CH(CH_3)_2$ | 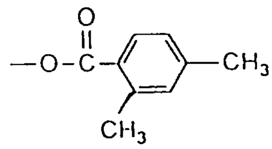 |
| $OCH_3$ | $OCH_3$ | $C(CH_3)_3$ | 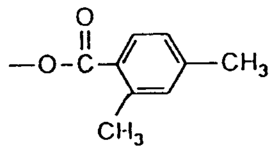 |
| $OCH_3$ | $OCH_3$ | $CH(CH_3)_2$ | 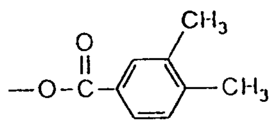 |
| $OCH_3$ | $OCH_3$ | $C(CH_3)_3$ | 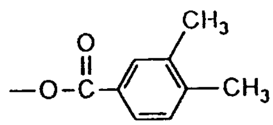 |
| $OCH_3$ | $OCH_3$ | $CH(CH_3)_2$ | 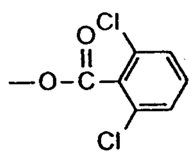 |
| $OCH_3$ | $OCH_3$ | $C(CH_3)_3$ | 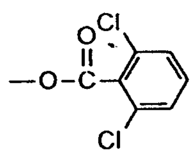 |
| $OCH_3$ | $OCH_3$ | $CH(CH_3)_2$ | 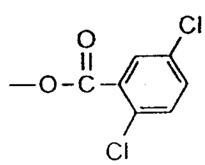 |

Table 1 (continued)

| R¹ | R² | R³ | R⁴ |
|---|---|---|---|
| $OCH_3$ | $OCH_3$ | $C(CH_3)_3$ | 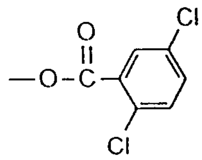 |
| $OCH_3$ | $OCH_3$ | $CH(CH_3)_2$ | 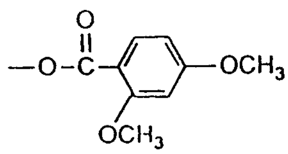 |
| $OCH_3$ | $OCH_3$ | $C(CH_3)_3$ | 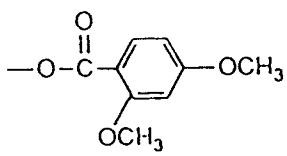 |
| $OCH_3$ | $OCH_3$ | $CH(CH_3)_2$ | 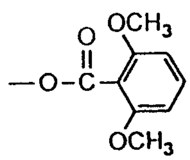 |
| $OCH_3$ | $OCH_3$ | $C(CH_3)_3$ | 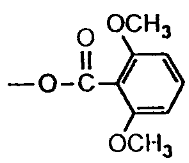 |
| $OCH_3$ | $OCH_3$ | $CH(CH_3)_2$ | 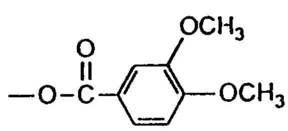 |
| $OCH_3$ | $OCH_3$ | $C(CH_3)_3$ | 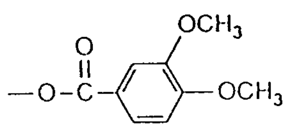 |

Table 1 (continued)

| $R^1$ | $R^2$ | $R^3$ | $R^4$ |
|---|---|---|---|
| $OCH_3$ | $OCH_3$ | $CH(CH_3)_2$ | 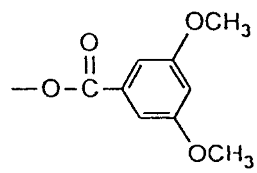 |
| $OCH_3$ | $OCH_3$ | $C(CH_3)_3$ | 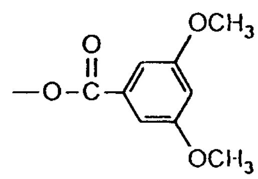 |
| $OCH_3$ | $OCH_3$ | $CH(CH_3)_2$ | 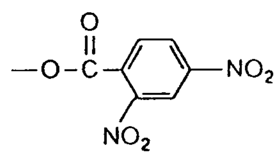 |
| $OCH_3$ | $OCH_3$ | $C(CH_3)_3$ | 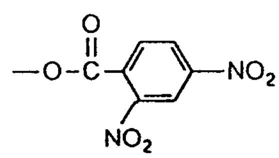 |
| $OCH_3$ | $OCH_3$ | $CH(CH_3)_2$ | 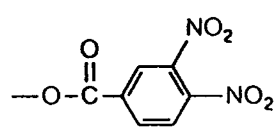 |
| $OCH_3$ | $OCH_3$ | $C(CH_3)_3$ | 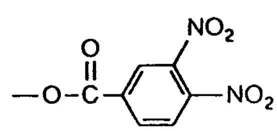 |
| $OCH_3$ | $OCH_3$ | $CH(CH_3)_2$ | 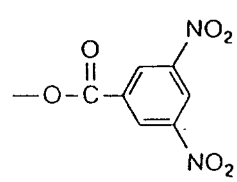 |

Table 1 (continued)

| $R^1$ | $R^2$ | $R^3$ | $R^4$ |
|---|---|---|---|
| $OCH_3$ | $OCH_3$ | $CH(CH_3)_2$ | 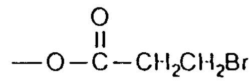 |
| $OCH_3$ | $OCH_3$ | $C(CH_3)_3$ | 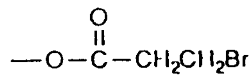 |
| $OCH_3$ | $OCH_3$ | $CH(CH_3)_2$ | 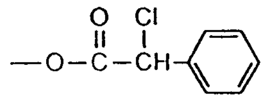 |
| $OCH_3$ | $OCH_3$ | $C(CH_3)_3$ | 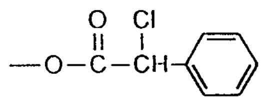 |
| $OCH_3$ | $OCH_3$ | 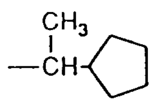 | 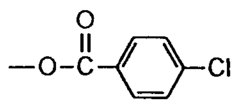 |
| $OCH_3$ | $OCH_3$ | 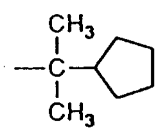 | 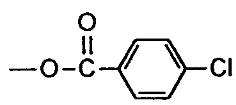 |
| $OCH_3$ | $OCH_3$ | 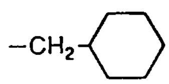 | 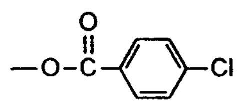 |
| $OCH_3$ | $OCH_3$ | 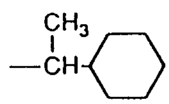 | 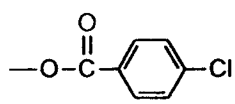 |
| $OCH_3$ | $OCH_3$ | 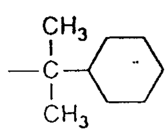 | 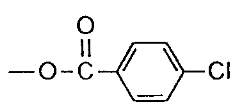 |
| $OCH_3$ | $OCH_3$ | 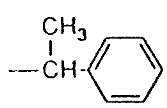 | 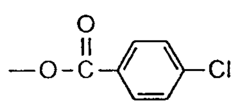 |

Table 1 (continued)

| $R^1$ | $R^2$ | $R^3$ | $R^4$ |
|---|---|---|---|
| $OCH_3$ | $OCH_3$ | $C_2H_5$ | 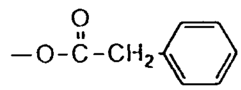 |
| $OCH_3$ | $OCH_3$ | $CH(CH_3)_2$ | 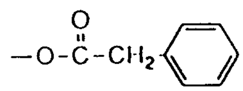 |
| $OCH_3$ | $OCH_3$ | $C(CH_3)_3$ | 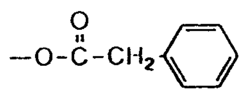 |
| $OCH_3$ | $OCH_3$ | $CH_2CH_2CH_3$ | 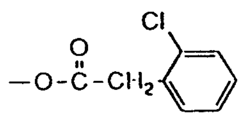 |
| $OCH_3$ | $OCH_3$ | $CH(CH_3)_2$ | 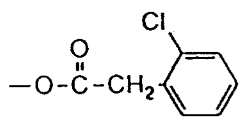 |
| $OCH_3$ | $OCH_3$ | $C(CH_3)_3$ | 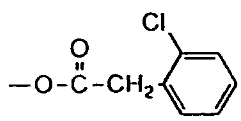 |
| $OCH_3$ | $OCH_3$ | $C_2H_5$ | 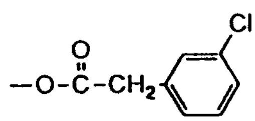 |
| $OCH_3$ | $OCH_3$ | $CH(CH_3)_2$ | 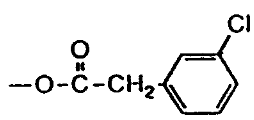 |
| $OCH_3$ | $OCH_3$ | $C(CH_3)_3$ | 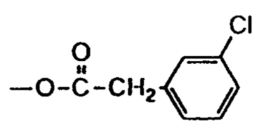 |
| $OCH_3$ | $OCH_3$ | $CH_2CH_2CH_3$ | 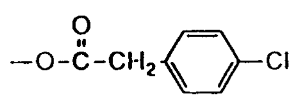 |
| $OCH_3$ | $OCH_3$ | $CH(CH_3)_2$ | 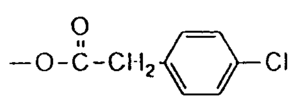 |

Table 1 (continued)

| $R^1$ | $R^2$ | $R^3$ | $R^4$ |
|---|---|---|---|
| $OCH_3$ | $OCH_3$ | $C(CH_3)_3$ | 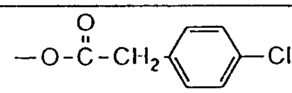 |
| $OCH_3$ | $OCH_3$ | $C_2H_5$ | 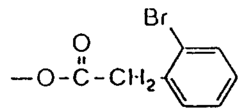 |
| $OCH_3$ | $OCH_3$ | $CH(CH_3)_2$ | 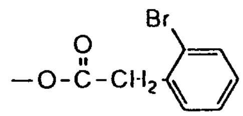 |
| $OCH_3$ | $OCH_3$ | $C(CH_3)_3$ | 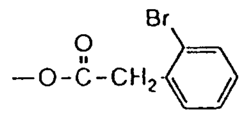 |
| $OCH_3$ | $OCH_3$ | $CH_2CH_2CH_3$ | 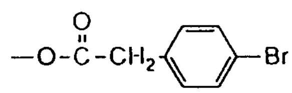 |
| $OCH_3$ | $OCH_3$ | $CH(CH_3)_2$ | 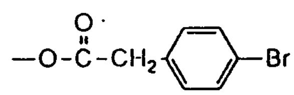 |
| $OCH_3$ | $OCH_3$ | $C(CH_3)_3$ | 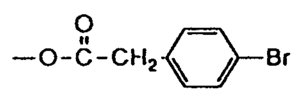 |
| $OCH_3$ | $OCH_3$ | $CH_2CH_2CH_3$ | 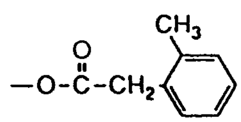 |
| $OCH_3$ | $OCH_3$ | $CH(CH_3)_2$ | 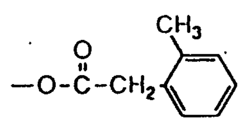 |
| $OCH_3$ | $OCH_3$ | $C(CH_3)_3$ | 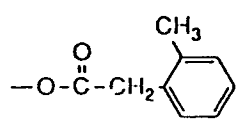 |
| $OCH_3$ | $OCH_3$ | $C_2H_5$ | 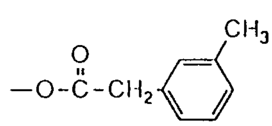 |

Table 1 (continued)

| $R^1$ | $R^2$ | $R^3$ | $R^4$ |
|---|---|---|---|
| $OCH_3$ | $OCH_3$ | $CH(CH_3)_2$ | 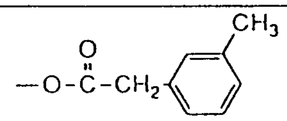 |
| $OCH_3$ | $OCH_3$ | $C(CH_3)_3$ | 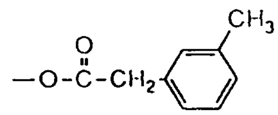 |
| $OCH_3$ | $OCH_3$ | $CH_2CH_2CH_3$ | 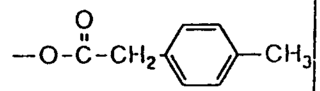 |
| $OCH_3$ | $OCH_3$ | $CH(CH_3)_2$ | 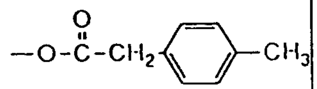 |
| $OCH_3$ | $OCH_3$ | $C(CH_3)_3$ | 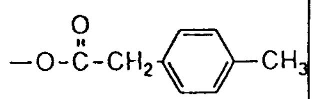 |
| $OCH_3$ | $OCH_3$ | $C_2H_5$ | 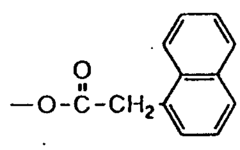 |
| $OCH_3$ | $OCH_3$ | $CH(CH_3)_2$ | 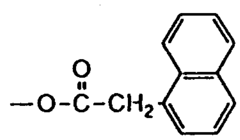 |
| $OCH_3$ | $OCH_3$ | $C(CH_3)_3$ | 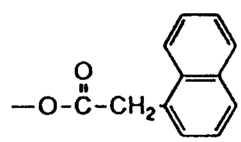 |
| $OCH_3$ | $OCH_3$ | $CH_2CH_2CH_3$ | 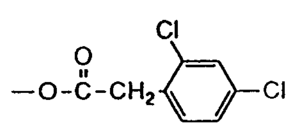 |
| $OCH_3$ | $OCH_3$ | $CH(CH_3)_2$ | 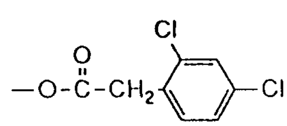 |
| $OCH_3$ | $OCH_3$ | 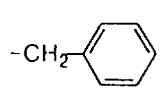 | 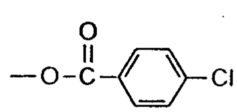 |

Table 1 (continued)

| $R^1$ | $R^2$ | $R^3$ | $R^4$ |
|---|---|---|---|
| $OCH_3$ | $OCH_3$ | $C(CH_3)_3$ | 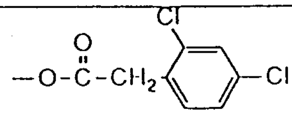 |
| $OCH_3$ | $OCH_3$ | $CH_2CH_2CH_3$ | 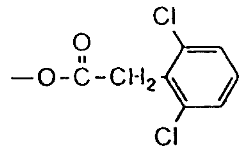 |
| $OCH_3$ | $OCH_3$ | $CH(CH_3)_2$ | 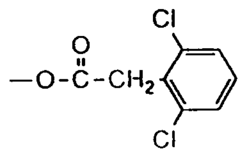 |
| $OCH_3$ | $OCH_3$ | $C(CH_3)_3$ | 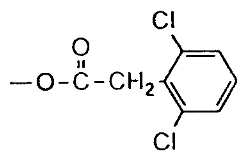 |
| $OCH_3$ | $OCH_3$ | $CH_2CH_2CH_3$ | 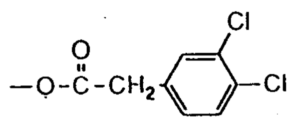 |
| $OCH_3$ | $OCH_3$ | $CH(CH_3)_2$ | 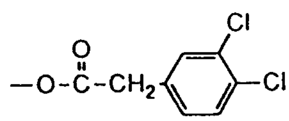 |
| $OCH_3$ | $OCH_3$ | $C(CH_3)_3$ | 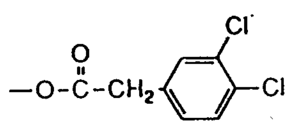 |
| $OCH_3$ | $OCH_3$ | 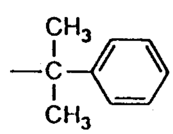 | 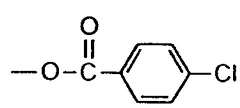 |
| $OCH_3$ | $OCH_3$ | 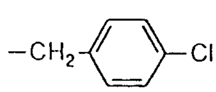 | 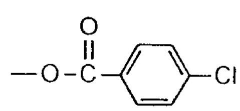 |

Table 1 (continued)

| R1 | R2 | R3 | R4 |
|---|---|---|---|
| $OCH_3$ | $OCH_3$ | $C_2H_5$ | 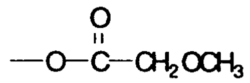 |
| $OCH_3$ | $OCH_3$ | $CH_2CH_2CH_3$ | 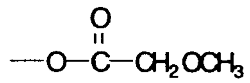 |
| $OCH_3$ | $OCH_3$ | $CH(CH_3)_2$ | 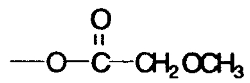 |
| $OCH_3$ | $OCH_3$ | $CH_2CH_2CH_2CH_3$ | 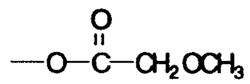 |
| $OCH_3$ | $OCH_3$ | $CH(CH_3)CH_2CH_3$ | 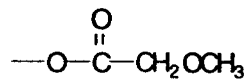 |
| $OCH_3$ | $OCH_3$ | $CH_2CH(CH_3)_2$ | 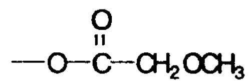 |
| $OCH_3$ | $OCH_3$ | $C(CH_3)_3$ | 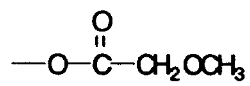 |
| $OCH_3$ | $OCH_3$ | $C_2H_5$ | 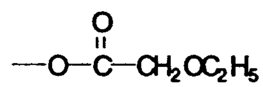 |
| $OCH_3$ | $OCH_3$ | $CH_2CH_2CH_3$ | 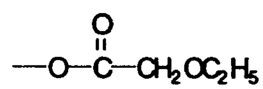 |
| $OCH_3$ | $OCH_3$ | $CH(CH_3)_2$ | 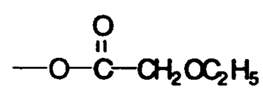 |
| $OCH_3$ | $OCH_3$ | $CH_2CH_2CH_2CH_3$ | 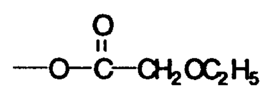 |
| $OCH_3$ | $OCH_3$ | $CH(CH_3)CH_2CH_3$ | 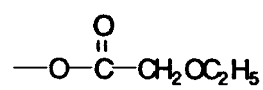 |
| $OCH_3$ | $OCH_3$ | $CH_2CH(CH_3)_2$ | 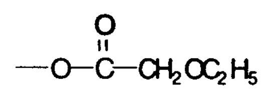 |
| $OCH_3$ | $OCH_3$ | $C(CH_3)_3$ | 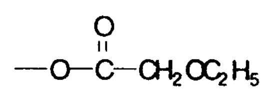 |
| $OCH_3$ | $OCH_3$ | $C_2H_5$ | 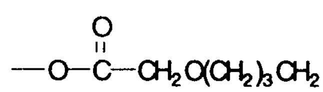 |

Table 1 (continued)

| R1 | R2 | R3 | R4 |
|---|---|---|---|
| $OCH_3$ | $OCH_3$ | $CH_2CH_2CH_3$ | 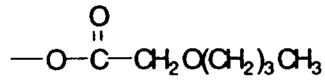 |
| $OCH_3$ | $OCH_3$ | $CH(CH_3)_2$ | 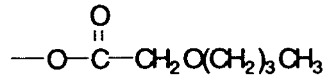 |
| $OCH_3$ | $OCH_3$ | $CH_2CH_2CH_2CH_3$ | 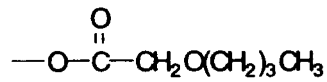 |
| $OCH_3$ | $OCH_3$ | $CH(CH_3)CH_2CH_3$ | 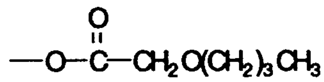 |
| $OCH_3$ | $OCH_3$ | $CH_2CH(CH_3)_2$ | 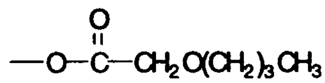 |
| $OCH_3$ | $OCH_3$ | $C(CH_3)_3$ | 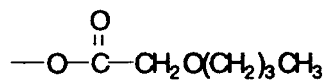 |
| $OCH_3$ | $OCH_3$ | $C_2H_5$ | 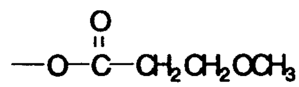 |
| $OCH_3$ | $OCH_3$ | $CH_2CH_2CH_3$ | 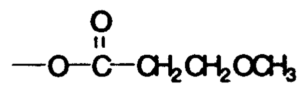 |
| $OCH_3$ | $OCH_3$ | $CH(CH_3)_2$ | 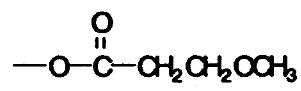 |
| $OCH_3$ | $OCH_3$ | $CH_2CH_2CH_2CH_3$ | 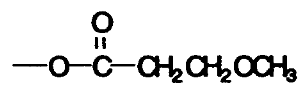 |
| $OCH_3$ | $OCH_3$ | $CH(CH_3)CH_2CH_3$ | 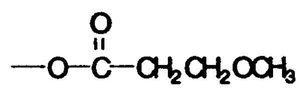 |
| $OCH_3$ | $OCH_3$ | $CH_2CH(CH_3)_2$ | 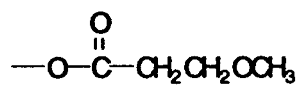 |
| $OCH_3$ | $OCH_3$ | $C(CH_3)_3$ | 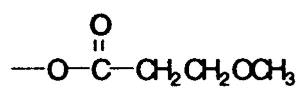 |
| $OCH_3$ | $OCH_3$ | $C_2H_5$ | 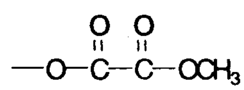 |
| $OCH_3$ | $OCH_3$ | $CH_2CH_2CH_3$ | 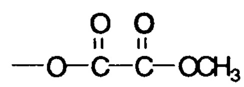 |

Table 1 (continued)

| R1 | R2 | R3 | R4 |
|---|---|---|---|
| $OCH_3$ | $OCH_3$ | $CH(CH_3)_2$ | 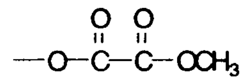 |
| $OCH_3$ | $OCH_3$ | $CH_2CH_2CH_2CH_3$ | 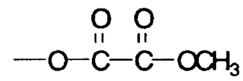 |
| $OCH_3$ | $OCH_3$ | $CH(CH_3)CH_2CH_3$ | 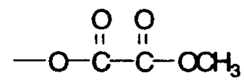 |
| $OCH_3$ | $OCH_3$ | $CH_2CH(CH_3)_2$ | 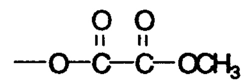 |
| $OCH_3$ | $OCH_3$ | $C(CH_3)_3$ | 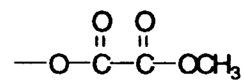 |
| $OCH_3$ | $OCH_3$ | $C_2H_5$ | 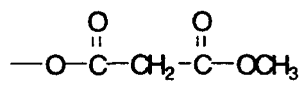 |
| $OCH_3$ | $OCH_3$ | $CH_2CH_2CH_3$ | 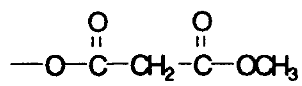 |
| $OCH_3$ | $OCH_3$ | $CH(CH_3)_2$ | 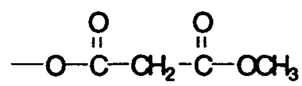 |
| $OCH_3$ | $OCH_3$ | $CH_2CH_2CH_2CH_3$ | 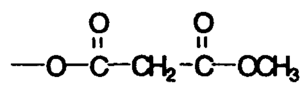 |
| $OCH_3$ | $OCH_3$ | $CH(CH_3)CH_2CH_3$ | 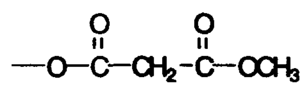 |
| $OCH_3$ | $OCH_3$ | $CH_2CH(CH_3)_2$ | 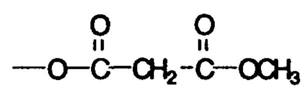 |
| $OCH_3$ | $OCH_3$ | $C(CH_3)_3$ | 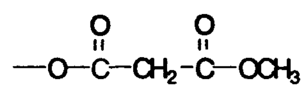 |
| $OCH_3$ | $OCH_3$ | $C_2H_5$ | 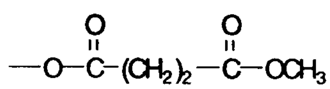 |
| $OCH_3$ | $OCH_3$ | $CH_2CH_2CH_3$ | 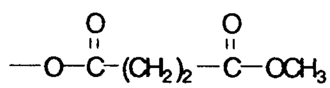 |
| $OCH_3$ | $OCH_3$ | $CH(CH_3)_2$ | 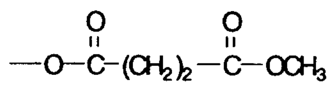 |

Table 1 (continued)

| R1 | R2 | R3 | R4 |
|---|---|---|---|
| $OCH_3$ | $OCH_3$ | $CH_2CH_2CH_2CH_3$ | 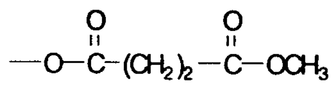 |
| $OCH_3$ | $OCH_3$ | $CH(CH_3)CH_2CH_3$ | 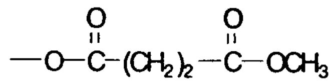 |
| $OCH_3$ | $OCH_3$ | $CH_2CH(CH_3)_2$ | 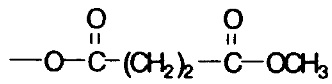 |
| $OCH_3$ | $OCH_3$ | $C(CH_3)_3$ | 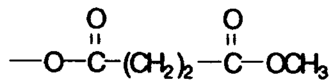 |
| $OCH_3$ | $OCH_3$ | $C_2H_5$ | 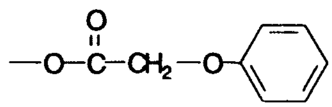 |
| $OCH_3$ | $OCH_3$ | $CH_2CH_2CH_3$ | 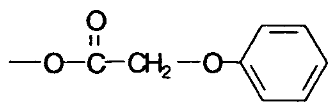 |
| $OCH_3$ | $OCH_3$ | $CH(CH_3)_2$ | 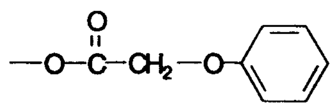 |
| $OCH_3$ | $OCH_3$ | $CH_2CH_2CH_2CH_3$ | 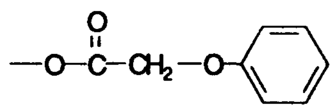 |
| $OCH_3$ | $OCH_3$ | $CH(CH_3)CH_2CH_3$ | 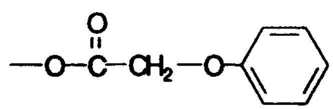 |
| $OCH_3$ | $OCH_3$ | $CH_2CH(CH_3)_2$ | 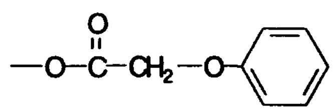 |
| $OCH_3$ | $OCH_3$ | $C(CH_3)_3$ | 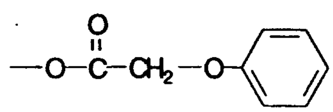 |
| OCH3 | OCH3 | $C_2H_5$ | 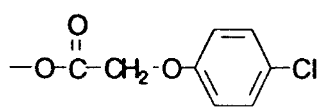 |
| OCH3 | OCH3 | $CH_2CH_2CH_3$ | 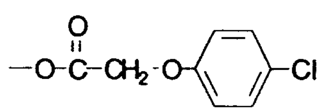 |

Table 1 (continued)

| R1 | R2 | R3 | R4 |
|---|---|---|---|
| $OCH_3$ | $OCH_3$ | $CH(CH_3)_2$ | 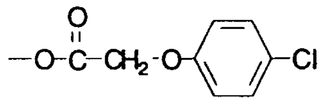 |
| $OCH_3$ | $OCH_3$ | $CH_2CH_2CH_2CH_3$ | 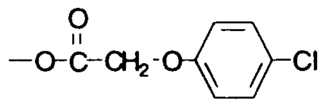 |
| $OCH_3$ | $OCH_3$ | $CH(CH_3)CH_2CH_3$ | 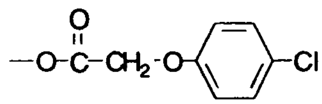 |
| $OCH_3$ | $OCH_3$ | $CH_2CH(CH_3)_2$ | 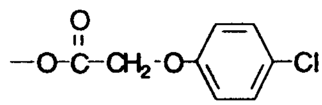 |
| $OCH_3$ | $OCH_3$ | $C(CH_3)_3$ | 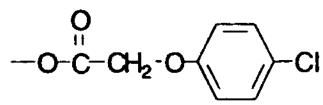 |
| $OCH_3$ | $OCH_3$ | $C_2H_5$ | 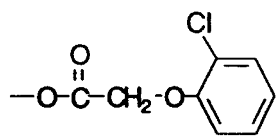 |
| $OCH_3$ | $OCH_3$ | $CH_2CH_2CH_3$ | 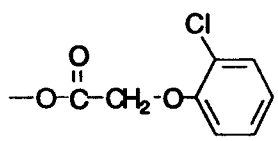 |
| $OCH_3$ | $OCH_3$ | $CH(CH_3)_2$ | 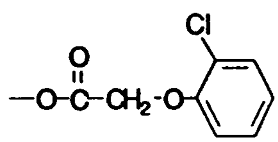 |
| $OCH_3$ | $OCH_3$ | $CH_2CH_2CH_2CH_3$ | 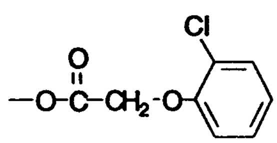 |
| $OCH_3$ | $OCH_3$ | $CH(CH_3)CH_2CH_3$ | 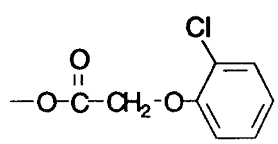 |
| $OCH_3$ | $OCH_3$ | $CH_2CH(CH_3)_2$ | 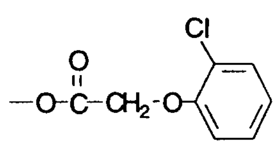 |

Table 1 (continued)

| R1 | R2 | R3 | R4 |
|---|---|---|---|
| $OCH_3$ | $OCH_3$ | $C(CH_3)_3$ | 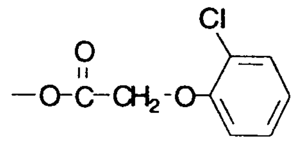 |
| $OCH_3$ | $OCH_3$ | $C_2H_5$ | 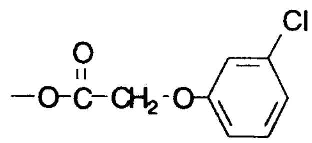 |
| $OCH_3$ | $OCH_3$ | $CH_2CH_2CH_3$ | 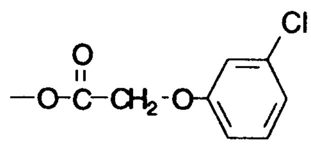 |
| $OCH_3$ | $OCH_3$ | $CH(CH_3)_2$ | 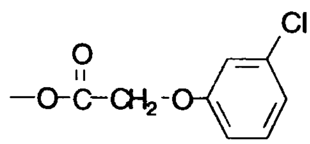 |
| $OCH_3$ | $OCH_3$ | $CH_2CH_2CH_2CH_3$ | 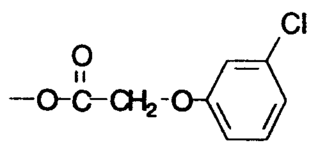 |
| $OCH_3$ | $OCH_3$ | $CH(CH_3)CH_2CH_3$ | 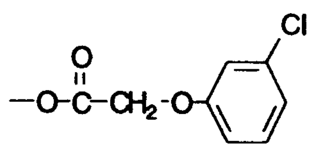 |
| $OCH_3$ | $OCH_3$ | $CH_2CH(CH_3)_2$ | 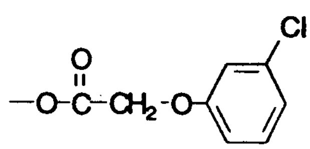 |
| $OCH_3$ | $OCH_3$ | $C(CH_3)_3$ | 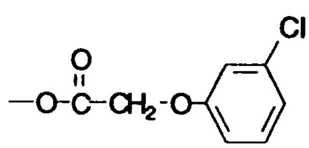 |
| $OCH_3$ | $OCH_3$ | $C_2H_5$ | 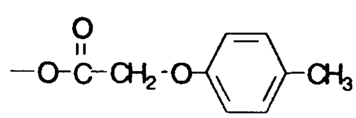 |
| $OCH_3$ | $OCH_3$ | $CH_2CH_2CH_3$ | 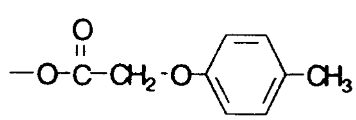 |

Table 1 (continued)

| R1 | R2 | R3 | R4 |
| --- | --- | --- | --- |
| $OCH_3$ | $OCH_3$ | $CH(CH_3)_2$ | 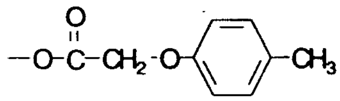 |
| $OCH_3$ | $OCH_3$ | $CH_2CH_2CH_2CH_3$ | 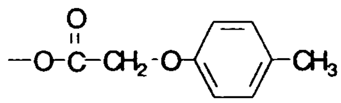 |
| $OCH_3$ | $OCH_3$ | $CH(CH_3)CH_2CH_3$ | 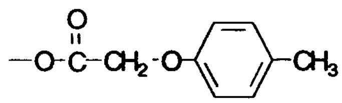 |
| $OCH_3$ | $OCH_3$ | $CH_2CH(CH_3)_2$ | 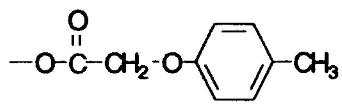 |
| $OCH_3$ | $OCH_3$ | $C(CH_3)_3$ | 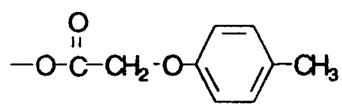 |
| $OCH_3$ | $OCH_3$ | $C_2H_5$ | 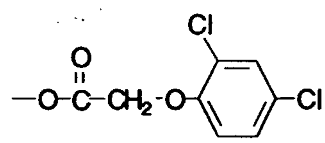 |
| $OCH_3$ | $OCH_3$ | $CH_2CH_2CH_3$ | 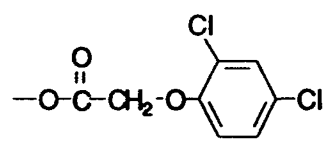 |
| $OCH_3$ | $OCH_3$ | $CH(CH_3)_2$ | 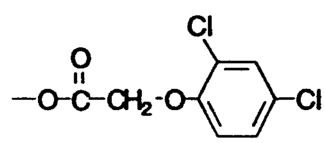 |
| $OCH_3$ | $OCH_3$ | $CH_2CH_2CH_2CH_3$ | 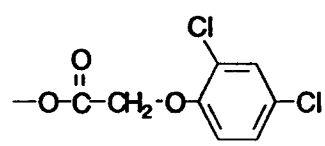 |
| $OCH_3$ | $OCH_3$ | $CH(CH_3)CH_2CH_3$ | 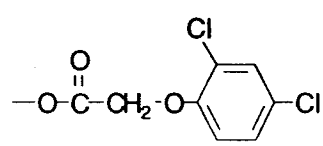 |
| $OCH_3$ | $OCH_3$ | $CH_2CH(CH_3)_2$ | 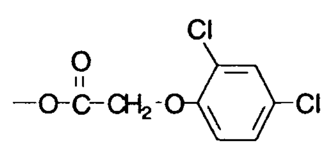 |

Table 1 (continued)

| R1 | R2 | R3 | R4 |
|---|---|---|---|
| $OCH_3$ | $OCH_3$ | $C(CH_3)_3$ | 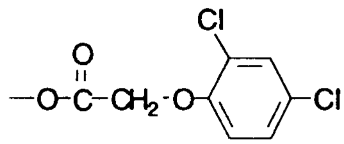 |
| $OCH_3$ | $OCH_3$ | $C_2H_5$ | 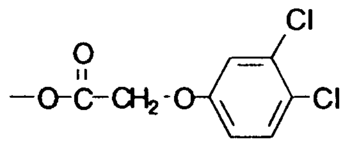 |
| $OCH_3$ | $OCH_3$ | $CH_2CH_2CH_3$ | 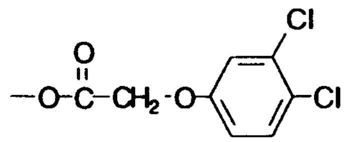 |
| $OCH_3$ | $OCH_3$ | $CH(CH_3)_2$ | 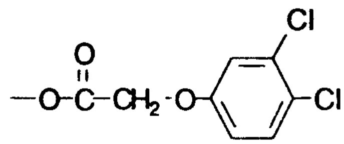 |
| $OCH_3$ | $OCH_3$ | $CH_2CH_2CH_2CH_3$ | 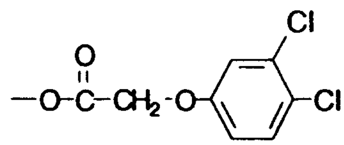 |
| $OCH_3$ | $OCH_3$ | $CH(CH_3)CH_2CH_3$ | 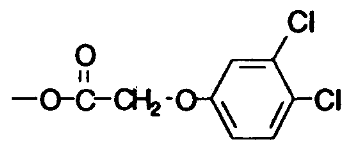 |
| $OCH_3$ | $OCH_3$ | $CH_2CH(CH_3)_2$ | 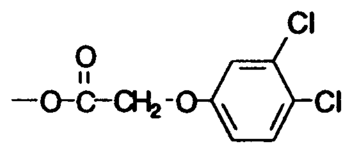 |
| $OCH_3$ | $OCH_3$ | $C(CH_3)_3$ | 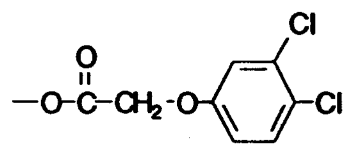 |
| $OCH_3$ | $OCH_3$ | $C_2H_5$ | 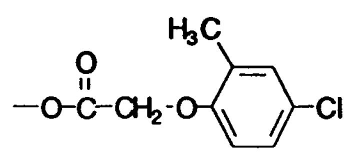 |
| $OCH_3$ | $OCH_3$ | $CH_2CH_2CH_3$ | 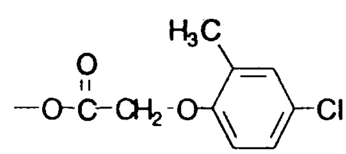 |

Table 1 (continued)

| R1 | R2 | R3 | R4 |
|---|---|---|---|
| $OCH_3$ | $OCH_3$ | $CH(CH_3)_2$ | 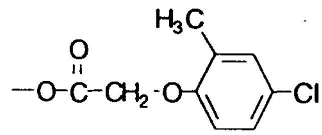 |
| $OCH_3$ | $OCH_3$ | $CH_2CH_2CH_2CH_3$ | 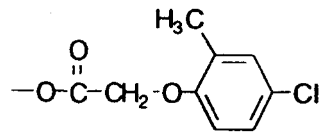 |
| $OCH_3$ | $OCH_3$ | $CH(CH_3)CH_2CH_3$ | 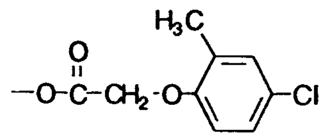 |
| $OCH_3$ | $OCH_3$ | $CH_2CH(CH_3)_2$ | 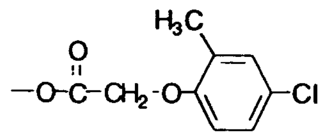 |
| $OCH_3$ | $OCH_3$ | $C(CH_3)_3$ | 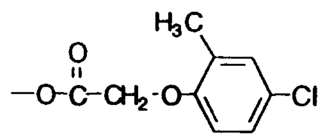 |
| $OCH_3$ | $OCH_3$ | $C_2H_5$ | 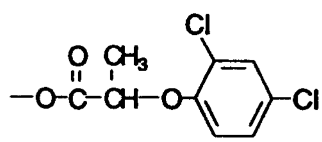 |
| $OCH_3$ | $OCH_3$ | $CH_2CH_2CH_3$ | 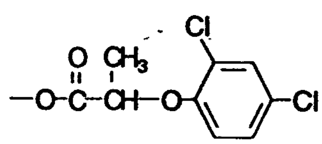 |
| $OCH_3$ | $OCH_3$ | $CH(CH_3)_2$ | 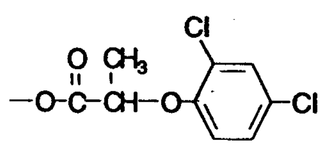 |
| $OCH_3$ | $OCH_3$ | $CH_2CH_2CH_2CH_3$ | 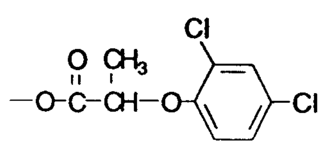 |
| $OCH_3$ | $OCH_3$ | $CH(CH_3)CH_2CH_3$ | 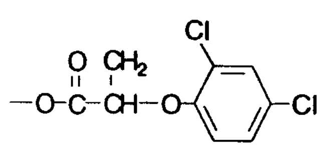 |

Table 1 (continued)

| R1 | R2 | R3 | R4 |
|---|---|---|---|
| $OCH_3$ | $OCH_3$ | $CH_2CH(CH_3)_2$ | 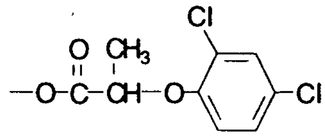 |
| $OCH_3$ | $OCH_3$ | $C(CH_3)_3$ | 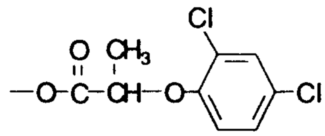 |
| $OCH_3$ | $OCH_3$ | $C_2H_5$ | 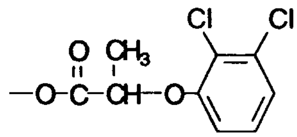 |
| $OCH_3$ | $OCH_3$ | $CH_2CH_2CH_3$ | 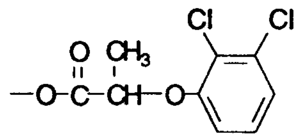 |
| $OCH_3$ | $OCH_3$ | $CH(CH_3)_2$ | 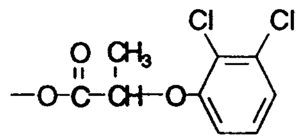 |
| $OCH_3$ | $OCH_3$ | $CH_2CH_2CH_2CH_3$ | 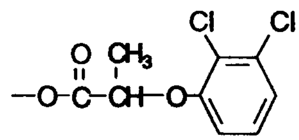 |
| $OCH_3$ | $OCH_3$ | $CH(CH_3)CH_2CH_3$ | 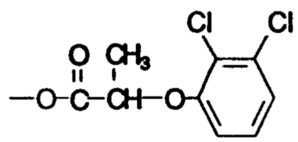 |
| $OCH_3$ | $OCH_3$ | $CH_2CH(CH_3)_2$ | 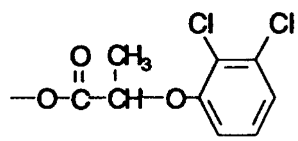 |
| $OCH_3$ | $OCH_3$ | $C(CH_3)_3$ | 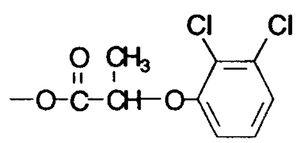 |
| $OCH_3$ | $OCH_3$ | $C_2H_5$ | 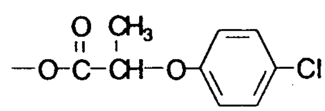 |

Table 1 (continued)

| R1 | R2 | R3 | R4 |
|---|---|---|---|
| $OCH_3$ | $OCH_3$ | $CH_2CH_2CH_3$ | 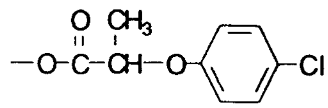 |
| $OCH_3$ | $OCH_3$ | $CH(CH_3)_2$ | 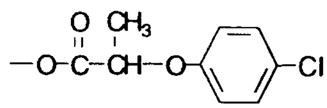 |
| $OCH_3$ | $OCH_3$ | $CH_2CH_2CH_2CH_3$ | 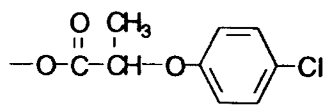 |
| $OCH_3$ | $OCH_3$ | $CH(CH_3)CH_2CH_3$ | 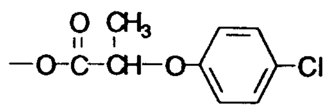 |
| $OCH_3$ | $OCH_3$ | $CH_2CH(CH_3)_2$ | 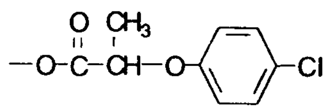 |
| $OCH_3$ | $OCH_3$ | $C(CH_3)_3$ | 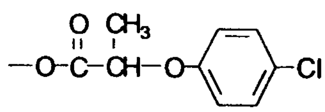 |
| $OCH_3$ | $OCH_3$ | $C_2H_5$ | 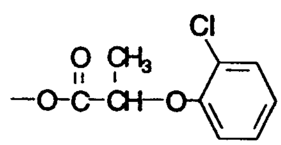 |
| $OCH_3$ | $OCH_3$ | $CH_2CH_2CH_3$ | 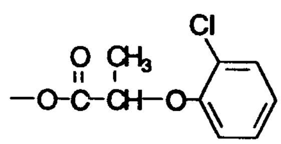 |
| $OCH_3$ | $OCH_3$ | $CH(CH_3)_2$ | 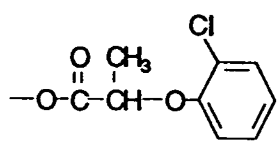 |
| $OCH_3$ | $OCH_3$ | $CH_2CH_2CH_2CH_3$ | 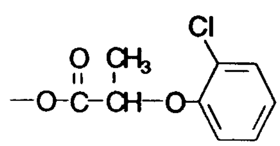 |
| $OCH_3$ | $OCH_3$ | $CH(CH_3)CH_2CH_3$ | 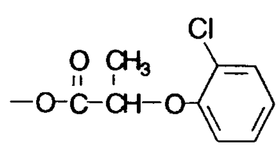 |

Table 1 (continued)

| R1 | R2 | R3 | R4 |
|---|---|---|---|
| $OCH_3$ | $OCH_3$ | $CH_2CH(CH_3)_2$ | 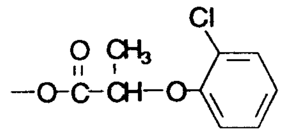 |
| $OCH_3$ | $OCH_3$ | $C(CH_3)_3$ | 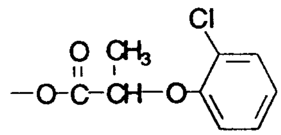 |
| $OCH_3$ | $OCH_3$ | $C_2H_5$ | 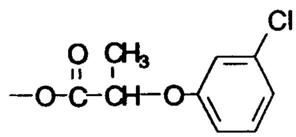 |
| $OCH_3$ | $OCH_3$ | $CH_2CH_2CH_3$ | 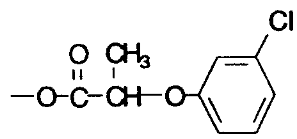 |
| $OCH_3$ | $OCH_3$ | $CH(CH_3)_2$ | 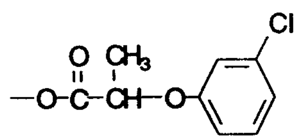 |
| $OCH_3$ | $OCH_3$ | $CH_2CH_2CH_2CH_3$ | 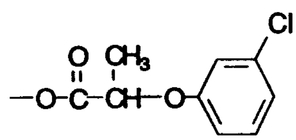 |
| $OCH_3$ | $OCH_3$ | $CH(CH_3)CH_2CH_3$ | 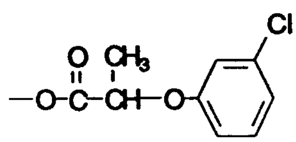 |
| $OCH_3$ | $OCH_3$ | $CH_2CH(CH_3)_2$ | 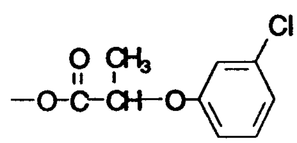 |
| $OCH_3$ | $OCH_3$ | $C(CH_3)_3$ | 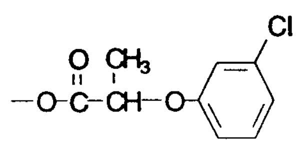 |
| $OCH_3$ | $OCH_3$ | $C_2H_5$ | 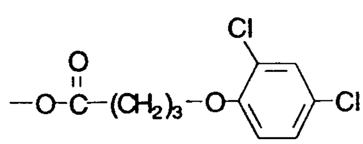 |

Table 1 (continued)

| R1 | R2 | R3 | R4 |
|---|---|---|---|
| $OCH_3$ | $OCH_3$ | $CH_2CH_2CH_3$ | 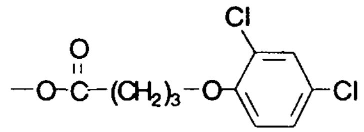 |
| $OCH_3$ | $OCH_3$ | $CH(CH_3)_2$ | 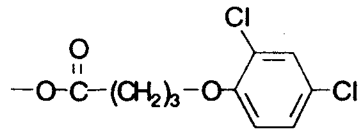 |
| $OCH_3$ | $OCH_3$ | $CH_2CH_2CH_2CH_3$ | 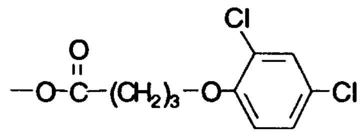 |
| $OCH_3$ | $OCH_3$ | $CH(CH_3)CH_2CH_3$ | 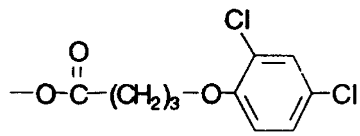 |
| $OCH_3$ | $OCH_3$ | $CH_2CH(CH_3)_2$ | 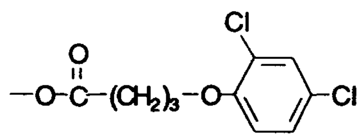 |
| $OCH_3$ | $OCH_3$ | $C(CH_3)_3$ | 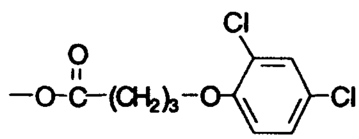 |
| $OCH_3$ | $OCH_3$ | $C_2H_5$ | 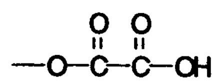 |
| $OCH_3$ | $OCH_3$ | $CH_2CH_2CH_3$ | 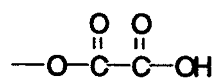 |
| $OCH_3$ | $OCH_3$ | $CH(CH_3)_2$ | 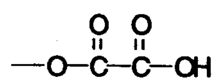 |
| $OCH_3$ | $OCH_3$ | $CH_2CH_2CH_2CH_3$ | 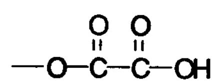 |
| $OCH_3$ | $OCH_3$ | $CH(CH_3)CH_2CH_3$ | 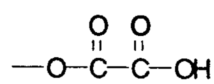 |
| $OCH_3$ | $OCH_3$ | $CH_2CH(CH_3)_2$ | 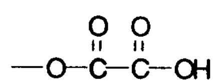 |
| $OCH_3$ | $OCH_3$ | $C(CH_3)_3$ | 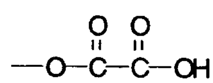 |

Table 1 (continued)

| R1 | R2 | R3 | R4 |
|---|---|---|---|
| $OCH_3$ | $OCH_3$ | $C_2H_5$ | 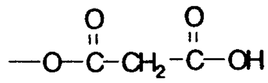 |
| $OCH_3$ | $OCH_3$ | $CH_2CH_2CH_3$ | 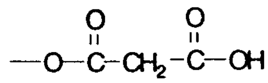 |
| $OCH_3$ | $OCH_3$ | $CH(CH_3)_2$ | 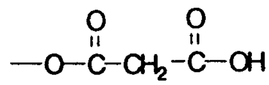 |
| $OCH_3$ | $OCH_3$ | $CH_2CH_2CH_2CH_3$ | 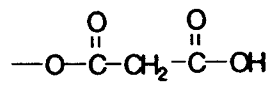 |
| $OCH_3$ | $OCH_3$ | $CH(CH_3)CH_2CH_3$ | 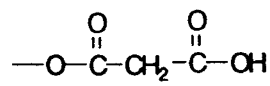 |
| $OCH_3$ | $OCH_3$ | $CH_2CH(CH_3)_2$ | 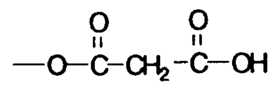 |
| $OCH_3$ | $OCH_3$ | $C(CH_3)_3$ | 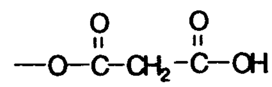 |
| $OCH_3$ | $OCH_3$ | $C_2H_5$ | 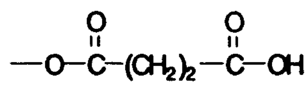 |
| $OCH_3$ | $OCH_3$ | $CH_2CH_2CH_3$ | 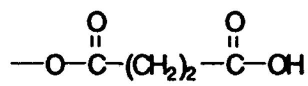 |
| $OCH_3$ | $OCH_3$ | $CH(CH_3)_2$ | 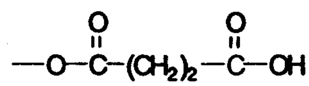 |
| $OCH_3$ | $OCH_3$ | $CH_2CH_2CH_2CH_3$ | 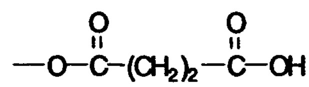 |
| $OCH_3$ | $OCH_3$ | $CH(CH_3)CH_2CH_3$ | 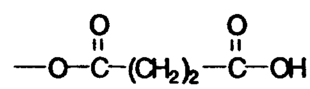 |
| $OCH_3$ | $OCH_3$ | $CH_2CH(CH_3)_2$ | 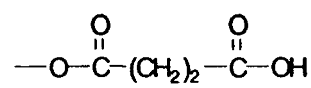 |
| $OCH_3$ | $OCH_3$ | $C(CH_3)_3$ | 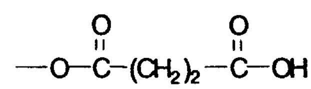 |
| $OCH_3$ | $OCH_3$ | $C_2H_5$ | 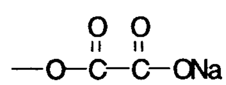 |

Table 1 (continued)

| R1 | R2 | R3 | R4 |
|---|---|---|---|
| $OCH_3$ | $OCH_3$ | $CH_2CH_2CH_3$ | 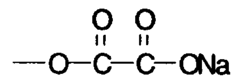 |
| $OCH_3$ | $OCH_3$ | $CH(CH_3)_2$ | 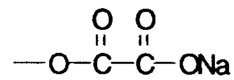 |
| $OCH_3$ | $OCH_3$ | $CH_2CH_2CH_2CH_3$ | 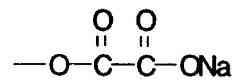 |
| $OCH_3$ | $OCH_3$ | $CH(CH_3)CH_2CH_3$ | 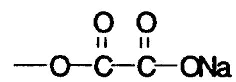 |
| $OCH_3$ | $OCH_3$ | $CH_2CH(CH_3)_2$ | 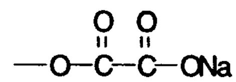 |
| $OCH_3$ | $OCH_3$ | $C(CH_3)_3$ | 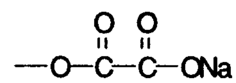 |
| $OCH_3$ | $OCH_3$ | $C_2H_5$ | 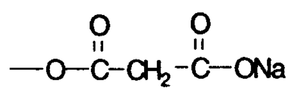 |
| $OCH_3$ | $OCH_3$ | $CH_2CH_2CH_3$ | 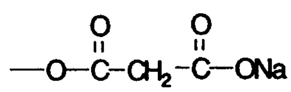 |
| $OCH_3$ | $OCH_3$ | $CH(CH_3)_2$ | 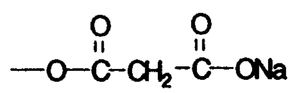 |
| $OCH_3$ | $OCH_3$ | $CH_2CH_2CH_2CH_3$ | 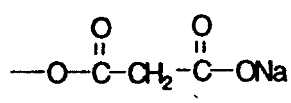 |
| $OCH_3$ | $OCH_3$ | $CH(CH_3)CH_2CH_3$ | 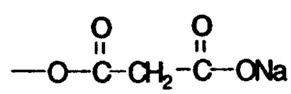 |
| $OCH_3$ | $OCH_3$ | $CH_2CH(CH_3)_2$ | 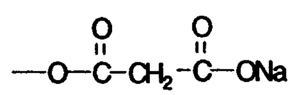 |
| $OCH_3$ | $OCH_3$ | $C(CH_3)_3$ | 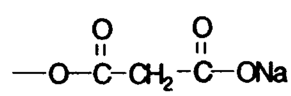 |
| $OCH_3$ | $OCH_3$ | $C_2H_5$ | 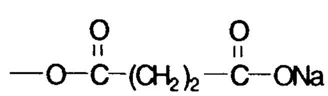 |
| $OCH_3$ | $OCH_3$ | $CH_2CH_2CH_3$ | 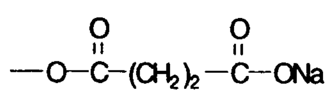 |

Table 1 (continued)

| R1 | R2 | R3 | R4 |
|---|---|---|---|
| $OCH_3$ | $OCH_3$ | $CH(CH_3)_2$ | 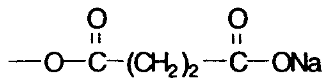 |
| $OCH_3$ | $OCH_3$ | $CH_2CH_2CH_2CH_3$ | 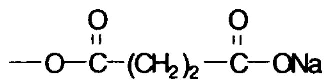 |
| $OCH_3$ | $OCH_3$ | $CH(CH_3)CH_2CH_3$ | 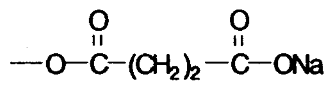 |
| $OCH_3$ | $OCH_3$ | $CH_2CH(CH_3)_2$ | 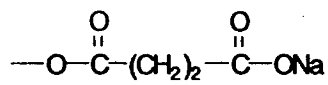 |
| $OCH_3$ | $OCH_3$ | $C(CH_3)_3$ | 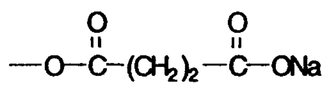 |
| $OCH_3$ | $OCH_3$ | $C_2H_5$ | 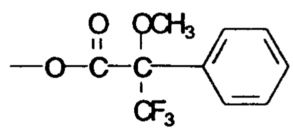 |
| $OCH_3$ | $OCH_3$ | $CH_2CH_2CH_3$ | 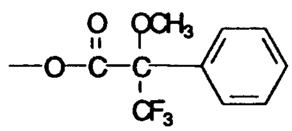 |
| $OCH_3$ | $OCH_3$ | $CH(CH_3)_2$ | 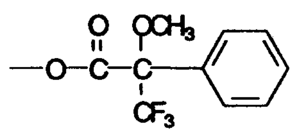 |
| $OCH_3$ | $OCH_3$ | $CH_2CH_2CH_2CH_3$ | 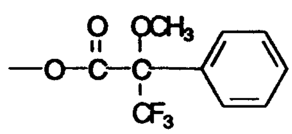 |
| $OCH_3$ | $OCH_3$ | $CH(CH_3)CH_2CH_3$ | 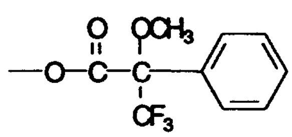 |
| $OCH_3$ | $OCH_3$ | $CH_2CH(CH_3)_2$ | 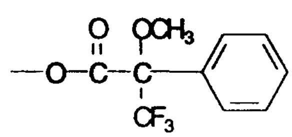 |
| $OCH_3$ | $OCH_3$ | $C(CH_3)_3$ | 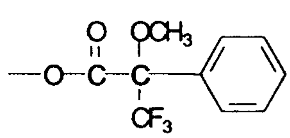 |

Table 1 (continued)

| R1 | R2 | R3 | R4 |
|---|---|---|---|
| $OCH_3$ | $OCH_3$ | $C_2H_5$ | 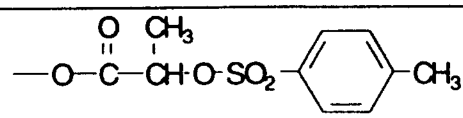 |
| $OCH_3$ | $OCH_3$ | $CH_2CH_2CH_3$ | 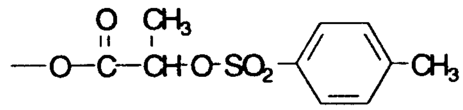 |
| $OCH_3$ | $OCH_3$ | $CH(CH_3)_2$ | 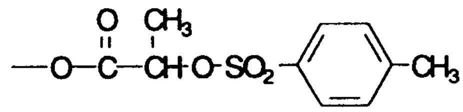 |
| $OCH_3$ | $OCH_3$ | $CH_2CH_2CH_2CH_3$ | 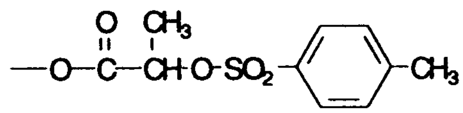 |
| $OCH_3$ | $OCH_3$ | $CH(CH_3)CH_2CH_3$ | 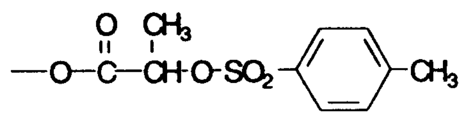 |
| $OCH_3$ | $OCH_3$ | $CH_2CH(CH_3)_2$ | 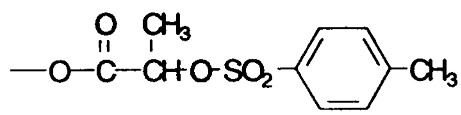 |
| $OCH_3$ | $OCH_3$ | $C(CH_3)_3$ | 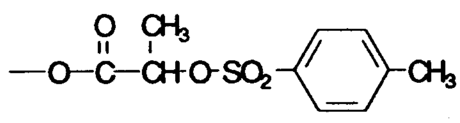 |
| $OCH_3$ | $OCH_3$ | $C_2H_5$ | 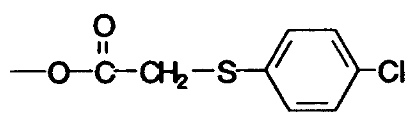 |
| $OCH_3$ | $OCH_3$ | $CH_2CH_2CH_3$ | 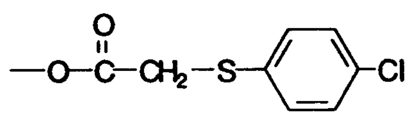 |
| $OCH_3$ | $OCH_3$ | $CH(CH_3)_2$ | 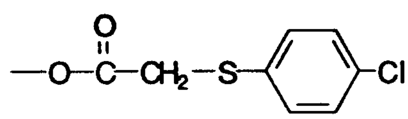 |
| $OCH_3$ | $OCH_3$ | $CH_2CH_2CH_2CH_3$ | 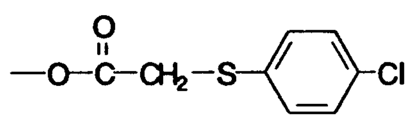 |
| $OCH_3$ | $OCH_3$ | $CH(CH_3)CH_2CH_3$ | 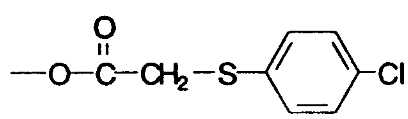 |
| $OCH_3$ | $OCH_3$ | $CH_2CH(CH_3)_2$ | 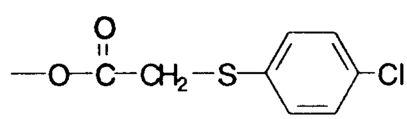 |

Table 1 (continued)

| R1 | R2 | R3 | R4 |
|---|---|---|---|
| $OCH_3$ | $OCH_3$ | $C(CH_3)_3$ | 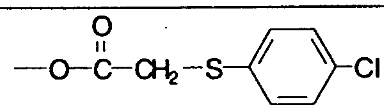 |
| $OCH_3$ | $OCH_3$ | $C_2H_5$ | 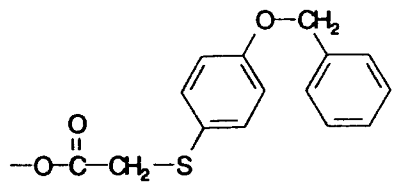 |
| $OCH_3$ | $OCH_3$ | $CH_2CH_2CH_3$ | 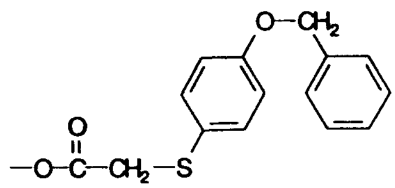 |
| $OCH_3$ | $OCH_3$ | $CH(CH_3)_2$ | 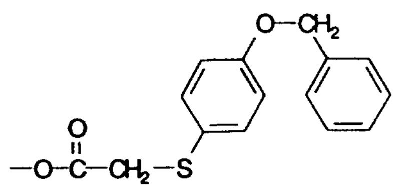 |
| $OCH_3$ | $OCH_3$ | $CH_2CH_2CH_2CH_3$ | 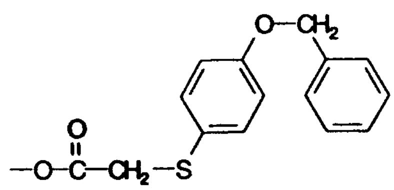 |
| $OCH_3$ | $OCH_3$ | $CH(CH_3)CH_2CH_3$ | 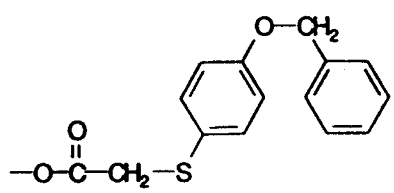 |
| $OCH_3$ | $OCH_3$ | $CH_2CH(CH_3)_2$ | 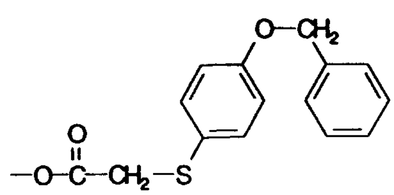 |
| $OCH_3$ | $OCH_3$ | $C(CH_3)_3$ | 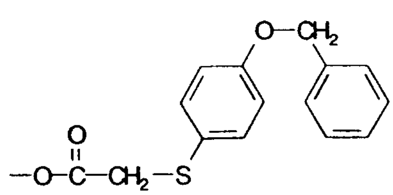 |

Table 1 (continued)

| R1 | R2 | R3 | R4 |
|---|---|---|---|
| $OCH_3$ | $OCH_3$ | $C_2H_5$ | 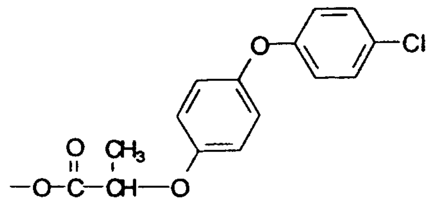 |
| $OCH_3$ | $OCH_3$ | $CH_2CH_2CH_3$ | 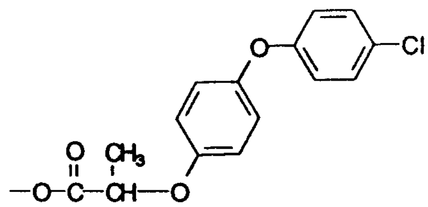 |
| $OCH_3$ | $OCH_3$ | $CH(CH_3)_2$ | 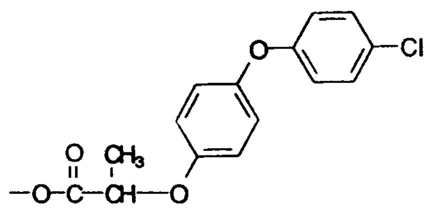 |
| $OCH_3$ | $OCH_3$ | $CH_2CH_2CH_2CH_3$ | 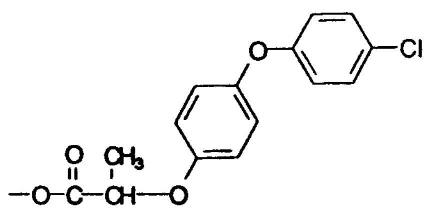 |
| $OCH_3$ | $OCH_3$ | $CH(CH_3)CH_2CH_3$ | 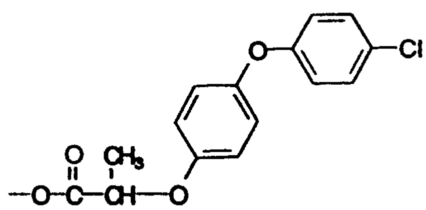 |
| $OCH_3$ | $OCH_3$ | $CH_2CH(CH_3)_2$ | 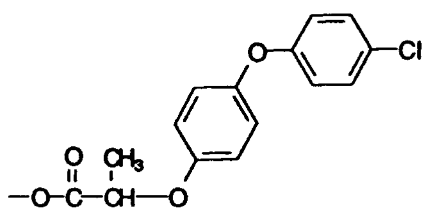 |
| $OCH_3$ | $OCH_3$ | $C(CH_3)_3$ | 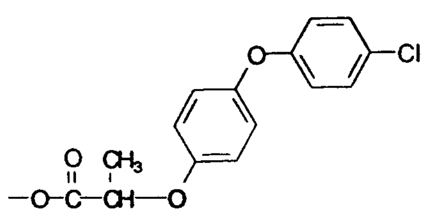 |

Table 1 (continued)

| R1 | R2 | R3 | R4 |
|---|---|---|---|
| OCH3 | OCH3 | C2H5 | 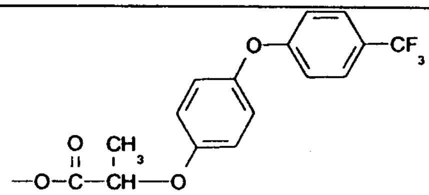 |
| $OCH_3$ | $OCH_3$ | $CH_2CH_2CH_3$ | 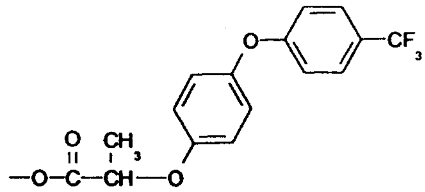 |
| $OCH_3$ | $OCH_3$ | $CH(CH_3)_2$ | 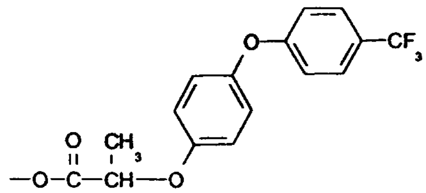 |
| $OCH_3$ | $OCH_3$ | $CH_2CH_2CH_2CH_3$ | 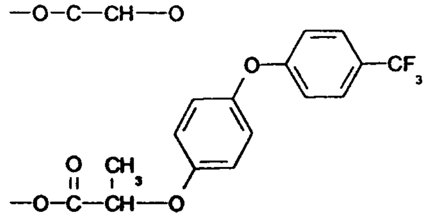 |
| $OCH_3$ | $OCH_3$ | $CH(CH_3)CH_2CH_3$ | 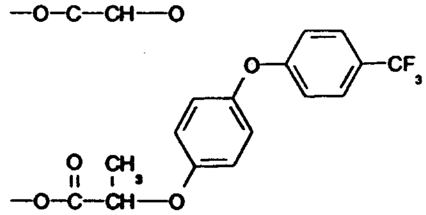 |
| $OCH_3$ | $OCH_3$ | $CH_2CH(CH_3)_2$ | 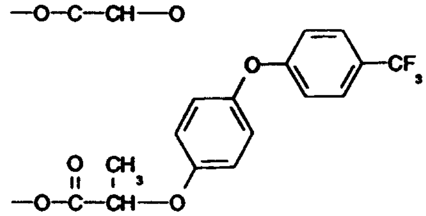 |
| $OCH_3$ | $OCH_3$ | $C(CH_3)_3$ | 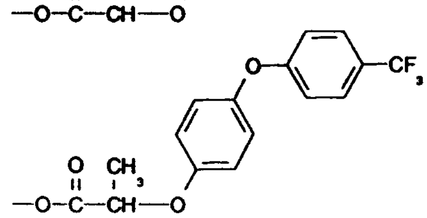 |
| OCH3 | OCH3 | C2H5 | 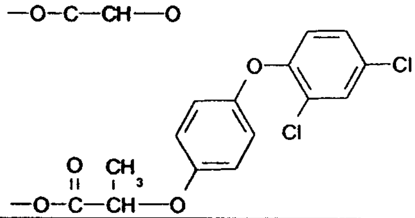 |

Table 1 (continued)

| R1 | R2 | R3 | R4 |
|---|---|---|---|
| $OCH_3$ | $OCH_3$ | $CH_2CH_2CH_3$ | 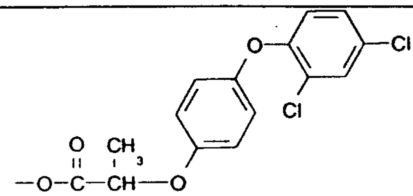 |
| $OCH_3$ | $OCH_3$ | $CH(CH_3)_2$ | 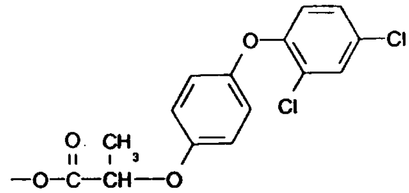 |
| $OCH_3$ | $OCH_3$ | $CH_2CH_2CH_2CH_3$ | 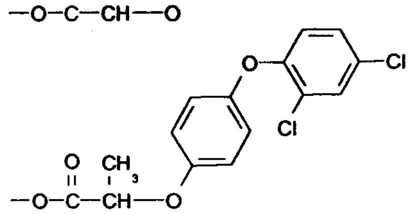 |
| $OCH_3$ | $OCH_3$ | $CH(CH_3)CH_2CH_3$ | 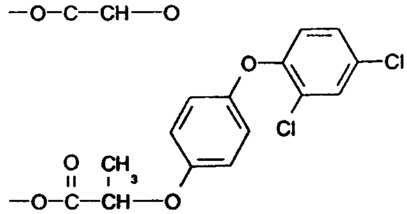 |
| $OCH_3$ | $OCH_3$ | $CH_2CH(CH_3)_2$ | 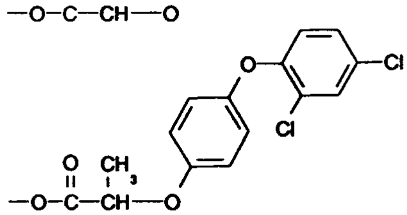 |
| $OCH_3$ | $OCH_3$ | $C(CH_3)_3$ | 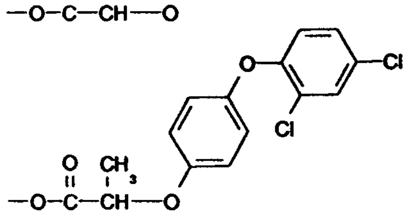 |
| OCH3 | OCH3 | C2H5 | 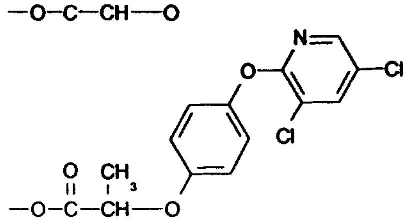 |
| $OCH_3$ | $OCH_3$ | $CH_2CH_2CH_3$ | 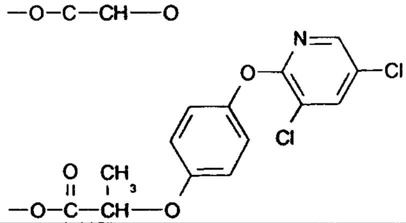 |

Table 1 (continued)

| R1 | R2 | R3 | R4 |
|---|---|---|---|
| $OCH_3$ | $OCH_3$ | $CH(CH_3)_2$ | 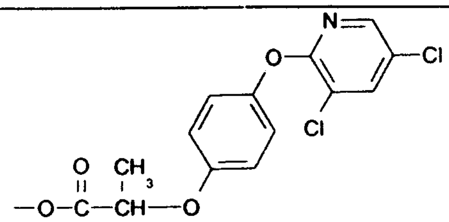 |
| $OCH_3$ | $OCH_3$ | $CH_2CH_2CH_2CH_3$ | 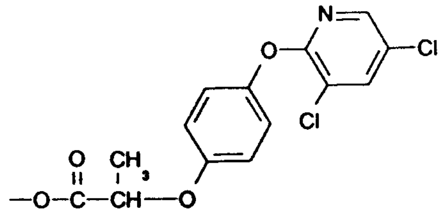 |
| $OCH_3$ | $OCH_3$ | $CH(CH_3)CH_2CH_3$ | 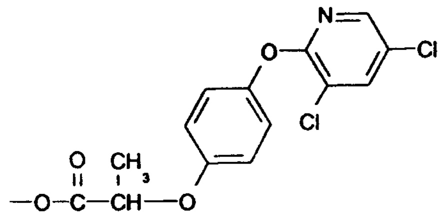 |
| $OCH_3$ | $OCH_3$ | $CH_2CH(CH_3)_2$ | 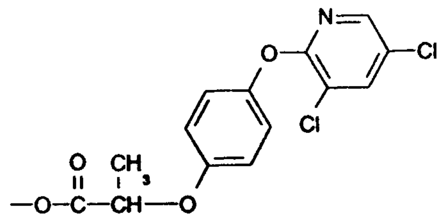 |
| $OCH_3$ | $OCH_3$ | $C(CH_3)_3$ | 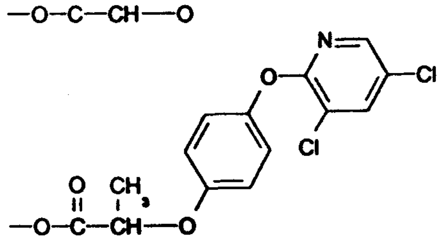 |
| $OCH_3$ | $OCH_3$ | $C_2H_5$ | 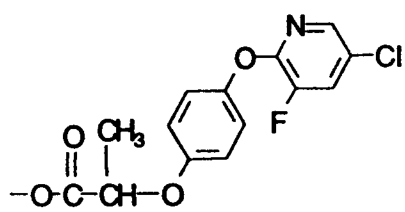 |
| $OCH_3$ | $OCH_3$ | $CH_2CH_2CH_3$ | 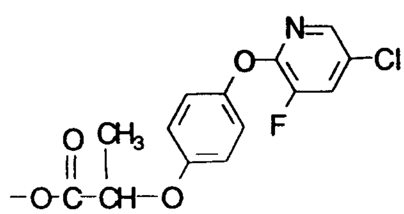 |

Table 1 (continued)

| R1 | R2 | R3 | R4 |
|---|---|---|---|
| $OCH_3$ | $OCH_3$ | $CH(CH_3)_2$ | 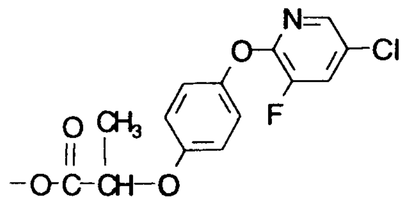 |
| $OCH_3$ | $OCH_3$ | $CH_2CH_2CH_2CH_3$ | 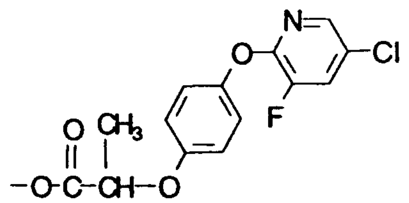 |
| $OCH_3$ | $OCH_3$ | $CH(CH_3)CH_2CH_3$ | 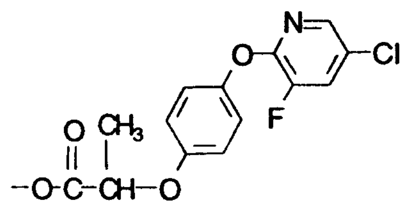 |
| $OCH_3$ | $OCH_3$ | $CH_2CH(CH_3)_2$ | 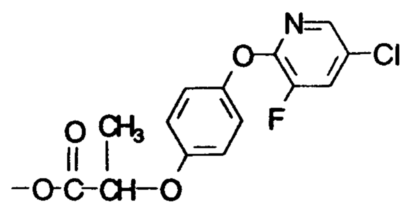 |
| $OCH_3$ | $OCH_3$ | $C(CH_3)_3$ | 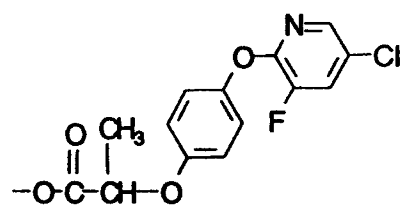 |
| $OCH_3$ | $OCH_3$ | $C_2H_5$ | 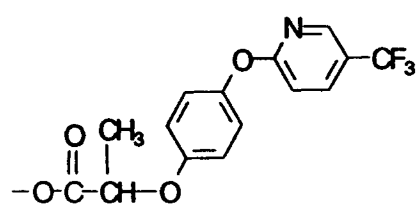 |
| $OCH_3$ | $OCH_3$ | $CH_2CH_2CH_3$ | 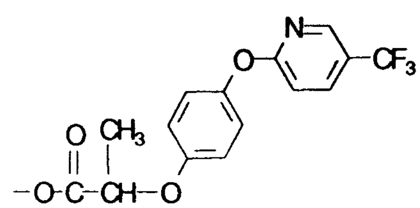 |

Table 1 (continued)

| R1 | R2 | R3 | R4 |
|---|---|---|---|
| $OCH_3$ | $OCH_3$ | $CH(CH_3)_2$ | 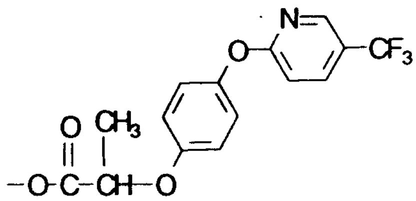 |
| $OCH_3$ | $OCH_3$ | $CH_2CH_2CH_2CH_3$ | 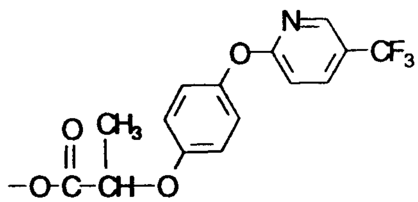 |
| $OCH_3$ | $OCH_3$ | $CH(CH_3)CH_2CH_3$ | 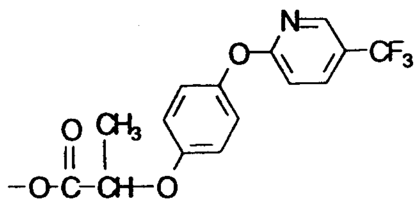 |
| $OCH_3$ | $OCH_3$ | $CH_2CH(CH_3)_2$ | 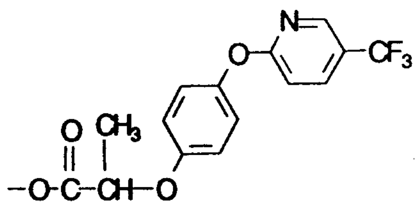 |
| $OCH_3$ | $OCH_3$ | $C(CH_3)_3$ | 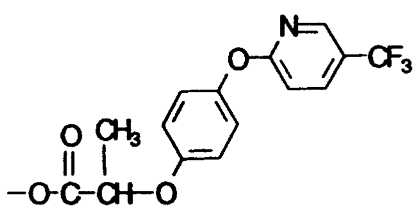 |
| $OCH_3$ | $OCH_3$ | $C_2H_5$ | 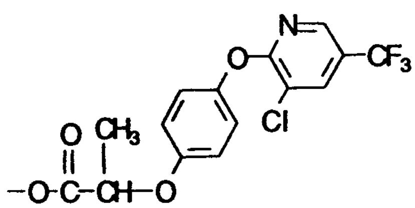 |
| $OCH_3$ | $OCH_3$ | $CH_2CH_2CH_3$ | 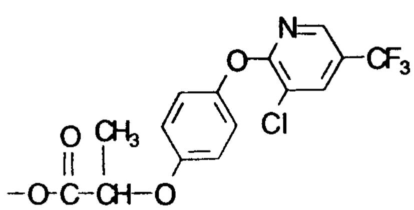 |

Table 1 (continued)

| R1 | R2 | R3 | R4 |
|---|---|---|---|
| $OCH_3$ | $OCH_3$ | $CH(CH_3)_2$ | 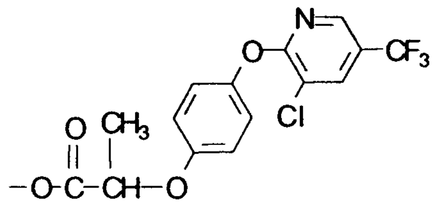 |
| $OCH_3$ | $OCH_3$ | $CH_2CH_2CH_2CH_3$ | 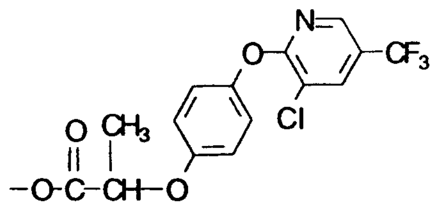 |
| $OCH_3$ | $OCH_3$ | $CH(CH_3)CH_2CH_3$ | 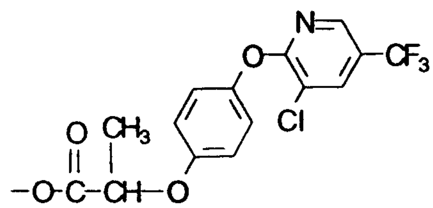 |
| $OCH_3$ | $OCH_3$ | $CH_2CH(CH_3)_2$ | 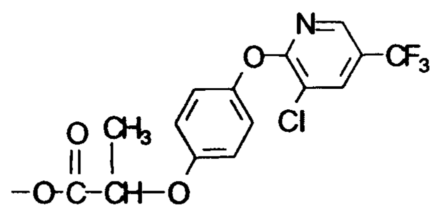 |
| $OCH_3$ | $OCH_3$ | $C(CH_3)_3$ | 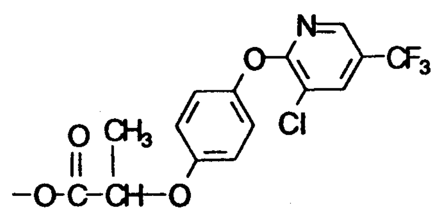 |
| $OCH_3$ | $OCH_3$ | $C_2H_5$ | 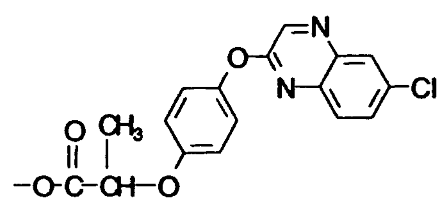 |
| $OCH_3$ | $OCH_3$ | $CH_2CH_2CH_3$ | 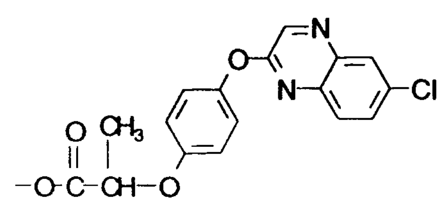 |

Table 1 (continued)

| R1 | R2 | R3 | R4 |
| --- | --- | --- | --- |
| $OCH_3$ | $OCH_3$ | $CH(CH_3)_2$ | 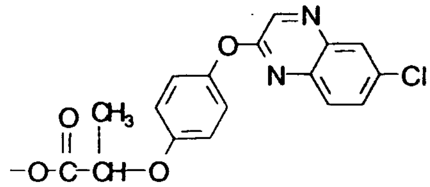 |
| $OCH_3$ | $OCH_3$ | $CH_2CH_2CH_2CH_3$ | 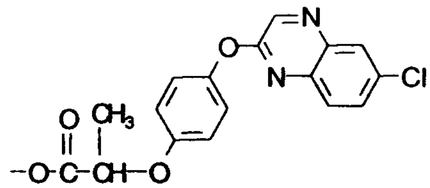 |
| $OCH_3$ | $OCH_3$ | $CH(CH_3)CH_2CH_3$ | 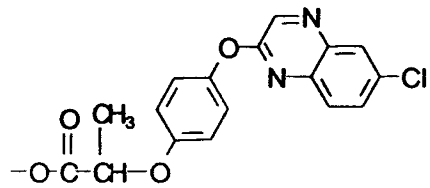 |
| $OCH_3$ | $OCH_3$ | $CH_2CH(CH_3)_2$ | 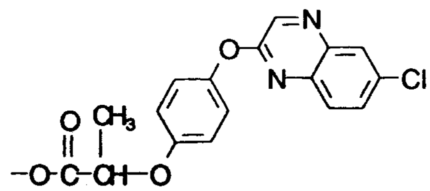 |
| $OCH_3$ | $OCH_3$ | $C(CH_3)_3$ | 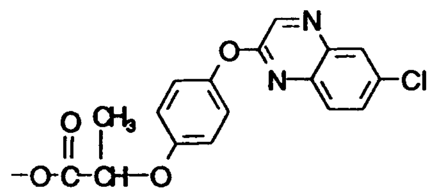 |
| $OCH_3$ | $OCH_3$ | $C_2H_5$ | 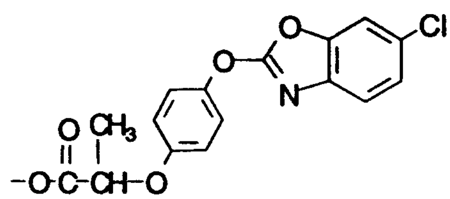 |
| $OCH_3$ | $OCH_3$ | $CH_2CH_2CH_3$ | 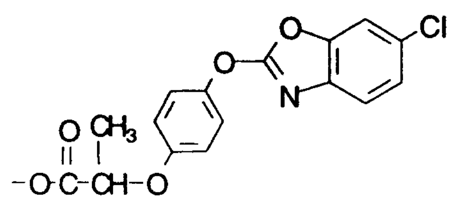 |

Table 1 (continued)

| R1 | R2 | R3 | R4 |
|---|---|---|---|
| $OCH_3$ | $OCH_3$ | $CH(CH_3)_2$ | 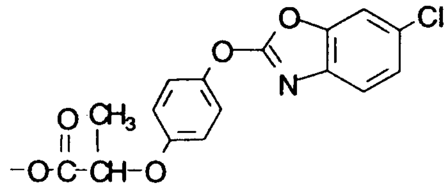 |
| $OCH_3$ | $OCH_3$ | $CH_2CH_2CH_2CH_3$ | 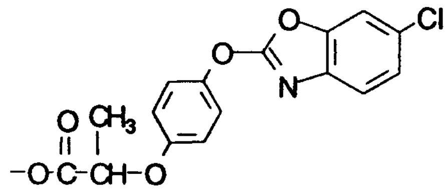 |
| $OCH_3$ | $OCH_3$ | $CH(CH_3)CH_2CH_3$ | 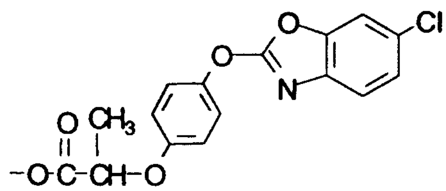 |
| $OCH_3$ | $OCH_3$ | $CH_2CH(CH_3)_2$ | 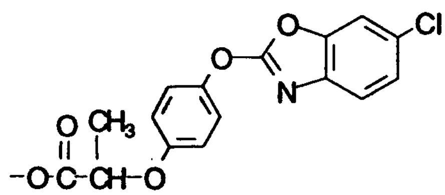 |
| $OCH_3$ | $OCH_3$ | $C(CH_3)_3$ | 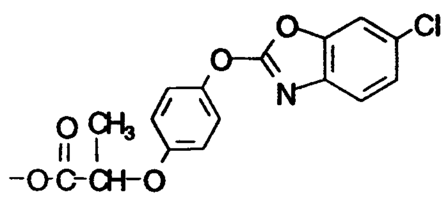 |
| $OCH_3$ | $OCH_3$ | $C_2H_5$ | 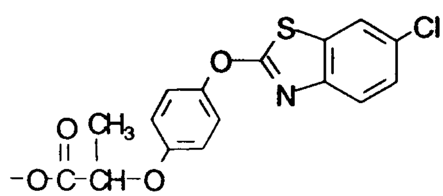 |

Table 1 (continued)

| R1 | R2 | R3 | R4 |
|---|---|---|---|
| $OCH_3$ | $OCH_3$ | $CH_2CH_2CH_3$ | 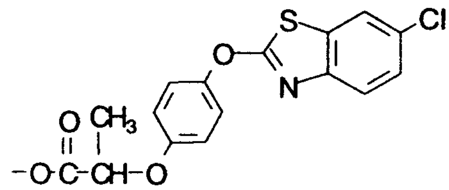 |
| $OCH_3$ | $OCH_3$ | $CH(CH_3)_2$ | 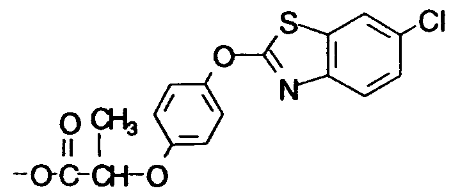 |
| $OCH_3$ | $OCH_3$ | $CH_2CH_2CH_2CH_3$ | 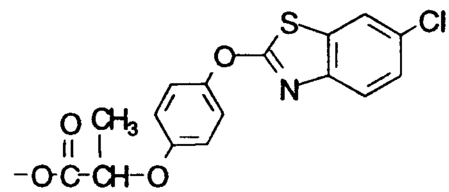 |
| $OCH_3$ | $OCH_3$ | $CH(CH_3)CH_2CH_3$ | 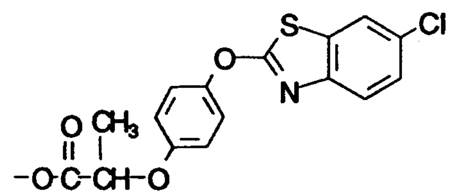 |
| $OCH_3$ | $OCH_3$ | $CH_2CH(CH_3)_2$ | 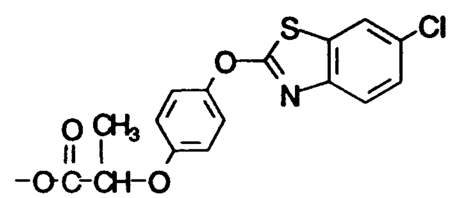 |
| $OCH_3$ | $OCH_3$ | $C(CH_3)_3$ | 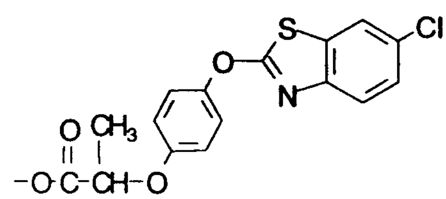 |

Table 1 (continued)

| R1 | R2 | R3 | R4 |
|---|---|---|---|
| $OCH_3$ | $OCH_3$ | $C_2H_5$ | 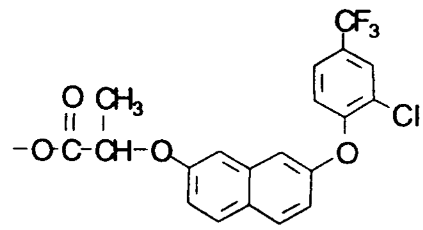 |
| $OCH_3$ | $OCH_3$ | $CH_2CH_2CH_3$ | 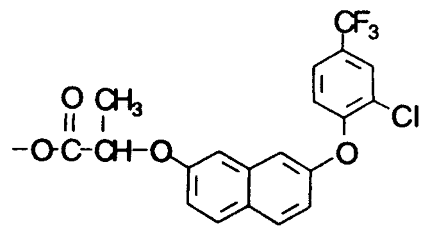 |
| $OCH_3$ | $OCH_3$ | $CH(CH_3)_2$ | 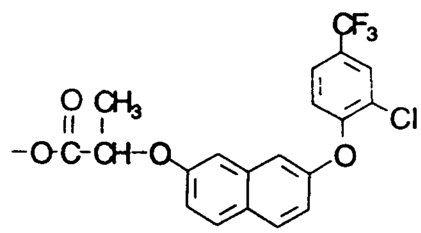 |
| $OCH_3$ | $OCH_3$ | $CH_2CH_2CH_2CH_3$ | 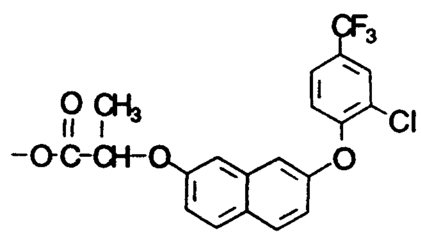 |
| $OCH_3$ | $OCH_3$ | $CH(CH_3)CH_2CH_3$ | 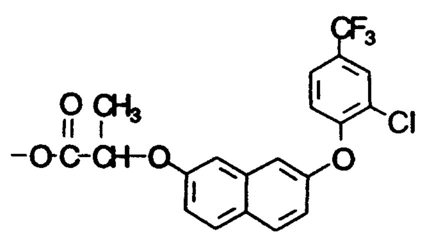 |
| $OCH_3$ | $OCH_3$ | $CH_2CH(CH_3)_2$ | 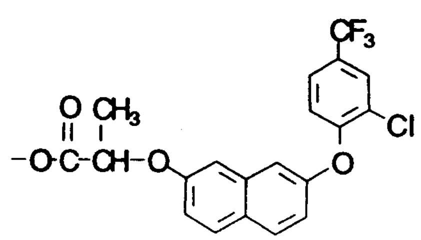 |

Table 1 (continued)

| R1 | R2 | R3 | R4 |
|---|---|---|---|
| $OCH_3$ | $OCH_3$ | $C(CH_3)_3$ | 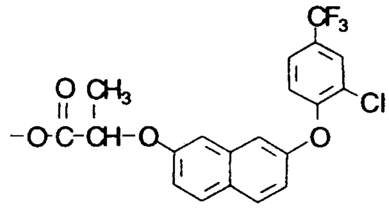 |
| $OCH_3$ | $OCH_3$ | $C_2H_5$ | 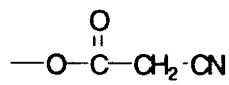 |
| $OCH_3$ | $OCH_3$ | $CH_2CH_2CH_3$ | 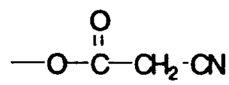 |
| $OCH_3$ | $OCH_3$ | $CH(CH_3)_2$ | 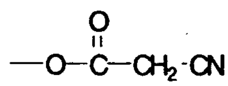 |
| $OCH_3$ | $OCH_3$ | $CH_2CH_2CH_2CH_3$ | 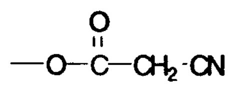 |
| $OCH_3$ | $OCH_3$ | $CH(CH_3)CH_2CH_3$ | 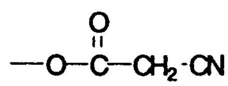 |
| $OCH_3$ | $OCH_3$ | $CH_2CH(CH_3)_2$ | 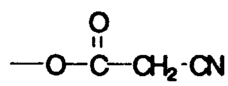 |
| $OCH_3$ | $OCH_3$ | $C(CH_3)_3$ | 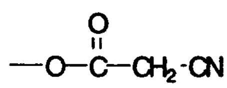 |
| $OCH_3$ | $OCH_3$ | $C_2H_5$ | 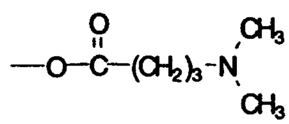 |
| $OCH_3$ | $OCH_3$ | $CH_2CH_2CH_3$ | 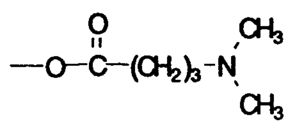 |
| $OCH_3$ | $OCH_3$ | $CH(CH_3)_2$ | 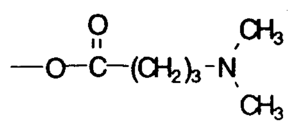 |
| $OCH_3$ | $OCH_3$ | $CH_2CH_2CH_2CH_3$ | 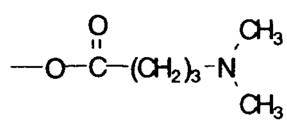 |

Table 1 (continued)

| R1 | R2 | R3 | R4 |
|---|---|---|---|
| $OCH_3$ | $OCH_3$ | $C(CH_3)_3$ | 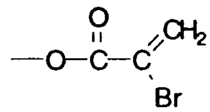 |
| $OCH_3$ | $OCH_3$ | 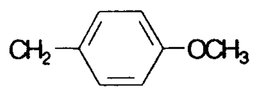 | 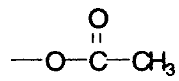 |
| $OCH_3$ | $OCH_3$ | $C(CH_3)_3$ | 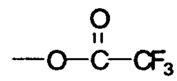 |
| $OCH_3$ | $OCH_3$ | $C(CH_3)_3$ | 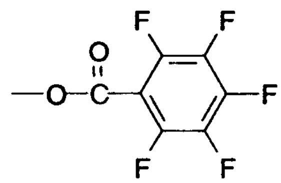 |
| $OCH_3$ | $OCH_3$ | $C(CH_3)_3$ | 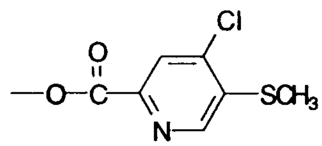 |
| $OCH_3$ | $OCH_3$ | $C(CH_3)_3$ | 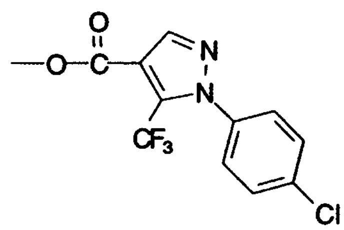 |
| $OCH_3$ | $OCH_3$ | $C(CH_3)_3$ | 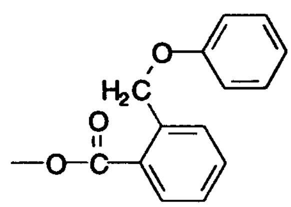 |
| $OCH_3$ | $OCH_3$ | $C(CH_3)_3$ | 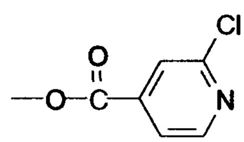 |
| $OCH_3$ | $OCH_3$ | $C(CH_3)_3$ | 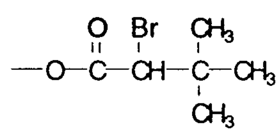 |
| $OCH_3$ | $OCH_3$ | $C(CH_3)_3$ | 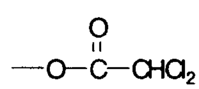 |

Table 1 (continued)

| R1 | R2 | R3 | R4 |
|---|---|---|---|
| $OCH_3$ | $OCH_3$ | $C(CH_3)_3$ | 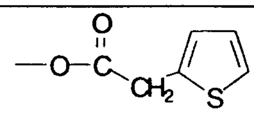 |
| $OCH_3$ | $OCH_3$ | $C(CH_3)_3$ | 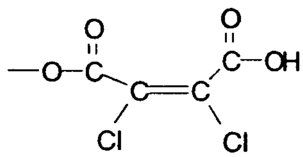 |
| $OCH_3$ | $OCH_3$ | $C(CH_3)_3$ | 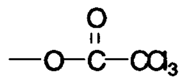 |
| $OCH_3$ | $OCH_3$ | $C(CH_3)_3$ | 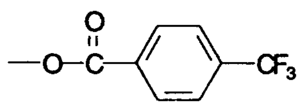 |
| $OCH_3$ | $OCH_3$ | $C(CH_3)_3$ | 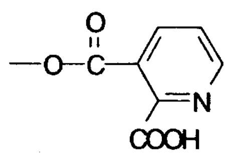 |
| $OCH_3$ | $OCH_3$ | $C(CH_3)_3$ | 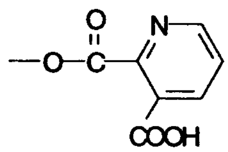 |
| $OCH_3$ | $OCH_3$ | $C(CH_3)_3$ | 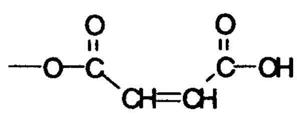 |
| $OCH_3$ | $OCH_3$ | $C(CH_3)_3$ | 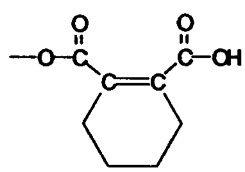 |
| $OCH_3$ | $OCH_3$ | $C(CH_3)_3$ | 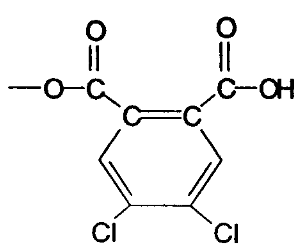 |
| $OCH_3$ | $OCH_3$ | $C(CH_3)_3$ | 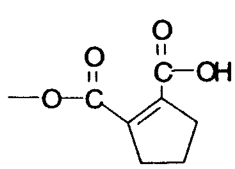 |

Table 1 (continued)

| R1 | R2 | R3 | R4 |
|---|---|---|---|
| $OCH_3$ | $OCH_3$ | $C(CH_3)_3$ | 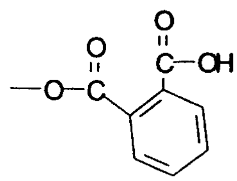 |
| $OCH_3$ | $OCH_3$ | $C(CH_3)_3$ | 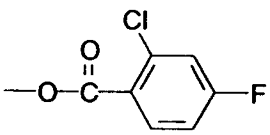 |
| $OCH_3$ | $OCH_3$ | $C(CH_3)_3$ | 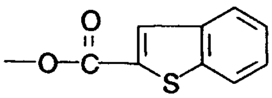 |
| $OCH_3$ | $OCH_3$ | $C(CH_3)_3$ | 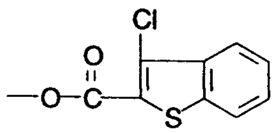 |
| $OCH_3$ | $OCH_3$ | $C(CH_3)_3$ | 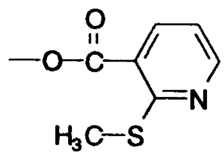 |
| $OCH_3$ | $OCH_3$ | $C(CH_3)_3$ | 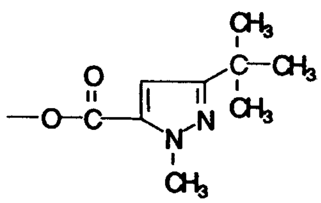 |
| $OCH_3$ | $OCH_3$ | $C(CH_3)_3$ | 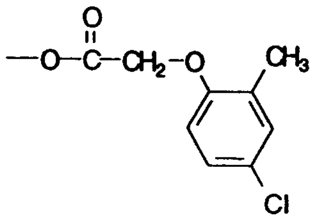 |
| $OCH_3$ | $OCH_3$ | $C(CH_3)_3$ | 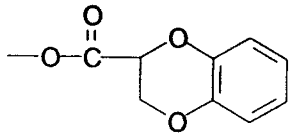 |
| $OCH_3$ | $OCH_3$ | $C(CH_3)_3$ | 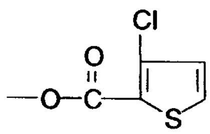 |

Table 1 (continued)

| R1 | R2 | R3 | R4 |
|---|---|---|---|
| $OCH_3$ | $OCH_3$ | $C(CH_3)_3$ | 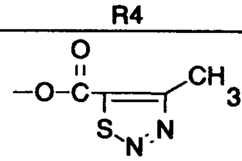 |
| $OCH_3$ | $OCH_3$ | $C(CH_3)_3$ | 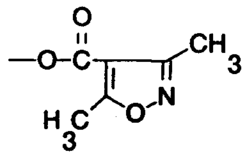 |
| $OCH_3$ | $OCH_3$ | $C(CH_3)_3$ | 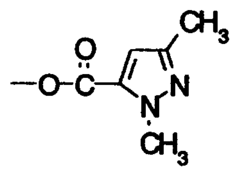 |
| $OCH_3$ | $OCH_3$ | $C(CH_3)_3$ | 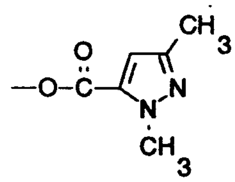 |
| $OCH_3$ | $OCH_3$ | $C(CH_3)_3$ | 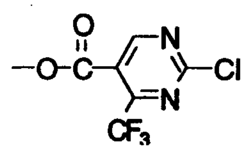 |
| $OCH_3$ | $OCH_3$ | $C(CH_3)_3$ | 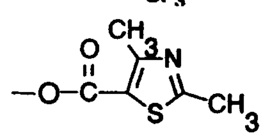 |
| $OCH_3$ | $OCH_3$ | $CH(CH_3)_2$ | 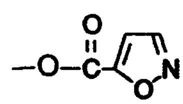 |
| $OCH_3$ | $OCH_3$ | $CH(CH_3)_2$ | 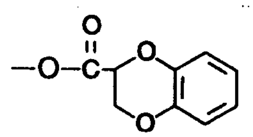 |
| $OCH_3$ | $OCH_3$ | $CH(CH_3)_2$ | 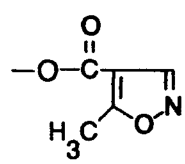 |
| $OCH_3$ | $OCH_3$ | $C(CH_3)_3$ | 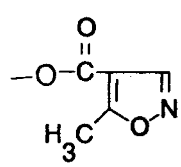 |

Table 1 (continued)

| R1 | R2 | R3 | R4 |
|---|---|---|---|
| $OCH_3$ | $OCH_3$ | $CH(CH_3)_2$ | 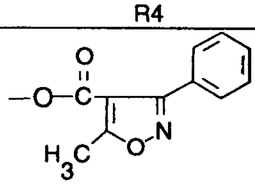 |
| $OCH_3$ | $OCH_3$ | $C(CH_3)_3$ | 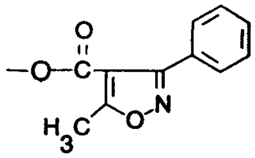 |
| $OCH_3$ | $OCH_3$ | $C(CH_3)_3$ | 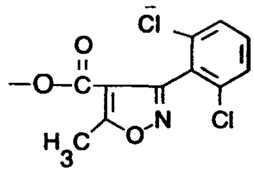 |
| $OCH_3$ | $OCH_3$ | $CH(CH_3)_2$ | 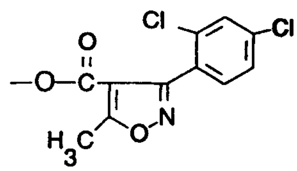 |
| $OCH_3$ | $OCH_3$ | $C(CH_3)_3$ | 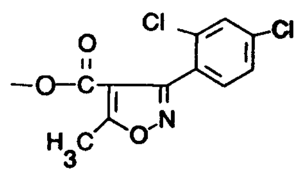 |
| $OCH_3$ | $OCH_3$ | $C(CH_3)_3$ | 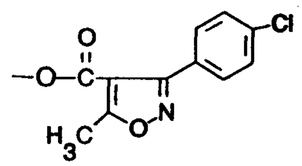 |
| $OCH_3$ | $OCH_3$ | $C(CH_3)_3$ | 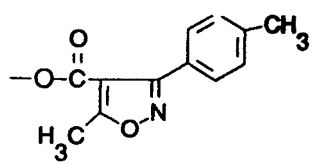 |
| $OCH_3$ | $OCH_3$ | $C(CH_3)_3$ | 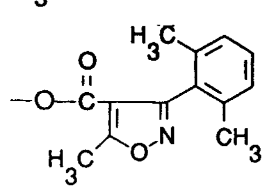 |
| $OCH_3$ | $OCH_3$ | $C(CH_3)_3$ | 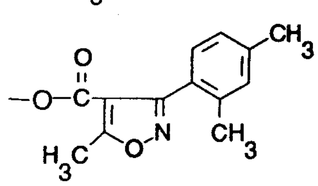 |

Table 1 (continued)

| R1 | R2 | R3 | R4 |
|---|---|---|---|
| $OCH_3$ | $OCH_3$ | $C(CH_3)_3$ | 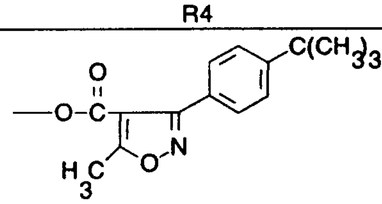 |
| $OCH_3$ | $OCH_3$ | $CH(CH_3)_2$ | 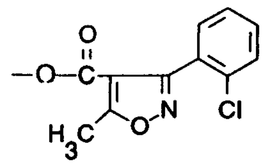 |
| $OCH_3$ | $OCH_3$ | $C(CH_3)_3$ | 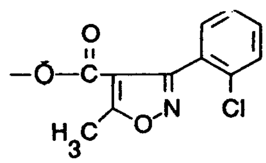 |
| $OCH_3$ | $OCH_3$ | $C(CH_3)_3$ | 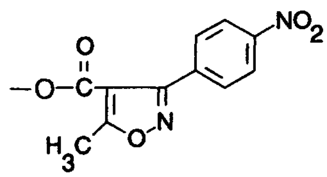 |
| $OCH_3$ | $OCH_3$ | $C(CH_3)_3$ | 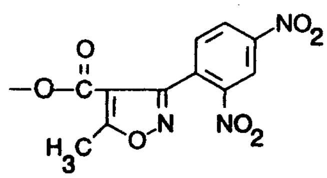 |
| $OCH_3$ | $OCH_3$ | $CH(CH_3)_2$ | 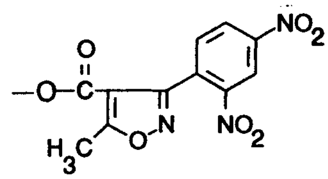 |
| $OCH_3$ | $OCH_3$ | $C(CH_3)_3$ | 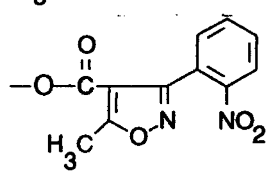 |
| $OCH_3$ | $OCH_3$ | $CH(CH_3)_2$ | 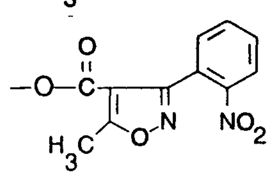 |
| $OCH_3$ | $OCH_3$ | $C(CH_3)_3$ | 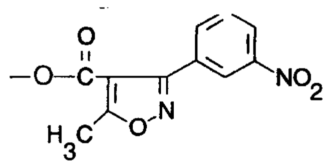 |

Table 1 (continued)

| R1 | R2 | R3 | R4 |
|---|---|---|---|
| $OCH_3$ | $OCH_3$ | $C(CH_3)_3$ | 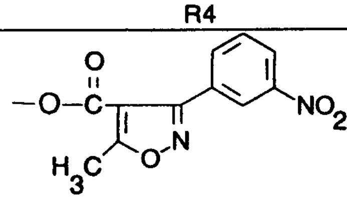 |
| $OCH_3$ | $OCH_3$ | $CH(CH_3)_2$ | 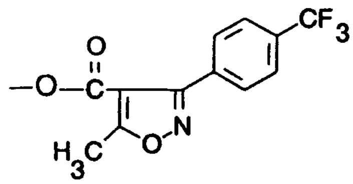 |
| $OCH_3$ | $OCH_3$ | $C(CH_3)_3$ | 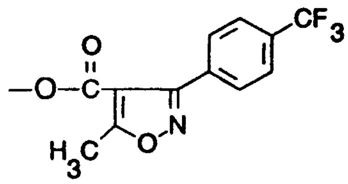 |
| $OCH_3$ | $OCH_3$ | $C(CH_3)_3$ | 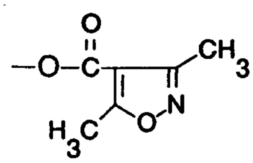 |
| $OCH_3$ | $OCH_3$ | $C(CH_3)_3$ | 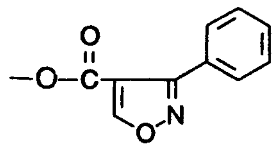 |
| $OCH_3$ | $OCH_3$ | $C(CH_3)_3$ | 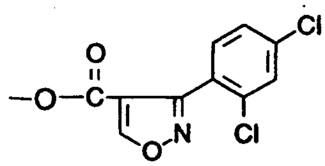 |
| $OCH_3$ | $OCH_3$ | $C(CH_3)_3$ | 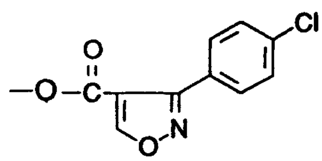 |
| $OCH_3$ | $OCH_3$ | $CH(CH_3)_2$ | 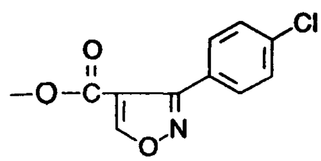 |
| $OCH_3$ | $OCH_3$ | $C(CH_3)_3$ | 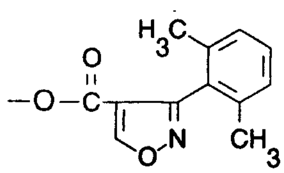 |

Table 1 (continued)

| R1 | R2 | R3 | R4 |
|---|---|---|---|
| $OCH_3$ | $OCH_3$ | $CH(CH_3)_2$ | 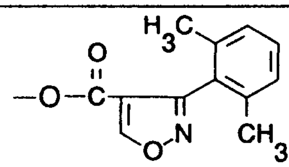 |
| $OCH_3$ | $OCH_3$ | $C(CH_3)_3$ | 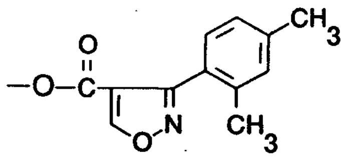 |
| $OCH_3$ | $OCH_3$ | $CH(CH_3)2$ | 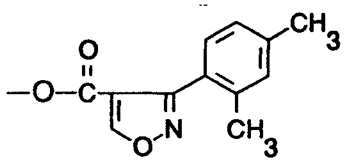 |
| $OCH_3$ | $OCH_3$ | $C(CH_3)_3$ | 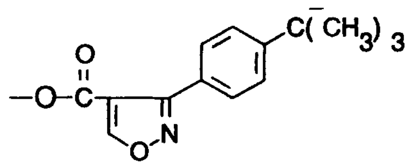 |
| $OCH_3$ | $OCH_3$ | $C(CH_3)_3$ | 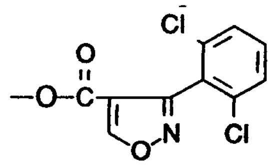 |
| $OCH_3$ | $OCH_3$ | $CH(CH_3)_2$ | 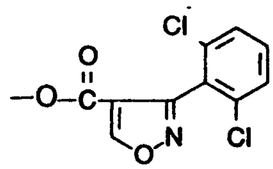 |
| $OCH_3$ | $OCH_3$ | $C(CH_3)_3$ | 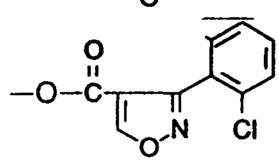 |
| $OCH_3$ | $OCH_3$ | $C(CH_3)_3$ | 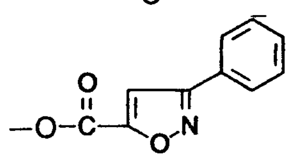 |
| $OCH_3$ | $OCH_3$ | $C(CH_3)_3$ | 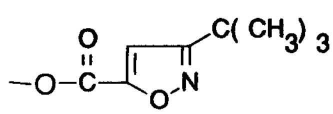 |
| $OCH_3$ | $OCH_3$ | $CH(CH_3)_2$ | 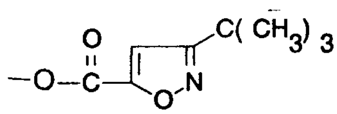 |

Table 1 (continued)

| R1 | R2 | R3 | R4 |
|---|---|---|---|
| $OCH_3$ | $OCH_3$ | $C(CH_3)_3$ | 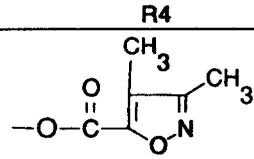 |
| $OCH_3$ | $OCH_3$ | $C(CH_3)_3$ | 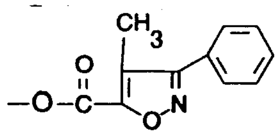 |
| $OCH_3$ | $OCH_3$ | $C(CH_3)_3$ | 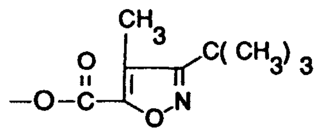 |
| $OCH_3$ | $OCH_3$ | $C(CH_3)_3$ | 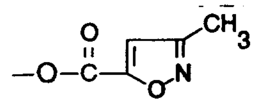 |
| $OCH_3$ | $OCH_3$ | $C(CH_3)_3$ | 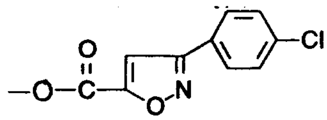 |
| $OCH_3$ | $OCH_3$ | $CH(CH_3)_2$ | 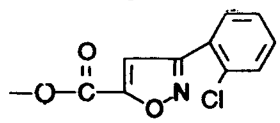 |
| $OCH_3$ | $OCH_3$ | $C(CH_3)_3$ | 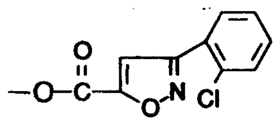 |
| $OCH_3$ | $OCH_3$ | $CH(CH_3)_2$ | 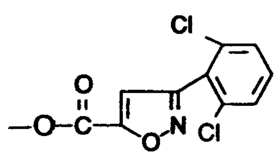 |
| $OCH_3$ | $OCH_3$ | $C(CH_3)_3$ | 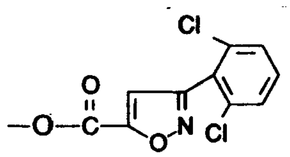 |
| $OCH_3$ | $OCH_3$ | $C(CH_3)_3$ | 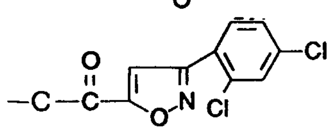 |
| $OCH_3$ | $OCH_3$ | $C(CH_3)_3$ | 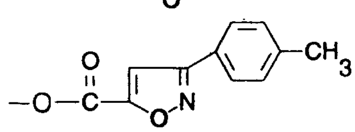 |
| $OCH_3$ | $OCH_3$ | $CH(CH_3)_2$ | 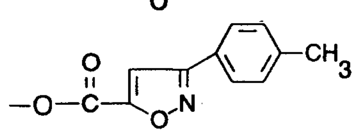 |

Table 1 (continued)

| R1 | R2 | R3 | R4 |
|---|---|---|---|
| $OCH_3$ | $OCH_3$ | $C(CH_3)_3$ | 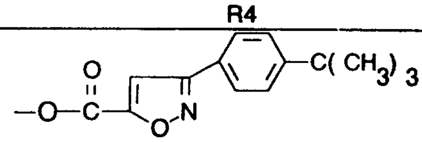 |
| $OCH_3$ | $OCH_3$ | $C(CH_3)_3$ | 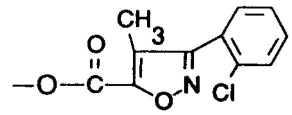 |
| $OCH_3$ | $OCH_3$ | $C(CH_3)_3$ | 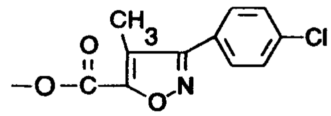 |
| $OCH_3$ | $OCH_3$ | $CH(CH_3)_2$ | 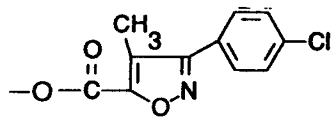 |
| $OCH_3$ | $OCH_3$ | $C(CH_3)_3$ | 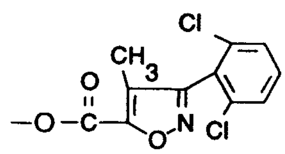 |
| $OCH_3$ | $OCH_3$ | $C(CH_3)_3$ | 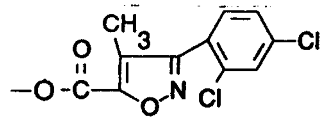 |
| $OCH_3$ | $OCH_3$ | $C(CH_3)_3$ | 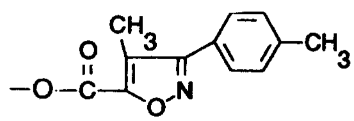 |
| $OCH_3$ | $OCH_3$ | $C(CH_3)_3$ | 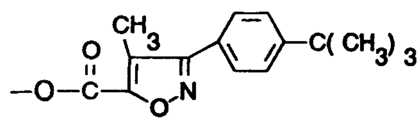 |
| $OCH_3$ | $OCH_3$ | $CH(CH_3)_2$ | 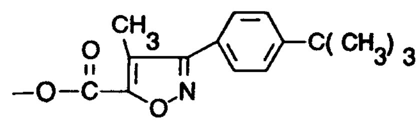 |
| $OCH_3$ | $OCH_3$ | $CH(CH_3)_2$ | 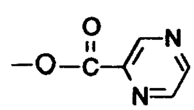 |
| $OCH_3$ | $OCH_3$ | $C(CH_3)_3$ | 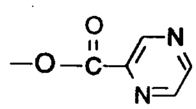 |
| $OCH_3$ | $OCH_3$ | $CH(CH_3)_2$ | 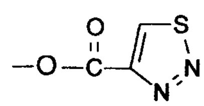 |
| $OCH_3$ | $OCH_3$ | $C(CH_3)_3$ | 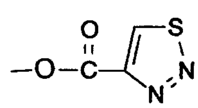 |

Table 1 (continued)

| R1 | R2 | R3 | R4 |
|---|---|---|---|
| $OCH_3$ | $OCH_3$ | $CH(CH_3)_2$ | 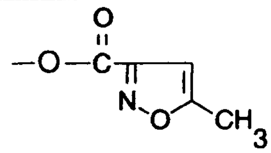 |
| $OCH_3$ | $OCH_3$ | $C(CH_3)_3$ | 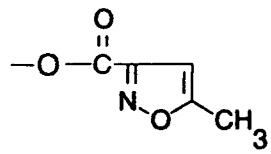 |
| $OCH_3$ | $OCH_3$ | $C(CH_3)_3$ | 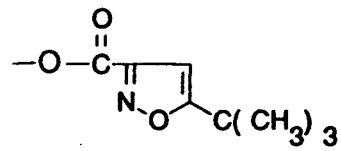 |
| $OCH_3$ | $OCH_3$ | $C(CH_3)_3$ | 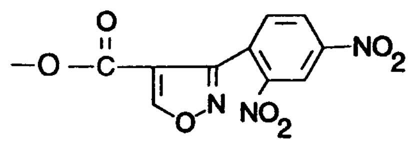 |
| $OCH_3$ | $OCH_3$ | $CH(CH_3)_2$ | 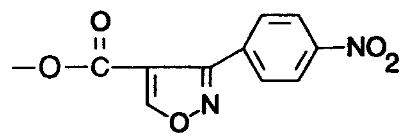 |
| $OCH_3$ | $OCH_3$ | $C(CH_3)_3$ | 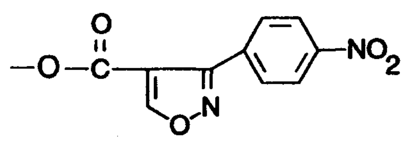 |
| $OCH_3$ | $OCH_3$ | $C(CH_3)_3$ | 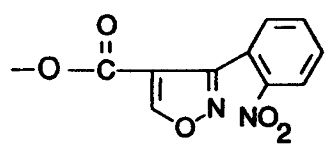 |
| $OCH_3$ | $OCH_3$ | $C(CH_3)_3$ | 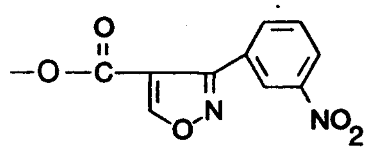 |
| $OCH_3$ | $OCH_3$ | $C(CH_3)_3$ | 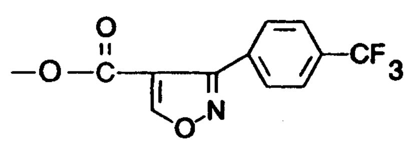 |
| $OCH_3$ | $OCH_3$ | $CH(CH_3)_2$ | 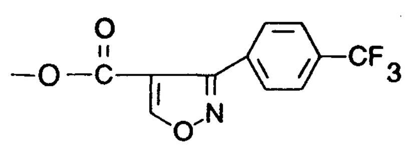 |

Table 1 (continued)

| R1 | R2 | R3 | R4 |
|---|---|---|---|
| $OCH_3$ | $OCH_3$ | $C(CH_3)_3$ | 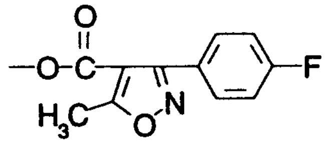 |
| $OCH_3$ | $OCH_3$ | $C(CH_3)_3$ | 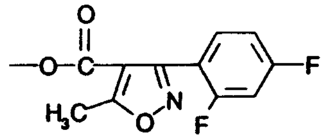 |
| $OCH_3$ | $OCH_3$ | $C(CH_3)_3$ | 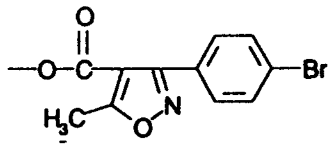 |
| $OCH_3$ | $OCH_3$ | $C(CH_3)_3$ | 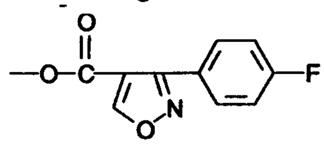 |
| $OCH_3$ | $OCH_3$ | $C(CH_3)_3$ | 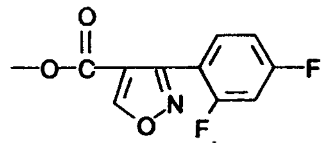 |
| $OCH_3$ | $OCH_3$ | $C(CH_3)_3$ | 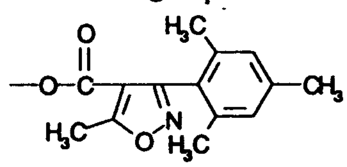 |
| $OCH_3$ | $OCH_3$ | $C(CH_3)_3$ | 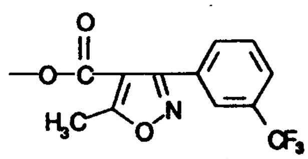 |
| $OCH_3$ | $OCH_3$ | $C(CH_3)_3$ | 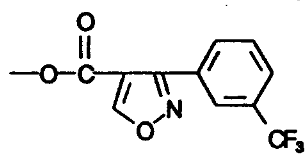 |
| $OCH_3$ | $OCH_3$ | $C(CH_3)_3$ | 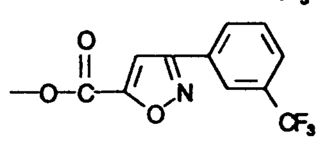 |
| $OCH_3$ | $OCH_3$ | $C(CH_3)_3$ | 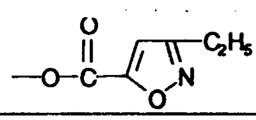 |

Table 1 (continued)

| R1 | R2 | R3 | R4 |
|---|---|---|---|
| $OCH_3$ | $OCH_3$ | $C(CH_3)_3$ | 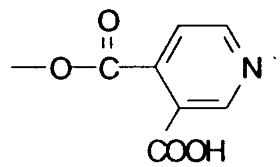 |
| $OCH_3$ | $OCH_3$ | $CH(CH_3)CH_2CH_3$ | 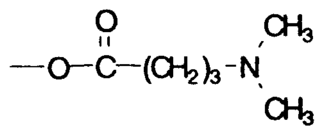 |
| $OCH_3$ | $OCH_3$ | $CH_2CH(CH_3)_2$ | 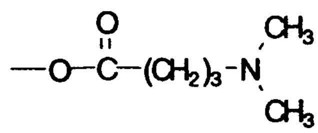 |
| $OCH_3$ | $OCH_3$ | $C(CH_3)_3$ | 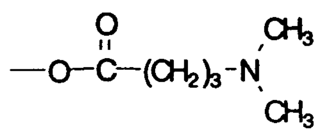 |
| $OCH_3$ | $OCH_3$ | $C(CH_3)_3$ | 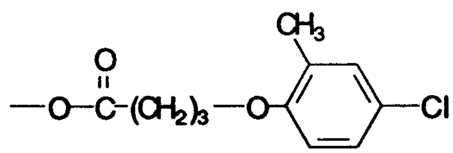 |

Table 2

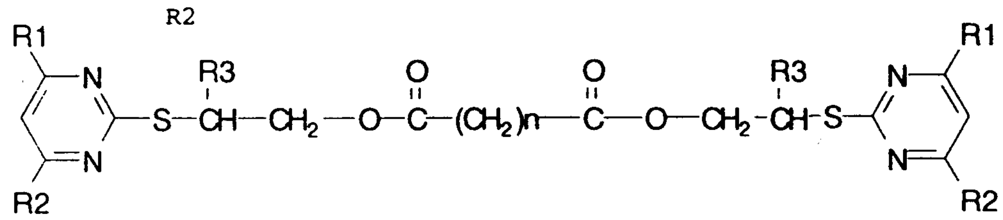

| R1 | R2 | R3 | n |
|---|---|---|---|
| $OCH_3$ | $OCH_3$ | $CH_3$ | 0 |
| $OCH_3$ | $OCH_3$ | $CH_2CH_3$ | 0 |
| $OCH_3$ | $OCH_3$ | $CH_2CH_2CH_3$ | 0 |
| $OCH_3$ | $OCH_3$ | $CH_2CH_2CH_3$ | 2 |
| $OCH_3$ | $OCH_3$ | $CH(CH_3)_2$ | 0 |
| $OCH_3$ | $OCH_3$ | $CH(CH_3)_2$ | 2 |
| $OCH_3$ | $OCH_3$ | $CH(CH_3)_2$ | 4 |
| $OCH_3$ | $OCH_3$ | $CH(CH_3)_2$ | 6 |
| $OCH_3$ | $OCH_3$ | $C(CH_3)_3$ | 0 |
| $OCH_3$ | $OCH_3$ | $C(CH_3)_3$ | 1 |
| $OCH_3$ | $OCH_3$ | $C(CH_3)_3$ | 2 |
| $OCH_3$ | $OCH_3$ | $C(CH_3)_3$ | 3 |
| $OCH_3$ | $OCH_3$ | $C(CH_3)_3$ | 4 |
| $OCH_3$ | $OCH_3$ | $C(CH_3)_3$ | 5 |
| $OCH_3$ | $OCH_3$ | $CH_2CH_2CH_2CH_3$ | 6 |
| $OCH_3$ | $OCH_3$ | $CH_2CH_2CH_2CH_3$ | 0 |
| $OCH_3$ | $OCH_3$ | $CH_2CH_2CH_2CH_3$ | 1 |
| $OCH_3$ | $OCH_3$ | $CH_2CH_2CH_2CH_3$ | 2 |
| $OCH_3$ | $OCH_3$ | $CH_2CH_2CH_2CH_3$ | 4 |
| $OCH_3$ | $OCH_3$ | $CH_2CH(CH_3)_2$ | 0 |
| $OCH_3$ | $OCH_3$ | $CH_2CH(CH_3)_2$ | 2 |
| $OCH_3$ | $OCH_3$ | $CH(CH_3)CH_2CH_3$ | 2 |
| $OCH_3$ | $OCH_3$ | $CH(CH_3)CH_2CH_3$ | 0 |

Table 3

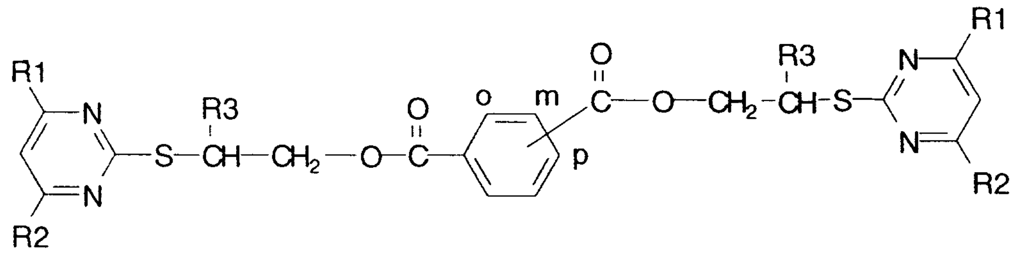

| R1 | R2 | R3 | 結合位置 |
|---|---|---|---|
| $OCH_3$ | $OCH_3$ | $CH_3$ | p |
| $OCH_3$ | $OCH_3$ | $CH_2CH_3$ | p |
| $OCH_3$ | $OCH_3$ | $CH_2CH_2CH_3$ | m |
| $OCH_3$ | $OCH_3$ | $CH_2CH_2CH_3$ | p |
| $OCH_3$ | $OCH_3$ | $CH(CH_3)_2$ | o |
| $OCH_3$ | $OCH_3$ | $CH(CH_3)_2$ | m |
| $OCH_3$ | $OCH_3$ | $CH(CH_3)_2$ | p |
| $OCH_3$ | $OCH_3$ | $CH_2CH_2CH_2CH_3$ | m |
| $OCH_3$ | $OCH_3$ | $CH_2CH_2CH_2CH_3$ | p |
| $OCH_3$ | $OCH_3$ | $CH(CH_3)CH_2CH_3$ | o |
| $OCH_3$ | $OCH_3$ | $CH(CH_3)CH_2CH_3$ | m |
| $OCH_3$ | $OCH_3$ | $CH(CH_3)CH_2CH_3$ | p |
| $OCH_3$ | $OCH_3$ | $CH_2CH(CH_3)_2$ | o |
| $OCH_3$ | $OCH_3$ | $CH_2CH(CH_3)_2$ | m |
| $OCH_3$ | $OCH_3$ | $CH_2CH(CH_3)_2$ | p |
| $OCH_3$ | $OCH_3$ | $C(CH_3)_3$ | o |
| $OCH_3$ | $OCH_3$ | $C(CH_3)_3$ | m |
| $OCH_3$ | $OCH_3$ | $C(CH_3)_3$ | p |

When in the process (a), if, for example, 2-(4,6-dimethoxy-2-pyrimidinylthio)-3-methyl-1-butanol and thionyl chloride are used as starting materials, the course of the reaction can be represented by the following equation:

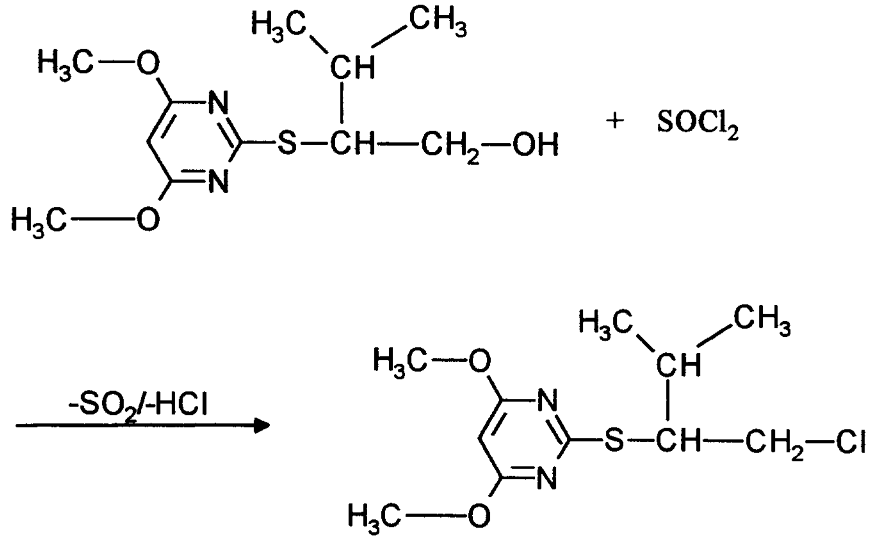


When in the process (b), if, for example, 2-4(4,6-dimethoxy-2-pyrimidinylthio)-3-methyl-1-butanol and 4-chlorophenyl chloride are used as starting materials, the course of the reaction can be represented by the following equation:

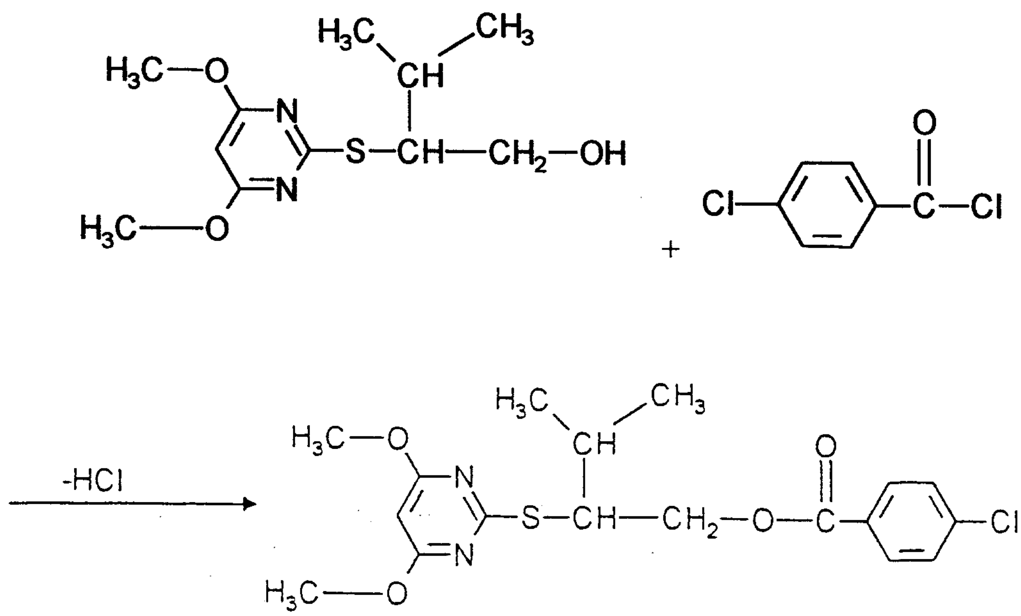


When in the process (c), if, for example, 2-(4,6-dimethoxy-2-pyrimidinylthio)-3-methyl-1-butanol and acetic anhydride are used as starting materials, the course of the reaction can be represented by the following equation:

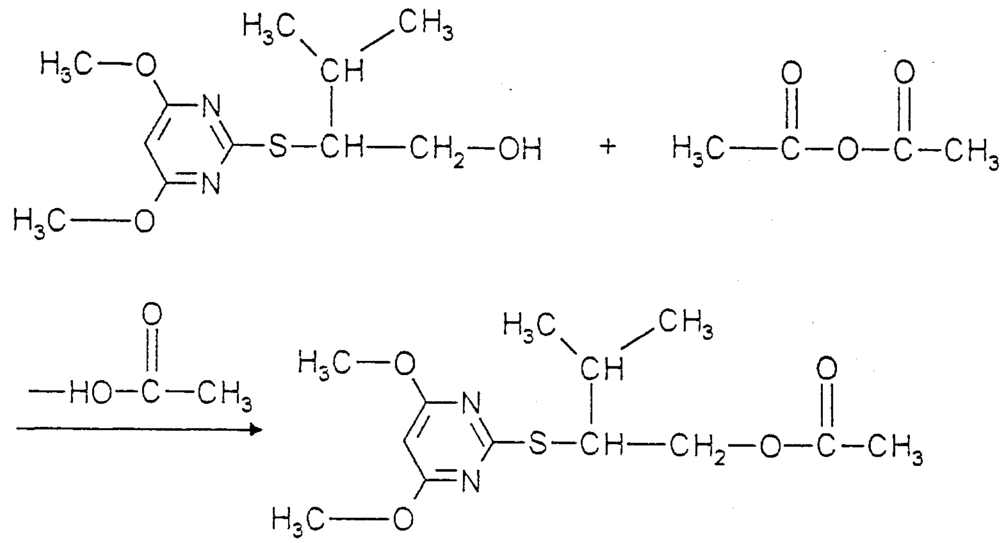

When in the process (d), if, for example, 1-chloro-2-(4,6-dimethoxy-2-pyrimidinylthio)-3,3-dimethyl-1-butane and thioacetic acid are used as starting materials, the course of the reaction can be represented by the following equation:

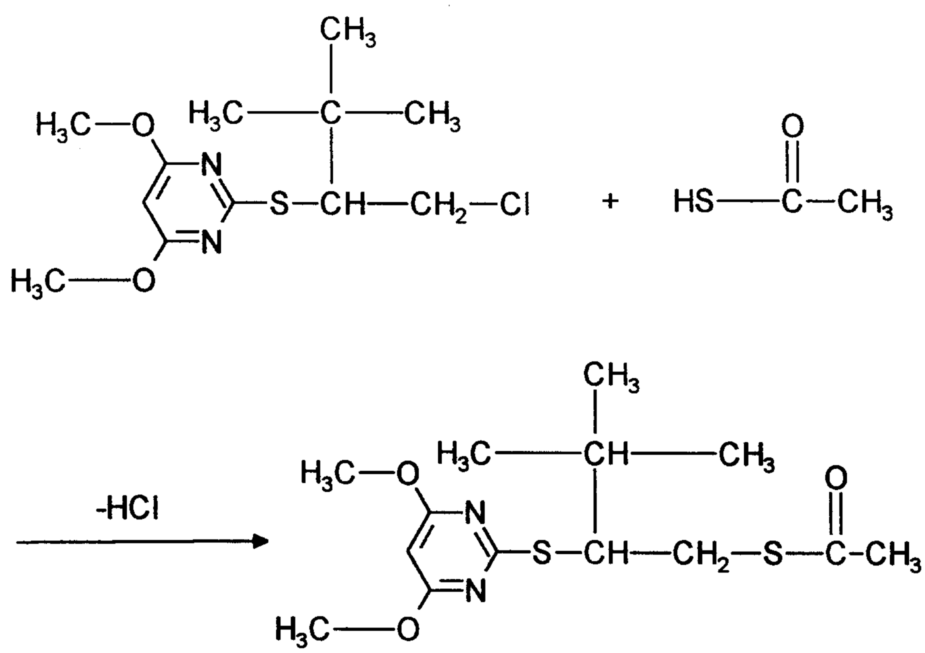


In the process (a), (b) and (c), the starting compounds of the formula (II) mean compounds based on the above definitions of $R^1$, $R^2$ and $R^3$ and preferably substituents based on the above preferred definitions of $R^1$, $R^2$ and $R^3$.

The compounds of the formula (II) can be obtained in the same way as described in Japanese patent application No. 194529/1992.

As specific examples of the formula (II), there may be mentioned:

2-(4,6-dimethoxy-2-pyrimidinylthio)-3-methyl-1-butanol,
2-(4,6-dimethoxy-2-pyrimidinylthio)-3,3-dimethyl-1-butanol,
2-(4,6-dimethoxy-2-pyrimidinylthio)butanol,
2-(4,6-dimethoxy-2-pyrimidinylthio)pentanol,
2-(4,6-dimethoxy-2-pyrimidinylthio)-3-methyl-1-pentanol,

2-(4,6-dimethoxy-2-pyrimidinylthio)-4-methyl-1-pentanol, and
2-(4,6-dimethoxy-2-pyrimidinylthio)-2-cyclopentylethanol.

In the process (a), as specific examples of the halogenating agents, there may be mentioned: methanesulfonyl chloride, thionyl chloride, and thionyl bromide.

In the process (b), the starting compounds of the formula (III) mean compounds based on the above definitions of $R^6$ and $R^7$, preferably substituents based on the above preferred definitions of $R^6$, and $R^7$ are chloro and bromine.

The starting compounds of the formula (III) are well known in the field of organic chemistry.

As specific examples of the formula (III), there may be mentioned:
benzoyl chloride or bromide,
cinnamyl chloride or bromide,
propionyl chloride or bromide,
nicotinyl chloride or bromide,
p-chlorobenzoyl chloride or bromide,
chloroacetyl chloride or bromide,
4-chloro-2-methylphenoxyacetyl chloride, and
3-phenylpropionyl chloride.

In the process (c), the starting compounds of the formula (IV) mean compounds based on the above definitions of $R^6$.

The starting compounds of the formula (IV) are well known in the field of organic chemistry. As specific examples of the formula (IV), there may be mentioned: acetic anhydride, propionic anhydride, and acetic formic anhydride.

In the process (d), the starting compounds of the formula (V) mean compounds based on the above definitions of $R^1$, $R^2$, $R^3$ and $R^7$.

The compounds of the formula (V) can be obtained in the above mentioned process (a).

As specific examples of the formula (V), there may be mentioned:
1-chloro-2-(4,6-dimethoxy-2-pyrimidinylthio)-3-methylbutane,
1-bromo-2-(4,6-dimethoxy-2-pyrimidinylthio)-3-methylbutane,
1-chloro-2-(4,6-dimethoxy-2-pyrimidinylthio)-3,3-dimethylbutane, and
1-bromo-2-(4,6-dimethoxy-2-pyrimidinylthio)-3,3-dimethylbutane.

In the process (d), the starting compounds of the formula (VI) mean compounds based on the above definition of $R^6$.

As specific examples of the formula (VI), there may be mentioned:
thioacetic acid, thiobenzoic acid and thiopropionic acid.

In carrying out the process (a) mentioned above, use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, dimethyl ether, methyl ethyl ether, isopropyl ether, butyl ether, dioxane, dimethoxyethane (DME), tetrahydrofurane (THF), diethyleneglycol dimethyl ether (DGM) and the like;
ketones such as acetone, methylethyl ketone (MEK), methylisopropyl ketone, methyl-isobutyl ketone (MIBK) and the like; nitriles such as acetonitrile, propionitrile, acrylonitrile and the like;
esters such as ethyl acetate, amyl acetate and the like, acid amides such as dimethyl formamide (DMF), dimethyl acetamide (DMA), N-methyl-pyrrolidinone, 1,3-dimethyl-2-imidazlidinone, hexamethylphosphoric triamide (HMPA) and the like;
sulfones and sulfoxides such as dimethyl sulfoxide (DMSO), sulfolane and the like.

In the above mentioned process (a), the reaction temperature can be varied within a substantially wide range. In general, the reaction is carried out at a temperature of from about -20 °C to about 120 °C, preferably from 0 °C to about 60 °C.

Further, the reaction is carried out under normal pressure, although it is also possible to employ a higher or reduced pressure.

When the above mentioned process (a) according to the present invention is carried out, use is made, for example, about 1.0 to 1.2 mols of the halogenating agent in a diluent such as chloroform per 1 mol of the compounds represented by the general formula (II) to obtain the desired compounds.

In carrying out the process (b) mentioned above, use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like;
ethers such as diethyl ether, methyl ethyl ether, diisopropyl ether, dibutyl ether, dioxane, dimethoxyethane (DME), tetrahydrofurane (THF) diethyleneglycol dimethyl ether (DGM) and the like;
ketones such as acetone, methylethyl ketone (MEK), methyl-isopropyl ketone, methyl-isobutyl ketone (MIBK) and the like; nitriles such as acetonitrile, propionitrile, acrylonitrile and the like;
esters such as ethyl acetate, amyl acetate and the like, acid amides such as dimethyl formamide (DMF), dimethyl acetamide (DMA) N-methyl-pyrrolidone, 1,3-dimethyl-2-imidazolidinone, hexamethylphosphoric triamide (HPMA) and the like;
sulfones and sulfoxides such as dimethyl sulfoxide (DMSO), sulfolane and the like; and base such as pyridine.

The process (b) according to the invention is carried out preferably in the presence of an acid binder.

As example of such acid binder may be mentioned:
inorganic bases including hydroxide, carbonate, bicarbonate of alkali metals such as, for example, sodium hydrogen carbonate, potassium hydrogen carbonate, sodium carbonate, potassium carbonate, l ithium hydroxide, sodium hydroxide, potassium hydroxide, calcium hydroxide and the like, inorganic alkali metal amide including lithium amide, sodium amide, potassium amide, and the like,
organic bases including alkoxide, tertiary amines, dialkylaminoanilines, and pyridines such as, for example, triethylamine, tributylamine, 1,1,4,4-tetramethylenediamine (TMEDA), N,N-dimethylaniline, N,N-diethylaniline, pyridine, 4-dimethylaminopyridine (DMAP), 1,4-diaza-bicyclo-[2,2,2]octane (DABCO), 1,8-diazabicyclo[5,4,0]-undec-7-ENE (DBU) and the like,
organic lithiums including methyllithium, n-butyllithium, sec-butyllithium, tert-butyllithium, phenyllithium, dimethyl copper lithium, lithiumisopropylamide, lithiumcyclohexylisopropyl-amide, lithiumdicyclohexylamide, n-butyllithium DABCO, n-butyllithium TMEDA.

In the above mentioned process (b), the reaction temperature can be varied within a substantially wide range. In general, the reaction is carried out at a temperature of from about -40 °C to 100 °C, preferably from 0 °C to about 60 °C.

Further, the reaction is carried out under normal pressure, although it is also possible to employ a higher or reduced pressure.

When the above mentioned process (b) according to the present invention is carried out, use is made, for example, about 1.0 to 1.2 mols of the compound of the formula (III) in a diluent such as pyridine, per 1 mol of the compounds represented by the general formula (II) to obtain the desired compounds.

In carrying out the process (c) mentioned above, use may be made, as suitable diluent, of an inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like;
ethers such as diethyl ether, methyl ethyl ether, diisopropyl ether, dibutyl ether, dioxane, dimethoxyethane (DME), tetrahydrofurane (THF) diethyleneglycol dimethyl ether (DGM) and the like;
ketones such as acetone, methylethyl ketone (MEK), methylisopropyl ketone, methyl-isobutyl ketone (MIBK) and the like; nitriles such as acetonitrile, propionitrile acrylonitrile and the like;
esters such as ethyl acetate, amyl acetate and the like, acid amides such as dimethyl formamide (DMF), dimethyl acetamide (DMA) N-methyl-pyrrolidinone, 1,3-dimethyl-2-imidazolidinone, hexamethylphosphoric triamide (HMPA) and the like;
sulfones and sulfoxides such as dimethyl sulfoxide (DMSO), sulfolane and the like; and base such as pyridine.

The process (c) according to the invention is carried out preferably in the presence of an acid binder.

As example of such an acid binder may be mentioned:
inorganic bases including hydroxide, carbonate, bicarbonate, of alkali metals such as, for example, sodium hydrogen carbonate, potassium hydrogen carbonate, sodium carbonate, potassium carbonate, lithium hydroxide, sodium hydroxide, potassium hydroxide, calcium hydroxide and the like, inorganic alkali metal amide including lithium amide, sodium amide, potassium amide, and the like,
organic bases including alkoxide, tertiary amines, dialkylaminoanilines, and pyridines such as, for example, triethylamine, tributylamine, 1,1,4,4-tetramethylenediamine (TMEDA), N,N-dimethylaniline, N,N-diethylaniline, pyridine, 4-dimethylaminopyridine (DMPA), 1,4-diaza-bicyclo-[2,2,2]octane (DABCO), 1,8-diazabicyclo[5,4,0]-undec-7-ENE (DBU) and the like,
organic lithiums including methyllithium, n-butyllithium, sec-butyllithium, tert-butyllithium, phenyllithium, dimethyl copper lithium, lithiumdiisopropylamide,
lithiumcyclohexylisopropyl-amide, lithiumdicyclohexylamide, n-butyllithium DABCO, n-butyllithium TMEDA.

In the above mentioned process (c), the reaction temperature can be varied within a substantially wide range. In general, the reaction is carried out at a temperature of from about -40 °C to about 100 °C, preferably from 0 °C to about 60 °C.

Further, the reaction is carried out under normal pressure, although it is also possible to employ a higher or reduced pressure.

When the above mentioned process (c) according to the present invention is carried out, use is made, for example, about 1.0 to 1.2 mols of the compound of the formula (IV) in a diluent such as pyridine per 1 mol of the compounds represented by the general formula (II) to obtain the desired compounds.

In carrying out the process (d) mentioned above, use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like;
ethers such as diethyl ether, methyl ethyl ether, diisopropyl ether, dibutyl ether, dioxane, dimethoxyethane (DME), tetrahydrofurane (THF) diethyleneglycol dimethyl ether (DGM) and the like;
ketones such as acetone, methylethyl ketone (MEK), methylisopropyl ketone, methyl-isobutyl ketone (MIBK) and the like; nitriles such as acetonitrile, propionitrile, acrylonitrile and the like;
esters such as ethyl acetate, amyl acetate and the like, acid amides such as dimethyl formamide (DMF), dimethyl acetamide (DMA) N-methyl-pyrrolidone, 1,3-dimethyl-2-imidazolidinone, hexamethylphosphoric triamide (HMPA) and the like;
sulfones and sulfoxides such as dimethyl sulfoxide (DMSO), sulfolane and the like; and base such as pyridine.

The process (d) according to the invention is carried out preferably in the presence of an acid binder.

As example of such an acid binder may be mentioned:
inorganic bases including hydroxide, carbonate, bicarbonate, of alkali metals such as, for example, sodium hydrogen carbonate, potassium hydrogen carbonate, sodium carbonate, potassium carbonate, lithium hydroxide, sodium hydroxide, potassium hydroxide, calcium hydroxide and the like, inorganic alkali metal amide including lithium amide, sodium amide, potassium amide and the like, organic bases including alkoxide, tertiary amines, dialkylaminoanilines, and pyridines, such as, for example, triethylamine, tributylamine, 1,1,4,4-tetra-methylenediamine (TMEDA), N,N-dimethylaniline, N,N-diethylaniline, pyridine, 4-dimethylaminopyridine (DMAP), 1,4-diaza-bicyclo-[2,2,2]octane (DABCO), 1,8-diazabicyclo[5,4,0]-undec-7-ENE (DBU) and the like,
organic lithiums including methyllithium, n-butyllithium, sec-butyllithium, tert-butyllithium, phenyllithium, dimethyl copper lithium, lithiumdiisopropylamide, lithiumcyclohexylisopropyl-amide, lithiumdicyclohexylamide, n-butyllithium DABCO, n-butyllithium TMEDA.

In the above mentioned process (d), the reaction temperature can be varied within a substantially wide range. In general, the reaction is carried out at a temperature of from about -10 °C to 120 °C, preferably from 0 °C to about 50 °C.

Further, the reaction is carried out under normal pressure, although it is also possible to employ a higher or reduced pressure.

When the above mentioned process (d) according to the present invention is carried out, use is made, for example, about 1.0 to 1.2 mols of the compound of the formula (VI) in a diluent such as dimethyl formamide, in the presence of an acid binder such as sodium hydride per 1 mol of the compounds represented by the formula (V) to obtain the desired compounds.

The active compounds according to the invention can be used as defoliants, desiccants, agents for destroying broad-leaf plants and, especially, as weedkillers.

By weeds, in the broadest sense, there are to be understood all plants which grow in locations where they are undesired. Whether the substances according to the invention act as total or selective herbicides depends essentially on the amount used.

The active compounds according to the invention can be used, for example, in connection with the following plants:

Dicotyledon weeds of the genera:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea. Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.

Monocotyledon weeds of the genera:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactylocterium, Agrostis, Alopecurus and Apera.

Monocotyledon cultures of the genera:

Oryza-, Zea, Triticum, Hordeum, Avena, Secale, Sorghum, Panicum, Saccharum, Ananas, Asparagus ad Allium.

However, the use of the active compounds according to the invention is in no way restricted to these genera, but also extends in the same manner to other plants. The compounds are suitable, depending on the concentration, for the total combating of weeds, for example on industrial terrain and rail tracks, and on parths and squares with or without tree plantings.

Equally, the compounds can be employed for combating weeds in perennial cultures, for example, afforestations, decorative tree plantings, orchards, vineyards, citrusgroves, nut orchards, banana plantations, coffee plantations, tea plantations, rubber plantations, oil palm plantations, cocoa plantations, soil fruit plantings and hopfields, and for the selective combating of weeds in annual cultures.

The active compounds can be converted into the customary formulations, such as solutions, emulsions, wettable powders, suspensions, powders, foams, pastes, granules, aerosols, natural ad synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquefied solvents diluents or carriers, there are suitable in the main aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutylketone or cyclohexanone, or strongly polar solvents, such as dimethylformamide and dimethylsulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates.

As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-acid esters, polyoxyethylene-alcoholethers, for example alkylaryl polyglycol ethers, alkylsulphonates, alkyl sulphates, aryl sulphonates as well as albumin hydrolysis products.

Dispersing agents include, for example, lignin sulphite waste liquors and methyl-cellulose. Adhesives such as carboxymethyl-cellulose and natural and synthetic polymers in the form of powders, granules or latices, such as gum Arabic polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts of iron, manganese boron, copper, cobalt, molybdenum and zinc.

The formulations in general contain from 0.1 to 95 percent by weight of active compound, preferably from 0.5 to 90 percent by weight.

The active compounds according to the invention, as such or in the form of their formulations, can also be used for combating weeds, as mixtures with known herbicides, finished formulations or tank mixes being possible.

Mixtures with other known active compounds, such as herbicides, fungicides, insecticides, acaricides, nematicides, bird repellents, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomizing or scattering.

The active compounds according to the invention can be applied either before or after emergence of the plants. They can also be incorporated into the soil before sowing. They are used, in particular, after emergence of the plants.

The amount of active compound used can vary within a substantial range. It depends essentially on the nature of the desired effect. In general, the amounts used are between 0.001 and 10 kg of active compound per hectare of soil surface, preferably between 0.01 and 5 kg per ha.

The preparation and use of the active compounds according to the invention can be seen from the following examples.

**Example 1**

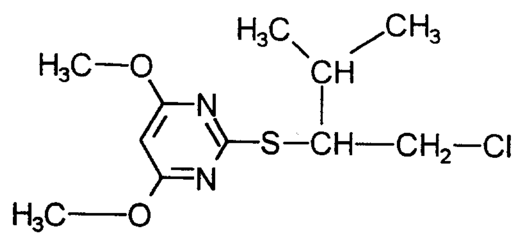

0.02 mol (5.0 g) 2-(4,6-dimethoxy-2-pyrimidinylthio)-3-methyl-1-butanol) was dissolved in chloroform (30 ml).To the resulting mixture was dropwise added 0.05 mol (2.5 g) thionyl chloride at room temperature.The mixture was stirred for one hour at a temperature from about 40 ° C to 50 ° C. Then the reaction mixture was cooled to room temperature, and the solvent was distilled off under reduced pressure. The oily residue thus obtained was dissolved in ethyl acetate, and washed with water, and aqueous sodium bicarbonate solution and further with water in that order, followed by drying over anhydrous sodium sulfate. The organic layer was distilled off under reduced pressure to give 1-chloro-2-(4,6-dimethoxy-2-pyrimidinylthio)-3-methylbutane (4.5g).$n_D^{20}$ = 1.5178.

**Example 2**

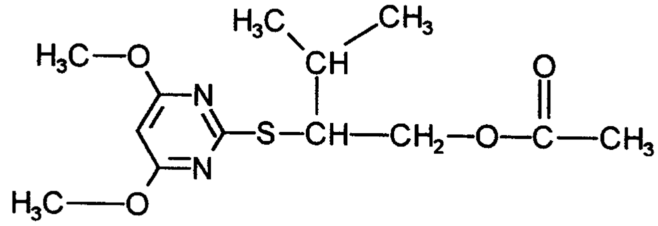

0.0073 mol (1.9g) 2-(4,6-dimethoxy-2-pyrimidinylthio)-3-methyl-1-butanol was dissolved in pyridine (4 ml). To the resulting solution was dropwise added acetic anhydride (4 ml) at room temperature. The reaction mixture was stirred for 3 hours, and then poured into ice water, followed by an extraction operation with ethyl acetate, and thereafter by a washing operation with 1N-aqueous hydrochloric acid solution and then water in that order. The organic solvent was removed by reduced pressure. The oily residue which was obtained was refined by a silica gel column chromatography to give 2-(4,6-dimethoxy-2-pyrimidinylthio)-3-methyl-1-butyl acetate (1.0 g). $n_D^{20}$ = 1.4974.

**Example 3**

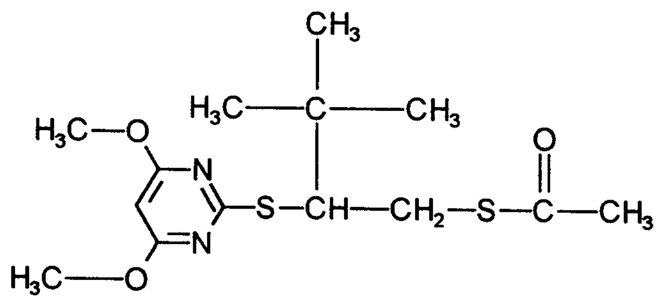

To a solution of thioacetic acid (1.1 g) in dimethylformamide (30 ml) was added 0.015 mol (2.1 g) potassium carbonate (2.1 g) at room temperature. The reaction mixture was stirred for 1 hour, and a solution of 0.007 mol (2.1 g) 1-chloro-2-(4,6-dimethoxy-2-pyrimidinylthio)-3,3-dimethylbutane in dimethylformamide was dropwise added to the reaction mixture while maintaining the reaction mixture at room temperature. After completion of this addition, the reaction mixture was stirred for 8 hours, admixed with water, extracted with ethyl acetate, and dried over anhydrous sodium sulfate.Thereafter, the solvent was removed by a distillation operation under reduced pressure. The resultant oily residue was refined by silica gel column chromatography to give 2-(4,6-dimethoxy-2-pyrimidinylthio)-3,3-dimethyl-1-butyl thioacetate (1.5 g). $n_D^{20}$ = 1.5430.

In the following are shown the compounds that were prepared likewise the above-mentioned Examples 1 to 3, together with those that were actually prepared in said Examples.

No. 1: 1-chloro-2-(4,6-dimethoxy-2-pyrimidinylthio)-3-methylbutane $n_D^{20}$ = 1.5667
No.2: 2-(4,6-dimethoxy-2-pyrimidinylthio)-3-methyl-1-butyl acetate $n_D^{20}$ = 1.4974
No. 3: 1-chloro-2-(4,6-dimethoxy-2-pyrimidinylthio)-butane mp. 68~70.5 °C
No. 4: 2-(4,6-dimethoxy-2-pyrimidinylthio)-1-butyl acetate $n_D^{20}$ = 1.5138
No. 5: 2-(4,6-dimethoxy-2-pyrimidinylthio)-1-butyl p-chlorobezoate $n_D^{20}$ = 1.5628
No. 6: 2-(4,6-dimethoxy-2-pyrimidinylthio)-3,3-dimethyl-butyl acetate $n_D^{20}$ = 1.5156
No. 7: 2-(4,6-dimethoxy-2-pyrimidinylthio)-3,3-dimethyl-1-butyl n-propionate $n_D^{20}$ = 1.5060
No. 8: 2-(4,6-dimethoxy-2-pyrimidinylthio)-3,3-dimethyl-1-butyl n-butyrate $n_D^{20}$ = 1.5043
No. 9: 2-(4,6-dimethoxy-2-pyrimidinylthio)-3,3-dimethyl-1-butyl cyclopropanecarboxylate $n_D^{20}$ = 1.5222
No. 10: 2-(4,6-dimethoxy-2-pyrimidinylthio)-3,3-dimethyl-1-butyl benzoate $n_D^{20}$ = 1.5520
No. 11: 1-(4,6-dimethoxy-2-pyrimidinylthio)-4-methyl-1-pentyl acetate $n_D^{20}$ = 1.5112
No. 12: 3-cyclohexyl-2-(4,6-dimethoxy-2-pyrimidinylthio)-1-propyl-n-propionate $n_D^{20}$ = 1.5153

No. 13: 2-(4,6-dimethoxy-2-pyrimidinylthio)-3-phenyl-1-butyl acetate $n_D^{20}$ = 1.5445
No. 14: 2-(4,6-dimethoxy-2-pyrimidinylthio)-3-methyl-1-butyl valerate $n_D^{20}$ = 1.4807
No. 15: 2-(4,6-dimethoxy-2-pyrimidinylthio)-3-methyl-1-butyl octanoate $n_D^{20}$ = 1.4930
No. 16: 2-(4,6-dimethoxy-2-pyrimidinylthio)-3-methyl-1-butyl laurate $n_D^{20}$ = 1.4825
No. 17: 2-(4,6-dimethoxy-2-pyrimidinylthio)-3-methyl-1-butyl myristate $n_D^{20}$ = 1.4923
No. 18: 2-(4,6-dimethoxy-2-pyrimidinylthio)-3-methyl-1-butyl p-methylbenzoate $n_D^{20}$ = 1.5576
No. 19: 2-(4,6-dimethoxy-2-pyrimidinylthio)-3-methyl-1-butyl pivalate $n_D^{20}$ = 1.4858
No. 20: 2-(4,6-dimethoxy-2-pyrimidinylthio)-3-methyl-1-butyl p-tert-butylbenzoate $n_D^{20}$ = 1.5437
No. 21: 1-chloro-2-(4,6-dimethoxy-2-pyrimidinylthio)-3,3-dimethylbutane $n_D^{20}$ = 1.5334
No. 22: 2-(4,6-dimethoxy-2-pyrimidinylthio)-3,3-dimethyl-1-butyl tert-butylacetate $n_D^{20}$ = 1.4868
No. 23: 2-(4,6-dimethoxy-2-pyrimidinylthio)-3,3-dimethyl-1-butyl-thioacetate $n_D^{20}$ = 1.5430
No. 24: 2-(4,6-dimethoxy-2-pyrimidinylthio)-3,3-dimethyl-1-butyl-valerate $n_D^{20}$ = 1.4819
No. 25: 2-(4,6-dimethoxy-2-pyrimidinylthio)-3,3-dimethyl-1-butyl-p-chlorobenzoatae $n_D^{20}$ = 1.5229
No. 26: 2-(4,6-dimethoxy-2-pyrimidinylthio)-3,3-dimethyl-1-butyl-α-chlorophenylacetate $n_D^{20}$ = 1.5481
No. 27: 2-(4,6-dimethoxy-2-pyrimidinylthio)-3-methyl-1-pentylacetate $n_D^{20}$ = 1.5168
No. 28: 2-(4,6-dimethoxy-2-pyrimidinylthio)-3,3-dimethyl-1-butyl-vinylacetate $n_D^{20}$ = 1.5079
No. 29: 2-(4,6-dimethoxy-2-pyrimidinylthio)-3,3-dimethyl-1-butyl-2-thiophenecarboxylate $n_D^{20}$ = 1.5548
No. 30: 2-(4,6-dimethoxy-2-pyrimidinylthio)-3,3-dimethyl-1-butyl-3-chloropropionate $n_D^{20}$ = 1.5006
No. 31: 2-(4,6-dimethoxy-2-pyrimidinylthio)-3,3-dimethylbutyl-3-bromopropionate $n_D^{20}$ = 1.5052
No. 32: 2-(4,6-dimethoxy-2-pyrimidinylthio)3,3-dimethylbutyl methacrylate $n_D^{20}$ = 1.5054
No. 33: 2-(4,6-dimethoxy-2-pyrimidinylthio)-3,3-dimethyl-butyl-2-bromoacrylate $n_D^{20}$ = 1.5148
No. 34: 3-(4-methoxyphenyl)-2-(4,6-dimethoxy-2-pyrimidinylthio)-1-propylacetate $n_D^{20}$ = 1.5404
No. 35: 2-(4,6-dimethoxy-2-pyrimidinylthio)-3,3-dimethyl-1-butyl-isovalerate $n_D^{20}$ = 1.5042
No. 36: 2-(4,6-dimethoxy-2-pyrimidinylthio)-3,3-dimethyl-1-butyl-o-chlorobenzoate $n_D^{20}$ = 1.5378
No. 37: 2-(4,6-dimethoxy-2-pyrimidinylthio)-3,3-dimethyl-1-butyl-m-chlorobenzoate $n_D^{20}$ = 1.5493
No. 38: 2-(4,6-dimethoxy-2-pyrimidinylthio)-3,3-dimethyl-1-butyl-2-chloropropionatae $n_D^{20}$ = 1.5223
No. 39: 2-(4,6-dimethoxy-2-pyrimidinylthio)-3,3-dimethyl-1-butyl-trifluoroacetate mp. 67.5-69.5 °C
No. 40: 2-(4,6-dimethoxy-2-pyrimidinylthio)-3,3-dimethyl-1-butyl-n-octanoatae $n_D^{20}$ = 1.4940
No. 41: 2-(4,6-dimethoxy-2-pyrimidinylthio)-3,3-dimethyl-1-butyl-n-decanoate $n_D^{20}$ = 1.4859
No. 42: 2-(4,6-dimethoxy-2-pyrimidinylthio)-3,3-dimethyl-1-butyl-laurate $n_D^{20}$ = 1.4903
No. 43: 2-(4,6-dimethoxy-2-pyrimidinylthio)-3,3-dimethyl-1-butyl-myristate $n_D^{20}$ = 1.4820
No. 44: 2-(4,6-dimethoxy-2-pyrimidinylthio)-3,3-dimethyl-1-butylpentafluorobenzoate $n_D^{20}$ = 1.5122
No. 45: 2-(4,6-dimethoxy-2-pyrimidinylthio)-3,3-dimethyl-1-butyl-5-chloro-6-methylthionicotinate $n_D^{20}$ = 1.5438
No. 46: 2-(4,6-dimethoxy-2-pyrimidinylthio)-3,3-dimethyl-1-butyl 1-(p-chlorophenyl)-5-trifluoromethyl-4-pyrazolcarboxylate $n_D^{20}$ = 1.5382
No. 47: 2-(4,6-dimethoxy-2-pyrimidinylthio)-2-cyclopentylethyl acetate $n_D^{20}$ = 1.5335
No. 48: 2-(4,6-dimethoxy-2-pyrimidinylthio)-3,3-dimethyl-1-butyl-2-phenoxymethylbenzoate $n_D^{20}$ = 1.5507
No. 49: 2-(4,6-dimethoxy-2-pyrimidinylthio)-3,3-dimethyl-1-butyl-α-picolinate $n_D^{20}$ = 1.5378
No. 50: 2-(4,6-dimethoxy-2-pyrimidinylthio)-3,3-dimethyl-1-butyl-nicotinate $n_D^{20}$ = 1.5452
No. 51: 2-(4,6-dimethoxy-2-pyrimidinylthio)-3,3-dimethyl-1-butyl-chloroacetate $n_D^{20}$ = 1.5200
No. 52: 2-(4,6-dimethoxy-2-pyrimidinylthio)-3,3-dimethyl-1-butyl-isonicotinate $n_D^{20}$ = 1.5172
No. 53: 2-(4,6-dimethoxy-2-pyrimidinylthio)-3,3-dimethyl-1-butyl-2-chloro-isonicotinate $n_D^{20}$ = 1.5320
No. 54: bis[2-(4,6-dimethoxy-2-pyrimidinylthio)-3,3-dimethyl-1-butyl]-oxalate $n_D^{20}$ = 1.5336
No. 55: bis[2-(4,6-dimethoxy-2-pyrimidinylthio)-3,3-dimethyl-1-butyl]-phthalate $n_D^{20}$ = 1.5298
No. 56: bis[2-(4,6-dimethoxy-2-pyrimidinylthio)-3,3-dimethyl-1-butyl]-terephthalate $n_D^{20}$ = 1.5361
No. 57: 2-(4,6-dimethoxy-2-pyrimidinylthio)-3-methyl-1-butyl-methoxyacetate $n_D^{20}$ = 1.5182
No. 58: 2-(4,6-dimethoxy-2-pyrimidinylthio)-3,3-dimethyl-1-butyl-methoxyacetate $n_D^{20}$ = 1.5041
No. 59: 2-(4,6-dimethoxy-2-pyrimidinylthio)-3,3-dimethyl-1-butyl-phenoxyacetate $n_D^{20}$ = 1.5408
No. 60: 2-(4,6-dimethoxy-2-pyrimidinylthio)-3,3-dimethyl-1-butyl-(2,4-dichlorophenoxy)acetate $n_D^{20}$ = 1.5309
No. 61: 2-(4,6-dimethoxy-2-pyrimidinylthio)-3,3-dimethyl-1-butyl-(4-chloro-2-methylphenoxy)acetate $n_D^{20}$ = 1.5312
No. 62: 2-(4,6-dimethoxy-2-pyrimidinylthio)-3,3-dimethyl-1-butyl-α-trifluoromethyl-α-methoxyphenylacetate $n_D^{20}$ = 1.5191

No. 63: 2-(4,6-dimethoxy-2-pyrimidinylthio)-3,3-dimethyl-1-butyl-2-(p-tolylsulfonyloxy)propionate mp. 81-85 °C
No. 64: 2-(4,6-dimethoxy-2-pyrimidinylthio)-3,3-dimethyl-1-butyl-p-chlorophenylmercaptoacetate $n_D^{20}$ = 1.5704
No. 65: 2-(4,6-dimethoxy-2-pyrimidinylthio)-3,3-dimethyl-1-butyl-p-benzyloxyphenylmercaptoacetate $n_D^{20}$ = 1.5701
No. 66: 2-(4,6-dimethoxy-2-pyrimidinylthio)-3,3-dimethyl-1-butyl-methoxycarbonylcarboxylate $n_D^{20}$ = 1.5112
No. 67: 2-(4,6-dimethoxy-2-pyrimidinylthio)-3,3-dimethyl-1-butyl-methoxycarbonylacetate $n_D^{20}$ = 1.5142
No. 68: 2-(4,6-dimethoxy-2-pyrimidinylthio)-3-methyl-1-butyl-3-methoxycarbonylpropionate $n_D^{20}$ = 1.5142
No. 69: 2-(4,6-dimethoxy-2-pyrimidinylthio)-3,3-dimethyl-1-butyl-3-methoxycarbonylpropionate $n_D^{20}$ = 1.5039
No. 70: 2-(4,6-dimethoxy-2-pyrimidinylthio)-3,3-dimethyl-1-butyl-hydrogen succiniate $n_D^{20}$ = 1.5141
No. 71: sodium-2-(4,6-dimethoxy-2-pyrimidinylthio)-3,3-dimethyl-1-butyl succinate melting point and refractive index cannot be measured
No. 72: 2-(4,6-dimethoxy-2-pyrimidinylthio)-3,3-dimethyl-1-butyl-cyclohexanecarboxylate $n_D^{20}$ = 1.5122
No. 73: 2-(4,6-dimethoxy-2-pyrimidinylthio)-3,3-dimethyl-1-butyl-1-bromo-2,2-dimethylbutyrate $n_D^{20}$ = 1.5181
No. 74: 2-(4,6-dimethoxy-2-pyrimidinylthio)-3,3-dimethyl-1-butyl-dichloroacetate mp. 87-89 °C
No. 75: 2-(4,6-dimethoxy-2-pyrimidinylthio)-3,3-dimethyl-1-butyl-thienylacetate $n_D^{20}$ = 1.5632
No. 76: 2-(4,6-dimethoxy-2-pyrimidinylthio)-3,3-dimethyl-1-butyl-hydrogen-1,2-dichloromaleate amorphous
No. 77: 2-(4,6-dimethoxy-2-pyrimidinylthio)-3,3-dimethyl-1-butyl-trichloroacetate $n_D^{20}$ = 1.5283
No. 78: 2-(4,6-dimethoxy-2-pyrimidinylthio)-3,3-dimethyl-1-butyl-4-trifluoromethylbenzoate $n_D^{20}$ = 1.5126
No. 79: 2-(4,6-dimethoxy-2-pyrimidinylthio)-3,3-dimethyl-1-butyl-6-chloronicotinate mp. 78-81 °C
No. 80: 2-(4,6-dimethoxy-2-pyrimidinylthio)-3,3-dimethyl-1-butyl-2-hydroxycarbonylpicolinate $n_D^{20}$ = 1.5485
No. 81: 2-(4,6-dimethoxy-2-pyrimidinylthio)-3,3-dimethyl-1-butyl-hydrogen maleate $n_D^{20}$ = 1.5323
No. 82: 2-(4,6-dimethoxy-2-pyrimidinylthio)-3,3-dimethyl-1-butyl-2-hydroxycarbonyl-1-cyclohexenecarboxylate $n_D^{20}$ = 1.5210
No. 83: 2-(4,6-dimethoxy-2-pyrimidinylthio)-3,3-dimethyl-1-butyl-hydrogen-4,5-dichlorophthalate $n_D^{20}$ = 1.5629
No. 84: 2-(4,6-dimethoxy-2-pyrimidinylthio)-3,3-dimethyl-1-butyl-3-phenylpropionate $n_D^{20}$ = 1.5379
No. 85: 2-(4,6-dimethoxy-2-pyrimidinylthio)-3,3-dimethyl-1-butyl-2-hydroxycarbonylcyclohexanecarboxylate $n_D^{20}$ = 1.5066
No. 86: 2-(4,6-dimethoxy-2-pyrimidinylthio)-3,3-dimethyl-1-butyl-2-hydroxycarbonyl-1-cyclopentencarboxylate mp. 85.5-88 °C
No. 87: 2-(4,6-dimethoxy-2-pyrimidinylthio)-3,3-dimethyl-1-butyl-hydrogen phthalate $n_D^{20}$ = 1.5492
No. 88: 2-(4,6-dimethoxy-2-pyrimidinylthio)-3,3-dimethyl-1-butyl-2-chloro-4-fluorobenzoate $n_D^{20}$ = 1.5439
No. 89: 2-(4,6-dimethoxy-2-pyrimidinylthio)-3,3-dimethyl-1-butyl-benzo[b]thiophene-2-carboxylate $n_D^{20}$ = 1.5879
No. 90: 2-(4,6-dimethoxy-2-pyrimidinylthio)-3,3-dimethyl-1-butyl-3-chlorobenzo[b]thiophene-2-carboxylate $n_D^{20}$ = 1.5865
No. 91: 2-(4,6-dimethoxy-2-pyrimidinylthio)-3,3-dimethyl-1-butyl-2-methylthionicotinate $n_D^{20}$ = 1.5653
No. 92: 2-(4,6-dimethoxy-2-pyrimidinylthio)-3,3-dimethyl-1-butyl-3-tert-butyl-2-methylpyrazole-3-carboxylate $n_D^{20}$ = 1.5213
No. 93: 2-(4,6-dimethoxy-2-pyrimidinylthio)-3,3-dimethyl-1-butyl-cinnamate $n_D^{20}$ = 1.5724
No. 94: 2-(4,6-dimethoxy-2-pyrimidinylthio)-3,3-dimethyl-1-butyl-2-[4-(6-chloro-2-quinoxalyloxy)-phenoxy]-propionate $n_D^{20}$ = 1.5691
No. 95: 2-(4,6-dimethoxy-2-pyrimidinylthio)-3,3-dimethyl-1-butyl-phenylacetate $n_D^{20}$ = 1.5441
No. 96: 2-(4,6-dimethoxy-2-pyrimidinylthio)-3,3-dimethyl-1-butyl-4-n-butylbenzoate $n_D^{20}$ = 1.5389
No. 97: 2-(4,6-dimethoxy-2-pyrimidinylthio)-3,3-dimethyl-1-butyl-2,5-dichlorobenzoate $n_D^{20}$ = 1.5515

No. 98: 2-(4,6-dimethoxy-2-pyrimidinylthio)-3,3-dimethyl-1-butyl-1,4-benzoxane-2-carboxylate $n_D^{20}$ = 1.5202

No. 99: 2-(4,6-dimethoxy-2-pyrimidinylthio)-3,3-dimethyl-1-butyl-isoxazolyl-5-carboxylate mp. 77.5-79.5 °C

No. 100: 2-(4,6-dimethoxy-2-pyrimidinylthio)-3,3-dimethyl-1-butyl-5-bromonicotinate $n_D^{20}$ = 1.5441

No. 101: 2-(4,6-dimethoxy-2-pyrimidinylthio)-3,3-dimethyl-1-butyl-2,6-dichloroisonicotinate $n_D^{20}$ = 1.5306

No. 102: 2-(4,6-dimethoxy-2-pyrimidinylthio)-3,3-dimethyl-1-butyl-4-(chloro-2-methylphenoxy)butyrate $n_D^{20}$ = 1.5349

No. 103: 2-(4,6-dimethoxy-2-pyrimidinylthio)-3,3-dimethyl-1-butyl-2,4,5-trichlorophenoxyacetate $n_D^{20}$ = 1.5526

No. 104: 2-(4,6-dimethoxy-2-pyrimidinylthio)-3,3-dimethyl-1-butyl-3-hydroxycarbonylisonicotinate $n_D^{20}$ = 1.5353

No. 105: 2-(4,6-dimethoxy-2-pyrimidinylthio)-3,3-dimethyl-1-butyl-3-chlorothiophene-2-carboxylate $n_D^{20}$ = 1.5523

No. 106: 2-(4,6-dimethoxy-2-pyrimidinylthio)-3,3-dimethyl-1-butyl-2-methylpropionate $n_D^{20}$ = 1.4995

**Example 4**

Post-emergence foliage treatment on upland weeds

Formulation of Active Compounds

| | |
|---|---|
| Carrier: | 5 parts by weight of acetone |
| Emulsifier: | 1 part by weight of benzyloxy polyglycol ester |

To produce a suitable formulation each of the active compounds, 1 part by weight of each of the active compounds was mixed with the stated amount of carrier and the stated amount of emulsifier, and the resulting emulsifiable concentrate was then diluted with water to the desired concentrations.

Test procedure

In a greenhouse a number of test pots each having an area of seeds of barnyard grass (Echinochloa crus-gali) and redroot pigweed (Amaranthus Blitum), respectively, were sown onto the soil surface in the respective test pots, followed by cultivation for ten days. Thereafter, the predetermined dosages of the active compound formulations, prepared in the manner mentioned above, were uniformly sprayed onto the foliate portions of the weeds in the respective test pots. Three weeks after the application of the active compound formulations, the degree of the herbicidal effect on the weeds was determined based on the following assessment:

| | |
|---|---|
| Completely killed | 100% |
| Status equivalent to non-treated pots | 0% |

**Example 5**

Pre-emergence soil treatment test on upland

In a greenhouse, a number of test pots each having an area of 120 $cm^2$ were charged with soil taken out from a cultivated field. Onto the soil surface in the respective test pots were sown seeds of barnyard grass and redroot pigweed, respectively, followed by soil covering thereon. Thereafter, predetermined dosages of the active compound formulations, prepared as in Example 4, were uniformly sprayed onto the soil surfaces of the respective test pots.

Four weeks after the spraying of the active compound formulations, the degree of herbicidal effect on the weeds were determined.

In both tests for example the compounds 1, 2, 3, 4, 5, 6, 7, 8, 9, 10, 11, 14, 15, 16, 17, 18, 19, 20, 21, 22, 23, 24, 25, 28, 29, 31, 35, 37, 38, 41, 48, 50, 51, 52, 53, 54, 58, 59, 61, 64, 65, 68, 69, 71, 72, 74, 78, 79, 80, 81 and 82 exhibit powerful herbicidal activities showing degrees of herbicidal effect on the weeds between 80 and 100% in pre-emergence treatment and between 50 and 100% in post-emergence treatment.

## Claims

**1.** Pyrimidinylthioalkane derivatives of the formula (I)

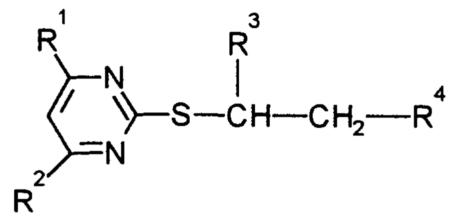

(I)

wherein

- $R^1$ represents $C_{1-4}$alkyl, $C_{1-4}$alkoxy, halogen, halogeno-$C_{1-4}$alkyl or halogeno-$C_{1-4}$alkoxy,
- $R^2$ represents $C_{1-4}$alkoxy, halogen, halogeno-$C_{1-4}$alkyl or halogeno-$C_{1-4}$alkoxy,
- $R^3$ represents optionally $C_{1-4}$alkyl substituted or unsubstituted $C_{3-7}$cycloalkyl or optionally substituted $C_{1-15}$alkyl,
- $R^4$ represents halogen or

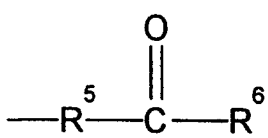

,

- $R^5$ represents oxygen or sulfur,
- $R^6$ represents hydrogen, in each case optionally substituted $C_{1-20}$ saturated aliphatic hydrocarbon, $C_{2-20}$ unsaturated aliphatic hydrocarbon, phenyl, $C_{3-8}$cycloalkyl, $C_{3-8}$cycloalkenyl, $C_{1-4}$alkoxycarbonyl, carboxyl and its salts, an optionally substituted heterocyclic ring, an optionally substituted condensed heterocyclic ring or the following formulae:

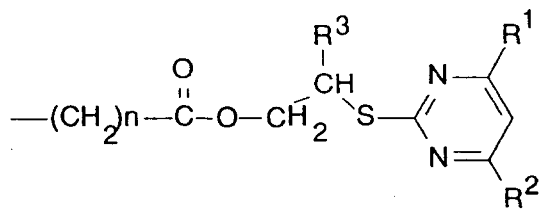

wherein $R^1$, $R^2$ and $R^3$ have the same meanings as mentioned above and n represents an integer of 0 to 6, or

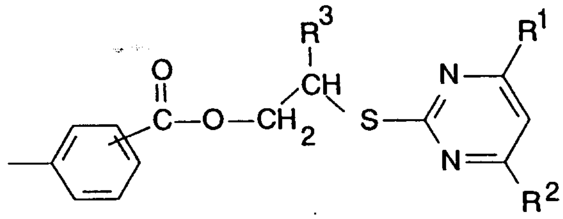

wherein $R^1$, $R^2$ and $R^3$ have the same meanings as mentioned above.

**2.** The compounds of formula (I) according to claim 1, characterized in that

$R^1$ represents, methoxy, difluoromethoxy or trifluoromethoxy,

$R^2$ represents methoxy, difluoromethoxy or trifluoromethoxy,

$R^3$ represents optionally $C_{1-2}$alkyl-substituted $C_{3-6}$cycloalkyl or $C_{1-7}$alkyl which may be unsubstituted or substituted by at least one substituent selected from the group consisting of halogen, $C_{3-6}$cycloalkyl and phenyl which may be substituted by cyano, nitro, halogen, $C_{1-4}$alkyl, $C_{1-4}$alkoxy, halogeno-$C_{1-4}$alkyl or halogeno-$C_{1-4}$alkoxy,

$R^4$ represents, chloro, bromo and

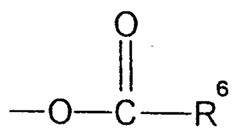 ,

$R^6$ represents, hydrogen,

$R^6$ further represents $C_{1-12}$alkyl which may be unsubstituted or substituted by at least one substituent selected from the group consisting of halogen, cyano, nitro, $C_{3-8}$cycloalkyl which may be substituted by $C_{1-4}$alkyl, $C_{1-4}$alkoxy, $C_{1-4}$alkylthio, halogeno-$C_{1-4}$alkoxy, halogeno-$C_{1-4}$alkylthio, carboxyl or its salt, $C_{1-4}$alkylcarbonyl, $C_{1-4}$alkoxycarbonyl, $C_{1-4}$alkylthiocarbonyl, amino, $C_{1-4}$alkylamino, and di-$C_{1-4}$alkylamino,

$R^6$ further represents phenyl-$C_{1-12}$alkyl, said phenyl is unsubstituted or substituted at least by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, and halogeno-$C_{1-4}$alkylthio,

$R^6$ further represents phenoxy-$C_{1-12}$alkyl, said phenoxy may be unsubstituted or substituted at least by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, halogeno-$C_{1-4}$alkylthio, phenoxy which may be unsubstituted or substituted by one of the group consisting of halogen, $C_{1-4}$alkyl and halogeno-$C_{1-4}$alkyl,
pyridyloxy which may be unsubstituted or substituted by one of the group consisting of halogen, $C_{1-4}$alkyl, and halogeno-$C_{1-4}$alkyl,
quinoxalyloxy which may be unsubstituted or substituted by one of the group consisting of halogen, $C_{1-4}$alkyl, and halogeno-$C_{1-4}$alkyl,
benzothiazolyloxy which may be unsubstituted or substituted by one of the group consisting of halogen, $C_{1-4}$alkyl, and halogeno-$C_{1-4}$alkyl, and
benzoxazolyloxy which may be unsubstituted or substituted by one of the group consisting of halogen, $C_{1-4}$alkyl and halogeno-$C_{1-4}$alkyl,

$R^6$ further represents phenylthio-$C_{1-12}$alkyl, said phenylthio may be unsubstituted or substituted by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, halogeno-$C_{1-4}$alkylthio, phenyl-$C_{1-4}$alkoxy, phenoxy which may be unsubstituted or substituted by one of the group consisting of halogen, $C_{1-4}$alkyl and halogeno-$C_{1-4}$alkyl,
pyridyloxy which may be unsubstituted or substituted by one of the group consisting of halogen, $C_{1-4}$alkyl, and halogeno-$C_{1-4}$alkyl,
quinoxalyloxy which may be unsubstituted or substituted by one of the group consisting of

halogen, $C_{1-4}$alkyl, and halogeno-$C_{1-4}$alkyl,
benzothiazolyloxy which may be unsubstituted or substituted by one of the group consisting of halogen, $C_{1-4}$alkyl, and halogeno-$C_{1-4}$alkyl, and
benzoxazolyloxy which may be unsubstituted or substituted by one of the group consisting of halogen, $C_{1-4}$alkyl, and halogeno-$C_{1-4}$alkyl,

$R^6$ further represents naphthyl-$C_{1-12}$alkyl or naphthoxy-$C_{1-12}$alkyl, said naphthyl or naphthoxy may be unsubstituted or substituted by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, halogeno-$C_{1-4}$alkylthio, phenyl-$C_{1-4}$alkoxy, phenoxy which may be unsubstituted or substituted by one of the group consisting of halogen, $C_{1-4}$alkyl and halogeno-$C_{1-4}$alkyl,
pyridyloxy which may be unsubstituted or substituted by one of the group consisting of halogen, $C_{1-4}$alkyl and halogeno-$C_{1-4}$alkyl,
quinoxalyloxy which may be unsubstituted or substituted by one of the group consisting of halogen, $C_{1-4}$alkyl, and halogeno-$C_{1-4}$alkyl,
benzothiazolyloxy which may be unsubstituted or substituted by one of the group consisting of halogen, $C_{1-4}$alkyl, and halogeno-$C_{1-4}$alkyl, and
benzoxazolyloxy which may be unsubstituted or substituted by one of the group consisting of halogen, $C_{1-4}$alkyl, and halogeno-$C_{1-4}$alkyl,

$R^6$ further represents $C_{1-4}$alkylsulfonyloxy-$C_{1-12}$alkyl, or benzenesulfonyloxy-$C_{1-12}$alkyl, said benzene may be unsubstituted or substituted by one of the group consisting of halogen and $C_{1-4}$alkyl,

$R^6$ further represents $C_{2-12}$alkenyl which may be unsubstituted or substituted by one of the group consisting of halogen, $C_{1-4}$alkyl, carboxyl or its salts and phenyl which may be substituted by halogen or $C_{1-4}$alkyl,

$R^6$ further represents $C_{3-12}$alkynyl, $C_{3-12}$alkadienyl,

$R^6$ further represents phenyl which may be unsubstituted or substituted by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, and carboxyl or its salts,

$R^6$ further represents a 5- or 6-memberd heterocyclic group that has one to four heteroatom(s) each being independently oxygen, sulfur or nitrogen, said heterocyclic group is unsubstituted or substituted by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, phenyl which may be unsubstituted or substituted by halogen, nitro, $C_{1-4}$ alkyl, $C_{1-4}$ alkoxy, halogeno-$C_{1-4}$alkyl, halogeno-$C_{1-4}$alkoxy, phenoxy, and carboxyl or its salts,

$R^6$ further represents a 9- or 10-membered condensed heterocyclic group having one to four heteroatom(s) each being independently oxygen, sulfur or nitrogen, said condensed heterocyclic group may be unsubstituted or substituted by one of the group consisting of cyano, nitro, halogen, $C_{1-4}$alkyl, $C_{1-4}$alkoxy, $C_{1-4}$alkylthio, halogeno-$C_{1-4}$alkyl, halogeno-$C_{1-4}$alkoxy, phenyl which may be unsubstituted or substituted by halogen, phenoxy or carboxyl and its salts,

$R^6$ further represents $C_{3-8}$cycloalkyl which may be substituted by one of the group consisting of $C_{1-4}$alkyl, or carboxyl and its salts, $C_{3-8}$cycloalkenyl Which may be substituted by one of the group consisting of $C_{1-4}$alkyl, $C_{1-4}$alkoxycarbonyl or carboxyl and its salts,

$R^6$ further represents the following formulae

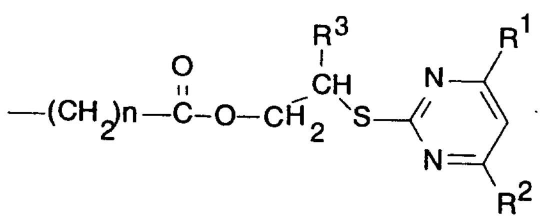

wherein $R^1$, $R^2$ and $R^3$ have the same meanings as defined above, and n represents an integer of 0 to 6 or

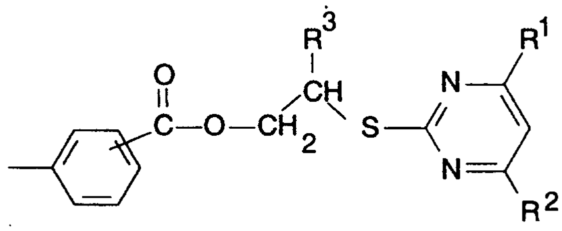

wherein $R^1$, $R^2$ and $R^3$ have the same meanings as defined above.

**3.** The compound of the general formula (I) according to claim 1, wherein

$R^1$ represents methoxy,

$R^2$ represents methoxy,

$R^3$ represents cyclopentyl which may be substituted by methyl, optionally unsubstituted or methyl-substituted cyclohexyl, $C_{1-4}$alkyl which may be unsubstituted or substituted by one of the group consisting of fluoro, chloro, bromo, cyclopropane, cyclopentane, cyclohexane and phenyl which may be unsubstituted or substituted by one of the group consisting of cyano, nitro, fluoro, chloro, bromo, methyl, methoxy, trifluoromethyl and trifluoromethoxy,

$R^4$ represents chloro, bromo or

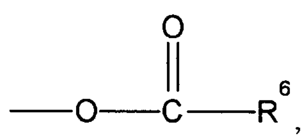

$R^6$ represents hydrogen,

$R^6$ further represents $C_{1-6}$alkyl which may be unsubstituted or substituted by one of the group consisting of fluoro, chloro, bromo, cyano, nitro, cyclopentyl which may be substituted by methyl, cyclohexyl which may be substituted by methyl, $C_{1-4}$alkoxy, carboxyl and its sodium salt, methylcarbonyl, methoxycarbonyl, amino and dimethylamino,

$R^6$ further represents phenyl-$C_{1-6}$alkyl, said phenyl may be unsubstituted or substituted by one of the group consisting of cyano, nitro, fluoro, chloro, methyl, methoxy or carboxyl,

$R^6$ further represents phenoxy-$C_{1-6}$alkyl, said phenoxy may be unsubstituted or substituted at least by one of the group consisting of cyano, nitro, fluoro, chloro, methyl, unsubstituted or substituted phenoxy (said substituent(s) is/are selected from the group consisting of fluoro, chloro and trifluoromethyl), unsubstituted or substituted pyridin-2-yloxy (said substituent(s) is/are selected from the group consisting of fluoro, chloro and trifluoromethyl), unsubstituted or substituted quinoxaline-2-yloxy (said substituent(s) is/are selected from the group consisting of fluoro, chloro and trifluoromethyl), unsubstituted or substituted benzothiazole-2-yloxy (said substituent(s) is/are selected from the group consisting of fluoro, chloro and trifluoromethyl) and unsubstituted or substituted benzoxazole-2-yloxy (said substituent(s) is/are selected from the group consisting of fluoro, chloro and trifluoromethyl),

$R^6$ further represents phenylthio-$C_{1-6}$alkyl, said phenylthio may be unsubstituted or substituted at least by one of the group consisting of fluoro, chloro, methyl, phenylmethoxy, unsubstituted or substituted phenoxy (said substituent(s) is/are selected from the group consisting of fluoro, chloro, $C_{1-4}$alkyl or halogeno-$C_{1-4}$alkyl), unsubstituted or substituted pyridin-2-yloxy (said substituent(s) is/are selected from the group consisting of fluoro, chloro or trifluoromethyl), unsubstituted or substituted quinoxaline-2-yloxy (said substituent(s) is/are selected from the group consisting of fluoro, chloro or trifluoromethyl), unsubstituted or substituted benzothiazole-2-yloxy (said substituent(s) is/are selected from the group consisting of fluoro, chloro or trifluoromethyl) and unsubstituted or substituted benzoxazole-2-yloxy (said sustituent(s) is/are selected from the group consisting of fluoro, chloro or trifluoromethyl),

$R^6$ further represents naphthyl-$C_{1-6}$alkyl or naphtoxy-$C_{1-6}$alkyl, said naphthyl or naphthoxy may be unsubstituted or substituted, and the substituent(s) is/are phenoxy (which may be substituted by fluoro, chloro or trifluoromethyl), and unsubstituted or substituted benzenesulfonyloxy (said substituent(s) is/are selected from the group consisting of fluoro, chloro and methyl),

$R^6$ further represents unsubstituted or substituted $C_{3-7}$alkenyl, said substituent is selected from one of the group consisting of fluoro, chloro, bromo, carboxyl and unsubstituted or substituted phenyl (said substituents are selected from the group consisting of fluoro and chloro),

$R^6$ further represents $C_{3-6}$alkynyl or unsubstituted or substituted phenyl, said substituent is selected from one of the group consisting of cyano, nitro, fluoro, chloro, bromo, ethyl, propyl, isopropyl, (n- or tert-)butyl, methoxy, ethoxy, trifluoromethyl and trifluoromethoxy,

$R^6$ further represents an unsubstituted or substituted 5- or 6-memberd heterocyclic ring, said heterocyclic group is selected from the group consisting of thienyl, thiazolyl, isoxazolyl, imidazolyl, pyrazolyl, furyl, pyridyl, pyrimidinyl, pyridazinyl, pyrazinyl and oxazolyl, and the substituent(s) is/are selected from the group consisting of fluoro, chloro, bromo, methyl, methoxy, methylthio, trifluoromethyl, trifluoromethoxy, carboxyl and phenyl which may be substituted by fluoro, chloro, nitro, methyl, ethyl methoxy and trifluoromethyl,

$R^6$ further represents an unsubstituted or substituted 9- or 10-membered condensed heterocyclic ring, said condensed heterocyclic group is selected from the group consisting of quinolyl and indolyl and the substituen(s) is/are selected from the group consisting of fluoro, chloro, bromo, methyl, methoxy, methylthio, trifluoromethyl and trifluoromethoxy,

$R^6$ further represents unsubstituted or substituted cyclopropyl, cyclopentyl or cyclohexyl (said substituent(s) is/are selected from the group consisting of methyl, ethyl and carboxyl),

$R^6$ further represents unsubstituted or substituted cyclopentenyl or cyclohexenyl (said substituent(s) is/are selected from the group consisting of methyl, ethyl and carboxyl), methoxycarbonyl, carboxyl and its salts and the following formulae

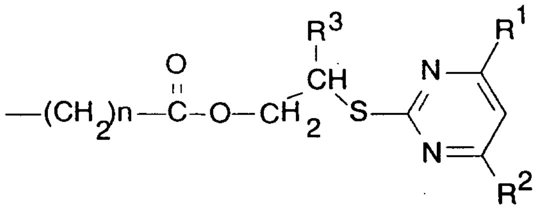

wherein $R^1$, $R^2$ and $R^3$ have the same meanings as defined above and n represents an integer of 0 to 6 or

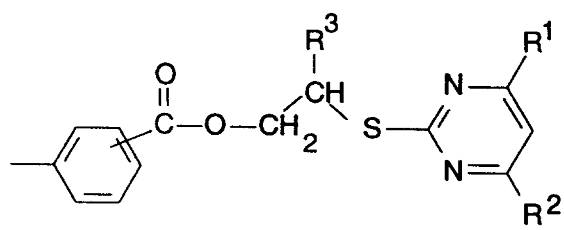

wherein $R^1$,$R^2$ and $R^3$ have the same meanings as defined above.

**4.** Process for the preparation of pyrimidinylthioalkane derivatives of the formula (I)

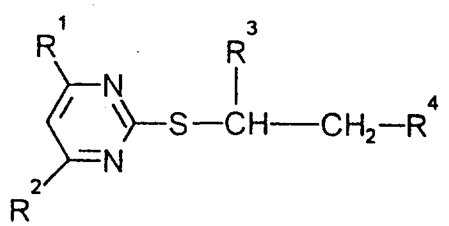 (I)

wherein

$R^1$ represents $C_{1-4}$alkyl, $C_{1-4}$alkoxy, halogen, halogeno-$C_{1-4}$alkyl or halogeno-$C_{1-4}$alkoxy,

$R^2$ represents $C_{1-4}$alkoxy, halogen, halogeno-$C_{1-4}$alkyl or halogeno-$C_{1-4}$alkoxy,

$R^3$ represents optionally $C_{1-4}$alkyl substituted or unsubstituted $C_{3-7}$cycloalkyl or optionally substituted $C_{1-15}$alkyl,

$R^4$ represents halogen or

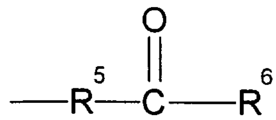

,

$R^5$ represents oxygen or sulfur,

$R^6$ represents hydrogen, in each case optionally substituted $C_{1-20}$saturated aliphatic hydrocarbon, $C_{2-20}$ unsaturated aliphatic hydrocarbon, phenyl, $C_{3-8}$cycloalkyl, $C_{3-8}$cycloalkenyl, $C_{1-4}$alkoxycarbonyl, carbonyl and its salts, an optionally substituted heterocyclic ring, an optionally substituted condensed heterocyclic ring or the following formulae:

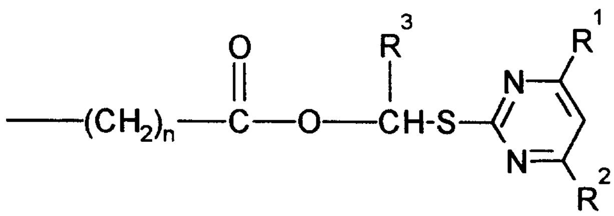

wherein $R^1$, $R^2$ and $R^3$ have the same meanings as mentioned above and n represents an integer of 0 to 6, or

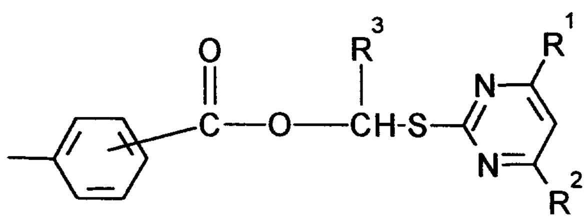

wherein $R^1$, $R^2$ and $R^3$ have the same meanings as defined above,

characterized in that

(a) in the case where $R^4$ represents halogen:

compounds of the formula (II)

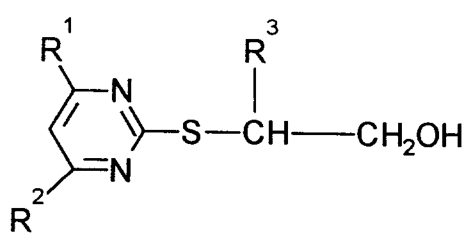

(II)

wherein

$R^1$, $R^2$ and $R^3$ have the same meanings as defined above,

are reacted with a halogenation agent, if appropriate, in the presence of inert solvents,

or

(b) in the case where the $R^4$ represents

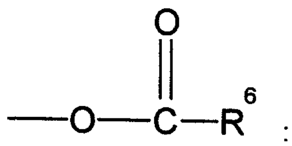

above mentioned compounds of the formula (II),
are reacted with acid halides of the formula (III)

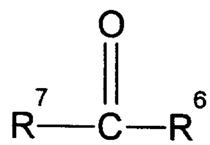

(III)

wherein $R^6$ has the meanings as defined above, and $R^7$ represents chlorine, bromine or iodine,
if appropriate, in the presence of inert solvents and, if appropriate in the presence of an acid binder,
or

(c) in the case where $R^4$ represents

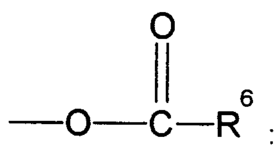

above mentioned compounds of the formula (II)
are reacted with acid anhydrides of the formula (IV)

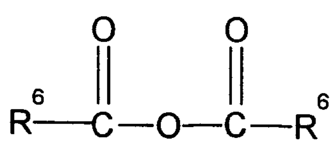

(IV)

wherein
$R^6$ has the same meanings as defined above and both $R^6$ may be the same or different to each other,
if appropriate, in the presence of inert solvents and, if appropriate in the presence of an acid binder,
or

(d) in the case where $R^4$ represents

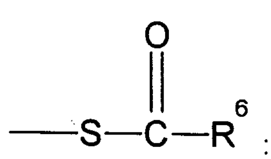

compounds of the formula (V)

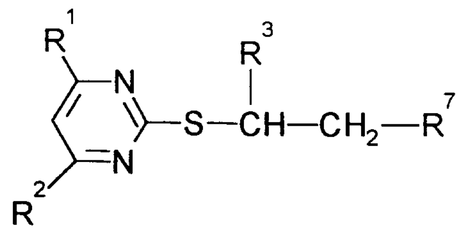

(V)

wherein
$R^1$, $R^2$, $R^3$ and $R^7$ have the same meanings as defined above,
are reacted with thiocarboxylic acids of the formula (VI)

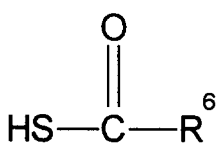

(VI)

wherein
$R^6$ has the same meaning as defined above,
in the presence of inert solvents and if appropriate in the presence of an acid binder.

**5.** Herbicidal compositions, characterized in that they contain at least one pyrimidinylthioalkane derivative of the formula (I) according to claim 1 to 4.

**6.** Process for combating weeds, characterized in that pyrimidinylthioalkane derivatives of the formula (I) according to claims 1 to 4 are allowed to act on weeds and/or their habitat.

**7.** Use of pyrimidinylthioalkane derivatives of the formula (I) according to claims 1 to 4 for combating weeds.

**8.** Process for the preparation of herbicidal compositions, characterized in that pyrimidinylthioalkane derivatives of the formula (I) according to claims 1 to 4 are mixed with extenders and/or surface active agents.

European Patent Office

# EUROPEAN SEARCH REPORT

Application Number

EP 93 11 6309

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 481 512 (UBE INDUSTRIES, LTD) 22 April 1992 * claim 1 * --- | 1-7 | C07D239/38 A01N43/54 C07D239/60 C07D401/12 C07D409/12 |
| A | EP-A-0 468 766 (MITSUBISHI PETROCHEMICAL CO., LTD) 29 January 1992 * claim 1 * --- | 1-7 | |
| A | EP-A-0 409 368 (SCHERING AKTIENGESELLSCHAFT) 23 January 1991 * claim 1 * --- | 1-7 | |
| A | EP-A-0 400 741 (SHELL INTERNATIONALE RESEARCH MAATSCHAPPIJ B.V.) 5 December 1990 * claim 1 * --- | 1-7 | |
| A | EP-A-0 347 811 (IHARA CHEMICAL INDUSTRY CO., LTD.) 27 December 1989 * claim 1 * ----- | 1-7 | TECHNICAL FIELDS SEARCHED (Int. Cl.5 ) C07D A01N |

The present search report has been drawn up for all claims

| Place of search | Date of completion of the search | Examiner |
|---|---|---|
| MUNICH | 06 DECEMBER 1993 | GETTINS M.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)